(19) Europäisches Patentamt
European Patent Office
Office européen des brevets

(11) EP 4 620 474 A1

(12) EUROPEAN PATENT APPLICATION

(43) Date of publication:
**24.09.2025 Bulletin 2025/39**

(21) Application number: **24164977.1**

(22) Date of filing: **20.03.2024**

(51) International Patent Classification (IPC):
***A61K 31/7088*** (2006.01) ***C07K 14/47*** (2006.01)

(52) Cooperative Patent Classification (CPC):
**A61K 31/713; C07K 14/472; C12N 15/115**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **APTARION biotech AG**
**10589 Berlin (DE)**

(72) Inventor: **The designation of the inventor has not yet been filed**

(74) Representative: **Bohmann, Armin K.**
**Bohmann**
**Anwaltssozietät**
**Nymphenburger Straße 1**
**80335 München (DE)**

Remarks:
The complete document including Reference Table(s) and the Sequence Listing(s) can be downloaded from the EPO website

# (54) METHOD OF TREATING A DISORDER WITH A C5A INHIBITOR

(57) The present invention is related to a C5a binding agent for use in a method of treating and/or preventing a disease in a subject, wherein the disease is associated with or caused by C5a, wherein the method comprises administering to the subject multiple doses of the C5a binding agent, wherein a dose of the multiple doses is administered to the subject when a level of the C5a binding agent in the blood of the subject is a level of 80 to 300 nM.

EP 4 620 474 A1

## Description

The present invention is related to a C5a binding agent for use in a method of treating and/or preventing a disease, and to a C5a binding agent for use in a method for reducing an adverse effect or for reducing the risk of an adverse effect of an anti-C5a therapy.

The complement system is part of the body's innate immune defence system against infections. Invading pathogens trigger a proteolytic cascade that results in the tagging and elimination of the microbial intruders and orchestrates immunological and inflammatory processes (Ricklin et al. 2010). Important terminal effector mechanisms are mediated by complement C5 cleavage products C5a and C5b which have distinct biological functions in the complement system.

C5a acts as a pro-inflammatory factor with enhancing effects on leukocyte recruitment and activation, vascular permeability, and coagulation. With regard to microbial invasion, C5a is produced in order to attract leukocytes to a site of microbial invasion and activivate leukocytes locally.

In certain conditions, C5a causes more damage than benefit, for example:

- in sepis and systemic inflammatory response (SIRS) where shock due to plama extravasation, endothelial glycocalyx shedding, and microcirculation disturbance due to disseminated intravascular coagulation may be observed;
- in pneumonia where pleural effusion and lung edema may be oserved; and
- in ischemia/reperfusion injury where postoperative kidney damage may be observed.

Because of that, C5a is an interesting target for treating such conditions.

C5b has an immediate anti-bacterial effect by initiating the formation of the membrane attack complex (MAC, also known as Terminal Complement Complex, TCC) consisting of C5b, C6, C7, C8 and C9 (also known as or referred to as 'CSb-9'), a multiprotein complex that forms pores in bacterial cell membranes and viral envelopes (Ricklin et al., 2010; Schiela et al., 2018).

Because of that important function of C5b, the function of C5b and/or formation of C5b (by cleavage of C5 into C5a and C5b) should not be hampered by any therapy targeting the complement system.

Blocking cleavage of C5 into C5a and C5b is associated with a risk of infections, such as meningococcal infections, and sepsis as the most serious side effects (McNamaraet al., 2017; Socie et al., 2019) due to hampering the formation of the membrane attack complex (MAC) consisting of C5b, C6, C7, C8 and C9 ('C5b-9'). Such blocking has been shown for the C5 binding monoclonal antibody eculizumab (McNamaraet al., 2017; Socie et al., 2019).

In contrast to the C5 binding antibody eculizumab, selective C5a inhibitors do not interfere with C5 cleavage and formation of the membrane attack complex (MAC). Different types of selective C5a inhibitors are known.

A first type of selective C5a inhibitors binds to C5a and C5 without interfering with C5 cleavage. Representative examples of this first type of selective C5a inhibitors are C5a binding nucleic acid molecules such as AON-D21 (also referred to as NOX-D21) and others as described in the international patent applications WO2009/040113, WO2010/108657 and WO2013104540. Such nucleic acid molecules specifically bind to a target molecule, such as C5a, through a mechanism different from Watson Crick base pairing and are also known as L-aptamers. L-aptamers consist of L-nucleotides and are, among other aptamers, described in 'The Aptamer Handbook' (ed. Klussmann, 2006).

A second type of selective C5a inhibitors binds to C5a, but does not bind to C5, thereby not interfering with C5 cleavage. A representative example of this second type of selective C5a inhibitors is the recombinant chimeric monoclonal IgG4 antibody vilobelimab.

A third type of selective C5a inhibitors binds to the C5a receptor, thereby not interfering with C5 cleavage. A representative example of this third type of selective C5a inhibitors is the C5a receptor antagonist avacopan (Bekker et al., 2016).

A selective C5a inhibitor of the first type that binds to both C5a and C5 (such as C5a binding nucleic acid molecule AON-D21 and others) is conofronted with the problem that the level of C5 in blood of both healthy individuals and individuals suffering from a disease associated with or caused by C5a is comparable, i.e. about 455 nM (mean concentration; corresponding to 84.5 μg/L) (Zelek et al., 2020). In contrast thereto, the titer of C5a in blood of both healthy individuals and individuals suffering from a disease associated with or caused by C5a is significantly lower compared to the titer of C5 and is below 3.0 nM (corresponding to 25 μg/ml) in healthy individuals (Zelek et al., 2020), and is in the range of 5.2 nM (mean concentration; corresponding to 43.0 μg/L)in individuals suffering from a disease associated with or caused by C5a, for example COVID-19 (Zelek et al., 2020).

Due to the above-described high surplus of C5 compared to C5a in blood of a subject, a person skilled in the art expects that most of the administered molecules of the first type of selective C5a inhibitors will bind to C5 and will hardly be available for binding to and thus inhibiting C5a, if one dose or several doses of the selective C5a inhibitor are administered to a subject and such dosage regimen results in similar levels of the selective C5a inhibitor and C5 in the subject's blood (or even lower levels of the selective C5a inhibitor), provided that the binding constant of the selective C5a inhibitor for C5a

and C5 is in the same range. Under such conditions, only a small or no effect of such selective C5a inhibitor on C5a function is expected. Increasing the doses of the selective C5a inhibitor is typically not an option with regard to costs and a potentially high risk of side effects during treatment.

The elimination half-life for IgG1, IgG2 and IgG4 antibodies is approx. 18 - 21 days, which is substantially longer that the half-life of other proteins with similar weight (Ryman and Meibohm, 2017). Such longer half-life may result in treament-emergent adverse events (TEAEs) as shown in a study using C5a binding antibody vilobelimab for patients with SARS-Co V-2 infection (Vlaar et al., 2022). In said study, the patients with SARS-Co V-2 infection received standard of care or vilobelimab at a dose of 800 mg intraveneously for a maximum of six dosis and observed treatment-emergent adverse events (TEAEs) were as follows: pneumonia (38 [21,7%] vs 26 [14,8%]), herpes simplex (11 [6,3%] vs 5 [2,6%]), Staphylococcus pneumonia (10 [5,7%] vs 5 [2,6%]) and Herpes simplex reactivation (4 [2,3%] vs 2 [1,1%]). It is strongly assumed that the TEAEs resulted from a prolonged blockade of C5a after end of treatment due to high terminal half life as known for antibodies in the human body and/or the high dosis of Vilobelimab used, thereby suppressing or diminishing C5a's positive role in the defense against infections based on microbial invasion for a lengthy period of time after end of treatment.

The problem underlying the present invention is the provision of a means for use in a method for treating and/or preventing a disease in a subject, wherein the disease is associated with or caused by C5a, wherein the method comprises administerting a C5a inhibitor to the subject.

A further problem underlying the present invention is the provision of means for use in a method for treating and/or preventing a disease in a subject, wherein the disease is associated with or caused by C5a, and wherein the method comprises administering a C5a inhibitor to the subject, wherein the C5a inhibitor binds to both C5a and C5.

A still further problem underlying the present invention is the provision of a means for use in a method for treating and/or preventing a disease in a subject, wherein the disease is associated with or caused by C5a, wherein the method comprises administering to the subject a C5a inhibitor, and wherein the method shows fewer side effects, preferably fewer side effects caused by the C5a inhibitor.

These and other problems underlying the present invention are solved by the subject matter of the attached independent claims. Preferred embodiments may be taken from the dependent claims.

These and other problems underlying the present invention are also solved by the following embodiments.

Embodiment 1. A C5a binding agent for use in a method of treating and/or preventing a disease in a subject, wherein the disease is associated with or caused by C5a, wherein the method comprises administering to the subject multiple doses of the C5a binding agent at a trough level of the C5a binding agent of 80 to 300 nM in the blood of the subject.

Embodiment 2. The C5a binding agent for use of Embodiment 1, wherein the multiple doses comprise a first dose, at least a second dose and optionally one or more subsequent dose(s).

Embodiment 3. The C5a binding agent for use of any one of Embodiments 1 to 2, wherein the trough level of the C5a binding agent is the level of the C5a binding agent in the blood of the subject before administering a or the second dose of the C5a binding agent and/or one or more of an or of the optional one or more subsequent dose(s) of the C5a binding agent.

Embodiment 4. The C5a binding agent for use of any of Embodiments 1, 2 and 3, wherein at the trough level of the C5a binding agent of about 100 to 300 nM or at a level of the C5a binding agent in the blood of the subject of ≥ about 100 nM 100% of an activity of C5a in the blood of the subject is inhibited, wherein preferably at the trough level of the C5a binding agent of 100 to 300 nM or at a level of the C5a binding agent in the blood of the subject of ≥ 100 nM 100% of an activity of C5a in the blood of the subject is inhibited.

Embodiment 5. The C5a binding agent for use of any one of Embodiments 1, 2, 3 and 4, wherein the trough level of the C5a binding agent is about 100 nM in the blood of the subject, preferably the trough level of the C5a binding agent is 100 nM in the blood of the subject.

Embodiment 6. The C5a binding agent for use of any of Embodiments 1, 2 and to 3, wherein at the trough level of the C5a binding agent of about 100 nM or at a level of the C5a binding agent in the blood of the subject of about 100 nM 100% of an activity of C5a in the blood of the subject is inhibited, preferably at the trough level of the C5a binding agent of 100 nM or at a level of the C5a binding agent in the blood of the subject of 100 nM 100% of an activity of C5a in the blood of the subject is inhibited.

Embodiment 7. The C5a binding agent for use of any of Embodiments 1, 2 and 3, wherein at the trough level of the C5a binding agent of 80 nM or at a level of the C5a binding agent in the blood of the subject of 80 nM at least 80% of an activity of C5a in the blood of the subject is inhibited.

Embodiment 8. The C5a binding agent for use of any of Embodiments 1, 2 and 3, wherein at the trough level of the C5a binding agent of 90 nM or at a level of the C5a binding agent in the blood of the subject of 90 nM at least 90% of an activity of C5a in the blood of the subject is inhibited.

Embodiment 9. A C5a binding agent for use in a method of treating and/or preventing a disease in a subject, wherein the disease is associated with or caused by C5a, wherein the method comprises administering to the subject multiple doses of the C5a binding agent at a level of the C5a binding agent in the blood of the subject of 80 to 300 nM.

Embodiment 10. The C5a binding agent for use of cl Embodiment 9, wherein the multiple doses comprise a first dose, at least a second dose and optionally one or more subsequent dose(s).

Embodiment 11. The C5a binding agent for use of any of Embodiments 9 to 10, wherein the level of the C5a binding agent is the level of the C5a binding agent in the blood of the subject before administering the a or the second dose of the C5a binding agent and/or one or more of an or of the optional one or more subsequent dose(s) of the C5a binding agent.

Embodiment 12. The C5a binding agent for use of any of Embodiments 9, 10 and 11, wherein at a level of the C5a binding agent in the blood of the subject of ≥ about 100 nM 100% of an activity of C5a is inhibited, wherein preferably at a level of the C5a binding agent in the blood of the subject of ≥ 100 nM 100% of an activity of C5a in the blood of the subject is inhibited.

Embodiment 13. The C5a binding agent for use of any of Embodiments 9, 10, 11 and 12, wherein the method comprises administering to the subject multiple doses of the C5a binding agent at the level of the C5a binding agent in the blood of the subject of about100 nM, preferably the method comprises administering to the subject multiple doses of the C5a binding agent at the level of the C5a binding agent in the blood of the subject of 100 nM.

Embodiment 14. The C5a binding agent for use of any of Embodiments 9, 10, 11, 12 and 13, wherein at a level of the C5a binding agent in the blood of the subject of about 100 nM 100 % of an activity of C5a is inhibited, more preferably at a level of the C5a binding agent in the blood of the subject of 100 nM 100 % of an activity of C5a in the blood of the subject is inhibited.

Embodiment 15. The C5a binding agent for use of any of Embodiments 9, 10 and 11, wherein at a level of the C5a binding agent in the blood of the subject of 80 nM at least 80 % of an activity of C5a in the blood of the subject is inhibited.

Embodiment 16. The C5a binding agent for use of any of Embodiments 9, 10 and 11, wherein at a level of the C5a binding agent in the blood of the subject of 90 nM at least 90 % of an activity of C5a in the blood of the subject is inhibited.

Embodiment 17. A C5a binding agent for use in a method of treating and/or preventing a disease in a subject, wherein the disease is associated with or caused by C5a, wherein the method comprises administering to the subject the C5a binding agent during a treatment period, wherein the administering of the C5a binding agent provides a level of the C5a binding agent in the blood of the subject, wherein the level of the C5a binding agent in the blood of the patient is

a) a minimum level of 80 to 300 nM maintained during the treatment period of the subject; or

b) a minimum level of 80 to 300 nM at the end of the treatment period of the subject.

Embodiment 18. The C5a binding agent for use of Embodiment 17, wherein the minimum level is reached within the treatment period, preferably the minimum level is reached within the treatment period at one or several points in time.

Embodiment 19. The C5a binding agent for use of any of Embodiments 17 to 18, wherein multiple doses of the C5a binding agent are administered during the treatment period.

Embodiment 20. The C5a binding agent for use of Embodiment 19, wherein the multiple doses comprise a first dose, at least a second dose and optionally one or more subsequent dose(s).

Embodiment 21. The C5a binding agent for use of any of Embodiments 19 to 20, wherein if multiple doses of the C5a binding agent are administered, the minimum level of the C5a binding agent is the level of the C5a binding agent in the blood of the subject before administering a or the second dose of the C5a binding agent and/or one or more of an or of the optional one or more subsequent dose(s) of the C5a binding agent the next dose is administered.

Embodiment 22. The C5a binding agent for use of any of Embodiments 17, 18, 19, 20 and 21, wherein at a level of the C5a binding agent in the blood of the subject of ≥ about100 nM 100% of an activity of C5a in the blood of the subject is inhibited, wherein preferably at a level of the C5a binding agent in the blood of the subject of ≥ 100 nM 100% of an activity of C5a in the blood of the subject is inhibited.

Embodiment 23. The C5a binding agent for use of any of Embodiments 17, 18, 19, 20, 21 and 22, wherein the minimum level of the C5a binding agent in the blood of the subject is about 100 nM, preferably the minimum level of the C5a binding agent in the blood of the subject is 100 nM.

Embodiment 24. The C5a binding agent for use of any of Embodiments 17, 18, 19, 20, 21 and 22, wherein at the minimum of the C5a binding agent in the blood of the subject of about 100 nM 100% of an activity of C5a in the blood of the subject is inhibited, wherein preferably at the minimum of the C5a binding agent in the blood of the subject of 100 nM 100% of an activity of C5a in the blood of the subject is inhibited.

Embodiment 25. The C5a binding agent for use of any of Embodiments 17, 18, 19, 20 and 21, wherein at the minimum level of the C5a binding agent in the blood of the subject of 80 nM at least 80% of an activity of C5a in the blood of the subject is inhibited.

Embodiment 26. The C5a binding agent for use of any of Embodiments 17, 18, 19, 20 and 21, wherein at the minimum level of the C5a binding agent in the blood of the subject of 90 nM at least 80% of an activity of C5a in the blood of the subject is inhibited.

Embodiment 27. The C5a binding agent for use of any one of Embodiments 17, 18, 19, 20, 21, 22, 23, 24, 25 and 26, wherein the treatment period of the subject starts with the administering of a first dose of the C5a binding agent and ends at the time at which the level of the C5a binding agent in the blood of the subject falls or is below 80 - 300 nM after the last dose of the C5a binding agent was administered, preferably the treatment period of the subject starts with the administering of a first dose of the C5a binding agent and ends at the time at which the level of the C5a binding agent in the blood of the subject falls or is below 80 - 100 nM after the last dose of the C5a binding agent was administered, wherein more preferably the treatment period of the subject starts with the administering of a first dose of the C5a binding agent and ends at the time at which the level of the C5a binding agent in the blood of the subject falls or is below about 100 nM after the last dose of the C5a binding agent was administered, wherein most preferably the treatment period of the subject starts with administering of a the first dose of the C5a binding agent and ends at the time at which the level of the C5a binding agent in the blood of the subject falls or is below 100 nM after the last dose of the C5a binding agent was administered.

Embodiment 28. The C5a binding agent for use of any one of Embodiments1, 2, 3, 4, 5, 6, 7, 8, 9, 10, 11, 12, 13, 14, 15, 16, 17, 18, 19, 20, 21, 22, 23, 24, 25, 26 and 27, wherein an adverse effect or a risk for an adverse effect of administering a C5a binding agent to the subject is reduced or avoided.

Embodiment 29. The C5a binding agent for use of Embodiment 28, wherein the adverse effect is associated with or caused by administering a C5a binding agent to the subject.

Embodiment 30. The C5a binding agent for use of any one of Embodiments 27 to 28, wherein the adverse effect is selected from the group comprising of shock, lung edema, postoperative kidney damage, pneumonia, herpes simplex, Staphylococcus pneumonia and Herpes simplex reactivation.

Embodiment 31. The C5a binding agent for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29 and 30, wherein the C5a binding agent has a blood plasma half-life of about 6 hours to about 15 hours within the first 12 to 48 hours.

Embodiment 32. A C5a binding agent for use in a method for treating and/or preventing a disease in a subject, wherein the disease is associated with or caused by C5a, wherein the method comprises administering the C5a binding agent to the subject, wherein an activity of C5a in the blood of the subject is reduced by 50% or less within 24 to 72 hours after administering the C5a binding agent to the subject.

Embodiment 33. The C5a binding agent for use of Embodiment 32, wherein an adverse effect or a risk for an adverse effect of administering the C5a binding agent to the subject is reduced or avoided.

Embodiment 34. The C5a binding agent for use of Embodiment 33, wherein the adverse effect is associated with or caused by administering the C5a binding agent to the subject.

Embodiment 35. The C5a binding agent for use of any one of Embodiments 33 to 34, wherein the adverse effect is selected from the group comprising of shock, lung edema, postoperative kidney damage, pneumonia, herpes simplex, Staphylococcus pneumonia and Herpes simplex reactivation.

Embodiment 36. The C5a binding agent for use of any one of Embodiments 32, 33, 34 and 35, wherein the C5a binding agent used has a blood plasma half-life of about 6 hours to about 15 hours within the first 12 to 48 hours after administering the C5a binding agent to the subject as determined in the blood of the subject.

Embodiment 37. A C5a binding agent for use in a method for treating and/or preventing a disease in a subject, wherein the disease is associated with or caused by C5a, wherein the treating and/or preventing comprises administering the C5a binding agent to the subject, wherein the treating and/or preventing of the disease is associated with or goes along with a reduced adverse effect or a reduced risk of an adverse effect, wherein the adverse effect is caused by or arising from the administering of the C5a binding agent, wherein an activity of C5a in the blood of the subject is reduced by 50 % or less within 24 hours to 72 hours after administering the C5a binding agent to the subject.

Embodiment 38. The C5a binding agent for use of Embodiment 37, wherein the adverse effect is selected from the group comprising of shock, lung edema, postoperative kidney damage, pneumonia, herpes simplex, Staphylococcus pneumonia and Herpes simplex reactivation.

Embodiment 39. The C5a binding agent for use of any one of Embodiments 37 to 38, wherein the C5a binding agent used has a plasma half-life of about 6 hours to about 15 hours within the first 12-48 hours after administering the C5a binding agent to the subject as determined in the blood of the subject.

Embodiment 40. A C5a binding agent for use in a method for treating and/or preventing a disease in a subject, wherein the disease is associated with or caused by C5a, wherein the treating and/or preventing comprises administering the C5a binding agent to the subject, wherein the treating and/or preventing of the disease is associated with or goes along with a reduced adverse effect or a reduced risk of an adverse effect, wherein the adverse effect is caused by or arising from the administering of the C5a binding agent, wherein the C5a binding agent has plasma half-life of about 6 hours to about 15 hours within the first 12 to 48 hours after administering the C5a binding agent to the subject as determined in the blood of the subj ect.

Embodiment 41. The C5a binding agent for use of Embodiment 40, wherein the adverse effect is selected from the group comprising of shock, lung edema, postoperative kidney damage, pneumonia, herpes simplex, Staphylococcus pneumonia and Herpes simplex reactivation.

Embodiment 42. A C5a binding agent for use in a method for reducing an adverse effect or for reducing the risk of an adverse effect of an anti-C5a therapy administered to a subject, wherein the method comprises administering to the subject a C5a binding agent, wherein an activity of C5a in the blood of the subject is reduced by 50 % or less within 24 hours to 72 hours after administering the C5a binding agent to the subject.

Embodiment 43. The C5a binding agent for use of Embodiment 42, wherein an adverse effect or a risk for an adverse effect of an anti-C5a therapy is reduced or avoided.

Embodiment 44. The C5a binding agent for use of Embodiment 43, wherein the adverse effect is associated with or caused by the anti-C5a therapy.

Embodiment 45. The C5a binding agent for use of any one of Embodiments 43 to 44, wherein the adverse effect is selected from the group comprising of shock, lung edema, postoperative kidney damage, pneumonia, herpes simplex, Staphylococcus pneumonia and Herpes simplex reactivation.

Embodiment 46. The C5a binding agent for use of any one of Embodiments 42, 43, 44 and 45, wherein the anti-C5a therapy comprises administering a C5a binding agent to the subject.

Embodiment 47. The C5a binding agent for use of any one of Embodiments 42, 43, 44, 45and 46, wherein the C5a binding agent used in the anti-C5a therapy has a plasma half-life of about 6 hours to about 15 hours within the first 12 to 48 hours after administering the C5a binding agent to the subject as determined in the blood of the subject.

Embodiment 48. A C5a binding agent for use in a method for reducing an adverse effect or for reducing the risk of an adverse effect of an anti-C5a therapy administered to a subject, wherein the method comprises administering to the subject a C5a binding agent, wherein the C5a binding agent has a plasma half-life of about 6 hours to about 15 hours within the first 12 to 48 hours after administering the C5a binding agent to the subject hours as determined in the blood of the subject.

Embodiment 49. The C5a binding agent for use of Embodiment 48, wherein an adverse effect or a risk for an adverse effect of an anti-C5a therapy is reduced or avoided.

Embodiment 50. The C5a binding agent for use of Embodiment 49, wherein the adverse effect is associated with or caused by the anti-C5a therapy.

Embodiment 51. The C5a binding agent for use of any one of Embodiments 49 to 50, wherein the adverse effect is selected from the group comprising of shock, lung edema, postoperative kidney damage, pneumonia, herpes simplex, Staphylococcus pneumonia and Herpes simplex reactivation.

Embodiment 52. The C5a binding agent for use of any one of Embodiments 48. 49, 50 and 51, wherein the anti-C5a therapy comprises administering a C5a binding agent to the subject.

Embodiment 53. The C5a binding agent for use of any of Embodiments 32, 33, 34, 35, 36, 37, 38, 39, 40, 41, 42, 43, 44, 45, 46, 47, 48, 49, 50, 51 and 52, wherein the level of the C5a binding agent in the blood of the subject falls below or is less than about 100 nM within 8 to 24 hours after a last dose of the C5a binding agent has been administered to the subject, preferably the concentration of the C5a binding agent in the blood of the subject falls below or is less than 100 nM within 8 to 24 hours after a last dose of the C5a binding agent has been administered to the subject.

Embodiment 54. The C5a binding agent according for use of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52 and 53, wherein the C5a binding agent is administered to the subject according to a dosage regimen in which successive doses of the C5a binding agent are administered to the subject.

Embodiment 55. The C5a binding agent for use of Embodiment 54, wherein the dosage regimen comprises administering the C5a binding agent every 48 hours, 24 hours, 12 hours or 8 hours.

Embodiment 56. The C5a binding agent for use of any one of Embodiments 54 and 55, wherein the dosage regimen comprises administering the C5a binding agent by continuous administration, wherein preferably the continuous administration comprises administering a loading dose at the beginning.

Embodiment 57. The C5a binding agent for use of any of Embodiments 54, 55 and 56, wherein the dosage regimen comprises administering the C5a binding agent by intravenous administration, subcutaneous administration or administration by inhalation.

Embodiment 58. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56 and 57, wherein the blood is full blood, blood plasma or blood serum, preferably plasma.

Embodiment 59. A C5a binding agent for use in a method of treating and/or preventing a disease in a subject, wherein the disease is which is associated with or caused by C5a, wherein the method comprises administering to the subject

a) a total amount of 4.18 to 220.25 nmol of the C5a binding agent per kg bodyweight of the subject over a period of 8 to 48 hours, or

b) a total amount of 0.055 to 2.9 mg of the C5a binding agent per kg bodyweight of the subject over a period of 8 to 48 hours.

Embodiment 60. The C5a binding agent for use of Embodiment 59, wherein the method comprises administering to the subject

a) a total amount of 12.53 to 72.12 nmol of the C5a binding agent per kg bodyweight of the subject every day, or
b) a total amount of 0.165 to 0.95 mg of the C5a binding agent per kg bodyweight of the subject every day.

Embodiment 61. The binding agent for use of any of Embodiments 59 and 60, wherein the method comprises administering to the subject

a) 98.73 to 220.25 nmol of the C5a binding agent per kg body weight of the subject every two days,
b) 25.82 to 72.12 nmol of the C5a binding agent per kg body weight of the subject every day,
c) 9.11 to 35.70 nmol of the C5a binding agent per kg body weight of the subject twice a day,
d) 4.18 to 24.30 nmol of the C5a binding agent per kg body weight of the subject three times a day,
e) 1.3 to 2.9 mg of the C5a binding agent per kg body weight of the subject every two days,
f) 0.34 to 0.95 mg of the C5a binding agent per kg body weight of the subject every day,
g) 0.12 to 0.47 mg of the C5a binding agent per kg body weight of the subject twice a day, or
h) 0.055 to 0.32 mg of the C5a binding agent per kg body weight of the subject three times a day.

Embodiment 62. The C5a binding agent for use of anyone Embodiments 59, 60 and 61, wherein the method comprises administering to the subject multiple doses of the C5a binding agent at a trough level of the C5a binding agent of 80 to 300 nM in the blood of the subject, wherein preferably blood is whole blood, blood plasma or blood serum, wherein more preferably blood is blood plasma.

Embodiment 63. The C5a binding agent for use of Embodiment 62, wherein the multiple doses comprise a first dose, at least a second dose and optionally one or more subsequent dose(s).

Embodiment 64. The C5a binding agent for use of any one Embodiments 62 to 63, wherein the trough level of the C5a binding agent is the level of the C5a binding agent in the blood of the subject before administering a or the second dose of the C5a binding agent and/or one or more of an or of the optional one or more subsequent dose(s) of the C5a binding agent.

Embodiment 65. The C5a binding agent for use of any of Embodiment 62, 63 and 64, wherein at the trough level of the C5a binding agent of about 100 to 300 nM or at a level of the C5a binding agent in the blood of the subject of ≥ about 100 nM 100% of an activity of C5a in the blood of the subject is inhibited, wherein preferably at the trough level of the C5a binding agent of 100 to 300 nM or at a level of the C5a binding agent in the blood of the subject of ≥ 100 nM 100% of an activity of C5a in the blood of the subject is inhibited.

Embodiment 66. The C5a binding agent for use of any one of Embodiments 62, 63, 64 and 65, wherein the trough level of the C5a binding agent is about 100 nM in the blood of the subject, preferably the trough level of the C5a binding agent is 100 nM in the blood of the subj ect.

Embodiment 67. The C5a binding agent for use of any of Embodiments 62, 63, 64, 65 and 66, wherein at the trough level of the C5a binding agent of about 100 nM or at a level of the C5a binding agent in the blood of the subject of about 100 nM 100% of an activity of C5a in the blood of the subject is inhibited, preferably at the trough level of the C5a binding agent of 100 nM or at a level of the C5a binding agent in the blood of the subject of 100 nM 100% of an activity of C5a in the blood of the subject is inhibited.

Embodiment 68. The C5a binding agent for use of any of Embodiment 62, 63, 64, 65, 66 and 67, wherein at the trough level of the C5a binding agent of 80 nM or at a level of the C5a binding agent in the blood of the subject of 80 nM at least 80% of an activity of C5a in the blood of the subject is inhibited.

Embodiment 69. The C5a binding agent for use of any of Embodiments 62, 63, 64, 65, 66, 67 and 68, wherein at the trough level of the C5a binding agent of 90 nM or at a level of the C5a binding agent in the blood of the subject of 90 nM at least 90% of an activity of C5a in the blood of the subject is inhibited.

Embodiment 70. The C5a binding agent for use of any of Embodiments 62, 63, 64, 65, 66, 67, 68 and 69, wherein

a) the trough level is reached after at least two to four doses of the dosage regimens, and/or
b) the trough level is reached after the first dose the dosage regimens and before administering the second dose according to dosage regimens.

Embodiment 71. The binding agent for use of Embodiment 59, wherein, wherein the method comprises administering to the subject

a) a total amount of 7.59 to 220.25 nmol of the C5a binding agent per kg bodyweight of the subject over a period of 8 to 48 hours, or

b) a total amount of 0.1 to 2.9 mg of the C5a binding agent per kg bodyweight of the subject over a period of 8 to 48 hours.

Embodiment 72. The C5a binding agent for use of Embodiment 59, wherein the method comprises administering to the subject

a) a total amount of 22.78 to 72.15 nmol of the C5a binding agent per kg bodyweight of the subject every day, or
b) a total amount of 0.3 to 0.95 mg of the C5a binding agent per kg bodyweight of the subject every day.

Embodiment 73. The binding agent for use of any of Embodiments 71 and 72, wherein the method comprises administering to the subject

a) 102.53 to 220.25 nmol of the C5a binding agent per kg body weight of the subject every two days,
b) 29.62 to 72.15 nmol of the C5a binding agent per kg body weight of the subject every day,
c) 12.15 to 35.70 nmol of the C5a binding agent per kg body weight of the subject twice a day,
d) 7.59 to 24.3 nmol of the C5a binding agent per kg body weight of the subject three times a day,
e) 1.35 to 2.9 mg of the C5a binding agent per kg body weight of the subject every two days,
f) 0.39 to 0.95 mg of the C5a binding agent per kg body weight of the subject every day,
g) 0.16 to 0.47 mg of the C5a binding agent per kg body weight of the subject twice a day, or
h) 0.1 to 0.32 mg of the C5a binding agent per kg body weight of the subject three times a day.

Embodiment 74. The C5a binding agent for use of any one of Embodiments 71 to 72, wherein the method comprises administering to the subject multiple doses of the C5a binding agent at a trough level of the C5a binding agent of 80 to 300 nM in the blood of the subject, wherein preferably blood is whole blood, blood plasma or blood serum, wherein more preferably blood is blood plasma.
Embodiment 75. The C5a binding agent for use of Embodiment 74, wherein the multiple doses comprise a first dose, at least a second dose and optionally one or more subsequent dose(s).
Embodiment 76. The C5a binding agent for use of any one of Embodiments 74 to 75 wherein the trough level of the C5a binding agent is the level of the C5a binding agent in the blood of the subject before administering a or the second dose of the C5a binding agent and/or one or more of an or of the optional one or more subsequent dose(s) of the C5a binding agent.
Embodiment 77. The C5a binding agent for use of any of Embodiments 74, 75 and 76, wherein at the trough level of the C5a binding agent of about 100 to 300 nM or at a level of the C5a binding agent in the blood of the subject of ≥ about 100 nM 100% of the activity of C5a is inhibited, wherein preferably at the trough level of the C5a binding agent of 100 to 300 nM or at a level of the C5a binding agent in the blood of the subject of ≥ 100 nM 100% of the activity of C5a is inhibited.
Embodiment 78. The C5a binding agent for use of any one of Embodiment 74, 75, 76 and 77, wherein the trough level of the C5a binding agent is about 100 nM in the blood of the subject, preferably the trough level of the C5a binding agent is 100 nM in the blood of the subj ect.
Embodiment 79. The C5a binding agent for use of any of Embodiments 74, 75, 76, 77 and 78, wherein at the trough level of the C5a binding agent of about 100 nM or at a level of the C5a binding agent in the blood of the subject of about 100 nM 100% of an activity of C5a in the blood of the subject is inhibited, preferably at the trough level of the C5a binding agent of 100 nM or at a level of the C5a binding agent in the blood of the subject of 100 nM 100% of an activity of C5a in the blood of the subject is inhibited.
Embodiment 80. The C5a binding agent for use of any of Embodiments 74, 75, 76, 77, 78 and 79, wherein at the trough level of the C5a binding agent of 80 nM or at a level of the C5a binding agent in the blood of the subject of 80 nM at least 80% of an activity of C5a in the blood of the subject is inhibited.
Embodiment 81. The C5a binding agent for use of any of Embodiments 74, 75, 76, 77, 78, 79 and 80, wherein at the trough level of the C5a binding agent of 90 nM or at a level of the C5a binding agent in the blood of the subject of 90 nM at least 90% of an activity of C5a in the blood of the subject is inhibited.
Embodiment 82. The binding agent for use of any of Embodiments 74, 75, 76, 77, 78, 79, 80 and 81, wherein the trough level is reached after the first dose the dosage regimens and before administering the second dose according to dosage regimens.
Embodiment 83. The binding agent for use of Embodiment 59, wherein, wherein the method comprises administering to the subject

a) a total amount of 4.18to 205.06 nmol of the C5a binding agent per kg bodyweight of the subject over a period of 8 to 48 hours, or
b) a total amount of 0.055 to 2.7 mg of the C5a binding agent per kg bodyweight of the subject over a period of 8 to 48

hours.

Embodiment 84. The C5a binding agent for use of Embodiment 83, wherein the method comprises administering to the subject

a) a total amount of 12.53 to 59.24 nmol of the C5a binding agent per kg bodyweight of the subject every day, or
b) a total amount of 0.165 to 0.78 mg of the C5a binding agent per kg bodyweight of the subject every day.

Embodiment 85. The binding agent for use of any of Embodiments 83 and 84, wherein the method comprises administering to the subject

a) 98.73 to 205.06 nmol of the C5a binding agent per kg body weight of the subject every two days,
b) 28.82 to 59.24 nmol of the C5a binding agent per kg body weight of the subject every day,
c) 9.11 to 22.78 nmol of the C5a binding agent per kg body weight of the subject twice a day,
d) 4.18 to 11.39 nmol of the C5a binding agent per kg body weight of the subject three times a day,
e) 1.3 to 2.7 mg of the C5a binding agent per kg body weight of the subject every two days,
f) 0.34 to 0.78 mg of the C5a binding agent per kg body weight of the subject every day,
g) 0.12 to 0.3 mg of the C5a binding agent per kg body weight of the subject twice a day, or
h) 0.055 to 0.15 mg of the C5a binding agent per kg body weight of the subject three times a day.

Embodiment 86. The C5a binding agent for use of any one of Embodiments 83, 84 and 85, wherein the method comprises administering to the subject multiple doses of the C5a binding agent at a trough level of the C5a binding agent of 80 to 300 nM in the blood of the subject, wherein preferably blood is whole blood, blood plasma or blood serum, wherein more preferably blood is blood plasma.

Embodiment 87. The C5a binding agent for use of Embodiment 86, wherein the multiple doses comprise a first dose, at least a second dose and optionally one or more subsequent dose(s).

Embodiment 88. The C5a binding agent for use of any one of Embodiments 86 to 87, wherein the trough level of the C5a binding agent is the level of the C5a binding agent in the blood of the subject before administering a or the second dose of the C5a binding agent and/or one or more of an or of the optional one or more subsequent dose(s) of the C5a binding agent.

Embodiment 89. The C5a binding agent for use of any of Embodiments 86, 87 and 88, wherein at the trough level of the C5a binding agent of about 100 to 300 nM or at a level of the C5a binding agent in the blood of the subject of ≥ about 100 nM 100% of an activity of C5a in the blood of the subject is inhibited, wherein preferably at the trough level of the C5a binding agent of 100 to 300 nM or at a level of the C5a binding agent in the blood of the subject of ≥ 100 nM 100% of an activity of C5a in the blood of the subject is inhibited.

Embodiment 90. The C5a binding agent for use of any one of Embodiments 86, 87, 88 and 89, wherein the trough level of the C5a binding agent is about 100 nM in the blood of the subject, preferably the trough level of the C5a binding agent is 100 nM in the blood of the subj ect.

Embodiment 91. The C5a binding agent for use of any of Embodiments 86, 87, 88, 89 and 90, wherein at the trough level of the C5a binding agent of about 100 nM or at a level of the C5a binding agent in the blood of the subject of about 100 nM 100% of an activity of C5a in the blood of the subject is inhibited, preferably at the trough level of the C5a binding agent of 100 nM or at a level of the C5a binding agent in the blood of the subject of 100 nM 100% of an activity of C5a in the blood of the subject is inhibited.

Embodiment 92. The C5a binding agent for use of any of Embodiments 86, 87, 88, 89, 90 and 91, wherein at the trough level of the C5a binding agent of 80 nM or at a level of the C5a binding agent in the blood of the subject of 80 nM at least 80% of an activity of C5a in the blood of the subject is inhibited.

Embodiment 93. The C5a binding agent for use of any of Embodiments 86, 87, 88, 89, 90, 91 and 92, wherein at the trough level of the C5a binding agent of 90 nM or at a level of the C5a binding agent in the blood of the subject of 90 nM at least 90% of an activity of C5a in the blood of the subject is inhibited.

Embodiment 94. The C5a binding agent for use of any of Embodiments 86, 87, 88, 89, 90, 91, 92 and 93, wherein the trough level is reached after at least two to four doses of the dosage regimens.

Embodiment 95. The C5a binding agent for use of any of Embodiments 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93 and 94, wherein the method comprises administering to the subject

a) 121.5 nmol of the C5a binding agent per kg body weight of the subject every two days,
b) 37.97 nmol of the C5a binding agent per kg body weight of the subject every day,
c) 15.19 nmol of the C5a binding agent per kg body weight of the subject twice a day,

d) 9.11 nmol of the C5a binding agent per kg body weight of the subject three times a day,
e) 1.6 mg of the C5a binding agent per kg body weight of the subject every two days,
f) 0.5 mg of the C5a binding agent per kg body weight of the subject every day,
g) 0.2 mg of the C5a binding agent per kg body weight of the subject twice a day, or
h) 0.12 mg of the C5a binding agent per kg body weight of the subject three times a day,

Embodiment 96. The C5a binding agent for use of any of Embodiments 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 and 95, wherein the method comprises administering to the subject

15.19 nmol of the C5a binding agent per kg body weight of the subject twice a day.
or

0.2 mg of the C5a binding agent per kg body weight of the subject twice a day.

Embodiment 97. The C5a binding agent for use of Embodiment 96, wherein the trough level is reached after the first dose the dosage regimens and before administering the second dose, and wherein the trough level of the C5a binding agent is about 100 nM in the blood of the subject, preferably the trough level of the C5a binding agent is 100 nM in the blood of the subj ect.

Embodiment 98. The C5a binding agent for use of any one of Embodiments 96 and 97, wherein the trough level is reached after the first dose the dosage regimens and before administering the second dose, and the trough level of the C5a binding agent is 100 to 200 nM in the blood of the subject.

Embodiment 99. The binding agent for use of any one of Embodiments 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97 and 98, wherein the method comprises administering to the subject C5a binding agent every two days, wherein every two days means every 42 to 54 hours, wherein preferably every two days means every 44 to 52, wherein more preferably every two days means every 46 to 50 hours, wherein most preferably every two days means every 48 hours.

Embodiment 100. The binding agent for use of any one of Embodiments 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 and 99, wherein the method comprises administering to the subject C5a binding agent every day, wherein every day means 20 to 28 hours, wherein preferably every day means every 22 to 26 hours, wherein more preferably every day means every 23 to 25 hours, wherein most preferably every day means every 24 hours.

Embodiment 101. The binding agent for use of any of Embodiments 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100, wherein the method comprises administering to the subject C5a binding agent twice a day, wherein twice day means every 10 to 14 hours, wherein preferably twice day means every 11 to 13 hours, wherein more preferably twice day means every 12 hours.

Embodiment 102. The binding agent for use of any one of Embodiments 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 and 101, wherein the method comprises administering to the subject C5a binding agent three times a day, wherein three times a day means every 6 to 10 hours, wherein preferably three times day means every 8 hours.

Embodiment 103. The binding agent for use of Embodiment 59, wherein the method comprises administering to the subject
a loading dose of 3.42 to 4.56 nmol of the C5a binding agent per kg body weight of the subject following a total amount of 9.11 to 12.15 nmol the C5a binding agent per kg body weight of the subject administered throughout the day, wherein preferably total amount of 9.11 to 12.15 nmol the C5a binding agent per kg body weight of the subject is administered throughout the day is administered by continuous infusion to the subject.

Embodiment 104. The binding agent for use of Embodiment 103, wherein the method comprises administering to the subject
a loading dose of 4.56 nmol of the C5a binding agent per kg body weight of the subject following a total amount of 12.15 nmol the C5a binding agent per kg body weight of the subject administered throughout the day, wherein preferably total amount of to 12.15 nmol the C5a binding agent per kg body weight of the subject is administered throughout the day is administered by continuous infusion.

Embodiment 105. The binding agent for use of Embodiment 59, wherein the method comprises administering to the subject
a loading dose of 0.045 to 0.06 mg of the C5a binding agent per kg body weight of the subject following a total amount of 0.12 to 0.16 mg the C5a binding agent per kg body weight of the subject administered throughout the day, wherein preferably total amount of 0.12 to 0.16 mg the C5a binding agent per kg body weight of the subject is administered throughout the day is administered by continuous infusion to the subject.

Embodiment 106. The binding agent for of Embodiment 105, wherein the method comprises administering to the subject

a loading dose of 0.06 mg of the C5a binding agent per kg body weight of the subject following a total amount of 0.16 mg the C5a binding agent per kg body weight of the subject administered throughout the day, wherein preferably total amount of 0.16 mg the C5a binding agent per kg body weight of the subject is administered throughout the day is administered by continuous infusion to the subject.

Embodiment 107. The C5a binding agent for use of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105 and 106, wherein administering the C5a binding agent comprises administering the C5a binding agent by intravenous administration, subcutaneous administration or administration by inhalation, wherein preferably administering the C5a binding agent is administering the C5a binding agent by intravenous administration.

Embodiment 108. The C5a binding agent for use of Embodiment 107, wherein the intravenous administration is an intravenous infusion of a solution of the C5a binding agent, preferably for 15 to 30 minutes.

Embodiment 109. The C5a binding agent for use of Embodiment 107, wherein the intravenous administration is an intravenous infusion of a solution of the C5a binding and the C5a binding is administered for a maximum of 10 days.

Embodiment 110. The C5a binding agent for use of any one of Embodiments 108, 109 and 110, wherein the C5a binding agent is administered in a solution, wherein the solution comprises water, buffer, PBS, glucose solution and/or 4% mannitol/0.05% EDTA; wherein preferably the glucose solution is 5% glucose solution, wherein more preferably 4% mannitol/0.05% EDTA is used as a storage solution, wherein most preferably 4% mannitol/0.05% EDTA is used as a storage solution and the C5a binding agent in the storage solution is diluted in 5% glucose solution before administration.

Embodiment 111. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109 and 110, wherein the disease associated with or caused by C5a is characterized by an increased level of C5a in blood of the subject.

Embodiment 112. The C5a binding agent for use of Embodiment 111, wherein the level of C5a in blood of the subject is increased if the level of C5a is higher than 3 nM.

Embodiment 113. The C5a binding agent for use of any one of Embodiments 111 to 112, wherein blood is whole blood, blood plasma or blood serum, preferably blood plasma.

Embodiment 114. The C5a binding agent for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112 and 113, wherein the disease associated with or caused by C5a is a disease selected from the group comprising associated with complement activation and C5a-mediated pathogenic mechanisms, and/or is selected from the group comprising of autoimmune disease, inflammatory disease, systemic inflammatory response syndrome, disease of the eye, ischemia/reperfusion injuries, delayed graft function, transplant rejection, cardiovascular disease, respiratory disease, acute reactions, infectious disease, neurological disease, neurodegenerative disease, fibrotic disease, hematological disease, metabolic disease, tumors and clinical complications associated with complement activation by biomaterials,

wherein preferably the systemic inflammatory response syndrome is selected from the group comprising sepsis and secondary damages of trauma or severe burns,

wherein preferably the tumour is caner, more preferably lung cancer, and wherein preferably the respiratory disease is pneumonia.

Embodiment 115. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113 and 114, wherein the C5a binding agent is capable of binding to C5a and/or C5, preferably capable of binding to C5a and C5.

Embodiment 116. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111,

112, 113, 114 and 115, wherein the C5a binding agent is an agent having a binding affinity to C5a with a $K_D$ of about 0.1 to 5 nM, preferably 0.1 to 2 nM, more preferably 0.1 to 1.0 nM, and/or the C5a binding agent is an agent having a binding affinity to C5 with a $K_D$ of about 0.1 to 5 nM, preferably 0.1 to 2 nM, more preferably 0.1 to 1.0 nM.

Embodiment 117. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115 and 116, wherein the C5a binding agent is an inhibitor of an activity mediated by human C5a, wherein preferably the inhibitor is an antagonist.

Embodiment 118. The C5a binding agent for use of Embodiment 117, wherein the C5a binding agent is an inhibitor of an activity mediated by human C5 having an inhibitory constant IC50 of about 0.1 to 5 nM, preferably 0.1 to 2 nM, more preferably 0.1 to 0.5 nM.

Embodiment 119. The C5a binding agent for use of any of Embodiments 117 to 118, wherein the C5a binding agent is capable inhibiting C5a activity in the presence of C5.

Embodiment 120. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118 and 119, wherein the C5a binding agent has plasma half-life of about 6 hours to about 15 hours within the first 12 to 48 hours after administering the C5a binding agent to the subject as determined in the blood of the subj ect.

Embodiment 121. The C5a binding agent for use of any of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 and 120, wherein the C5a binding agent inhibits C5a activity in a blood sample from the subject in the presence of C5, wherein the blood sample is taken after administering the agent to the subject for at least eight hours, wherein inhibition of C5a activity is selected from the group comprising at least 75 % inhibition, at least 80% inhibition, at least 85% inhibition, at least 90% inhibition, at least 95% inhibition, and 100 % inhibition, wherein 100% inhibition corresponds to the C5a activity measured in an healthy individual.

Embodiment 122. The C5a binding agent for use of Embodiment 121, wherein the C5a activity of an healthy individual is the activity resulting from less than 3 nM C5a in the blood.

Embodiment 123. The C5a binding agent for use of Embodiment 123, wherein the blood is whole blood, blood plasma or blood serum, preferably blood plasma.

Embodiment 124. The C5a binding agent for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122 and 123, wherein the subject is a subject who suffers from a disease or is at risk of suffering from a disease, wherein the disease is associated with or caused by C5a.

Embodiment 125. The C5a binding agent for use of Embodiment 124, wherein the disease associated with or caused by C5a is characterized by an increased level of C5a in blood of the subj ect.

Embodiment 126. The C5a binding agent for use of Embodiment 125, wherein the level of C5a in blood of the subject is increased if the level of C5a is higher than 3 nM.

Embodiment 127. The C5a binding agent for use of any one of Embodiments 125, 126 and 127, wherein blood is whole blood, blood plasma or blood serum, preferably blood plasma.

Embodiment 128. The C5a binding agent for use of any one of Embodiments 124, 125, 126 and 127, wherein the disease is associated with complement activation and C5a-mediated pathogenic mechanisms, and/or is selected from the group comprising of autoimmune disease, inflammatory disease, systemic inflammatory response syndrome, disease of the eye, ischemia/reperfusion injuries, delayed graft function, transplant rejection, cardiovascular disease, respiratory disease, acute reactions, infectious disease, neurological disease, neurodegenerative disease, fibrotic disease, hematological disease, metabolic disease, tumors and clinical complications associated with complement activation by biomaterials,

wherein preferably the systemic inflammatory response syndrome is selected from the group comprising sepsis and secondary damages of trauma or severe burns,

wherein preferably the tumour is caner, more preferably lung cancer, and wherein preferably the respiratory disease is pneumonia.

Embodiment 129. The C5a binding agent for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, wherein the C5a binding agent is selected from the group comprising a C5a binding L-aptamer, a C5a binding aptamer, a C5a binding peptide aptamer, a C5a binding antibody, C5a binding antibody fragment and C5a binding anticalin, preferably a C5a binding L-aptamer.

Embodiment 130. The C5a binding agent for use of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127 and 128, wherein the C5a binding agent is selected from the group comprising a C5a binding L-aptamer, a C5a binding aptamer, a C5a binding peptide aptamer, a C5a binding antibody, C5a binding antibody fragment and C5a binding anticalin, preferably a C5a binding L-aptamer.

Embodiment 131. The C5a binding agent for use of Embodiment 130, wherein the C5a binding L-aptamer is a nucleic acid molecule capable of binding to human C5a, optionally comprising a modification, wherein the nucleic acid molecule comprises a central stretch of nucleotides, wherein the central stretch of nucleotides comprises a nucleotide sequence of
wherein the nucleic acid molecule comprises a central stretch of nucleotides, wherein the central stretch of nucleotides comprises a nucleotide sequence of
5' AUG$n_1$GGUGKU$n_2n_3$RGGGHUGUKGGG$n_4$G$n_5$CGACGCA 3' [SEQ ID NO: 61],
wherein

$n_1$ is U or dU, $n_2$ is G or dG, $n_3$ is A or dA, $n_4$ is U or dU, $n_5$ is U or dU and

G, A, U, C, H, K, and R are ribonucleotides, and

dU, dG and dA are 2'-deoxyribonucleotides.

Embodiment 132. The C5a binding agent for use of Embodiment 131, wherein the central stretch of nucleotides comprises a nucleotide sequence selected from the group of

a) 5' AUG$n_1$GGUGUU$n_2n_3$AGGGUUGUGGGG$n_4$G$n_5$CGACGCA 3' [SEQ ID NO: 62],

b) 5' AUG$n_1$GGUGUU$n_2n_3$GGGGUUGUGGGG$n_4$G$n_5$CGACGCA 3' [SEQ ID NO: 63],

c) 5' AUG$n_1$GGUGUU$n_2n_3$AGGGUUGUUGGG$n_4$G$n_5$CGACGCA 3' [SEQ ID NO: 64],

d) 5' AUG$n_1$GGUGGU$n_2n_3$AGGGUUGUUGGG$n_4$G$n_5$CGACGCA 3' [SEQ ID NO: 65],

e) 5' AUG$n_1$GGUGGU$n_2n_3$GGGGUUGUGGGG$n_4$G$n_5$CGACGCA 3' [SEQ ID NO: 66],

f) 5' AUG$n_1$GGUGGU$n_2n_3$GGGGAUGUGGGG$n_4$G$n_5$CGACGCA 3'[SEQ ID NO: 67], and

g) 5' AUG$n_1$GGUGUU$n_2n_3$GGGGCUGUGGGG$n_4$G$n_5$CGACGCA 3' [SEQ ID NO: 68], wherein

$n_1$ is U or dU, $n_2$ is G or dG, $n_3$ is A or dA, $n_4$ is U or dU, $n_5$ is U or dU and

G, A, U and C are ribonucleotides, and

dU, dG and dA are 2'-deoxyribonucleotides.

Embodiment 133. The C5a binding agent for use of Embodiment 132, wherein the central stretch of nucleotides comprises a nucleotide sequence of

5' AUG$n_1$GGUGGU$n_2n_3$AGGGUUGUUGGG$n_4$G$n_5$CGACGCA 3' [SEQ ID NO: 65] wherein

$n_1$ is U or dU, $n_2$ is G or dG, $n_3$ is A or dA, $n_4$ is U or dU, $n_5$ is U or dU and

G, A, U and C are ribonucleotides, and

dU, dG and dA are 2'-deoxyribonucleotides.

Embodiment 134. The C5a binding agent for use of Embodiment 133, wherein the central stretch of nucleotides comprises a nucleotide sequence selected from the group of

a) 5' AUGdUGGUGGUGAAGGGUUGUUGGGUGUCGACGCA 3' [SEQ ID NO: 73],
b) 5' AUGUGGUGGUdGAAGGGUUGUUGGGUGUCGACGCA 3' [SEQ ID NO: 74],
c) 5' AUGUGGUGGUGdAAGGGUUGUUGGGUGUCGACGCA 3' [SEQ ID NO: 75],
d) 5' AUGUGGUGGUGAAGGGUUGUUGGGdUGUCGACGCA 3' [SEQ ID NO: 76],
e) 5' AUGUGGUGGUGAAGGGUUGUUGGGUGdUCGACGCA 3' [SEQ ID NO: 77],
f) 5' AUGdUGGUGGUGAAGGGUUGUUGGGdUGUCGACGCA 3' [SEQ ID NO: 78],
g) 5' AUGdUGGUGGUGAAGGGUUGUUGGGUGdUCGACGCA 3'[SEQ ID NO: 79],
h) 5' AUGUGGUGGUGAAGGGUUGUUGGGdUGdUCGACGCA 3' [SEQ ID NO: 80],
i) 5' AUGdUGGUGGUGAAGGGUUGUUGGGdUGdUCGACGCA 3'[SEQ ID NO: 81],
j) 5' AUGdUGGUGGUdGAAGGGUUGUUGGGdUGdUCGACGCA 3' [SEQ ID NO: 82],
k) 5' AUGdUGGUGGUGdAAGGGUUGUUGGGdUGdUCGACGCA 3' [SEQ ID NO: 83],
l) 5' AUGdUGGUGGUdGdAAGGGUUGUUGGGdUGdUCGACGCA 3' [SEQ ID NO: 84],

preferably the central stretch of nucleotides is

5' AUGdUGGUGGUGAAGGGUUGUUGGGdUGUCGACGCA 3' [SEQ ID NO: 78] or
5' AUGdUGGUGGUdGdAAGGGUUGUUGGGdUGdUCGACGCA 3' [SEQ ID NO: 84].

Embodiment 135. The C5a binding agent for use of Embodiment 132, wherein the central stretch of nucleotides comprises a nucleotide sequence of
5' AUG$n_1$GGUGGU$n_2$$n_3$GGGGUUGUGGGG$n_4$G$n_5$CGACGCA 3' [SEQ ID NO: 66],
wherein

$n_1$ is U or dU, $n_2$ is G or dG, $n_3$ is A or dA, $n_4$ is U or dU, $n_5$ is U or dU and

G, A, U and C are ribonucleotides, and

dU, dG and dA are 2'-deoxyribonucleotides.

Embodiment 136. The C5a binding agent for use of Embodiment 132, wherein the central stretch of nucleotides comprises a nucleotide sequence of
5' AUG$n_1$GGUGGU$n_2$$n_3$GGGGAUGUGGGG$n_4$G$n_5$CGACGCA 3' [SEQ ID NO: 67],
wherein

$n_1$ is U or dU, $n_2$ is G or dG, $n_3$ is A or dA, $n_4$ is U or dU, $n_5$ is U or dU and

G, A, U and C are ribonucleotides, and

dU, dG and dA are 2'-deoxyribonucleotides.

Embodiment 137. The C5a binding agent for use of Embodiment 132, wherein the central stretch of nucleotides comprises a nucleotide sequence of
5' AUG$n_1$GGUGUU$n_2$$n_3$AGGGUUGUUGGG$n_4$G$n_5$CGACGCA 3' [SEQ ID NO: 64],
wherein

$n_1$ is U or dU, $n_2$ is G or dG, $n_3$ is A or dA, $n_4$ is U or dU, $n_5$ is U or dU and

G, A, U and C are ribonucleotides, and

dU, dG and dA are 2'-deoxyribonucleotides.

Embodiment 138. The C5a binding agent for use of any one of Embodiments 131, 132, 133, 134, 135, 136 and 137, wherein the central stretch of nucleotides consists of ribonucleotides and 2'-deoxyribonucleotides.

Embodiment 139. The C5a binding agent for use of any one of Embodiments 131, 132, 133, 135, 136 and 137, wherein the central stretch of nucleotides consists of ribonucleotides.

Embodiment 140. The C5a binding agent for use of any one of Embodiments 131, 132, 133, 134, 135, 136, 137, 138 and 139, wherein the nucleic acid molecule comprises in 5'->3' direction a first terminal stretch of nucleotides, the central stretch of nucleotides and a second terminal stretch of nucleotides, wherein

the first terminal stretch of nucleotides comprises one to five nucleotides, and

the second terminal stretch of nucleotides comprises one to five nucleotides,
preferably

the first terminal stretch of nucleotides comprises three to five nucleotides, and

the second terminal stretch of nucleotides comprises three to five nucleotides,
more preferably

the first terminal stretch of nucleotides comprises three nucleotides, and

the second terminal stretch of nucleotides comprises three nucleotides.

Embodiment 141. The C5a binding agent for use of Embodiment 140, wherein the first terminal stretch of nucleotides comprises a nucleotide sequence of 5' $Z_1Z_2Z_3Z_4$G 3' and the second terminal stretch of nucleotides comprises a nucleotide sequence of 5' $Z_5Z_6Z_7Z_8$ $Z_9$ 3',
wherein

$Z_1$ is G or absent, $Z_2$ is S or absent, $Z_3$ is S or absent, $Z_4$ is B or absent, $Z_5$ is C or dC, $Z_6$ is V or absent, $Z_7$ is S or absent, $Z_8$ is S or absent, $Z_9$ is C or absent, and

G, S, B, C, V are ribonucleotides, and

dC is a 2'-deoxyribonucleotide,

preferably

a) $Z_1$ is G, $Z_2$ is S, $Z_3$ is S, $Z_4$ is B, $Z_5$ is C or dC, $Z_6$ is V, $Z_7$ is S, $Z_8$ is S, $Z_9$ is C, or
b) $Z_1$ is absent, $Z_2$ is S, $Z_3$ is S, $Z_4$ is B, $Z_5$ is C or dC, $Z_6$ is V, $Z_7$ is S, $Z_8$ is S, $Z_9$ is absent, or
c) $Z_1$ is absent, $Z_2$ is absent, $Z_3$ is S, $Z_4$ is B, $Z_5$ is C or dC, $Z_6$ is V, $Z_7$ is S, $Z_8$ is absent, $Z_9$ is absent, or
d) $Z_1$ is absent, $Z_2$ is absent, $Z_3$ is absent, $Z_4$ is B, $Z_5$ is C or dC, $Z_6$ is V, $Z_7$ is absent, $Z_8$ is absent, $Z_9$ is absent, or
e) $Z_1$ is absent, $Z_2$ is S, $Z_3$ is S, $Z_4$ is B, $Z_5$ is C or dC, $Z_6$ is V, $Z_7$ is S, $Z_8$ is S, $Z_9$ is C, or
f) $Z_1$ is absent, $Z_2$ is absent, $Z_3$ is S, $Z_4$ is B, $Z_5$ is C or dC, $Z_6$ is V, $Z_7$ is S, $Z_8$ is S, $Z_9$ is C, or
g) $Z_1$ is absent, $Z_2$ is absent, $Z_3$ is absent, $Z_4$ is B, $Z_5$ is C or dC, $Z_6$ is V, $Z_7$ is S, $Z_8$ is S, $Z_9$ is C, or
h) $Z_1$ is absent, $Z_2$ is absent, $Z_3$ is absent, $Z_4$ is absent, $Z_5$ is C or dC, $Z_6$ is V, $Z_7$ is S, $Z_8$ is S, $Z_9$ is C, or
i) $Z_1$ is absent, $Z_2$ is absent, $Z_3$ is S, $Z_4$ is B, $Z_5$ is C or dC, $Z_6$ is V, $Z_7$ is S, $Z_8$ is S, $Z_9$ is absent, or
j) $Z_1$ is absent, $Z_2$ is absent, $Z_3$ is absent, $Z_4$ is B, $Z_5$ is C or dC, $Z_6$ is V, $Z_7$ is S, $Z_8$ is S, $Z_9$ is absent, or
k) $Z_1$ is absent, $Z_2$ is absent, $Z_3$ is absent, $Z_4$ is absent, $Z_5$ is C or dC, $Z_6$ is V, $Z_7$ is S, $Z_8$ is S, $Z_9$ is absent, or
l) $Z_1$ is absent, $Z_2$ is S, $Z_3$ is S, $Z_4$ is B, $Z_5$ is C or dC, $Z_6$ is V, $Z_7$ is S, $Z_8$ is absent, $Z_9$ is absent, or
m) $Z_1$ is absent, $Z_2$ is S, $Z_3$ is S, $Z_4$ is B, $Z_5$ is C or dC, $Z_6$ is V, $Z_7$ is absent, $Z_8$ is absent, $Z_9$ is absent, or
n) $Z_1$ is absent, $Z_2$ is absent, $Z_3$ is absent, $Z_4$ is absent, $Z_5$ is C, $Z_6$ is V, $Z_7$ is S, $Z_8$ is absent, $Z_9$ is absent, or
o) $Z_1$ is absent, $Z_2$ is absent, $Z_3$ is absent, $Z_4$ is B, $Z_5$ is C or dC, $Z_6$ is V, $Z_7$ is S, $Z_8$ is absent, $Z_9$ is absent, or
p) $Z_1$ is absent, $Z_2$ is absent, $Z_3$ is S, $Z_4$ is B, $Z_5$ is C or dC, $Z_6$ is V, $Z_7$ is absent, $Z_8$ is absent, $Z_9$ is absent, or
q) $Z_1$ is absent, $Z_2$ is absent, $Z_3$ is S, $Z_4$ is B, $Z_5$ is C or dC, $Z_6$ is absent, $Z_7$ is absent, $Z_8$ is absent, $Z_9$ is absent.

Embodiment 142. The C5a binding agent for use of any one of Embodiments 140 to 141, wherein the first terminal stretch of nucleotides comprises a nucleotide sequence of 5' GCCUG 3' and the second terminal stretch of nucleotides comprises a nucleotide sequence of 5' CAGGC 3' or of 5' dCAGGC 3', wherein

C, A, G and U are ribonucleotides, and

dC is a 2'-deoxyribonucleotide.

Embodiment 143. The C5a binding agent for use of Embodiment 142, wherein the second terminal stretch of nucleotides comprises a nucleotide sequence of 5' dCAGGC 3'.

Embodiment 144. The C5a binding agent for use of Embodiment 142, wherein the second terminal stretch of nucleotides comprises a nucleotide sequence of 5' CAGGC 3'.

Embodiment 145. The nucleic acid molecule according to any one of Embodiments 140 to 141, wherein

the first terminal stretch of nucleotides comprises a nucleotide sequence of 5' CCUG 3' or 5' CUG 3' or 5' UG 3'or 5' G 3', and

the second terminal stretch of nucleotides comprises a nucleotide sequence of 5' dCAGGC 3', wherein

C, A, G and U are ribonucleotides, and

dC is a 2'-deoxyribonucleotide.

Embodiment 146. The C5a binding agent for use of Embodiment 145, wherein
the first terminal stretch of nucleotides comprises a nucleotide sequence of 5' CCUG 3'.

Embodiment 147. The C5a binding agent for use of Embodiment 145, wherein
the first terminal stretch of nucleotides comprises a nucleotide sequence of 5' CUG 3'.

Embodiment 148. The C5a binding agent for use of Embodiments 140 to 141, wherein

a) the first terminal stretch of nucleotides comprises a nucleotide sequence of 5' GCUG 3' and the second terminal stretch of nucleotides comprises a nucleotide sequence of 5' dCAGC 3'; or
b) the first terminal stretch of nucleotides comprises a nucleotide sequence of 5' GCCG 3' and the second terminal stretch of nucleotides comprises a nucleotide sequence of 5' dCGGC 3'; or
c) the first terminal stretch of nucleotides comprises a nucleotide sequence of 5' GGCG 3' and the second terminal stretch of nucleotides comprises a nucleotide sequence of 5' dCGCC 3'; wherein

C, A, G and U are ribonucleotides, and

dC is a 2'-deoxyribonucleotide.

Embodiment 149. The C5a binding agent for use of Embodiment 148, wherein
first terminal stretch of nucleotides comprises a nucleotide sequence of 5' GGCG 3' and the second terminal stretch of nucleotides comprises a nucleotide sequence of 5' dCGCC 3'.

Embodiment 150. The C5a binding agent for use of any one of Embodiments 140 to 141, wherein

the first terminal stretch of nucleotides comprises a nucleotide sequence of 5' CUG 3' or 5' UG 3'or 5' CG 3' or 5' G 3', and

the second terminal stretch of nucleotides comprises a nucleotide sequence of 5' dCAGC 3', wherein

C, A, G and U are ribonucleotides, and

dC is a 2'-deoxyribonucleotide.

Embodiment 151. The C5a binding agent for use of any one of Embodiments 140 to 141, wherein

the first terminal stretch of nucleotides comprises a nucleotide sequence of 5' GCUG 3', and

the second terminal stretch of nucleotides comprises a nucleotide sequence of 5' dCAC 3' or 5' dCC 3' or 5' dCA 3', wherein

C, A, G and U are ribonucleotides, and

dC is a 2'-deoxyribonucleotide.

Embodiment 152. The C5a binding agent for use of any one of Embodiments 140 to 141, wherein

a) the first terminal stretch of nucleotides comprises a nucleotide sequence of 5' GUG 3' and the second terminal stretch of nucleotides comprises a nucleotide sequence of 5' dCAC 3'; or
b) the first terminal stretch of nucleotides comprises a nucleotide sequence of 5' UG 3' and the second terminal stretch of nucleotides comprises a nucleotide sequence of 5' dCA 3'; or
c) the first terminal stretch of nucleotides comprises a nucleotide sequence of 5' GCG 3' and the second terminal stretch of nucleotides comprises a nucleotide sequence of 5' dCGC 3'; or
d) the first terminal stretch of nucleotides comprises a nucleotide sequence of 5' CG 3' and the second terminal stretch of nucleotides comprises a nucleotide sequence of 5' dCGC 3'; or
e) the first terminal stretch of nucleotides comprises a nucleotide sequence of 5' G 3' and the second terminal stretch of nucleotides comprises a nucleotide sequence of 5' dCGC 3'; or
f) the first terminal stretch of nucleotides comprises a nucleotide sequence of 5' GCG 3' and the second terminal stretch of nucleotides comprises a nucleotide sequence of 5' dCC 3'; or
g) the first terminal stretch of nucleotides comprises a nucleotide sequence of 5' GCG 3' and the second terminal stretch of nucleotides comprises a nucleotide sequence of 5' dC 3'; or
h) the first terminal stretch of nucleotides comprises a nucleotide sequence of 5' GG 3' and the second terminal stretch of nucleotides comprises a nucleotide sequence of 5' dCC 3';
i) the first terminal stretch of nucleotides comprises a nucleotide sequence of 5' GCG 3' and the second terminal stretch of nucleotides comprises a nucleotide sequence of 5' CGC 3'; wherein

C, A, U and G are ribonucleotides, and

dC is a 2'-deoxyribonucleotide.

Embodiment 153. The C5a binding agent for use of Embodiment 152, the first terminal stretch of nucleotides comprises a nucleotide sequence of 5' GCG 3' and the second terminal stretch of nucleotides comprises a nucleotide sequence of 5' dCGC 3'.

Embodiment 154. The C5a binding agent for use of any one of Embodiments 131, 132, 133, 135, 136, 137, 139, 140, 141, 142, and 144, wherein the nucleic acid molecule comprises a nucleotide sequence selected from the group of SEQ ID NO: 3, SEQ ID NO: 4, SEQ ID NO: 5, SEQ ID NO: 6 and SEQ ID NO: 90, or a nucleic acid molecule having an identity of at least 85% to the nucleic acid molecule comprising a nucleotide sequence selected from the group of SEQ ID NO: 3, SEQ ID NO: 4, SEQ ID NO: 5, SEQ ID NO: 6 and SEQ ID NO: 90, or a nucleic acid molecule which is homologous to the the nucleic acid molecule comprising a nucleotide sequence selected from the group of SEQ ID NO: 3, SEQ ID NO: 4, SEQ ID NO: 5, SEQ ID NO: 6 and SEQ ID NO: 90, wherein the homology is at least 85%.

Embodiment 155. The C5a binding agent for use of any one of Embodiments 131, 132, 133, 134, 138, 140, 141, 142 and 143, wherein the nucleic acid molecule comprises a nucleotide sequence selected from the group of SEQ ID NO: 14, SEQ ID NO: 21, SEQ ID NO: 24, SEQ ID NO: 25, SEQ ID NO: 26, SEQ ID NO: 27, SEQ ID NO: 37, SEQ ID NO: 57, SEQ ID NO: 59, SEQ ID NO: 60, SEQ ID NO: 91 and SEQ ID NO: 92, or a nucleic acid molecule having an identity of at least 85% to the nucleic acid molecule comprising a nucleotide sequence selected from the group of 14, SEQ ID NO: 21, SEQ ID NO: 24, SEQ ID NO: 25, SEQ ID NO: 26, SEQ ID NO: 27, SEQ ID NO: 37, SEQ ID NO: 57, SEQ ID NO: 59, SEQ ID NO: 60, SEQ ID NO: 91 and SEQ ID NO: 92, or a nucleic acid molecule which is homologous to the the nucleic acid molecule comprising a nucleotide sequence selected from the group of 14, SEQ ID NO: 21, SEQ ID NO: 24, SEQ ID NO: 25, SEQ ID NO: 26, SEQ ID NO: 27, SEQ ID NO: 37, SEQ ID NO: 57, SEQ ID NO: 59, SEQ ID NO: 60, SEQ ID NO: 91 and SEQ ID NO: 92, wherein the homology is at least 85%.

Embodiment 156. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154 and 155, wherein the nucleic acid molecule is capable of binding human C5a and mouse C5a.

Embodiment 157. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154, 155 and 156, wherein the nucleic acid molecule comprises at least one binding moiety which is capable of binding human C5a and mouse C5a, wherein such binding moiety consists of L-nucleotides.

Embodiment 158. The C5a binding agent for use of any one of Embodiments 131, 132, 133, 134, 135, 136, 137,

138, 139, 140, 141, 142, 143, 144, 145, 146, 147, 148, 149, 150, 151, 152, 153, 154, 155, 156 and 157, wherein the nucleotides of or the nucleotides forming the nucleic acid molecule are L-nucleotides.

Embodiment 159. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154, 155, 156, 157 and 158, wherein the nucleic acid molecule is an L-nucleic acid molecule.

Embodiment 160. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154, 155, 156, 157, 158 amd 159, wherein the nucleic acid is an antagonist of an activity mediated by human and/or mouse C5a.

Embodiment 161. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154, 155, 156, 157, 158, 159 and 160, wherein the nucleic acid molecule comprises a modification group, wherein excretion rate of the nucleic acid molecule comprising the modification group from an organism is decreased compared to a nucleic acid not comprising the modification group.

Embodiment 162. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154, 155, 156, 157, 158, 159 and 160, wherein the nucleic acid molecule comprises a modification group, wherein the nucleic acid molecule comprising the modification group has an increased retention time in an organism compared to a nucleic acid molecule not comprising the modification group.

Embodiment 163. The C5a binding agent for use of any one of Embodiments 161 and 162, wherein the modification group is selected from the group comprising biodegradable and non-biodegradable modifications, preferably the modification group is selected from the group comprising polyethylene glycol, linear polyethylene glycol, branched polyethylene glycol, hydroxyethyl starch, a peptide, a protein, a polysaccharide, a sterol, polyoxypropylene, polyoxyamidate and poly (2-hydroxyethyl)-L-glutamine.

Embodiment 164. The C5a binding agent for use of Embodiment 163, wherein the modification group is a polyethylene glycol, preferably consisting of a linear polyethylene glycol or branched polyethylene glycol, wherein the molecular weight of the polyethylene glycol is preferably from about 20,000 to about 120,000 Da, more preferably from about 30,000 to about 80,000 Da and most preferably about 40,000 Da.

Embodiment 165. The C5a binding agent for use of Embodiment 163, wherein the modification group is hydroxyethyl starch, wherein preferably the molecular weight of the hydroxyethyl starch is from about 50 to about 1000 kDa, more preferably from about 100 to about 700 kDa and most preferably from 200 to 500 kDa.

Embodiment 166. The C5a binding agent for use of any one of Embodiments 161, 162, 163, 164 and 165, whereby the modification group is coupled to the nucleic acid molecule via a linker, whereby preferably the linker is a biodegradable linker.

Embodiment 167. The C5a binding agent for use of any one of Embodiments 161, 162, 163, 164, 165 and 166, wherein the modification group is coupled to the 5'-terminal nucleotide and/or the 3'-terminal nucleotide of the nucleic acid molecule.

Embodiment 168. The C5a binding agent for use of any one of Embodiments 161, 162, 163, 164, 165, 166 and 167, wherein the organism is an animal or a human body, preferably a human body.

Embodiment 169. The C5a binding agent for use of any one of Embodiments131, 132, 133, 134, 135, 136, 137, 138, 139, 140, 141, 142, 143, 144, 145, 146, 147, 148, 149, 150, 151, 152, 153, 154, 155, 156, 157, 158, 159, 160,161, 162, 163, 164, 165, 166, 167 and 168, wherein the is nucleic acid molecule comprises a nucleotide sequence of SEQ ID NO: 59 or of a SEQ ID NO: 92 and a modification, wherein the modification has modification group that is a polyethylene glycol, preferably consisting of a branched polyethylene glycol, wherein the molecular weight of the polyethylene glycol is about 40,000 Da.

Embodiment 170. The C5a binding agent for use of Embodiment 169, wherein the nucleic acid molecule of a SEQ ID NO: 92 consists of phosphodiester-linked 37 L-RNA nucleotides and 3 L-DNA nucleotides that is conjugated at its 5'-end covalently to a hexylamino linker and 40 kDa PEG and the chemical name is L-cytidylyl-(3'→75')- L-guanylyl-(3'→75')- L-deoxycytidylyl-(3'→75')- L-adenylyl-(3'→5')-L-cytidylyl-(3'→75')- L-guanylyl-(3'→5')- L-cytidylyl-(3'→5')- L-adenylyl-(3'→5')-L-guanylyl-(3'→5')- L-cytidylyl-(3'→5')- L-uridylyl-(3'→5')- L-guanylyl-(3'→5')-L-deoxyuridylyl-(3'→5')- L-guanylyl-(3'→5')- L-guanylyl-(3'→5')- L-guanylyl-(3'→5')-L-uridylyl-(3'→5')- L-uridylyl-(3'→5')- L-guanylyl-(3'->5')- L-uridylyl-(3'->5')-L-uridylyl-(3'→5')- L-guanylyl-(3'→5')- L-guanylyl-(3'->5')- L-guanylyl-(3'→5')-L-adenylyl-(3'->5')- L-adenylyl-(3'→5')- L-guanylyl-(3'->5')- L-uridylyl-(3'->5')-L-guanylyl-(3'→5')- L-guanylyl-(3'→5')- L-uridylyl-(3'→5')- L-guanylyl-(3'->5')-L-guanylyl-(3'→5')- L-deoxyuridylyl-(3'-> 5')- L-guanylyl-(3'→5')- L-uridylyl-(3'→5')-L-adenylyl-(3'→5')- L-guanylyl-(3'→5')- L-cytidylyl-(3'→5')- L-guanylyl-(3'->5')-5'-ester with N-[ω-methoxypoly(oxy-1,2-ethanediyl], N'-[w-methoxypolγ(oxy-1,2-ethanediyi]-acetyl 9-amino-8-oxo-7-aza-nonyloxy phosphate, sodium salt.

Embodiment 171. The C5a binding agent for use of any one of Embodiments 131, 132, 133, 134, 135, 136, 137, 138, 139, 140, 141, 142, 143, 144, 145, 146, 147, 148, 149, 150, 151, 152, 153, 154, 155, 156, 157, 158, 159, 160,161, 162, 163, 164, 165, 166, 167, 168, 169 and 170, wherein the C5a binding agent is compound AON-D21.

Embodiment 172. The C5a binding agent for use of Embodiment 171, wherein

AON-D21 is represented by the following structural formula:

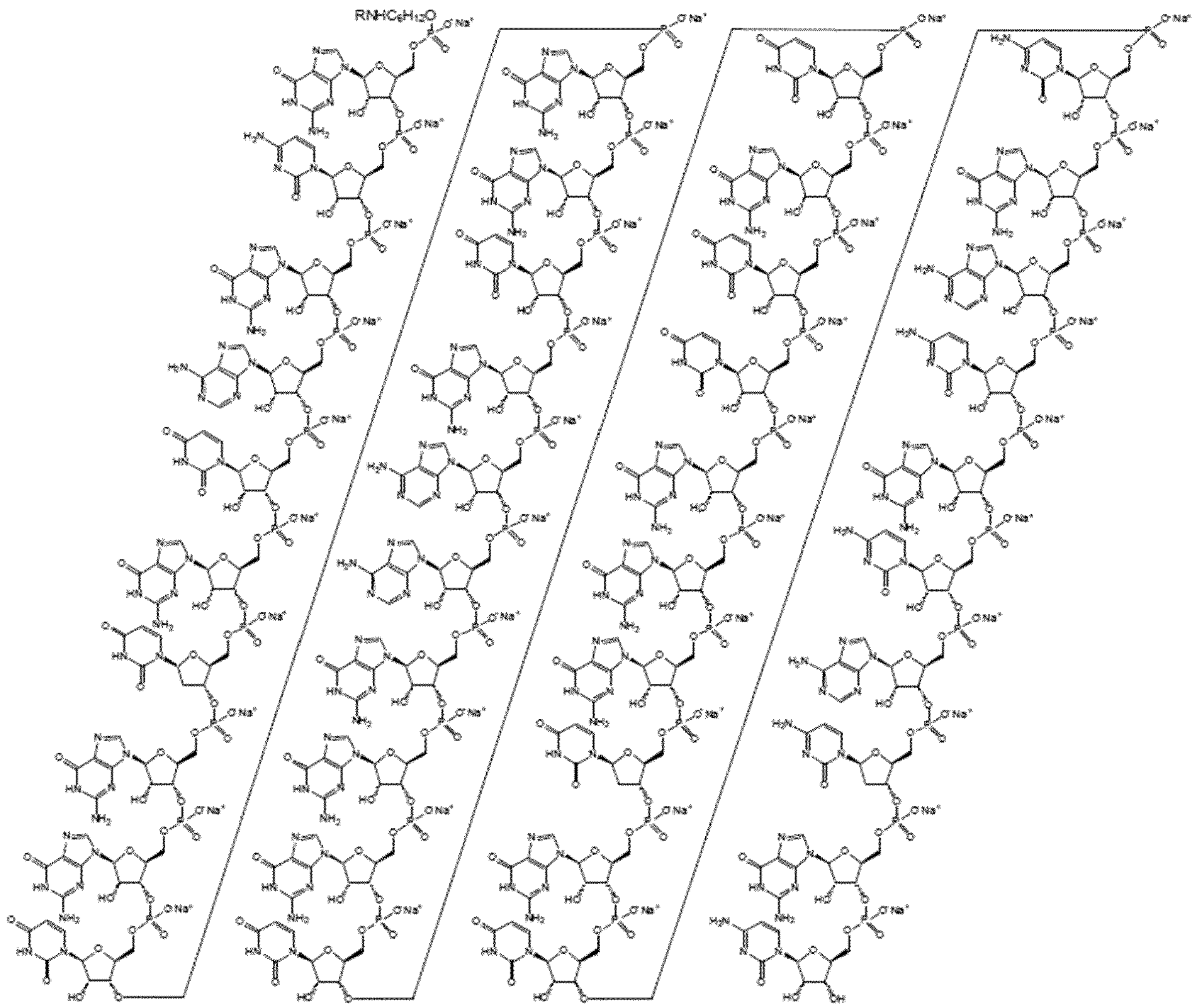

(structural formula I)

wherein NHC6H12O is a hexylamino linker and R is

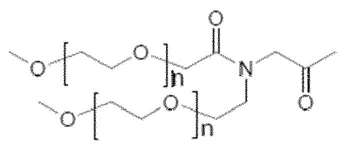

and n is approximately 450,

wherein the molecular formula is C398H458N160Na40O287P40[C2H4O]2n, wherein n is approximately 450.

Embodiment 173. The C5a binding agent for use of Embodiment 172, wherein

R is a PEG moiety with a molecular weight of about 40 kDa,

the oligonucleotide part of AON-D21 as an anhydrous free acid is represented by structural formula I without R (the PEG moiety), and

the molecular weight of the oligonucleotide part of AON-D21 as an anhydrous free acid is 13167 Da.

Embodiment 174. The C5a binding agent for use of any one of Embodiments 172 to 173, wherein AON-D21 comprises a nucleic acid moiety and a non-nucleic acid moiety, wherein the non-nucleic acid moiety is the PEG moiety preferably comprising the linker, and the nucleic acid moiety is the oligonucleotide part of AON-D21, preferably is the oligonucleotide part of AON-D21 as an anhydrous free acid.

Embodiment 175. The C5a binding agent for use of any one of Embodiments 172, 173 and 174, wherein 0.2 mg of AON-D21 per kg body weight of the subject corresponds to 15.19 nmol of AON-D21 per kg body weight of the subject.

Embodiment 176. The C5a binding agent for use of Embodiment 175, wherein the 0.2 mg of AON-D21 refers to the oligonucleotide part of AON-D21as an anhydrous, free acid.

Embodiment 177. The C5a binding agent for use of any one of Embodiments 171, 172, 173, 174, 175 and 176, wherein

the method comprises administering to the subject a dose of 0.2 mg of AON-D21 per kg body weight of the subject or a dose of 15.19 nmol of AON-D21 per kg body weight of the subject every 11 to 13 hours, preferably every 12 hours,

wherein the route of administration is an intravenous infusion, wherein the intravenous infusion is a central intravenous infusion or intravenous peripheral infusion,

wherein the duration of the infusion is 15 to 30 minutes.

The present inventors have found that a dosage regimen for a C5a binding agent that leads to a level of the C5a binding agent in the blood of a subject that is lower than a typically observed level of C5 in blood of a subject of about 450 nM, allows an inhibition of 100 % of an activity of C5a contained in blood of the subject. This finding is surprising because the level of C5a in blood of a subject, even in a subject suffering from a disease that is associated with or caused by C5a, is significantly lower, typically by at least one magnitude, than the level of C5 in blood of such subject.

This surprising finding has also been observed for a C5a binding agent that is capable of binding to both C5a and C5, and even further been observed for a C5a binding agent the binding affinity of which to both C5a and C5 is about the same.

Furthermore, the present inventors have surprisingly found that a dosing and, respectively, a dosage regimen according to the present invention reduces an activity of C5a in blood of a subject only for a period of time which is sufficiently long for achieving a desired therapeutic effect, but avoids or reduces treatment-emergent adverse events. Typically, a treatment-emergent adverse event results from suppressing the positive effect of C5a for too long after achieving the desired therapeutic effect upon which the treatment is usually terminated. In case of an anti-C5a therapy such as administering a C5a binding agent to a subject, a treatment-emergent adverse event may be an infection, typically a microbial infection arising from microbial invasion due to a missing C5a-mediated pro-inflammatory response of the subject's immune system.

The problems underlying the present invention are solved in a first aspect by a C5a binding agent for use in a method of treating and/or preventing a disease in a subject, wherein the disease is associated with or caused by C5a, wherein the method comprises administering to the subject multiple doses of the C5a binding agent at a trough level of the C5a binding agent of 80 to 300 nM in the blood of the subject. In such method two or more doses of the C5a binding agent are administered to the subject. A dose of the C5a binding agent is administered to the subject at a trough level which preferably means that the C5a binding agent is administered to the subject at a point in time when the level of the C5a binding agent in the subject is the same as or close to the trough level of the C5a binding agent in the subject. Determinig the level of the C5a binding agent in the subject is within the skills of a person of the art. In an embodiment, the level of the C5a binding agent in the subject is determined one or several times for determining whether the trough level has been reached. It will be appreciated by a person skilled in the art that based on considerations relying on pharmacokinetics and pharmacodynamics as well as empirical data, in particular empirical data of the individual subject, the reaching of the trough level can also be calculated and thus the administration of the next dose scheduled for a distinct point in time based on such calculation.

Preferably, a level of the C5a binding agent in the subject is close to the trough level of the C5a binding agent in the subject if the administration of the next dose of the C5a binding agent to the subject is suitable to increase the level of the C5a binding agent in the subject such that the desired therapeutic effect which is intended to be achieved by such next dose of the C5a binding agent is achieved. The range of levels which are regarded as being close to the trough level are known to a person skilled in the art such as a person managing the disease in the subject or may be determined by routine methods. In an embodiment, a level of the C5a binding agent is close to the trough level if said level of the C5a binding agent is ± 10 % of the trough level or less, preferably ± 5 % of the trough level or less and more prefebaly ± 3 % of the trough level or less.

It will be appreciated by a person skilled in the art that there may be a gap between the point in time when the level of the C5a binding agent is determined in the subject and the administration of any of the multiple doses of the C5a binding agent. Preferably, such gap in time does not have any impact on the treating and/or preventing of the disease associated with or caused by C5a and the tolerable length of such gap is known to a person skilled in the art such as a person managing the disease in the subject. Preferably, such gap in time comprises about 15 minutes and any multiples thereof up to one, two, three or four hours, whereby such gap also depende on the frequence with such any dose of the C5a binding agent is administered to the subject.

According to the present invention, the trough level of the C5a binding agent is from about 80 to about 300 nM. In an embodiment, the trough level is selected from 80 nM, 90 nM, 100 nM, 110 nM, 120 nM, 130 nM, 140 nM, 150 nM, 160 nM, 170 nM, 180 nM, 190 nM, 200 nM, 210 nM, 220 nM, 230 nM, 240 nM, 250 nM, 260 nM, 270 nM, 280 nM, 290 nM, and 300 nM; preferably, the trough level is selected from 80 nM, 90 nM, 100 nM, 110 nM, 120 nM and 130 nM; more preferably, the trough level is selected from 80 nM, 90 nM and 100 nM; most preferably the trough level is 100 nM.

The problems underyling the present invention are solved in a second aspect by a C5a binding agent for use in a method of treating and/or preventing a disease in a subject, wherein the disease is associated with or caused by C5a, wherein the method comprises administering to the subject multiple doses of the C5a binding agent at a level of the C5a binding agent in the blood of the subject of 80 to 300 nM. The level of the C5a binding agent in the blood of the subject of 80 to 300 nM may also be referred to as the minimum level.

In such method two or more doses of the C5a binding agent are administered to the subject. A dose of the C5a binding agent is administered to the subject at a minimum level which preferably means that the C5a binding agent is administered to the subject at a point in time when the level of the C5a binding agent in the subject is the same as or close to the minimum level of the C5a binding agent in the subject. Determinig the level of the C5a binding agent in the subject is within the skills of a person of the art. In an embodiment, the level of the C5a binding agent in the subject is determined one or several times for determining whether the minimum level has been reached. It will be appreciated by a person skilled in the art that based on considerations relying on pharmacokinetics and pharmacodynamics as well as empirical data, in particular empirical data of the individual subject, the reaching of the minimum level can also be calculated and thus the administration of the next dose scheduled for a distinct point in time based on such calculation.

Preferably, a level of the C5a binding agent in the subject is close to the minimum level of the C5a binding agent in the subject if the administration of the next dose of the C5a binding agent to the subject is suitable to increase the level of the C5a binding agent in the subject such that the desired therapeutic effect which is intended to be achieved by such next dose of the C5a binding agent is achieved. The range of levels which are regarded as being close to the minimum level are known to a person skilled in the art such as a person managing the disease in the subject or may be determined by routine methods. In an embodment, a level of the C5a binding agent is close to the minimum level if said level of the C5a binding agent is ± 10 % of the minimum level or less, preferably ± 5 % of the trough level or less and more prefebaly ± 3 % of the minimum level or less.

It will be appreciated by a person skilled in the art that there may be a gap between the point in time when the level of the C5a binding agent is determined in the subject and the administration of any of the multiple doses of the C5a binding agent. Preferably, such gap in time does not have any impact on the treating and/or preventing of the disease associated with or caused by C5a and the tolerable length of such gap is known to a person skilled in the art such as a person managing the disease in the subject. Preferably, such gap in time comprises about 15 minutes and any multiples thereof up to one, two, three or four hours, whereby such gap also depende on the frequence with such any dose of the C5a binding agent is administered to the subject.

According to the present invention, the trough level of the C5a binding agent is from about 80 to about 300 nM. In an embodiment, the trough level is selected from 80 nM, 90 nM, 100 nM, 110 nM, 120 nM, 130 nM, 140 nM, 150 nM, 160 nM, 170 nM, 180 nM, 190 nM, 200 nM, 210 nM, 220 nM, 230 nM, 240 nM, 250 nM, 260 nM, 270 nM, 280 nM, 290 nM, and 300 nM; preferably, the trough level is selected from 80 nM, 90 nM, 100 nM, 110 nM, 120 nM and 130 nM; more preferably, the trough level is selected from 80 nM, 90 nM and 100 nM; most preferably the trough level is 100 nM.

The problems underyling the present invention are solved in a third aspect by a C5a binding agent for use in a method of treating and/or preventing a disease in a subject, wherein the disease is associated with or caused by C5a, wherein the method comprises administering to the subject the C5a binding agent during a treatment period, wherein the administering of the C5a binding agent provides a level of the C5a binding agent in the blood of the subject, wherein the level of the C5a binding agent in the blood of the patient is

a) a minimum level of 80 to 300 nM maintained during the treatment period of the subject; or

b) a minimum level of 80 to 300 nM at the end of the treatment period of the subject.

According to this third aspect, the C5a binding agent is administered to a subject when the level of the C5a binding agent is the same as or close to a minimum level of the C5a binding, whereby such minimum level is from about 80 nM to

about 300 nM. In an embodiment, the minimum level is maintained during the treatment period of the subject, i.e. during the period in time when the subject is treated. In an alternative embodiment, the minimum level is maintained at the end of a treatment period of the subject, i.e. at a point in time when the subject is is no longer treated of the treatment of the subject has been terminated.

**In** such method two or more doses of the C5a binding agent are administered to the subject. A dose of the C5a binding agent is administered to the subject at a minimum level which preferably means that the C5a binding agent is administered to the subject at a point in time when the level of the C5a binding agent in the subject is the same as or close to the minimum level of the C5a binding agent in the subject. Determinig the level of the C5a binding agent in the subject is within the skills of a person of the art. In an embodiment, the level of the C5a binding agent in the subject is determined one or several times for determining whether the minimum level has been reached. It will be appreciated by a person skilled in the art that based on considerations relying on pharmacokinetics and pharmacodynamics as well as empirical data, in particular empirical data of the individual subject, the reaching of the minimum level can also be calculated and thus the administration of the next dose scheduled for a distinct point in time based on such calculation.

Preferably, a level of the C5a binding agent in the subject is close to the minimum level of the C5a binding agent in the subject if the administration of the next dose of the C5a binding agent to the subject is suitable to increase the level of the C5a binding agent in the subject such that the desired therapeutic effect which is intended to be achieved by such next dose of the C5a binding agent is achieved. The range of levels which are regarded as being close to the minimum level are known to a person skilled in the art such as a person managing the disease in the subject or may be determined by routine methods. In an embodment, a level of the C5a binding agent is close to the minimum level if said level of the C5a binding agent is ± 10 % of the minimum level or less, preferably ± 5 % of the trough level or less and more prefebaly ± 3 % of the minimum level or less.

It will be appreciated by a person skilled in the art that there may be a gap between the point in time when the level of the C5a binding agent is determined in the subject and the administration of any of the multiple doses of the C5a binding agent. Preferably, such gap in time does not have any impact on the treating and/or preventing of the disease associated with or caused by C5a and the tolerable length of such gap is known to a person skilled in the art such as a person managing the disease in the subject. Preferably, such gap in time comprises about 15 minutes and any multiples thereof up to one, two, three or four hours, whereby such gap also depende on the frequence with such any dose of the C5a binding agent is administered to the subject.

According to the present invention, the trough level of the C5a binding agent is from about 80 to about 300 nM. In an embodiment, the trough level is selected from 80 nM, 90 nM, 100 nM, 110 nM, 120 nM, 130 nM, 140 nM, 150 nM, 160 nM, 170 nM, 180 nM, 190 nM, 200 nM, 210 nM, 220 nM, 230 nM, 240 nM, 250 nM, 260 nM, 270 nM, 280 nM, 290 nM, and 300 nM; preferably, the trough level is selected from 80 nM, 90 nM, 100 nM, 110 nM, 120 nM and 130 nM; more preferably, the trough level is selected from 80 nM, 90 nM and 100 nM; most preferably the trough level is 100 nM.

According to this third aspect, the method comprises administering to the subject the C5a binding agent during a treatment period, whereby such treatment comprises administering to the subject at last a first dose of the C5a binding agent and the administering to the subject at least a last dose of the C5a binding agent. In an embodiment, the treatment period starts with the administering of the first dose of the C5a binding agent which is administered to the subject and ends at a time at which the level of the C5a binding agent in the blood of the subject falls below 80 to 300 nM or is lower than 80 to 300 nM after the last dose of the C5a binding agent was administered. In an alternative embodiment, the treatment period starts with a first dose of a C5a binding agent which is administered to the subject and ends when a medical practitioner terminates it or declares it to be terminated. Preferably the medical practitioner is a medical doctor, a nurse or a medical practitioner service provider. Alternatively or more preferably, the medical practitioner is a person who is in charge of managing the disease of the subject. Symptoms/side effects taking into consideration one or more characteristics of the subject selected from the group comprising the overall health condition, age, gender, course of the disease associated with or caused by C5a, other disease(s) from which the subject suffers or is at risk of suffering, prognosis of the disease associated with or caused by C5a, in particular taking into consideration genetic disposition and family history.

The problems underyling the present invention are solved in a fourth aspect by a C5a binding agent for use in a method for treating and/or preventing a disease in a subject, wherein the disease is associated with or caused by C5a, wherein the method comprises administering the C5a binding agent to the subject, wherein an activity of C5a in the blood of the subject is reduced by 50% or less within 24 to 72 hours after administering the C5a binding agent to the subject. According to this method an activity of C5a is reduced by 50 % or less which preferably means that an activity of C5a is back to at leat 50 % of its original activity in the blood of the subject within 24 to 72 hours. This preferably means that the activity of C5a is reduced by at least 50 % and any percentage lower than 50 % including but not limited to 45 %, 40 %, 35 %, 30 %, 25%, 20 %, 15 %, 10 %, 5 % and 0%. Recuding an activity of C5a by 0 % means that the activity of C5a is 100 %. In an embodimtent, the term within 24 to 72 hours means that as early as 24 hours and as late as 72 hours after administering the C5a binding agent to the subject, an activity of the C5a contained in the blood of the subject is reduced by 50 % or less, or in other words, an activity of C5a contained in the blood of a subject is at - again - least 50 % or more, preferably at least 50 % or more of the activity prior to the administering of the C5a binding agent to the subject.

The problems underyling the present invention are solved in a fifth aspect by a C5a binding agent for use in a method for treating and/or preventing a disease in a subject, wherein the disease is associated with or caused by C5a, wherein the treating and/or preventing comprises administering the C5a binding agent to the subject, wherein the treating and/or preventing of the disease is associated with or goes along with a reduced adverse effect or a reduced risk of an adverse effect, wherein the adverse effect is caused by or arising from the administering of the C5a binding agent, wherein an activity of C5a in the blood of the subject is reduced by 50 % or less within 24 hours to 72 hours after administering the C5a binding agent to the subject. According to this method an activity of C5a is reduced by 50 % or less which preferably means that an activity of C5a is back to at leat 50 % of its original activity in the blood of the subject within 24 to 72 hours. This preferably means that the activity of C5a is reduced by at least 50 % and any percentage lower than 50 % including but not limited to 45 %, 40 %, 35 %, 30 %, 25%, 20 %, 15 %, 10 %, 5 % and 0%. Recuding an activity of C5a by 0 % means that the activity of C5a is 100 %. In an embodimtent, the term within 24 to 72 hours means that as early as 24 hours and as late as 72 hours after administering the C5a binding agent to the subject, an activity of the C5a contained in the blood of the subject is reduced by 50 % or less, or in other words, an activity of C5a contained in the blood of a subject is at - again - least 50 % or more, preferably at least 50 % or more of the activity prior to the administering of the C5a binding agent to the subject.

The problems underyling the present invention are solved in a sixth aspect by a C5a binding agent for use in a method for treating and/or preventing a disease in a subject, wherein the disease is associated with or caused by C5a, wherein the treating and/or preventing comprises administering the C5a binding agent to the subject, wherein the treating and/or preventing of the disease is associated with or goes along with a reduced adverse effect or a reduced risk of an adverse effect, wherein the adverse effect is caused by or arising from the administering of the C5a binding agent, wherein the C5a binding agent has plasma half-life of about 6 hours to about 15 hours within the first 12-48 hours after administering the C5a binding agent to the subject as determined in the blood of the subject.

The problems underyling the present invention are solved in a seventh aspect by a C5a binding agent for use in a method for reducing an adverse effect or for reducing the risk of an adverse effect of an anti-C5a therapy administered to a subject, wherein the method comprises administering to the subject a C5a binding agent, wherein an activity of C5a in the blood of the subject is reduced by 50 % or less within 24 hours to 72 hours after administering the C5a binding agent to the subject. According to this method an activity of C5a is reduced by 50 % or less which preferably means that an activity of C5a is back to at leat 50 % of its original activity in the blood of the subject within 24 to 72 hours. This preferably means that the activity of C5a is reduced by at least 50 % and any percentage lower than 50 % including but not limited to 45 %, 40 %, 35 %, 30 %, 25%, 20 %, 15 %, 10 %, 5 % and 0%. Recuding an activity of C5a by 0 % means that the activity of C5a is 100 %. In an embodimtent, the term within 24 to 72 hours means that as early as 24 hours and as late as 72 hours after administering the C5a binding agent to the subject, an activity of the C5a contained in the blood of the subject is reduced by 50 % or less, or in other words, an activity of C5a contained in the blood of a subject is at - again - least 50 % or more, preferably at least 50 % or more of the activity prior to the administering of the C5a binding agent to the subject.

The problems underyling the present invention are solved in a eighth aspect by a C5a binding agent for use in a method for reducing an adverse effect or for reducing the risk of an adverse effect of an anti-C5a therapy administered to a subject, wherein the method comprises administering to the subject a C5a binding agent, wherein the C5a binding agent has a plasma half-life of about 6 hours to about 15 hours within the first 12-48 hours after administering the C5a binding agent to the subject hours as determined in the blood of the subject.

In an embodiment of any one of the fouth, fifth, sixth, seventh and eighth aspect, including any embodiment thereof, at least a first and a least a second dose of the C5a binding agent are administered to the subject. In such embodiment the at least second dose is the last dose. If subsequent to the second dose subsequent doses are administered to the subject the last of such subsequent doses to be administered to the subject is the last dose administered.

The problems underyling the present invention are solved in a ninth aspect by a C5a binding agent for use in a method of treating and/or preventing a disease in a subject, wherein the disease is which is associated with or caused by C5a, wherein the method comprises administering to the subject

c) a total amount of 4.18 to 222.25 nmol of the C5a binding agent per kg bodyweight of the subject over a period of 8 to 48 hours, or

d) a total amount of 0.055 to 2.9 mg of the C5a binding agent per kg bodyweight of the subject over a period of 8 to 48 hours.

The problems underyling the present invention are solved in a tenth aspect by a C5a binding agent for use in a method of treating and/or preventing a disease in a subject, wherein the disease is which is associated with or caused by C5a, wherein the method comprises administering to the subject 15.19 nmol of AON-D21 per kg body weight of the subject every 11 to 13 hours, preferably every 12 hours.

It will be appreciated by a person skilled in the art that further dosage regimes may be used in the practicing of the present invention as disclosed herein. Such further dosage regimens include but are not limited to those disclosed in

Example 14 which are incorporated into this general part of the description by reference. It will be further appreciated that the dosage regimens disclosed in Example 14 can be applied to any C5a binding agent, preferably a C5a binding agent having binding characteristics similar to compound AON-D21. Preferably, such binding characteristics consisit of binding to both C5a and C5 expressed by an affinity of about 0.1 to 5 nM, preferably 0.1 to 2 nM, more preferably 0.1 to 1.0 nM. for C5a and C5a and of one molecule of the C5a binding agent binding to one molecule of C5a and C5, respectively.

In an embodiment of each and any aspect and as preferably used herein, a trough level or trough concentration (Ctrough) is the concentration or level reached by a drug such as a C5a binding agent, immediately before the next dose is administered. The name comes from the idea that on a graph of concentration versus time, the line forms a U-shaped trough at the lowest region, before a new dose sends it higher again. It will be appreciated by a person skilled in the art that the concept of a trough level is preferably applicable to a dosage regimen where multiple doses are administered to a subject. Upon administration of the first dose of such dosage regimen comprising multiple doses to be administered to a subject, the level of the administered drug will typically be lower than the intended level. After administration of more of the multiple doses a steady state will be achieved. It is preferred that the level achieved after the administration of more of the multiple doses is the trough level which triggers the administration of any subsequent dose to the subject and, respectively, the subsequent administration of a doses to the subject. In an embodiment, the first dose with which the drug is adminsterd to the subject is higher so as to make sure that such steady state of the drug in the subject is established faster.

In an embodiment of each and any aspect of the invention, including any embodiment thereof, and as preferably used herein, administering to a or the subject multiple doses of the C5a binding agent means that at least a first dose of the C5a binding agent and at least a second dose of the C5a binding agent are administered to a or the subject, whereby the at least second dose of the C5a binding agent is administered subsequent to the at least first dose of the C5a binding agent. In this embodiment, the at least second dose of the C5a binding agent may also be referred to as the subsequent or the next dose of the C5a binding agent.

In a preferred embodiment of each and any aspect of the invention, including any embodiment thereof, and as preferably used herein, administering to a or the subject multiple doses of the C5a binding agent means that at least a first dose of the C5a binding agent, at least a second dose of the C5a binding agent and at least one subsequent dose of the C5a binding agent are administered to a or the subject, whereby the at least second dose of the C5a binding agent is administered to a or the subject subsequent to the at least first dose of the C5a binding agent, and the at least one subsequent dose of the C5a binding agent is administered to a or the subject subsequent to the at least second dose of the C5a binding agent. In this embodiment, the at least second dose of the C5a binding agent and the at last one subsequent dose of the C5a binding agent may each also be referred to as next dose of the C5a binding agent.

In a more preferred embodiment of each and any aspect of the invention, including any embodiment thereof, and as preferably used herein, administering to a or the subject multiple doses of the C5a binding agent means that at least a first dose of the C5a binding agent, at least a second dose of the C5a binding agent and more subsequent doses of the C5a binding agent are administered to a or the subject, whereby the at least second dose of the C5a binding agent is administered to a or the subject subsequent to the at least first dose of the C5a binding agent, a first of the more subsequent doses of the C5a binding agent is administered to a or the subject subsequent to the at least second dose of the C5a binding agent and a second of the more subsequent doses of the C5a binding agent is administered to a or the subject subsequent to the first of the more subsequent doses of the C5a binding agent etc. Depending on the number of the more subsequent doses the above outlined sequence of administering the individual doses of the more doses is continued accordingly.

In an alternative embodiment of each and any aspect of the invention and as preferably used herein, administering to the subject multiple doses of the C5a binding agent at a level of the C5a binding agent, such as a trough level, means that one of the multiple doses of the C5a binding agent is administered to the subject at distinct points in time in the method of treating or preventing a disease or a method for reducing an adverse effect or for reducing the risk of an adverse effect of an anti-C5a therapy administered to a subject, whereby such distinct points in time are defined by a level of the C5a binding agent in the blood of the subject, whereby such level is the indicated level, for example 80 to 300 nM. More preferably, at a single point in time only one of the multiple doses is administered to the subject. In an exemplary embodiment where the multiple doses comprises at first dose of the C5a binding agent, a second dose of the C5a binding agent and a third dose of the C5a binding agent, the first dose of the multiple doses of the C5a binding agent is administered to the subject at a first point in time in the method of treating and/or preventing a disease or in the method for reducing an adverse effect or for reducing the risk of an adverse effect of an anti-C5a therapy administered to a subject when the level of the C5a binding agent in the blood of the subject is a level of 80 to 300 nM,

the second dose of the C5a binding agent is administered to the subject at a second point in time in the method of treating and/or preventing a disease or in the method for reducing an adverse effect or for reducing the risk of an adverse effect of an anti-C5a therapy administered to a subject when, after the administration of the first dose of the C5a binding agent, the level of the C5a binding agent in the blood of the subject is - again - a level of 80 to 300 nM, and the third dose of the C5a binding agent is administered to the subject at third point in time in the method of treating and/or preventing a disease or in the method for reducing an adverse effect or for reducing the risk of an adverse effect of an anti-C5a therapy administered

to a subject when, after administration of the second dose of the C5a binding agent, the level of the C5a binding agent in the blood of the subject is - again - a level of 80 to 300 nM

In an embodiment of each and any aspect of the invention, including any embodiment thereof, and as preferably used herein, an activity of C5a is an activity of C5a as determined in a cell-based assay, wherein the cell-based assay makes use of a reporter cell line expressing a C5a receptor, whereby the reporter cell line generates a bioluminescent signal proportional to C5a and the level of C5a, respectively. An example of such a cell-based assay and a protocol for its use are described in Examples 13.1.3 and 8, whereby such cell-based assay and protocol are incorporated into this general part of the description by reference.

In an alternative embodiment of each and any aspect of the invention, including any embodiment thereof, and as preferably used herein, an activity of C5a is selected from group comprising activity as a chemoattractant for inflammatory cells including leukocytes, activity stimulating respiratory burst, a cytokine and chemokine release activity and an activity for increasing vascular permeability. Methods and assays for determining such activity are known to a person skilled in the art.

In an embodiment of each and any aspect of the invention, including any embodiment thereof, activity of C5a is directly linked to the concentration of C5a. This means that in such embodiment, any inhibition of the activity of C5a in a subject or a sample from such subject goes along with or is caused by a reduction in the level of C5a in the subject or in the sample from the subject. More preferably the concentration of C5a is the concentration of active C5a, whereby more preferably the concentration of active C5a is determined by tests and methods known in the art; such tests and method known in the art include the cell-based assay using a reporter cell line expressing C5a receptor is disclosed herein.

In an embodiment of each and any aspect of the invention, including any embodiment thereof, an activity of the C5a is inhibited. The degree of such inhibition may range from 100 % to 0 %. Means and methods for determining such inhibition are known in the art and include, but are not limited to, the assay and test disclosed in Examples 8 and 13.1.3 of the instant application. In a more preferred embodiment, the activity of C5a is completely inhibited, i.e. 100 % of the activity of the C5a in blood of a subject is inhibited if the level of the C5a binding agent in the blood of the subject is 100 nM or higher. This applies in particular if the C5a binding agent is compound AON-D21. A person skilled in the art will acknowledge that the activity of the C5a in blood of a subject will also be completely inhibited, i.e. 100 % of the activity of the C5a in blood of a subject is inhibited if the level of the C5a binding agent in the blood of the subject is higher than 100 nM, for example is 150 nM, 200 nM, 250 nM or 300 nM.

It has been found that if, among others, the C5a antagonist is compound AON-D21, 80 % of the activity of C5a in blood of a subject is inhibited if the concentration of said C5a antagonist in the blood of the subject is about 80 nM. Similarly, it has been found that if, among others, the C5a antagonist is compound AON-D21, 90 % of the activity of C5a in blood of a subject is inhibited if the concentration of said C5a antagonist in the blood of the subject is about 90 nM.

In an embodiment of each and any aspect of the invention, including any embodiment thereof, an adverse effect is reduced if the symptoms of or associated with such adverse effect are reduced. In an embodiment of each and any aspect of the invention, including any embodiment thereof, an adverse effect is avoided if the symptoms of or associated with such adverse effect are avoided.

In an embodiment of each and any aspect of the invention, including any embodiment thereof, the risk for an adverse effect is reduced if the risks for symptoms or the risk of the subject to develop or show such symptoms of or associated with such adverse effect is reduced. In an embodiment of each and any aspect of the invention, including any embodiment thereof, the risk for an adverse effect is avoided if the risk for symptoms or the risk of the subject to develop or show such symptoms of or associated with such adverse effect are avoided.

In an embodiment of each and any aspect of the invention, including any embodiment thereof, the half-life of the C5a binding agent is determined. Such determining and methods for determining the half-life of a compound including of a C5a binding agent are known to a person skilled in the art In an embodiment thereof, the half-life is determined in the blood of the subject. In a preferred embodiment, the half-life of the C5a binding agent is about 15 hours or less, preferably about 6 hours to about 15 hours within the first 12 to 48 hours in the blood of the subject, more preferably the half-life of the C5a binding agent is about 15 hours or less within the first 12 to 48 hours in the blood of the subject after administering the C5a binding agent to the subject.

In an embodiment of each and any aspect of the invention, including any embodiment thereof, any reference to a level of a C5a binding agent in the blood of a subject refers to a level of a C5a binding agent in a blood sample of the subject.

In an embodiment of each and any aspect of the invention, including any embodiment thereof, any reference to an activity of C5a in the blood of a subject refers to an activity of C5a in a blood sample of the subject.

In an embodiment of each and any aspect of the invention, including any embodiment thereof, any reference to a level of C5a in the blood of a subject refers to a level of C5a in a blood sample of the subject.

In an embodiment of each and any aspect of the invention, including any embodiment thereof, an adverse effect of an anti-C5a therapy is an adverse effect caused by or associated with a C5a therapy, preferably the C5a therapy is administered to a subject which shows or is at risk of showing such adverse effect. More preferably such adverse effect is selected from the group consisting of shock, lung edema, and postoperative kidney damage. In a further embodiment the

C5a therapy is a therapy comprising administering to a subject a C5a binding agent, including a C5a binding agent as disclosed herein.

In an embodiment of each and any aspect of the invention, including any embodiment thereof, the term every two days means every 44 to 52, wherein more preferably every two days means every 46 to 50 hours, wherein most preferably every two days means every 48 hours.

In an embodiment of each and any aspect of the invention, including any embodiment thereof, the term every day means every 20 to 28 hours, wherein preferably every day means every 22 to 26 hours, wherein more preferably every day means every 23 to 25 hours, wherein most preferably every day means every 24 hours.

In an embodiment of each and any aspect of the invention, including any embodiment thereof, the term twice day means every 10 to 14 hours, wherein preferably twice day means every 11 to 13 hours, wherein more preferably twice day means every 12 hours.

In an embodiment of each and any aspect of the invention, including any embodiment thereof, the term three times a day means every 6 to 10 hours, wherein preferably three times day means every 8 hours.

In an embodiment of each and any aspect of the present invention, including any embodiment thereof, the C5a binding agent is compound AON-D21 as defined herein.

In an embodiment of the present invention, including any embodiment thereof, the subject is a mammal, preferably a human being. It is, however, also within the present invention that such mammal is a mammal selected from the group comprising a monkey, an ape, a mouse, a rat, a dog, a cat, a horse, a cattle, a sheep, a goat and a pig.

It will be acknowledged that any embodiment disclosed herein is an embodiment of each and any aspect of the present invention. Furthermore, any embodiment of a particular aspect of the present invention is also an embodiment of each and any other aspects of the present invention, including any embodiment thereof. The terms "of the present invention", "of the invention", "according to the present invention" and "according to the invention" are used interchangeably herein and refer to each and any aspect of such invention disclosed herein.

The terms "level", "concentration" and "titer" are used synonymously herein.

The terms "subject" and "individual" are used synonymously herein.

The terms "subject", "individual" and "patient" are used synonymously herein, unless indicated otherwise or differently.

According to the present invention the C5a binding agent is capable of binding to C5a and/or C5, preferably capable of binding to C5a and C5.

In an embodiment of invention the C5a binding agent is an agent having a binding affinity to C5a with a $K_D$ of about 0.1 to 5 nM, preferably 0.1 to 2 nM, more preferably 0.1 to 1.0 nM.

In an embodiment the C5a binding agent is an inhibitor of an activity mediated by human C5a, wherein preferably the inhibitor is an antagonist. In an preferred embodiment is the C5a binding agent an inhibitor of an activity mediated by human C5 having an inhibitory constant IC50 of about 0.1 to 5 nM, preferably 0.1 to 2 nM, more preferably 0.1 to 0.5 nM. In another preferred embodiment is the C5a binding agent capable of inhibiting C5a activity in the presence of C5. In a preferred embodiment the binding affinity of the C5a binding agent to C5 and C5a is below 10nM, more preferably below 10 nM and most preferably below 1 nM.

In another preferred embodiment the dosing regime leads to plasma concentrations of the C5a binding agent of more than 100 nM.

In a preferred embodiment the C5a binding agent is an antibody or antigen-binding fragment thereof, a peptide, an anticalin protein or a nucleic acid molecule, wherein the nucleic acid molecule is preferably an L-aptamer or a spiegelmer.

The term "antibody" typically refers to a glycoprotein comprising at least two heavy (H) chains and two light (L) chains inter-connected by disulfide bonds, or an antigen-binding portion thereof. The term "antibody" also includes all recombinant forms of antibodies, e.g. antibodies expressed in prokaryotes, unglycosylated antibodies, and any antigen-binding antibody fragments and derivatives as described below. Each heavy chain is comprised of a heavy chain variable region (abbreviated herein as VH or VH) and a heavy chain constant region. Each light chain is comprised of a light chain variable region (abbreviated herein as VL or VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding fragment" of an antibody (or simply "binding portion"), as used herein, refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) Fab fragments, monovalent fragments

consisting of the VL, VH, CL and CH domains; (ii) F(ab')2 fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments, which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv);). Such single chain antibodies are also intended to be encompassed within the term "antigen-binding fragment" of an antibody. A further example is a binding-domain immunoglobulin fusion protein comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies. Further examples of "antigen- binding fragments" are so-called microantibodies, which are derived from single CDRs.

Thus, the term "antibody or antigen-binding fragment thereof," as used herein, refers to immunoglobulin molecules and immunologically active portions of immunoglobulin molecules, i.e. molecules that contain an antigen-binding site that immunospecifically binds an antigen.

The term "peptide" typically refers to any polymer of (same or different) amino acids joined via peptide bonds.

The term "anticalin proteins" typically refers to artificial proteins that are able to bind to antigens, either to proteins or to small molecules. They are not structurally related to antibodies, which makes them a type of antibody mimetic. Instead, they are derived from human lipocalins which are a family of naturally binding proteins. Anticalin proteins are being used in lieu of monoclonal antibodies, but are about eight times smaller with a size of about 180 amino acids and a mass of about 20 kDa.

The term "dosage regimen" (also referred to as "dosage regime", "dosing regime", "dosing regimen", "dose regimen", "dose regime") ntypically refers to a schedule of doses of a medicine, including the time between doses, the duration of treatment and the amount to be taken each time. Dosage regimens also include how a medicine is to be taken, and in what formulation (dosage form).

The nucleic acid molecule according to the present invention binds specifically and with high affinity to both mouse C5a and human C5a, thereby inhibiting the binding of C5a to its C5a receptor, although the sequence homology of mouse C5a and human C5a is only 64 % in the homologous region and mouse C5a having 3 additional N-terminal amino acids. Moreover, in contrast to human C5a, mouse C5a is not glycosylated. Asparagine$^{64}$, the glycosylation site in human C5a is mutated to glutamate in mouse.

It is within the present invention that the nucleic acid according to the present invention is a nucleic acid molecule. Insofar the terms nucleic acid and nucleic acid molecule are used herein in a synonymous manner if not indicated to the contrary. Moreover, such nucleic acid(s) is/are preferably also referred to herein as the nucleic acid molecule(s) according to the (present) invention, the nucleic acid(s) according to the present invention, the inventive nucleic acid(s) or the inventive nucleic acid molecule(s).

The features of the nucleic acids according to the present invention as described herein can be realised in any aspect of the present invention where the nucleic acid is used, either alone or in any combination.

As to the various diseases, conditions and disorders which may be treated or prevented by using the C5a binding agent in a dosage regimen according to the present invention and compositions, preferably pharmaceutical compositions comprising the same, it has to be acknowledged that such diseases, conditions and disorders are those which are described herein, including and in particular those described and set forth in the introductory part of the instant application. Insofar, the respective passages of the specification and the introductory part of the specification form an integral part of the present disclosure teaching the suitability of the dosage regimen using a C5a binding agent of the present invention for the prevention and treatment, respectively, for said diseases, conditions, and disorders. Additionally, the dosage regimen using a C5a binding agent according to the present invention is preferred if the physiological effect of the C5a - C5a receptor axis is related to higher plasma levels of C5a.

As used herein the term C5a refers to any C5a including, but not limited to, mammalian C5a. Preferably, the mammalian C5a is selected from the group comprising human, rat, mouse, monkey C5a (see C5a species alignment in Fig. 11). More preferably the C5a is human C5a. Human C5a is a basic protein having the amino acid sequence according to SEQ. ID. No. 50. Mouse C5a is a basic protein having the amino acid sequence according to SEQ. ID. No. 52.

As outlined in more detail in the claims and example 1, the present inventors could more surprisingly identify a number of different binding nucleic acid molecules capable of binding both human and mouse C5a.

As outlined in more detail herein, the present inventors have identified a number of different C5a binding nucleic acid molecules capable of binding both, human and mouse C5a, whereby the nucleic acid molecules can be characterised

in terms of stretches of nucleotides which are also referred to herein as disclosed (see Example 1). As experimentally shown in examples the inventors could surprisingly demonstrate in several systems that the nucleic acid molecule according to the present invention issuitbale for the treatment of a disease.

Each of the different types of C5a binding nucleic acid molecules of the invention that bind to C5a comprises three different stretches of nucleotides: a first terminal stretch of nucleotides, a central stretch of nucleotides and a second terminal stretch of nucleotides. In general, C5a binding nucleic acid molecules of the present invention comprise at their 5'-end and the 3'-end each one of the terminal stretches of nucleotides, i.e. the first terminal stretch of nucleotides or the second terminal stretch of nucleotides (also referred to as 5'-terminal stretch of nucleotides and 3'-terminal stretch of nucleotides). The first terminal stretch of nucleotides and the second terminal stretch of nucleotides can, in principle due to their base complementarity, hybridize to each other, whereby upon hybridization a double-stranded structure is formed. However, such hybridization is not necessarily realized in the molecule under physiological and/or non-physiological conditions. The three stretches of nucleotides of C5a binding nucleic acid molecules - the first terminal stretch of nucleotides, the central stretch of nucleotides and second terminal stretch of nucleotides - are arranged to each other in 5' $\rightarrow$ 3'-direction: the first terminal stretch of nucleotides - the central stretch of nucleotides - the second terminal stretch of nucleotides. Alternatively, the second terminal stretch of nucleotides, the central stretch of nucleotides and the terminal first stretch of nucleotides are arranged to each other in 5' $\rightarrow$ 3'-direction.

The length of the central stretch of nucleotides of the nucleic acids according to the present invention is preferably 34.

The length of the first terminal stretch of nucleotides of the nucleic acids according to the present invention is between one and five nucleotides, preferably between three and five nucleotides, more preferably three nucleotides.

The length of the second terminal stretch of nucleotides of the nucleic according to the present invention is between one and five nucleotides, preferably between three and five nucleotides, more preferably three nucleotides.

The terms 'stretch' and 'stretch of nucleotides' are used herein in a synonymous manner if not indicated to the contrary.

The differences in the sequences of the defined stretches between the different C5a binding nucleic acid molecules may influence the binding affinity to C5a. Based on binding analysis of the different C5a binding nucleic acid molecules of the present invention the central stretch and the nucleotides forming the same are individually and more preferably in their entirety essential for binding of the C5a binding nucleic acid molecule to C5a.

In a preferred embodiment the nucleic acid molecule according to the present invention is a single nucleic acid molecule. In a further embodiment, the single nucleic acid molecule is present as a multitude of the single nucleic acid molecule or as a multitude of the single nucleic acid molecule species.

It will be acknowledged by the ones skilled in the art that the nucleic acid molecule in accordance with the invention preferably consists of nucleotides which are covalently linked to each other, preferably through phosphodiester links or linkages.

It is within the present invention that the nucleic acid molecule according to the present invention comprises two or more stretches or part(s) thereof that can, in principle, hybridise with each other. Upon such hybridisation a double-stranded structure is formed. It will be acknowledged by the ones skilled in the art that such hybridisation may or may not occur, particularly under *in vitro* and/or *in vivo* conditions. Also, in case of hybridisation, such hybridisation does not necessarily occur over the entire length of the two stretches where, at least based on the rules for base pairing, such hybridisation and thus formation of a double-stranded structure may, in principle, occur. As preferably used herein, a double-stranded structure is a part of a nucleic acid molecule or a structure formed by two or more separate strands or two spatially separated stretches of a single strand of a nucleic acid molecule, whereby at least one, preferably two or more base pairs exist which are base pairing preferably in accordance with the Watson-Crick base pairing rules. It will also be acknowledged by the one skilled in the art that other base pairing such as Hoogsten base pairing may exist in or may form such double-stranded structure. It is also to be acknowledged that the feature that two stretches hybridize preferably indicates that such hybridization is assumed to happen due to base complementarity of the two stretches regardless of whether such hybridization actually occurs *in vivo* and/or *in vitro.* In connection with the present invention such stretches are the first terminal stretch of nucleotides and the second stretch of nucleotides which, in an embodiment, may hybridize as defined above.

In a preferred embodiment the term arrangement as used herein, means the order or sequence of structural or functional features or elements described herein in connection with the nucleic acids molecule(s) disclosed herein.

It will be acknowledged by the person skilled in the art that the C5a binding agent is capable of binding to C5a or to both C5a and C5. It will be acknowledged by the person skilled in the art that the a nucleic acid according to the present invention, is capable of binding to both C5a and C5. This binding characteristic arises from the fact that for the identification of the nucleic acids a moiety of C5a was used which is present in both C5a and C5. Also, it will be acknowledged by the person skilled in the art that the nucleic acid molecule according to the present invention is an antagonist to both C5 and C5a. Because of this the nucleic acids according to the present invention are suitable for the treatment and prevention, respecticely, of any disease which is associated with or caused by either C5a or C5 or both. The scientific rational may be

taken from the prior art which establishes that C5a and C5, respectively, are involved or associated with a variety of diseases and conditions, respectively, and which is incoroporated herein by reference.

The C5a binding nucleic acid molecule of present invention disclosed herein have been shown to recognize C5a in the context of C5 (see Example 1). Therefore, it was investigated whether C5 cleavage to the anaphylatoxin C5a and C5b, which is part of the membrane attack complex (MAC) is inhibited by C5a binding nucleic acids. The MAC is the ultimate product of the complement cascade: a pore consisting of C5b-9. MAC is believed to insert into the cytoplasmic membranes of pathogens and kill them by induction of cytoplasmic leakage. The assay for C5 cleavage was achieved by using a complement-dependent sheep erythrocyte hemolysis test. The C5a binding molecule of the invention did not inhibit hemolysis (see Example 6). The C5a binding nucleic acid molecule of the invention does not interfere with C5 cleavage and MAC formation and are therefore selective antagonists of C5a only. If used as a medicament, this may be advantageous in many diseases, since the formation of the MAC that is beneficial in pathogen defense is not compromised in their presence.

The nucleic acid molecule according to the present invention shall also comprise nucleic acids which are essentially homologous to the particular sequences disclosed herein. The term substantially homologous shall be understood such as the homology is at least 75%, preferably 85%, more preferably 90% and most preferably more that 95 %, 96 %, 97 %, 98 % or 99%.

The actual percentage of homologous nucleotides present in the nucleic acid according to the present invention will depend on the total number of nucleotides present in the nucleic acid. The percent modification can be based upon the total number of nucleotides present in the nucleic acid.

The homology between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or nucleic acid molecule which is said to be homologous or to be tested whether it is homologous, and if so, to what extent, to a different nucleic acid molecule, whereby such different nucleic acid molecule is also referred to as the reference sequence. In an embodiment, the reference sequence is a nucleic acid molecule as described herein, preferably a nucleic acid molecule having a sequence according to any one of SEQ ID NO: 3, SEQ ID NO: 4, SEQ ID NO: 5, SEQ ID NO: 6, SEQ ID NO: 90, SEQ ID NO: 14, SEQ ID NO: 21, SEQ ID NO: 24, SEQ ID NO: 25, SEQ ID NO: 26, SEQ ID NO: 27, SEQ ID NO: 37, SEQ ID NO: 57, SEQ ID NO: 59, SEQ ID NO: 60, SEQ ID NO: 91 and SEQ ID NO: 92. Optimal alignment of sequences for comparison can be conducted, e.g., by the local homology algorithm of Smith & Waterman (Smith & Waterman, 1981) by the homology alignment algorithm of Needleman & Wunsch (Needleman & Wunsch, 1970) by the search for similarity method of Pearson & Lipman (Pearson & Lipman, 1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al (Altschul et al. 1990 and Altschul et al, 1997). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al (McGinnis et al, 2004).

The nucleic acids according to the present invention shall also comprise nucleic acids which have a certain degree of identity relative to the nucleic acids disclosed herein and defined by their nucleotide sequence. More preferably, the instant invention also comprises those nucleic acid molecules which have an identity of at least 75%, preferably 85%, more preferably 90% and most preferably more than 95 %, 96 %, 97 %, 98 % or 99% relative to the nucleic acids disclosed herein and defined by their nucleotide sequence or a part thereof.

The term inventive nucleic acid or nucleic acid according to the present invention shall also comprise those nucleic acids comprising the nucleic acids sequences disclosed herein or part thereof, preferably to the extent that the nucleic acids or said parts are involved in the binding to human C5a. Such nucleic acid is, in an embodiment, one of the nucleic acid molecules described herein, or a derivative and/ or a metabolite thereof, whereby such derivative and/ or metabolite are preferably a truncated nucleic acid compared to the nucleic acid molecules described herein. Truncation may be related to either or both of the ends of the nucleic acids as disclosed herein. Also, truncation may be related to the inner sequence of nucleotides of the nucleic acid, i.e. it may be related to the nucleotide(s) between the 5' and the 3' terminal nucleotide, respectively. Moreover, truncation shall comprise the deletion of as little as a single nucleotide from the sequence of the nucleic acids disclosed herein. Truncation may also be related to more than one stretch of the inventive nucleic acid(s), whereby the stretch can be as little as one nucleotide long. The binding of a nucleic acid according to the present invention can be determined by the ones skilled in the art using routine experiments or by using or adopting a method as described herein, preferably as described herein in the example part.

The nucleic acid molecule according to the present invention may be either a D-nucleic acid molecule or an L-nucleic acid molecule. Preferably, the nucleic acid molecule according to the present invention is an L-nucleic acid

molecule. More preferably, the nucleic acid molecule of the present invention is a Spiegelmer or L-aptamer.

It is also within the present invention that, in an embodiment, each and any of the nucleic acid molecules described herein in their entirety in terms of their nucleic acid sequence(s) are limited to the particular indicated nucleotide sequence(s). In other words, the terms "comprising" or "comprise(s)" shall be interpreted in such embodiment in the meaning of containing or consisting of.

An L-nucleic acid as used herein is a nucleic acid or nucleic acid molecule consisting of L-nucleotides.

A D-nucleic acid as used herein is nucleic acid or nucleic aicd molecule consisting of D-nucleotides.

The terms nucleic acid and nucleic acid molecule are used herein in an interchangeable manner if not explicitly indicated to the contrary.

Also, if not indicated to the contrary, any nucleotide sequence is set forth herein in 5' $\rightarrow$ 3' direction.

As preferably used herein any position of a nucleotide is determined or referred to relative to the 5' end of a sequence, a stretch or a sub stretch containing such nucleotide. Accordingly, a second nucleotide is the second nucleotide counted from the 5' end of the sequence, stretch and substretch, respectively. Also, in accordance therewith, a penultimate nucleotide is the seond nucleotide counted from the 3' end of a sequence, stretch and substretch, respectively.

Irrespective of whether the nucleic acid molecule of the invention consists of D-nucleotides, L-nucleotides or a combination of both with the combination being e.g. a random combination or a defined sequence of stretches consisting of at least one L-nucleotide and at least one D-nucleic acid, the nucleic acid may consist of desoxyribonucleotide(s), ribonucleotide(s) or combinations thereof.

It is also within the present invention that the nucleic acid molecule consists of both ribonucleotides and 2'deoxyribonucleotides. The 2'deoxyribonucleotides and ribonucleotides are shown in Fig. 20 and 21. In order to distinguish between ribonucleotides and 2'deoxyribonucleotides in the sequences of the nucleic acid molecules according to the present invention the following reference code is used herein.

The nucleic acid molecule according to the present invention consists of 2'deoxyribonucleotides, wherein

dG is 2'deoxy-guanosine-5'-monophosphate,
dC is 2'deoxy-cytidine-5'-monophosphate,
dA is 2'deoxy-adenosine-5'-monophosphate,
dU is 2'deoxy-uridine 5'monophosphate

The nucleic acid molecule according to the present invention consists of ribonucleotides, wherein

G is guanosine-5'-monophosphate,
C is cytidine 5'-monophosphate,
A is adenosine-5'-monophosphate,
U is uridine-5'monophosphate.

For definition of ribonucleotide sequence motifs, the IUPAC abbreviations for ambiguous nucleotides are used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

Designing the nucleic acid molecule of the invention as an L-nucleic acid molecule is advantageous for several reasons. L-nucleic acid molecules are enantiomers of naturally occurring nucleic acids. D-nucleic acid molecules, however, are not very stable in aqueous solutions and particularly in biological systems or biological samples due to the widespread presence of nucleases. Naturally occurring nucleases, particularly nucleases from animal cells are not capable of degrading L-nucleic acids. Because of this, the biological half-life of an L-nucleic acid molecule is significantly increased in such a system, including the animal and human body. Due to the lacking degradability of L-nucleic acid

molecules no nuclease degradation products are generated and thus no side effects arising therefrom observed in such a system including the animal and human body. This aspect distinguishes L-nucleic acid moelcules from factually all other compounds which are used in the therapy of diseases and/or disorders involving the presence of C5. An L-nucleic acid molecule which specifically binds to a target molecule through a mechanism different from Watson Crick base pairing, or an aptamer which consists partially or completely of L-nucleotides, particularly with those parts of the aptamer being involved in the binding of the aptamer to the target molecule, is also called a Spiegelmer or L-aptamer. Aptamers and Spiegelmers or L-aptamers as such are known to a person skilled in the art and are, among others, described in 'The Aptamer Handbook' (eds. Klussmann, 2006).

It is also within the present invention that the nucleic acid molecule of the invention, regardless whether it is are present as a D-nucleic acid, L-nucleic acid or D,L-nucleic acid or whether it is DNA or RNA, may be present as single stranded or double stranded nucleic acid molecule. Typically, the nucleic acid molecule is a single stranded nucleic acid molecule which exhibits a defined secondary structure due to its primary sequence and may thus also form a tertiary structure. The nucleic acid molecule, however, may also be double stranded in the meaning that two strands which are complementary or partially complementary to each other are hybridised to each other.

A possibility to determine the binding constants of the C5a binding agent, preferably the nucleic acid molecules according to the present invention, is the use of the plasmon resonance plasmon resonance spectroscopy as described in example 4 which confirms the above finding that the nucleic acids according to the present invention exhibit a favourable $K_D$ value range. An appropriate measure in order to express the intensity of the binding between the individual nucleic acid molecule and the target which is in the present case C5a is the so-called $K_D$ value which as such as well the method for its determination are known to the one skilled in the art.

Preferably, the $K_D$ value of the C5a binding agent, preferably of the nucleic acids according to the present invention, is below 10 nM. The above-mentioned $K_D$ of about 10 nM is a preferred upper limit for the $K_D$ value for a C5a binding agent in dosage regimen for the treatment of a disease. The lower limit for the $K_D$ of a C5a binding agent, preferably a target binding nucleic acid, can be as little as about 10 picomolar or can be higher. It is within the present invention that the $K_D$ values of C5a binding agent, preferably an individual nucleic acid of the nucleic acids of the present invention, binding to C5a is preferably within this range. Preferred ranges can be defined by choosing any first number within this range and any second number within this range. Preferred upper $K_D$ values are 10 nM and 1 nM, preferred lower $K_D$ values are 100 pM and 10 pM. The more preferred upper $K_D$ value is 1 nM, the more preferred lower $K_D$ value is 100 pM.

In addition to the binding properties of the C5a binding agent; preferably a nucleic acid molecule according to the present invention, the C5a binding agent, preferably a nucleic acid molecule according to the present inventions inhibits the function of the respective target molecule which is in the present case C5a. The inhibition of the function of C5a - for instance the stimulation of the respective receptors as described previously - is achieved by binding of the C5a binding agent, preferably a nucleic acid molecule according to the present invention, to C5a and forming a complex of a C5a binding agent, preferably a nucleic acid molecule according to the present invention, and C5a. Such complex of a C5a binding agent, preferably a nucleic acid molecule, and C5a cannot stimulate the receptors that normally are stimulated by C5a, i.e. C5a which is not present in a complex with a C5a binding agent, preferably a nucleic acid molecule of the invention. Accordingly, the inhibition of receptor function by a C5a binding agent, preferably a nucleic acid molecule according to the present invention, is independent from the respective receptor that can be stimulated by C5a but results from preventing the stimulation of the receptor by C5a by the C5a binding agent, preferably the nucleic acid molecule according to the present invention.

A possibility to determine the inhibitory constant of a C5a binding agent, preferably a nucleic acid molecule according to the present invention, is the use of the methods as described in example 5 which confirms the above finding that the C5a binding agent, preferably the nucleic acids according to the present invention, exhibits a favourable inhibitory constant which allows the use of said C5a binding agent, preferably the nucleic acid, in a therapeutic treatment scheme, preferably the dosage regimen according to the present invention. An appropriate measure in order to express the intensity of the inhibitory effect of the individual C5a binding agent, preferably a nucleic acid molecule, on interaction of the target which is in the present case C5a and the respective receptor, is the so-called half maximal inhibitory concentration (abbr. $IC_{50}$) which as such as well the method for its determination are known to the one skilled in the art.

Preferably, the $IC_{50}$ value shown by a C5a binding agent, preferably a nucleic acid molecule according to the present invention, is below 1 $\mu$M. An $IC_{50}$ value of about 1 $\mu$M is said to be characteristic for a non-specific inhibition of target functions by a C5a binding agent, preferably a nucleic acid molecule. As will be acknowledged by the ones skilled in the art, the $IC_{50}$ value of a group of C5a binding agents such as the nucleic acid molecules according to the present invention is within a certain range. The above-mentioned $IC_{50}$ of about 1 $\mu$M is a preferred upper limit for the $IC_{50}$ value. The lower limit for the $IC_{50}$ of a C5a binding agent, preferably a nucleic acid molecule, can be as little as about 10 picomolar or can be higher. It is within the present invention that the $IC_{50}$ values of individual C5a binding agent, preferably a nucleic acid, binding to C5a is preferably within this range. Preferred ranges can be defined by choosing any first number within this range and any second number within this range. Preferred upper $IC_{50}$ values are 10 nM and 1 nM, preferred lower $IC_{50}$ values are 100 pM and 10 pM. The more preferred upper $IC_{50}$ value is 1 nM, the more preferred lower $IC_{50}$ value is 100 pM.

The nucleic acid molecules according to the present invention may have any length provided that they are still able to bind to the target molecule. It will be acknowledged in the art that there are preferred lengths of the nucleic acids according to the present inventions. Typically, the length is between 15 and 120 nucleotides. It will be acknowledged by the ones skilled in the art that any integer between 15 and 120 is a possible length for the nucleic acids according to the present invention. More preferred ranges for the length of the nucleic acids according to the present invention are lengths of about 20 to 100 nucleotides, about 20 to 80 nucleotides, about 20 to 60 nucleotides, about 38 to 44 nucleotides and about 40 nucleotides.

It is within the present invention that the C5a binding agent, preferably the nucleic acid molecule of the present invention, comprises a moiety which preferably is a high molecular weight moiety and/or which preferably allows to modify the characteristics of the C5a binding agent, preferably the nucleic acid molecule in terms of, among others, residence time in the animal body, preferably the human body. A particularly preferred embodiment of such modification is PEGylation and HESylation of the C5a binding agent, preferably the nucleic acids according to the present invention. As used herein PEG stands for poly(ethylene glycole) and HES for hydroxyethly starch. PEGylation as preferably used herein is the modification of a C5a binding agent, preferably an nucleic acid molecule according to the present invention, whereby such modification consists of a PEG moiety which is attached to an C5a binding agent, preferably a nucleic acid molecule according to the present invention. HESylation as preferably used herein is the modification of a C5a binding agent, preferably a nucleic acid molecule according to the present invention, whereby such modification consists of a HES moiety which is attached to a C5a binding agent, preferably a nucleic acid molecule according to the present invention. These modifications as well as the process of modifying a C5a binding agent, preferably a nucleic acid molecule using such modifications, is described in European patent application EP 1 306 382, the disclosure of which is herewith incorporated in its entirety by reference.

In the case of PEG being such high molecular weight moiety the molecular weight is preferably about 20,000 to about 120,000 Da, more preferably from about 30,000 to about 80,000 Da and most preferably about 40,000 Da. In the case of HES being such high molecular weight moiety the molecular weight is preferably from about 50 kDa to about 1000 kDa, more preferably from about 100 kDa to about 700 kDa and most preferably from 200 kDa to 500 kDa. HES exhibits a molar substitution of 0.1 to 1.5, more preferably of 1 to 1.5 and exhibits a substitution grade expressed as the C2/C6 ratio of approximately 0.1 to 15, preferably of approximately 3 to 10.

The process of HES modification is, e.g., described in German patent application DE 1 2004 006 249.8 the disclosure of which is herewith incorporated in its entirety by reference.

The modification can, in principle, be made to an C5a binding agent at any position thereof. In case of a nucleic acid molecule of the present invention preferably such modification is made either to the 5' -terminal nucleotide, the 3'-terminal nucleotide and/or any nucleotide between the 5' nucleotide and the 3' nucleotide of the nucleic acid molecule.

The modification and preferably the PEG and/or HES moiety can be attached to the C5a binding agent, preferably the nucleic acid molecule of the present invention, either directly or indirectly, preferably indirectly through a linker. It is also within the present invention that the a C5a binding agent, preferably the nucleic acid molecule according to the present invention, comprises one or more modifications, preferably one or more PEG and/or HES moiety. In an embodiment the individual linker molecule attaches more than one PEG moiety or HES moiety to a C5a binding agent, preferably a nucleic acid molecule according to the present invention. The linker used in connection with the present invention can itself be either linear or branched. This kind of linkers are known to the ones skilled in the art and are further described in international patent applications WO2005/074993 and WO2003/035665.

In a preferred embodiment the linker is a biodegradable linker. The biodegradable linker allows to modify the characteristics of the C5a binding agent, preferably the nucleic acid molecule according to the present invention, in terms of, among other, residence time in an animal body, preferably in a human body, due to release of the modification from a C5a binding agent, preferably a nucleic acid molecule according to the present invention. Usage of a biodegradable linker may allow a better control of the residence time of a C5a binding agent, preferably a nucleic acid molecule according to the present invention. A preferred embodiment of such biodegradable linker is a biodegradable linker as described in, but not limited to, international patent applications WO2006/052790, WO2008/034122, WO2004/092191 and WO2005/099768.

It is within the present invention that the modification or modification group is a biodegradable modification, whereby the biodegradable modification can be attached to the C5a binding agent, preferably the nucleic acid molecule of the present invention, either directly or indirectly, preferably through a linker. The biodegradable modification allows modifying the characteristics of the C5a binding agent, preferably the nucleic acid molecule according to the present invention, in terms of, among other, residence time in an animal body, preferably in a human body, due to release or degradation of the modification from the C5a binding agent, preferably the nucleic acid molecule according to the present invention. Usage of a biodegradable modification may allow a better control of the residence time of the C5a binding agent, preferably the nucleic acid molecule according to the present invention. A preferred embodiment of such biodegradable modification is biodegradable as described in, but not restricted to, international patent applications WO2002/065963, WO2003/070823, WO2004/113394 and WO2000/41647, preferably in WO2000/41647, page 18, line 4 to 24.

Beside the modifications as described above, other modifications can be used to modify the characteristics of the

C5a binding agent, preferably the nucleic acid molecule according to the present invention, whereby such other modifications may be selected from the group of proteins, lipids such as cholesterol and sugar chains such as amylase, dextran etc..

Without wishing to be bound by any theory, by modifying a C5a binding agent, preferably the nucleic acid molecule according to the present invention, with a high molecular weight moiety such as a polymer and more particularly one or several of the polymers disclosed herein, which are preferably physiologically acceptable, the excretion kinetic of the thus a C5a binding agent, preferably the modified nucleic acid molecule of the invention from an animal or human body to which the modified a C5a binding agent, preferably the nucleic acid molecule of the invention, is administered is changed is changed. More particularly, due to the increased molecular weight of the thus modified a C5a binding agent, preferably the nucleic acid molecule of the invention, excretion from an animal body, preferably from a mammalian body and more preferably from a human body is decreased. As excretion typically occurs via the kidneys, the present inventors assume that the glomerular filtration rate of the thus modified the C5a binding agent, preferably the nucleic acid molecule, is significantly reduced compared to a C5a binding agent, preferably a nucleic acid molecule, not having this kind of high molecular weight modification which results in an increase in the residence time of the C5a binding agent, preferably the modified nucleic acid molecule, in the animal body. In connection therewith it is particularly noteworthy that, despite such high molecular weight modification the specificity of the C5a binding agent, preferably the nucleic acid molecule according to the present invention is not affected in a detrimental manner.

However, it is also within the present invention that the C5a binding agent, preferably the nucleic acid molecule according to the present invention, does not comprise any modification and particularly no high molecular weight modification such as PEG or HES.

The inventive C5a binding agent, preferably the nucleic acids according to the present invention, and/or the antagonists according to the present invention may be used for the generation or manufacture of a medicament. Such medicament or a pharmaceutical composition according to the present invention contains at least one of the inventive C5a binding agent, preferably the nucleic acids, optionally together with further pharmaceutically active compounds, whereby the inventive C5a binding agent, preferably the nucleic acid, preferably acts as pharmaceutically active compound itself. Such medicaments comprise in preferred embodiments at least a pharmaceutically acceptable carrier. Such carrier may be, e.g., water, buffer, PBS, glucose solution, preferably a 5% glucose salt balanced solution, starch, sugar, gelatine or any other acceptable carrier substance. Such carriers are generally known to the one skilled in the art. It will be acknowledged by the person skilled in the art that any embodiments, use and aspects of or related to the medicament of the present invention is also applicable to the pharmaceutical composition of the present invention and vice versa.

The indication, diseases and disorders for the treatment and/or prevention of which the C5a binding agent, preferably the nucleic acid, the pharmaceutical compositions and medicaments in accordance with or prepared in accordance with the present invention result from the involvement, either direct or indirect, of C5a in the respective pathogenetic mechanism.

The local release of C5a at sites of inflammation results in powerful pro-inflammatory stimuli. Thus, neutralization of C5a might be beneficial in many acute or chronic conditions, such as immune complex associated diseases in general (Heller et al., 1999); neurodegeneration and inflammation, e.g. in Alzheimer's disease (Bonifati & Kishore, 2007), where the complement C5a receptor antagonist PMX205 improved behavioral parameters and a reduction of pathological markers such as fibrillar deposits and activated glia (Fonseca *et al.* 2009). Other inflammatory diseases with C5a involvement are systemic lupus erythematosus (Jacob *et al.* 2010a; Jacob *et al.* 2010b), asthma (Kohl, 2001); secondary damages of trauma (Yao et al. 1998); septic shock (Huber-Lang et al., 2001); systemic inflammtory response syndrome (SIRS); multiorgan failure (MOF); acute respiratory distress syndrome (ARDS); inflammatory bowel syndrome (IBD) (Woodruff et al., 2003); immune-complex-mediated renal disease (Wang, 2006), e.g. as a complication of systemic lupus erythematosus (Manderson et al, 2004); infections and their consequences (e.g. vascular leakage or bone loss such as bone loss secondary to periodontitis (Breivik *et al.* 2011)); severe burns (Piccolo et al., 1999); reperfusion injury of organs such as heart, spleen, bladder, pancreas, stomach, lung, liver, kidney, limbs, brain, sceletal muscle or intestine (Riley et al., 2000)(Gueler *et al.* 2008; Khan *et al.* 2011; van der Pals *et al.* 2010; Zheng *et al.* 2008) that may lead amongst others to delayed graft function (Lewis et al, 2008) or fibrosis and/or remodelling of the organ, e.g. after an infarction of heart, brain or lung leading to secondary damage; psoriasis (Bergh et al., 1993); myocarditis; multiple sclerosis (Muller-Ladner et al., 1996); paroxysmal nocturnal hemoglobinuria (PNH), hemolysis, thromboembolism (Hillmern et al. 2007) and rheumatoid arthritis (RA) (Woodruff et al., 2002), resection of renal cell carcinoma and activation of osteoclasts promoting bone destruction, e.g. resulting in osteoarthtitis or delayed healing. Complement C5a has also been found in elevated amounts in drusen in age-related macular degeneration and it has been shown to lead to increased VEGF-expression and to promote choroidal neovascularization that may lead to vision impairment and loss (Nozaki et al, 2006).

Activation of the complement system has also been shown to raise susceptibility to develop cerebral malaria in a mouse model. C5a or C5a receptor blockade using serum from mice immunized with theses molecules conferred resistance to cerebral malaria. Therefore, blocking the C5 C5a axis may be beneficial in the prevention of developing malaria, especially cerebral malaria in humans (Patel *et al.* 2008).

Autoimmune inflammatory diseases with complement involvement have been reviewed recently (Chen *et al.* 2010). An expert review on possible and already pursued complement-targeted therapies appeared in Nature Biotechnology (Ricklin & Lambris, 2007). An update was published in Molecular Medicine in 2011 (Ehrnthaller *et al.* 2011).

Of course, because the C5a binding agent, preferably aC5a binding nucleic acid according to the present invention, interact with or bind to human C5a, a skilled person will generally understand that the C5a binding agent, preferably a C5a binding nucleic acid according to the present invention can easily be used for the treatment, prevention and/or diagnosis of any disease of humans and animals as described herein. In connection therewith, it is to be acknowledged that the C5a binding agent, preferably a nucleic acid molecule according to the present invention can be used for the treatment and prevention of any of the diseases, disorder or condition described herein, irrespective of the mode of action underlying such disease, disorder and condition.

In the following, and without wishing to be bound by any theory, the rational for the use of the C5a binding agent, preferably a nucleic acid molecule according to the present invention, in connection with the various diseases, disorders and conditions is provided, thus rendering the claimed therapeutic, preventive and diagnostic applicability of the C5a binding agent, preferably a nucleic acid molecule according to the present invention, plausible. In order to avoid any unnecessary repetition, it should be acknowledged that due to the involvement of the C5a - C5a receptor axis as outlined in connection therewith said axis may be addressed by the C5a binding agent, preferably a nucleic acid molecule according to the present invention such that the claimed therapeutic, preventive and diagnostic effect is achieved. It should furthermore be acknowledged that the particularities of the diseases, disorders and conditions, of the patients and any detail of the treatment regimen described in connection therewith, may be subject to preferred embodiments of the instant application.

Myeloid-derived suppressor (abbr. MDS) cells were originally observed in cancer patient > 30 years ago, their role as spoilers of anti-tumor immunity is only now being appreciated. A heterogeneous population of normal myeloid cells trapped in intermediate stages of differentiation, MDS cells accumulate in the blood, lymph nodes and at tumor sites in virtually all cancer patients. In healty individuals, these cells differentiate into macrophages, dendritic cells and neutrophils, but the tumors secrete a range of factors that disrupt diffentiation of immune progenitor cells (Ostrand-Rosenberg, 2008). As shown for isolated MSD cells from the peripheral blood and the spleens of healty mice, the MDS cells express the C5a receptor on their surface to a similar extent - an abundant expression - to that of their mature counterparts granulocytes and monocytes. As well in tumor bearing mice, the MDS cells express the C5a receptor, but the expression level is lower on the surface of tumor associated MSD cells than on MSD cells in the peripheral blood and spleen. The reason is that the C5a receptor is internatilzed in tumor associated cells as shown by Markiewski et al, a C5a receptor antagonist can block the function of the C5a receptor on the surface of the MSD cells and led to an impaired tumor growth (Markiewski et al. 2008). C5a also acts as a suppressor of natural killer cell (NK cell) functions providing an explanation for negative impact of complement on tumor surveillance and NK function disorders in patients with certain immune diseases (Li *et al.* 2012; Min *et al.* 2012).

Accordingly, disease and/or disorders and/or diseased conditions for the treatment and/or prevention of which the medicament according to the present invention may be used include, but are not limited to tumor associated diseases and/ or disorders and/or diseased conditions.

In a preferrred embodiment, tumor or tumour is the name for a swelling or lesion formed by an abnormal growth of cells (termed neoplastic). A tumor can be benign, pre-malignant or malignant tumor. Moreover a tumor can be a solid tumor, preferably carcininoma, sarcomaa, aoteoma, fibrosarcoma, and chondrosoma

The tumors which can in particular be treated by a medicament or a C5a binding agent, preferably a nucleic acid molecule according to the present invention, are preferably those tumors which are selected from the group comprising tumors of the endocrine system, the eye, the gastrointestinal tract, the genital system, the haematopoietic system (including mixed and embryonic tumours), the mammary gland, the nervous system, the respiratory system, the skeleton, the skin, the soft tissues, the urinary outflow system

Preferably, these tumors are selected from the group comprising breast cancer, ovary carcinoma, prostate carcinoma, osteosarcoma, glioblastoma, melanoma, small-cell lung carcinoma and colorectal carcinoma.

It is within the present invention that the medicament and pharmaceutical composition, resepectively, containing a C5a binding agent preferably a nucleic acid according to the present invention, may be used for the treatment in such way.

In a further embodiment, the medicament comprises a further pharmaceutically active agent for the treament of tumors. Such further pharmaceutically active compounds are, among others but not limited thereto, those known to antineoplastic-active substances as alkylating agents, antimetabolites, antiangiogenic agents, mitose inhibiting agents, topoisomerase inhibitors, inhibitors of the cellular signal transduction, hormones, antibodies, immune conjugates and fusion proteins.

Other pharmaceutically active compounds are, among others but not limited thereto, those known to Bleomycin, inhibitors of thymidylatsynthase such as Raltitrexed and Pemetrexed, enzymes such as L-asparaginase, Miltefosin and ANAgrelid, inhibitors of the proteasome such as Bortezomib.

Moreover, the medicament according to the present invention may be used for the treatment and/or prevention of chronic obtructive pulmonary disease.

Chronic obstructive pulmonary disease (abbr. COPD) is a lung ailment that is characterized by a persistent blockage of airflow from the lungs. It is an under-diagnosed, life-threatening lung disease that interferes with normal breathing and is not fully reversible. COPD includes a few lung diseases: the most common are chronic bronchitis and emphysema. Many people with COPD have both of these diseases. The emphysema is a damage to the air sacs at the tips of the airways what makes it hard for the body to take in the oxygen it needs. During chronic bronchitis the airways are irritated, red, and make too much sticky mucus. The walls of the airways are swollen and partly block the air from passing through.

Neutrophils are attracted towards a C5a gradient, release superoxide radicals to kill pathogens and release beta-glucuronidase to hydrolyse complex glucuronide conjugates at the site of inflammation. However, these valuable defense mechanisms can be damaging to the body if the neutrophils are recruited to sites of pathogen-free inflammation, e.g. at reperfused sites after infaction, stroke or organ transplantation or in cases of autoimmune disease, alzheimer's disease and others that are listed below.

In turn, excessively high C5a concentrations - especially if they are not only locally elevated as they appear during sepsis - lead to a systemic activation of the neutrophils (leading to organ damage) with subsequent exhaustion and deactivation by means of reduced C5a receptor expression on the neutrophils' cell surface. This renders the patient even more vulnerable to the pathogens that persist in his body (Huber-Lang *et al.* 2002).

A C5a binding agent, preferably a C5a-binding nucleic acid, that is also inhibitory for the C5a-mediated effects on its receptor (CD88) (as shown by chemotaxis assays using differentiated BAF-3 cells) has therefore the potenital to block the above named consequences of C5a signaling and could prove beneficial as part of a medicament in a number of diseases and conditions which aberrant C5a signaling is implicated in. One of these conditions is polymicrobial sepsis. There is a rich body of literature collecting evidence for the detrimental role of C5a signaling in sepsis (Ward 2010b). Nucleic acids according to the present inventions were found to improve survival of mice and organ function parameters in cecal ligation and puncture (CLP) studies. CLP is a well-established rodent model for polymicrobial sepsis. Recently the role of complement C5 in sepsis induced by cecal ligation and puncture was investigated in wild-type and CS-deficient mice (Flierl *et al.* 2008). C5-/- mice had no survival advantage compared to WT mice and displayed a 400-fold increase of blood-borne bacteria when compared to wild-type mice. These effects were linked to the inability of C5 (-/-) mice to assemble the terminal membrane attack complex (MAC). The authors conclude that, during sepsis, selective blockade of C5a or its receptor (rather than C5) seems a more promising strategy, because C5a-blockade still allows for MAC formation while the adverse effects of C5a are prevented. In agreement, genetic deletion of C5a receptors CD88 and C5L2, pharmacological blockade of CD88 or pharmacological neutralization of C5a has been shown to be protective in cecal ligation and puncture (CLP) -induced sepsis (Czermak *et al.* 1999; Rittirsch *et al.* 2008). A translational in vitro model of meningococcal sepsis using human whole blood confirms that selective C5a inhibition (in contrast to blockade of C5 cleavage) prevents potentially harmful leukocyte activation without comprising bacterial clearance (Sprong *et al.* 2003). Inhibition of C5a prevents multiorgan failure in experimental sepsis by limiting systemic inflammation, coagulation and other pathogenic mechanisms (Huber-Lang *et al.* 2001; Huber-Lang *et al.* 2002; Laudes *et al.* 2002; Rittirsch *et al.* 2008; Ward 2010a).. The nucleic acids according to this invention, though binding to C5 do not block C5 cleavage to C5a and CSb which is required for MAC formation. On the contray, the inventive nucleic acids occupy the protein C5, which also increases terminal plasma half-life and selectively block the action of C5a once this has been liberated, e.g. by the C5 convertase. In a sepsis model, nucleic acids according to this invention have been shown to limit inflammation, prevent multi organ failue and edema formation and to improve survival.

Sepsis patients often require mechanical ventilation due to acute lung injury (ALI) and acute respiratory distress syndrome (ARDS). ALI/ARDS may also develop from a direct infection of the lung by community- or hospital-acquired infections in pneumonia. There is abundant evidence for a pathogenic role of C5a in ALI/ARDS. C5a induced tissue factor expression contributes to fibrin deposition within pulmonary alveoli of ALI/ARDS patients (Kambas *et al.* 2008). Experimental ALI is attenuated in C5-/- mice and CSa-neutralisation or silencing of C5aR in the lungs suppresses the inflammatory response and prevents vascular leakage (Bosmann & Ward 2012).

Moreover, other disease and/or disorders and/or diseased conditions for the treatment and/or prevention of which the medicament according to the present invention may be used include, but are not limited to are autoimmune diseases such as rheumatoid arthritis (abbr. RA), ankylosing spodylitis (abbr. AS), systemic lupus erythematosus (abbr. SLE), multiple sclerosis (abbr. MS), psoriasis, alopecia areata, warm and cold autoimmune hemolytic anemia (abbr. AIHA), atypical haemolytic uremia, pernicious anemia, acute inflammatory diseases, autoimmune adrenalitis, chronic inflammatory demyelinating polyneuropathy (abbr. CIDP), Churg-Strauss syndrome, Cogan syndrome, CREST syndrome, pemphigus vulgaris and pemphigus foliaceus, bullous pemphigoid, polymyalgia rheumatica, polymyositis, primary biliary cirrhosis, pancreatitis, peritonitis, psoriatic arthritis, rheumatic fever, sarcoidosis, Sjörgensen syndrome, scleroderma, celiac disease, stiff-man syndrome, Takayasu arteritis, transient gluten intolerance, autoimmune uveitis, vitiligo, polychondritis, dermatitis herpetiformis (abbr. DH) or Duhring's disease, fibromyalgia, Goodpasture syndrome, Guillain-Barré syndrome, Hashimoto thyroiditis, autoimmune hepatitis, inflammatory bowel disease (abbr. IBD), Crohn's disease, colitis ulcerosa, myasthenia gravis, immune complex disorders, glomerulonephritis, polyarteritis nodosa, anti-phospholipid syndrome, polyglandular autoimmune syndrome, idiopatic pulmonar fibrosis, idiopathic thrombocytopenic purpura (abbr. ITP), urticaria, autoimmune infertility, juvenile rheumatoid arthritis, sarcoidosis, autoimmune cardiomyopathy, Lambert-Eaton syndrome, lichen sclerosis, Lyme disease, Graves disease, Behcet's disease, Ménière's disease, reactive arthritis (Reiter's syndrome); infections with viruses such as HIV, HBV, HCV, CMV or intracellular parasites such as Leishmania, Rickettsia, Chlamydia, Coxiella, Plasmodium, Brucella, mycobacteria, Listeria, Toxoplasma and Trypanosoma; secondary damages of trauma; local inflammation, shock, anaphylactic shock, burn, septic shock, haemorrhagic shock, systemic inflammatory response syndrome (abbr. SIRS), multiple organ failure (abbr. MOF), asthma and allergy, vasculitides such as arteritis temporalis, vasculitis, vascular leakage, and atherosclerosis; acute injuries of the central nervous system, myocarditis, dermatomyositis, gingivitis, acute respiratory insufficiency, chronic obstructive pulmonary disease, stroke, myocardial infarction, reperfusion injury, neurocognitive dysfunction, burn, inflammatory diseases of the eye such as uveitis, age-related macular degeneration (abbr. AMD), diabetic retinopathy (abbr. DR), diabetic macular edema (abbr. DME), ocular pemphigoid, keratoconjunctivitis, Stevens-Johnson syndrome, and Graves ophthalmopathy; local manifestations of systemic diseases, inflammatory diseases of the vasculature, acute injuries of the central nervous system, type 1 and 2 diabetes, the manifestations of diabetes, SLE, and rheumatic disease in the eye, brain, vasculature, heart, lung, kidneys, liver, gastrointestinal tract, spleen, skin, bones, lymphatic system, blood or other organ systems, for the prevention and/or support and/or post-operative treatment of coronary artery bypass graft (abbr. CABG), off-pump coronary artery bypass graft (abbr. OPCABG), minimally invasive direct coronary artery bypass graft (abbr. MIDCAB), percutaneous transluminal coronary angioplasty (abbr. PTCA), thrombolysis, organ transplantation, and vessel clamping surgery; for the prevention of organ damage of a transplanted organ or of an organ to be transplanted or for use of treatment of transplant rejection for transplanted organs such as liver, kidney, intestine, lung, heart, skin, limb, cornea, Langerhans islet, bone marrow, blood vessels and pancreas; fetal rejection.

The various diseases and disorders for the treatment and/or prevention of which C5a binding agent, preferably a nucleic acid, can be used, may be grouped as follows:

1. Autoimmune/inflammatory diseases

1.1 Systemic autoimmune and/or inflammatory diseases comprising allergy, septic shock, secondary damages of trauma, warm and cold autoimmune hemolytic anemia (abbr. AIHA), systemic inflammatory response syndrome (abbr. SIRS), hemorrhagic shock, diabetes type 1, diabetes type 2, the manifestations of diabetes, diffuse scleroderma, periodontitis and its associates bone loss, polychondritis, polyglandular autoimmune syndrome, rheumatoid arthritis, systemic lupus erythematosus (abbr. SLE) and manifestations thereof, reactive arthritis (also known as Reiter's syndrome).

1.2 Autoimmune and/or inflammatory diseases of the gastro-intestinal tract comprising Crohn's disease, colitis ulcerosa, celiac disease, transient gluten intolerance, inflammatory bowel disease (abbr. IBD), pancreatitis, gastrointestinal allergic hypersensitivity, necrotizing enterocolitis.

1.3 Autoimmune and/or inflammatory diseases of the skin comprising psoriasis, urticaria, dermatomyositis, pemphigus vulgaris, pemphigus foliaceus, bullous pemphigoid, morphea/linear scleroderma, vitiligo, dermatitis herpetiformis (abbr. DH) or Duhring's disease, lichen sclerosis.

1.4 Autoimmune and/or inflammatory diseases of the vasculature comprising vasculitides (preferably arteritis temporalis), vasculitis, Henoch Schönlein purpura, anti-neutrophil cytoplasmic antibody (ANCA)-associated vasculitis, vascular leakage, polymyalgia rheumatica, atherosclerosis, Churg-Strauss syndrome, Takayasu arteritis, Goodpasture syndrome (= antiglomerular basement membrane disease; mostly affecting the kidneys glomeruli and the lungs), glomerulonephritis, polyarteritis nodosa, Behcet's disease

1.5 Autoimmune and/or inflammatory diseases of the nervous system comprising multiple sclerosis (abbr. MS), chronic inflammatory demyelinating polyneuropathy (abbr. CIDP), neurocognitive dysfunction, stiff-man syndrome, Guillain-Barré syndrome, myasthenia gravis, Lambert-Eaton syndrome, neuromyelitis optica (Devic syndrome).

1.6 Muscular skeletal autoimmune and/or inflammatory diseases comprising rheumatoid arthritis, rheumatic disease in the eye, brain, lung, kidneys, heart, liver, gastrointestinal tract, spleen, skin, bones, lymphatic system, blood or other organs, ankylosing spodylitis (abbr. AS), sarcoidosis, periodontitis and associated bone loss, polymyalgia rheumatica, polymyositis, psoriatic arthritis, rheumatic fever, polychondritis, fibromyalgia, juvenile rheumatoid arthritis, Lyme disease, reactive arthritis (also known as Reiter's syndrome).

1.7 Other autoimmune and/or inflammatory diseases comprise Cogan syndrome , autoimmune adrenalitis, immune complex disordes, Ménière's disease, local inflammations, alopecia areata, acute inflammatory diseases, primary biliary cirrhosis, Sjörgen's syndrome, scleroderma, diffuse scleroderma, CREST syndrome, Morphea/linear scleroderma, autoimmune uveitis, Hashimoto thyroiditis (autoimmune thyroid destruction),

Graves disease, autoimmune hepatitis, non-alcoholic steatohepatitis, glomerulonephritis, peritonitis, anti-phospholipid syndrome, idiopathic pulmonary fibrosis, renal fibrosis, hepatic fibrosis, autoimmune infertility, fetal rejection or miscarriage and graft-versus-host disease.

2. Diseases of the eye comprising uveitis, conjunctivitis, age-related macular degeneration (abbr. AMD), diabetic retinopathy (abbr. DR), diabetic macular edema (abbr. DME), retinal vessel occlusion, glaucoma, cataract, autoimmune retinal and intraocular inflammatory disease, ocular pemphigoid, keratoconjunctivitis, Stevens-Johnson syndrome, and Graves ophthalmopathy.
3. Reperfusion injuries and transplant rejections comprising stroke, myocardial infarction, reperfusion injuries, posttransplant thrombotic microangiopathy or organ damage to transplanted organs, such as liver (Arumugam *et al.* 2004), kidney (Arumugam *et al.* 2003), intestine, lung, heart, skin, limb , cornea, islets of Langerhans (Tokodai *et al.* 2010), bone marrow, blood vessels and pancreas, kidney damage after organ or bone marrow transplantation.
4. Prevention of transplant rejection comprising transplant rejection of transplanted organs, such as liver, kidney, intestine, lung, heart, skin, limb, cornea, islets of Langerhans, bone marrow, blood vessels and pancreas.
5. Cardiovascular diseases comprising atherosclerosis, myocarditis, myocardial infarction, stroke, pulmonary arterial hypertension (PAH), Abdominal Aortic Aneurism, inflammatory diseases of the vasculature, vasculitides, preferably arteritis temporalis, vasculitis, vascular leakage, the manifestations of diabetes, pre-eclempsia, autoimmune cardiomyopathy, vein host disease, arrythmogenic right ventrivular dysplasia/cardiomyopathy, for the prevention and/or support and/or post-operative treatment of coronary artery bypass graft (abbr. CABG).
6. Metabolic dysfunction comprising insulin resistance, glucose intolerance, adipose inflammation, and cardiovascular dysfunction in diet-induced obesity.
7. Respiratory diseases comprising asthma, acute respiratory insufficiency, acute lung injury, transfusion related lung injury, adult respiratory distress syndrome, chronic obstructive pulmonary disease, ventilator-induced lung injury, pneumonia and complications thereof.
8. Inflammatory diseases comprising inflammatory disease of the eye, autoimmune uveitis (Copland *et al.* 2010), conjunctivitis, vernal conjunctivitis, local manifestations of systemic diseases.
9. Acute reactions comprising secondary damages of trauma and fractures, shock, burn, anaphylactic shock, hemorrhagic shock, multiple organ failure (abbr. MOF), acute injuries of the central nervous system, acute injuries of the central nervous system, acute damage due to excessive C5a production by an activated coagulation system such as after organ or islet transplantation,
10. pain, acute pain, chronic pain, neuropathic pain, morphine tolerance and withdrawal-induced hyperalgesia.
11. Neurological and neurodegenerative disorders comprising neuropathies, Amyotrophic Lateral Sclerosis (ALS), Alzheimer's disease and Parkinson's disease (Farkas et al. 1998).
12. Infectious diseases comprising

12.1 bacterial infections, preferably meningitis, Lyme disease, reactive arthritis (also known as Reiter's syndrome), urinary tract and kidney infection, sepsis and its complications such as organ failure, cardiac dysfunction, systemic hypoperfusion, acidosis, adult respiratory distress syndrome, infections with intracellular pathogens (Klos et al. 2009),
12.2 viral infections, preferably HIV, HBV, HCV, CMV, viral meningitis
12.3 intracellular parasites, preferably Leishmania, Rickettsia, Chlamydia, Coxiella, plasmodium, especially cerebral malaria, Brucella, mycobacteria, Listeria, Toxoplasma and Trypanosoma.

13. Hemotological diseases comprising diseases associated with activation of coagulation and fibrinolytic systems disseminated intravascular coagulation (DIC) and/or thrombosis, pernicious anemia, warm and cold autoimmune hemolytic anemia (abbr. AIHA), anti-phospholipid syndrome and its associated complications, arterial and venous thrombosis, pregnancy complications such as recurrent miscarriage and fetal death, preeclampsia, placental insufficiency, fetal growth restriction, cervical remodeling and preterm birth, idiopathic thrombocytopenic purpura (abbr. ITP), atypical hemolytic uremic syndrome (aHUS), paroxysmal nocturnal hemoglobinuria (PNH) and allergic transfusion reactions.
14. Clinical complications associated with activation by biomaterials of complement and coagulation cascades occurring in procedures comprising hemodialysis, apheresis, visco-supplementation of arthritic joints, cardiopulmonary bypass, prosthetic vascular grafts and use of cardio vascular devices.

The C5a binding agent, preferably a nucleic acid according to the present invention, may also be used in an intraoperative manner to avoid deleterious effects of the patient's immune system, more preferably for the prevention and/or support and/or post-operative treatment of coronary artery bypass graft (abbr. CABG), off-pump coronary artery bypass graft (abbr. OPCABG), minimally invasive direct coronary artery bypass graft (abbr. MIDCAB), percutaneous transluminal

coronary angioplasty (abbr. PTCA), thrombolysis, organ transplantation, brain and spinal cord surgery, reconstructive surgery, and vessel clamping surgery, during any treatment with artificial ventilation or ventilation assistance to avoid lung ventilator-induced lung injury or secondary damages, such as vascular leakage and/ or emphysema, for the prevention of organ damage of a transplanted organ or of an organ to be transplanted or for use of treatment of transplant rejection and reperfusion injury for transplanted organs, such as liver, kidney, intestine, lung, heart, skin, limb, cornea, islets of Langerhans, bone marrow, blood vessels and pancreas.

It is within the present invention that the medicament and pharmaceutical composition, resepectively, containing a nucleic acid according to the present inventors may be used for the treatment in such way.

In a further embodiment, the medicament comprises a further pharmaceutically active agent. Such further pharmaceutically active compounds are, among others but not limited thereto, those known to suppress the immune system such as calcineurin inhibitors, cyclosporin A, methotrexate, azathioprin, tacrolimus, rapamycin, chlorambucil, leflunomide, mycophenolate mofetil, brequinar, mizoribin, thalidomide, or deoxyspergualin. The further pharmaceutically active compound can be, in a further embodiment, also one of those compounds which reduce histamine production such as meclozin, clemastin, dimetinden, bamipin, ketotifen, cetirizin, lovecetirizin, cesloratadin, azelastin, mizolastin, levocabastin, terfenadin, fexofenadin, or ebastin. Such compounds can also be, but are not limited to, steroids and are preferably selected from the group comprising corticosteroids like prednisone, methylprednisolone, hydrocortisone, dexamethasone, triamcinolone, betamethasone, effervescent, or budesonide. Further, such compound can be one or several antibiotics such as, but not restricted to, aminoglycosides, β-lactam antibiotics, gyrase inhibitors, glycopeptide antibiotics, lincosamide, macrolide antibiotics, nitroimidazole derivatives, polypeptide antibiotics, sulfonamides, trimethoprim and tetracycline. Additionally, more specific anti-inflammatory or antiangiogenic biologics can be used in combination such as bevacizumab, ranibizumab, IL-10, erlizumab, tolermab, rituximab, gomiliximab, basiliximab, daclizumab, HuMax-TAC, visilizumab, HuMaxCD4, clenoliximab, MAX 16H5, TNX 100, toralizumab, alemtuzumab, CY 1788, galiximab, pexelizumab, eculizumab, PMX-53, ETI 104, FG 3019, bertilimumab, 249417 (anti-factor IX) abciximab, YM 337, omalizumab, talizumab, fontolizumab, J695 (anti-IL12), HuMaxIL-15, mepolizumab, elsilimomab, HuDREG, anakinra, Xoma-052, adalimumab, infliximab, certolizumab, afelimomab, CytoFab, AME 527, Vapaliximab, bevacizumab, ranibizumab, vitaxin, belimumab, MLN 1202, volociximab, F200 (anti-α5β1), efalizumab, m60.11 (anti.CD11b), etanercept, onercept, rilonacept, abatacept, natalizumab, or siplizumab, tocilizumab, ustekinumab, ABT-874. Finally, the further pharmaceutically active agent may be a modulator of the activity of any other chemokine which can be a chemokine agonist or antagonist or a chemokine receptor agonist or antagonist whereby the chemokine can also be a chemotactic lipid. An example is the S1P receptor modulator fingolimod. Alternatively, or additionally, such further pharmaceutically active agent is a further C5a binding agent, preferably a nucleic acid according to the present invention. Alternatively, the medicament comprises at least one more C5a binding agent which binds to a target molecule different from C5a or exhibits a function which is different from the one of the nucleic acids according to the present invention.

In general the C5a antagonist can be combined with inhibitors of other proinflammatory molecules or their receptors. Examples for proinflammatory molecules whose action can be attenuated in combination with the C5a antagonist are IL-1, IL-2, IL-5, IL-6, IL-8, IL-10, IL-12, IL-13, IL-15, IL-16, IL-17, IL-18, IL-23, TNF, α4β7, α5β1, BlyS, cadherin, CCR2, CD11a, CD11b, CD125, CD130, CD16, CD18, CD2, CD20,CD22, CD23, CD25, CD28, CD3, CD30, CD4, CD40, CD40L, CD44, CD45R, CD54, CD62E, CD62L, CD68, CD8, CD80, CD86, CD95, CEP, gastrin-R, C1, C1-esterase, C5, factor D, MBL, complement receptor 1, CRTH2-receptor, CTGF, E- and P-selectin, eotaxin, factor IX, FGF-20, Fgl-2, GM-CSF, GP IIb/IIIa receptor, HMG1, ICAM-1, IgE, thymocytes, IFNγ, IFNr, IP-10, MCP-1, M-CSF receptor, MIF, MMP9, PDGF-D, SDF-1, TGFβ1, tissue factor, tyrosine kinase receptor, VAP-1, VCAM-1, VEGF, VLA1, von Willebrandt factor, sphingosine 1 Phosphate, ceramide-1 phopshate, and inibitors of mitogens, e.g. inhibitors of lysophosphatidic acid.

Finally, the further pharmaceutically active agent may be a modulator of the activity of any other chemokine which can be a chemokine agonist or antagonist or a chemokine receptor agonist or antagonist. Alternatively, or additionally, such further pharmaceutically active agent is a further C5a binding agent. Alternatively, the medicament comprises at least one more C5a binding agent which binds to a target molecule different from C5a or exhibits a function which is different from the one of the nucleic acids according to the present invention.

It is within the present invention that the medicament is alternatively or additionally used, in principle, for the prevention of any of the diseases disclosed in connection with the use of the medicament for the treatment of said diseases. Respective markers therefore, i.e. for the respective diseases are known to the ones skilled in the art. Preferably, the respective marker is C5a.

In one embodiment of the medicament of the present invention, such medicament is for use in combination with other treatments for any of the diseases disclosed herein, particularly those for which the medicament of the present invention is to be used.

"Combination therapy" (or "co-therapy") includes the administration of a medicament of the invention and at least a second agent as part of a specific treatment regimen intended to provide the beneficial effect from the co-action of these therapeutic agents, i. e. the medicament of the present invention and said second agent. The beneficial effect of the

combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected).

"Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to a subject a single capsule having a fixed ratio of each therapeutic agent or in multiple, single capsules for each of the therapeutic agents.

Sequential or substantially simultaneous administration of each therapeutic agent can be effected by any appropriate route including, but not limited to, topical routes, oral routes, intravenous routes, intramuscular routes, and direct absorption through mucous membrane tissues. The therapeutic agents can be administered by the same route or by different routes. For example, a first therapeutic agent of the combination selected may be administered by injection while the other therapeutic agents of the combination may be administered topically.

Alternatively, for example, all therapeutic agents may be administered topically or all therapeutic agents may be administered by injection. The sequence in which the therapeutic agents are administered is not narrowly critical unless noted otherwise. "Combination therapy" also can embrace the administration of the therapeutic agents as described above in further combination with other biologically active ingredients. Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

As outlined in general terms above, the medicament according to the present invention can be administered, in principle, in any form known to the ones skilled in the art. A preferred route of administration is systemic administration, more preferably by parenteral administration, preferably by injection. Alternatively, the medicament may be administered locally. Other routes of administration comprise intramuscular, intraperitoneal, and subcutaneous, per orum, intranasal, intratracheal or pulmonary with preference given to the route of administration that is the least invasive, while ensuring efficiancy.

Parenteral administration is generally used for subcutaneous, intramuscular or intravenous injections and infusions. Additionally, one approach for parenteral administration employs the implantation of a slow-release or sustained-released systems, which assures that a constant level of dosage is maintained, that are well known to the ordinary skill in the art.

Furthermore, preferred medicaments of the present invention can be administered in intranasal form via topical use of suitable intranasal vehicles, inhalants, or via transdermal routes, using those forms of transdermal skin patches well known to those of ordinary skill in that art. To be administered in the form of a transdermal delivery system, the dosage administration will, of course, be continuous rather than intermittent throughout the dosage regimen. Other preferred topical preparations include creams, ointments, lotions, aerosol sprays and gels, wherein the concentration of active ingredient would typically range from 0.01% to 15%, w/w or w/v.

The medicament of the present invention will generally comprise an effective amount of the active component(s) of the therapy, including, but not limited to, a C5a binding agent preferably a nucleic acid molecule of the present invention, dissolved or dispersed in a pharmaceutically acceptable medium. Pharmaceutically acceptable media or carriers include any and all solvents, dispersion media, coatings, antibacterial and antifungal agents, isotonic and absorption delaying agents and the like. The use of such media and agents for pharmaceutical active substances is well known in the art. Supplementary active ingredients can also be incorporated into the medicament of the present invention.

In a further aspect the present invention is related to a pharmaceutical composition. Such pharmaceutical composition comprises at least one of the C5a binding agent, preferably a nucleic acid according to the present, invention and preferably a pharmaceutically acceptable vehicle. Such vehicle can be any vehicle or any binder used and/or known in the art. More particularly such binder or vehicle is any binder or vehicle as discussed in connection with the manufacture of the medicament disclosed herein. In a further embodiment, the pharmaceutical composition comprises a further pharmaceutically active agent.

The preparation of a medicament and a pharmaceutical composition will be known to those of skill in the art in light of the present disclosure. Typically, such compositions may be prepared as injectables, either as liquid solutions or suspensions; solid forms suitable for solution in, or suspension in, liquid prior to injection; as tablets or other solids for oral administration; as time release capsules; or in any other form currently used, including eye drops, creams, lotions, salves, inhalants and the like. The use of sterile formulations, such as saline-based washes, by surgeons, physicians or health care workers to treat a particular area in the operating field may also be particularly useful. Compositions may also be

delivered via microdevice, microparticle or sponge.

Upon formulation, a medicament will be administered in a manner compatible with the dosage formulation, and in such amount as is pharmacologically effective. The formulations are easily administered in a variety of dosage forms, such as the type of injectable solutions described above, but drug release capsules and the like can also be employed.

In this context, the quantity of active ingredient and volume of composition to be administered depends on the individual or the subject to be treated. Specific amounts of active compound required for administration depend on the judgment of the practitioner and are peculiar to each individual.

A minimal volume of a medicament required to disperse the active compounds is typically utilized. Suitable regimes for administration are also variable, but would be typified by initially administering the compound and monitoring the results and then giving further controlled doses at further intervals.

For instance, for oral administration in the form of a tablet or capsule (e.g., a gelatin capsule), the active drug component, i. e. a C5a binding agent, preferably a nucleic acid molecule of the present invention, and/or any further pharmaceutically active agent, also referred to herein as therapeutic agent(s) or active compound(s) can be combined with an oral, non-toxic, pharmaceutically acceptable inert carrier such as ethanol, glycerol, water and the like. Moreover, when desired or necessary, suitable binders, lubricants, disintegrating agents, and coloring agents can also be incorporated into the mixture. Suitable binders include starch, magnesium aluminum silicate, starch paste, gelatin, methylcellulose, sodium carboxymethylcellulose and/or polyvinylpyrrolidone, natural sugars such as glucose or beta-lactose, corn sweeteners, natural and synthetic gums such as acacia, tragacanth or sodium alginate, polyethylene glycol, waxes, and the like. Lubricants used in these dosage forms include sodium oleate, sodium stearate, magnesium stearate, sodium benzoate, sodium acetate, sodium chloride, silica, talcum, stearic acid, its magnesium or calcium salt and/or polyethyleneglycol, and the like. Disintegrators include, without limitation, starch, methyl cellulose, agar, bentonite, xanthan gum starches, agar, alginic acid or its sodium salt, or effervescent mixtures, and the like. Diluents, include, e.g., lactose, dextrose, sucrose, mannitol, sorbitol, cellulose and/or glycine.

The medicament of the invention can also be administered in such oral dosage forms as timed release and sustained release tablets or capsules, pills, powders, granules, elixirs, tinctures, suspensions, syrups and emulsions. Suppositories are advantageously prepared from fatty emulsions or suspensions.

The pharmaceutical composition or medicament may be sterilized and/or contain adjuvants, such as preserving, stabilizing, wetting or emulsifying agents, solution promoters, salts for regulating the osmotic pressure and/or buffers. In addition, they may also contain other therapeutically valuable substances. The compositions are prepared according to conventional mixing, granulating, or coating methods, and typically contain about 0.1% to 75%, preferably about 1% to 50%, of the active ingredient.

Liquid, particularly injectable compositions can, for example, be prepared by dissolving, dispersing, etc. The active compound is dissolved in or mixed with a pharmaceutically pure solvent such as, for example, water, saline, aqueous dextrose, glycerol, ethanol, and the like, to thereby form the injectable solution or suspension. Additionally, solid forms suitable for dissolving in liquid prior to injection can be formulated.

For solid compositions, excipients include pharmaceutical grades of mannitol, lactose, starch, magnesium stearate, sodium saccharin, talcum, cellulose, glucose, sucrose, magnesium carbonate, and the like. The active compound defined above, may be also formulated as suppositories, using for example, polyalkylene glycols, for example, propylene glycol, as the carrier. In some embodiments, suppositories are advantageously prepared from fatty emulsions or suspensions.

The medicaments and C5a binding agent, preferably the nucleic acid molecules, respectively, of the present invention can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, what is well known to the ordinary skill in the art. For example, the C5a binding agent, preferably a nucleic acid molecule described herein, can be provided as a complex with a lipophilic compound or non-immunogenic, high molecular weight compound constructed using methods known in the art. Additionally, liposomes may bear such nucleic acid molecules on their surface for targeting and carrying cytotoxic agents internally to mediate cell killing. An example of nucleic-acid associated complexes is provided in U.S. Patent No. 6,011,020.

The medicaments and C5a binding agent, preferably nucleic acid molecules, respectively, of the present invention may also be coupled with soluble polymers as targetable drug carriers. Such polymers can include polyvinylpyrrolidone, pyran copolymer, polyhydroxypropyl-methacrylamide-phenol, polyhydroxyethylaspanamidephenol, or polyethyleneoxidepolylysine substituted with palmitoyl residues. Furthermore, the medicaments and C5a binding agent, preferably nucleic acid molecules, respectively, of the present invention may be coupled to a class of biodegradable polymers useful in achieving controlled release of a drag, for example, polylactic acid, polyepsilon capro lactone, polyhydroxy butyric acid, polyorthoesters, polyacetals, polydihydropyrans, polycyanoacrylates and cross- linked or amphipathic block copolymers of hydrogels.

If desired, the pharmaceutical composition and medicament, respectively, to be administered may also contain minor amounts of non-toxic auxiliary substances such as wetting or emulsifying agents, pH buffering agents, and other substances such as for example, sodium acetate, and triethanolamine oleate.

The dosage regimen utilizing the nucleic acid molecules and medicaments, respectively, of the present invention is selected in accordance with a variety of factors including type, species, age, weight, sex and medical condition of the patient; the severity of the condition to be treated; the route of administration; the renal and hepatic function of the patient; and the particular aptamer or salt thereof employed. An ordinarily skilled physician or veterinarian can readily determine and prescribe the effective amount of the drug required to prevent, counter or arrest the progress of the condition.

Effective plasma levels of the nucleic acid according to the present invention preferably range from 500 fM to 500 µM in the treatment of any of the diseases disclosed herein.

The nucleic acid molecules and medicaments, respectively, of the present invention may preferably be administered in a single daily dose, every second or third day, weekly, every second week, in a single monthly dose or every third month.

It is within the present invention that the medicament as described herein constitutes the pharmaceutical composition disclosed herein.

In a further aspect the present invention is related to a method for the treatment of a subject who is in need of such treatment, whereby the method comprises the administration of a pharmaceutically active amount of at least one of the nucleic acids according to the present invention. In an embodiment, the subject suffers from a disease or is at risk to develop such disease, whereby the disease is any of those disclosed herein, particularly any of those diseases disclosed in connection with the use of any of the nucleic acids according to the present invention for the manufacture of a medicament.

As preferably used herein, the term treatment comprises in a preferred embodiment additionally or alternatively prevention and/or follow-up.

As preferably used herein, the terms disease and disorder shall be used in an interchangeable manner, if not indicated to the contrary.

As used herein, the term comprise is preferably not intended to limit the subject matter followed or described by such term. However, in an alternative embodiment the term comprises shall be understood in the meaning of containing and thus as limiting the subject matter followed or described by such term.

The various SEQ.ID. Nos., the chemical nature of the nucleic acid molecules according to the present invention and the target molecules C5a as used herein, the actual sequence thereof and the internal reference number is summarized in the following table.

**TABLE 1**

| SEQ ID NO. | Internal Reference | | Sequence |
|---|---|---|---|
| 1 | 274-B5-002 | L-RNA | GCCUGAUGUGGUGUUGAAGGGUUGUGGGGUGUCGACGCACAGGC |
| 2 | 274-D5-002 | L-RNA | GCCUGAUGUGGUGUUGAGGGGUUGUGGGGUGUCGACGCACAGGC |
| 3 | 274-C8-002 | L-RNA | GCCUGAUGUGGUGUUGAAGGGUUGUUGGGUGUCGACGCACAGGC |
| 4 | 274-C8-002-G14 (=NOX-D19001) | L-RNA | GCCUGAUGUGGUGGUGAAGGGUUGUUGGGUGUCGACGCACAGGC |
| 5 | 274-C5-002 | L-RNA | GCCUGAUGUGGUGGUGAGGGGUUGUGGGGUGUCGACGCACAGGC |
| 6 | 274-G6-002 | L-RNA | GCCUGAUGUGGUGGUGAGGGGAUGUGGGGUGUCGACGCACAGGC |
| 7 | 274-H6-002 | L-RNA | GCCUGAUGUGGUGUUGAGGGGCUGUGGGGUGUCGACGCACAGGC |
| 8 | NOX-D19001-D09 | L-RNA/**L-DNA** | GCCUGAUG**dU**GGUGGUGAAGGGUUGUUGGGUGUCGACGCACAGGC |
| 9 | NOX-D19001-D16 | L-RNA/**L-DNA** | GCCUGAUGUGGUGGU**dG**AAGGGUUGUUGGGUGUCGACGCACAGGC |
| 10 | NOX-D19001-D17 | L-RNA/**L-DNA** | GCCUGAUGUGGUGGUG**dA**AGGGUUGUUGGGUGUCGACGCACAGGC |
| 11 | NOX-D19001-D30 | L-RNA/**L-DNA** | GCCUGAUGUGGUGGUGAAGGGUUGUUGGG**dU**GUCGACGCACAGGC |
| 12 | NOX-D19001-D32 | L-RNA/**L-DNA** | GCCUGAUGUGGUGGUGAAGGGUUGUUGGGUG**dU**CGACGCACAGGC |
| 13 | NOX-D19001-D40 | L-RNA/**L-DNA** | GCCUGAUGUGGUGGUGAAGGGUUGUUGGGUGUCGACGCA**dC**AGGC |
| 14 | NOX-D19001-D09-30 | L-RNA/**L-DNA** | GCCUGAUG**dU**GGUGGUGAAGGGUUGUUGGG**dU**GUCGACGCACAGGC |
| 15 | NOX-D19001-D09-32 | L-RNA/**L-DNA** | GCCUGAUG**dU**GGUGGUGAAGGGUUGUUGGGUG**dU**CGACGCACAGGC |
| 16 | NOX-D19001-D09-40 | L-RNA/**L-DNA** | GCCUGAUG**dU**GGUGGUGAAGGGUUGUUGGGUGUCGACGCA**dC**AGGC |
| 17 | NOX-D19001-D30-32 | L-RNA/**L-DNA** | GCCUGAUGUGGUGGUGAAGGGUUGUUGGG**dU**G**dU**CGACGCACAGGC |
| 18 | NOX-D19001-D30-40 | L-RNA/**L-DNA** | GCCUGAUGUGGUGGUGAAGGGUUGUUGGG**dU**GUCGACGCA**dC**AGGC |
| 19 | NOX-D19001-D32-40 | L-RNA/**L-DNA** | GCCUGAUGUGGUGGUGAAGGGUUGUUGGGUG**dU**CGACGCA**dC**AGGC |
| 20 | NOX-D19001-D09-30-32 | L-RNA/**L-DNA** | GCCUGAUGdUGGUGGUGAAGGGUUGUUGGG**dU**G**dU**CGACGCACAGGC |
| 21 | NOX-D19001-D09-30-40 | L-RNA/**L-DNA** | GCCUGAUG**dU**GGUGGUGAAGGGUUGUUGGG**dU**GUCGACGCA**dC**AGGC |
| 22 | NOX-D19001-D09-32-40 | L-RNA/**L-DNA** | GCCUGAUG**dU**GGUGGUGAAGGGUUGUUGGGUG**dU**CGACGCA**dC**AGGC |
| 23 | NOX-D19001-D30-32-40 | L-RNA/**L-DNA** | GCCUGAUGUGGUGGUGAAGGGUUGUUGGG**dU**G**dU**CGACGCA**dC**AGGC |
| 24 | NOX-D19001-D09-30-32-40 | L-RNA/**L-DNA** | GCCUGAUG**dU**GGUGGUGAAGGGUUGUUGGG**dU**G**dU**CGACGCA**dC**AGGC |
| 25 | NOX-D19001-D09-16-30-32-40 | L-RNA/**L-DNA** | GCCUGAUG**dU**GGUGGU**dG**AAGGGUUGUUGGG**dU**G**dU**CGACGCA**dC**AGGC |

(continued)

| SEQ ID NO. | Internal Reference | | Sequence |
|---|---|---|---|
| 26 | NOX-D19001-D09-17-30-32-40 | L-RNA/**L-DNA** | GCCUGAUG**dU**GGUGGUG**dA**AGGGUUGUUGGG**dU**G**dU**CGACGCA**dC**AGGC |
| 27 | NOX-D19001-D09-16-17-30-32-40 (= NOX-D19001-6xDNA) | L-RNA/**L-DNA** | GCCUGAUG**dU**GGUGGU**dGdA**AGGGUUGUUGGG**dU**G**dU**CGACGCA**dC**AGGC |
| 28 | NOX-D19001-6xDNA-007 | L-RNA/**L-DNA** | CCUGAUG**dU**GGUGGU**dGdA**AGGGUUGUUGGG**dU**G**dU**CGACGCA**dC**AGGC |
| 29 | NOX-D19001-6xDNA-008 | L-RNA/**L-DNA** | CUGAUG**dU**GGUGGU**dGdA**AGGGUUGUUGGG**dU**G**dU**CGACGCA**dC**AGGC |
| 30 | NOX-D19001-6xDNA-009 | L-RNA/**L-DNA** | UGAUG**dU**GGUGGU**dGdA**AGGGUUGUUGGG**dU**G**dU**CGACGCA**dC**AGGC |
| 31 | NOX-D19001-6xDNA-010 | L-RNA/**L-DNA** | GAUG**dU**GGUGGU**dGdA**AGGGUUGUUGGGdUG**dU**CGACGCA**dC**AGGC |
| 32 | NOX-D19001-6xDNA-011 | L-RNA/**L-DNA** | GCUGAUG**dU**GGUGGU**dGdA**AGGGUUGUUGGG**dU**G**dU**CGACGCA**dC**AGC |
| 33 | NOX-D19001-6xDNA-012 | L-RNA/**L-DNA** | GUGAUG**dU**GGUGGU**dGdA**AGGGUUGUUGGG**dU**G**dU**CGACGCA**dC**AC |
| 34 | NOX-D19001-6xDNA-013 | L-RNA/**L-DNA** | UGAUG**dU**GGUGGU**dGdA**AGGGUUGUUGGG**dU**G**dU**CGACGCA**dC**A |
| 35 | NOX-D19001-6xDNA-018 | L-RNA/**L-DNA** | GCCGAUG**dU**GGUGGU**dGdA**AGGGUUGUUGGG**dU**G**dU**CGACGCA**dC**GGC |
| 36 | NOX-D19001-6xDNA-019 | L-RNA/**L-DNA** | GGCGAUG**dU**GGUGGU**dGdA**AGGGUUGUUGGG**dU**G**dU**CGACGCA**dC**GCC |
| 37 | NOX-D19001-6xDNA-020 (=NOX-D20001) | L-RNA/**L-DNA** | GCGAUG**dU**GGUGGU**dGdA**AGGGUUGUUGGG**dU**G**dU**CGACGCA**dC**GC |
| 38 | NOX-D19001-6xDNA-021 | L-RNA/**L-DNA** | CUGAUG**dU**GGUGGU**dGdA**AGGGUUGUUGGG**dU**G**dU**CGACGCAdCAGC |
| 39 | NOX-D19001-6xDNA-022 | L-RNA/**L-DNA** | UGAUG**dU**GGUGGU**dGdA**AGGGUUGUUGGG**dU**G**dU**CGACGCA**dC**AGC |
| 40 | NOX-D19001-6xDNA-023 | L-RNA/**L-DNA** | CGAUG**dU**GGUGGU**dGdA**AGGGUUGUUGGG**dU**G**dU**CGACGCA**dC**AGC |
| 41 | NOX-D19001-6xDNA-024 | L-RNA/**L-DNA** | GAUG**dU**GGUGGU**dGdA**AGGGUUGUUGGG**dU**G**dU**CGACGCA**dC**AGC |
| 42 | NOX-D19001-6xDNA-025 | L-RNA/**L-DNA** | GCUGAUG**dU**GGUGGU**dGdA**AGGGUUGUUGGG**dU**G**dU**CGACGCA**dC**AC |
| 43 | NOX-D19001-6xDNA-026 | L-RNA/**L-DNA** | GCUGAUG**dU**GGUGGU**dGdA**AGGGUUGUUGGG**dU**G**dU**CGACGCA**dC**C |
| 44 | NOX-D19001-6xDNA-027 | L-RNA/**L-DNA** | GCUGAUG**dU**GGUGGU**dGdA**AGGGUUGUUGGG**dU**G**dU**CGACGCA**dC**A |
| 45 | NOX-D19001-6xDNA-028 | L-RNA/**L-DNA** | CGAUG**dU**GGUGGU**dGdA**AGGGUUGUUGGG**dU**G**dU**CGACGCA**dC**GC |
| 46 | NOX-D19001-6xDNA-029 | L-RNA/**L-DNA** | GAUG**dU**GGUGGU**dGdA**AGGGUUGUUGGG**dU**G**dU**CGACGCA**dC**GC |
| 47 | NOX-D19001-6xDNA-030 | L-RNA/**L-DNA** | GCGAUG**dU**GGUGGU**dGdA**AGGGUUGUUGGG**dU**G**dU**CGACGCA**dC**C |
| 48 | NOX-D19001-6xDNA-032 | L-RNA/**L-DNA** | GCGAUG**dU**GGUGGU**dGdA**AGGGUUGUUGGG**dU**G**dU**CGACGCA**dC** |
| 49 | NOX-D19001-6xDNA-033 | L-RNA/**L-DNA** | GGAUG**dU**GGUGGU**dGdA**AGGGUUGUUGGG**dU**G**dU**CGACGCA**dC**C |

(continued)

| SEQ ID NO. | Internal Reference | | Sequence |
|---|---|---|---|
| 50 | human C5a | L-protein | TLQKKIEEIAAKYKHSVVKKCCYDGACVNNDETCEQRAARISLGPRCIKAF<br>TECCVVASQLRANISHKDMQLGR |
| 51 | rat C5a | L-protein | DLQLLHQKVEEQAAKYKHRVPKKCCYDGARENKYETCEQRVARVTIGPHCI<br>RAFNECCTIADKIRKESHHKGMLLGR |
| 52 | mouse C5a | L-protein | NLHLLRQKIEEQAAKYKHSVPKKCCYDGARVNFYETCEERVARVTIGPLCI<br>RAFNECCTIANKIRKESPHKPVQLGR |
| 53 | **Human C5, alpha chain** | L-protein | TLQKKIEEIAAKYKHSVVKKCCYDGACVNNDETCEQRAARISLGPRCIKAF<br>TECCVVASQLRANISHKDMQLGRLHMKTLLPVSKPEIRSYFPESWLWEVHL<br>VPRRKQLQFALPDSLTTWEIQGIGISNTGICVADTVKAKVFKDVFLEMNIP<br>YSVVRGEQIQLKGTVYNYRTSGMQFCVKMSAVEGICTSESPVIDHQGTKSS<br>KCVRQKVEGSSSHLVTFTVLPLEIGLHNINFSLETWFGKEILVKTLRVVPE<br>GVKRESYSGVTLDPRGIYGTISRRKEFPYRIPLDLVPKTEIKRILSVKGLL<br>VGEILSAVLSQEGINILTHLPKGSAEAELMSVVPVFYVFHYLETGNHWNIF<br>HSDPLIEKQKLKKKLKEGMLSIMSYRNADYSYSVWKGGSASTWLTAFALRV<br>LGQVNKYVEQNQNSICNSLLWLVENYQLDNGSFKENSQYQPIKLQGTLPVE<br>ARENSLYLTAFTVIGIRKAFDICPLVKIDTALIKADNFLLENTLPAQSTFT<br>LAISAYALSLGDKTHPQFRSIVSALKREALVKGNPPIYRFWKDNLQHKDSS<br>VPNTGTARMVETTAYALLTSLNLKDINYVNPVIKWLSEEQRYGGGFYSTQD<br>TINAIEGLTEYSLLVKQLRLSMDIDVSYKHKGALHNYKMTDKNFLGRPVEV<br>LLNDDLIVSTGFGSGLATVHVTTVVHKTSTSEEVCSFYLKIDTQDIEASHY<br>RGYGNSDYKRIVACASYKPSREESSSGSSHAVMDISLPTGISANEEDLKAL<br>VEGVDQLFTDYQIKDGHVILQLNSIPSSDFLCVRFRIFELFEVGFLSPATF<br>TVYEYHRPDKQCTMFYSTSNIKIQKVCEGAACKCVEADCGQMQEELDLTIS<br>AETRKQTACKPEIAYAYKVSITSITVENVFVKYKATLLDIYKTGEAVAEKD<br>SEITFIKKVTCTNAELVKGRQYLIMGKEALQIKYNFSFRYIYPLDSLTWIE<br>YWPRDTTCSSCQAFLANLDEFAEDIFLNGC |
| 54 | Rhesus monkey C5a | | MLQEKIEEIAAKYKHLVVKKCCYDGVRINHDETCEQRAARISVGPRCVKAF<br>TECCVVASQLRANNSHKDLQLGR |

(continued)

| SEQ ID NO. | Internal Reference | | Sequence |
|---|---|---|---|
| 55 | NOX-D19001-020 | | GCGAUGUGGUGGUGAAGGGUUGUUGGGUGUCGACGCACGC |
| 56 | NOX-D19001-1xDNA-020 | L-RNA/**L-DNA** | GCGAUG**dU**GGUGGUGAAGGGUUGUUGGGUGUCGACGCACGC |
| 57 | NOX-D19001-2xDNA-020 | L-RNA/**L-DNA** | GCGAUG**dU**GGUGGUGAAGGGUUGUUGGG**dU**GUCGACGCACGC |
| 58 | NOX-D19001-3xDNA-020 | L-RNA/**L-DNA** | GCGAUG**dU**GGUGGUGAAGGGUUGUUGGG**dU**G**dU**CGACGCACGC |
| 59 | NOX-D19001-2dU-1dC-020 (=NOX-D21001) | L-RNA/**L-DNA** | GCGAUG**dU**GGUGGUGAAGGGUUGUUGGG**dU**GUCGACGCA**dC**GC |
| 60 | NOX-D19001-3dU-1dC-020 | L-RNA/**L-DNA** | GCGAUG**dU**GGUGGUGAAGGGUUGUUGGG**dU**G**dU**CGACGCA**dC**GC |
| 61 | | L-RNA/**L-DNA** | AUG$n_1$GGUGKU$n_2n_3$RGGGHUGUKGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 62 | | L-RNA/**L-DNA** | AUG$n_1$GGUGUU$n_2n_3$AGGGUUGUGGGG$n_4$G$n_5$CGACGCA<br>wherein $n_1$ is U or dU, $n_2$ is G or **dG**, $n_3$ is A or **dA**, $n_4$ is U or **dU**, $n_5$ is U or **dU** |
| 63 | | L-RNA/**L-DNA** | AUG$n_1$GGUGUU$n_2n_3$GGGGUUGUGGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 64 | | L-RNA/**L-DNA** | AUG$n_1$GGUGUU$n_2n_3$AGGGUUGUUGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 65 | | L-RNA/**L-DNA** | AUG$n_1$GGUGGU$n_2n_3$AGGGUUGUUGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 66 | | L-RNA/**L-DNA** | AUG$n_1$GGUGGU$n_2n_3$GGGGUUGUGGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 67 | | L-RNA/**L-DNA** | AUG$n_1$GGUGGU$n_2n_3$GGGGAUGUGGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 68 | | L-RNA/**L-DNA** | AUG$n_1$GGUGUU$n_2n_3$GGGGCUGUGGGG$n_4$G$n_5$CGACGCA<br>wherein $n_1$ is U or **dU**, $n_2$ is G or **dG**, $n_3$ is A or **dA**, $n_4$ is U or **dU**, $n_5$ is U or **dU** |
| 69 | | L-RNA/**L-DNA** | AUGUGGUGKUGARGGGHUGUKGGGUGUCGACGCA |
| 70 | | L-RNA/**L-DNA** | AUGUGGUGUUGAAGGGUUGUUGGGUGUCGACGCA |
| 71 | | L-RNA/**L-DNA** | AUGUGGUGGUGAAGGGUUGUUGGGUGUCGACGCA |
| 72 | | L-RNA/**L-DNA** | AUGUGGUGGUGAGGGGUUGUGGGGUGUCGACGCA |
| 73 | | L-RNA/**L-DNA** | AUG**dU**GGUGGUGAAGGGUUGUUGGGUGUCGACGCA |

(continued)

| SEQ ID NO. | Internal Reference | | Sequence |
|---|---|---|---|
| 74 | | L-RNA/**L-DNA** | AUGUGGUGGU**dG**AAGGGUUGUUGGGUGUCGACGCA |
| 75 | | L-RNA/**L-DNA** | AUGUGGUGGUG**dA**AGGGUUGUUGGGUGUCGACGCA |
| 76 | | L-RNA/**L-DNA** | AUGUGGUGGUGAAGGGUUGUUGGG**dU**GUCGACGCA |
| 77 | | L-RNA/**L-DNA** | AUGUGGUGGUGAAGGGUUGUUGGGUG**dU**CGACGCA |
| 78 | | L-RNA/**L-DNA** | AUG**dU**GGUGGUGAAGGGUUGUUGGG**dU**GUCGACGCA |
| 79 | | L-RNA/**L-DNA** | AUG**dU**GGUGGUGAAGGGUUGUUGGGUG**dU**CGACGCA |
| 80 | | L-RNA/**L-DNA** | AUGUGGUGGUGAAGGGUUGUUGGG**dU**G**dU**CGACGCA |
| 81 | | L-RNA/**L-DNA** | AUG**dU**GGUGGUGAAGGGUUGUUGGG**dU**G**dU**CGACGCA |
| 82 | | L-RNA/**L-DNA** | AUG**dU**GGUGG**Ud**GAAGGGUUGUUGGG**dU**G**dU**CGACGCA |
| 83 | | L-RNA/**L-DNA** | AUG**dU**GGUGGUG**dA**AGGGUUGUUGGG**dU**G**dU**CGACGCA |
| 84 | | L-RNA/**L-DNA** | AUG**dU**GGUGGU**dGdA**AGGGUUGUUGGG**dU**G**dU**CGACGCA |
| 85 | 274-H6-002 | D-RNA | GCCUGAUGUGGUGUUGAGGGGCUGUGGGGUGUCGACGCACAGGC |
| 86 | 274-D5-002 | D-RNA | GCCUGAUGUGGUGUUGAGGGGUUGUGGGGUGUCGACGCACAGGC |
| 87 | revNOX-D19 | L-RNA/**L-DNA** | 40kDaPEG-<br>CGGACACGCAGCUGUGGGUUGUUGGGAAGUGGUGGUGUAGUCCG |
| 88 | revNOX-D21 | L-RNA/**L-DNA** | 40kDaPEG--<br>CGdCACGCAGCUGdUGGGUUGUUGGGAAGUGGUGGdUGUAGCG |
| 89 | Biotinylated mouse-D-C5a | D-protein | LLRQKIEEQAAKYKHSVPKKCCYDGARVNFYETCEERVARVTIGPLCIRAF<br>NECCTIANKIRKESPHKPVQLGR- Biotin |
| 90 | NOX-D19001-5'40kDa-PEG (= NOX-D19) | L-RNA/**L-DNA** | 40kDaPEG-<br>GCCUGAUGUGGUGGUGAAGGGUUGUUGGGUGUCGACGCACAGGC |
| 91 | NOX-D19001-6xDNA-020-5'40kDa-PEG (= NOX-D20) | L-RNA/**L-DNA** | 40kDaPEG-<br>GCGAUG**dU**GGUGGU**dGdA**AGGGUUGUUGGG**dU**G**dU**CGACGCA**dC**GC |

(continued)

| SEQ ID NO. | Internal Reference | | Sequence |
|---|---|---|---|
| 92 | NOX-D19001-2dU-1dC-020-5'40kDa-PEG (= NOX-D21) | L-RNA/**L-DNA** | 40kDaPEG-<br>GCGAUG**dU**GGUGGUGAAGGGUUGUUGGG**dU**GUCGACGCA**dC**GC |
| 93 | human des-ArgC5a | L-protein | TLQKKIEEIAAKYKHSVVKKCCYDGACVNNDETCEQRAARISLGPRCIKAF<br>TECCVVASQLRANISHKDMQLG<br>NLHLLRQKIEEQAAKYKHSVPKKCCYDGARVNFYETCEERVARVTIGPLCI<br>RAFNECCTIANKIRKESPHKPVQLG |
| 94 | mouse des-ArgC5a | L-protein | NLHLLRQKIEEQAAKYKHSVPKKCCYDGARVNFYETCEERVARVTIGPLCI<br>RAFNECCTIANKIRKESPHKPVQLG |
| 95 | **PNA-AON-D21-02;** | PNA | Atto425-OO-tgc gtc gac acc caa ca |

The present invention is further illustrated by the figures, examples and the sequence listing from which further features, embodiments and advantages may be taken, wherein

| | |
|---|---|
| Fig. 1 | shows an alignment of sequences of nucleic acid molecules capable of binding human and mouse C5a including the $K_D$ value and relative binding activity to human and mouse C5a as determined by suface plasmon resonance measurement; |
| Figs. 2 | shows derivatives of nucleic acid molecule NOX-D19001 with a single ribonucleotide to 2'-deoxyribonucleotide substitution including the $K_D$ value and relative binding activity to human C5a as determined by suface plasmon resonance measurement; |
| Figs. 3 | shows derivatives of nucleic acid molecule NOX-D19001 with two, three, four, five or six ribonucleotide to 2'-deoxyribonucleotide substitutions including the $K_D$ value and relative binding activity to human C5a as determined by suface plasmon resonance measurement; |
| Fig. 4+B | shows truncations of nucleic acid molecule NOX-D19001-6xDNA including the $K_D$ value and relative binding activity to human C5a as determined by suface plasmon resonance measurement; |
| Fig. 5 | shows derivatives of nucleic acid molecule NOX-D20001 with none, one, two, three or four ribonucleotide to 2'-deoxyribonucleotide substitutions including the $K_D$ value and relative binding activity to human C5a as determined by suface plasmon resonance measurement; |
| Fig. 6 | shows the kinetic evaluation by Biacore measurement of nucleic acid molecules NOX-D19001, NOX-D19001-D09 and NOX-D19001-D09-16-17-30-32-40 (also referred to as NOX-D19001-6xDNA) to human C5a whereby the data for 500 nM of L-aptamer NOX-D19001, NOX-D19001-D09 (abbr. D09) and NOX-D19001-D09-16-17-30-32-40 (abbr. D09-16-17-30-32-40) are shown; |
| Fig. 7 | is a diagram showing the efficacy of 5'-terminal 40kDa PEGylated C5a binding L-aptamers NOX-D19001-5'PEG (also referred as NOX-D19 and AON-D21$_{vD19}$) and NOX-D20 (also referred to as NOX-D19001-6xDNA-020-5'40kDa PEG and AON-D21$_{vD20}$) in chemotaxis assays, wherein cells were allowed to migrate towards 0.1 nM huC5a preincubated at 37°C with various amounts of L-aptamers, |
| Fig. 8 | shows the kinetic evaluation by Biacore measurement of nucleic acid molecules NOX-D20 (also referred to as NOX-D19001-6xDNA-020-5'40kDa PEG and AON-D21$_{vD20}$) to human C5a, rat C5a, mouse C5a, monkey C5a; whereby the data for 1000, 500, 250, 125, 62.5, 31.3, 15.6, 7.8, 3.9, and 1.95-0 nM of L-aptamer NOX-D20 are shown; |
| Fig. 9 | shows the kinetic evaluation by Biacore measurement of nucleic acid molecules NOX-D20 (also referred to as NOX-D19001-6xDNA-020-5'40kDa PEG and AON-D21$_{vD20}$) to human C5, and human desArg-CSa; whereby the data for 1000, 500, 250, 125, 62.5, 31.3, 15.6, 7.8, 3.9, and 1.95-0 nM of L-aptamer NOX-D20 are shown; |
| Fig. 10 | shows the kinetic evaluation by Biacore measurement of nucleic acid molecules NOX-D21 (also referred to as NOX-D19001-2dU-1dC-020-5'40kDa PEG and AON-D21) to human C5a, human C5, human desArg-C5a, mouse C5a and mouse desArg-CSa; whereby the data for 1000, 500, 250, 125, 62.5, 31.3, 15.6, 7.8, 3.9, and 1.95-0 nM of L-aptamer NOX-D21 binding to human C5a and human C5 are shown; |
| Fig. 11 | shows the polypeptide sequence aligment of C5a from human, rhesus monkey, mouse and rat; |
| Fig. 12A | is a diagram showing the efficacy of C5a binding L-aptamers NOX-D20 (also referred to as AON-D21$_{vD20}$) in chemotaxis assays with human C5a and mouse C5a, cells were allowed to migrate towards 0.1 nM huC5a or 0.3 nM mCSa preincubated at 37°C with various amounts of L-aptamer; wherein a) the cells counts were normalized to the largest value of each data set and depicted as per cent count against L-aptamer concentration, b) the L-aptamer concentrations at which the chemotaxis is inhibited by 50% ($IC_{50}$) were calculated using nonlinear regression (four parameter fit) with PrismS software; |
| Fig. 12B | is a diagram showing the efficacy of C5a binding L-aptamers NOX-D21 (also referred to as AON-D21) in chemotaxis assays with human C5a, cells were allowed to migrate towards 0.1 nM huC5a preincubated at 37°C with various amounts of L-aptamer; wherein a) the cells counts were normalized to the largest value of each data set and depicted as per cent count against L-aptamer concentration, b) the L-aptamer concentrations at which the chemotaxis is inhibited by 50% (IC50) were calculated using nonlinear regression (four parameter fit) with PrismS software; |
| Fig. 13 | shows the nuceic acid sequences, binding affinities and inhibitory constants of AON-D21 (also referred to as NOX-D21) and its variants; |
| Fig. 14 | is a diagram showing evaluation of C5 cleavage inhibition using a sheep erythrocyte hemolysis assay with L-aptamers (A) NOX-19 (also referred to as AON-D21$_{vD19}$) and NOX-D20 (also referred to as AON-D21$_{vD20}$), and (B) NOX-D21 (also referred to as AON-D21). A positive (C5C6) and negative controls (revNOX-D19 and revNOX-D21) are shown; |

Fig. 15 is a diagram showing survival in the cecal ligation and puncture (CLP) mouse model of polymicrobial sepsis; NOX-D19 (also referred to as AON-$D21_{vD19}$) at the indicated doses or vehicle was injected intraperitoneally daily starting right after CLP surgery. Sham animals received surgery without CLP, followed by vehicle injections;

Fig. 16 is a diagram showing (A) serum creatinine levels and (B) blood urea nitrogen (BUN) levels pre-surgery (day -4) and 1 day after CLP surgery in mice treated with NOX-D19 (also referred to as AON-$D21_{vD19}$) at indicated doses, in vehicle treated mice and in sham animals. Serum creatinine and BUN are biomarkers for renal function;

Fig. 17 is a diagram showing (A) serum levels of alanine aminotransferase (ALT) and (B) serum levels of aspartate aminotransferase (AST) pre-surgery (day -4) and 1 day after CLP surgery in mice treated with NOX-D19 (also referred to as AON-$D21_{vD19}$) at indicated doses, in vehicle treated mice and in sham animals. Serum ALT is a biomarker for hepatocellular damage. Serum AST is a biomarker for multiorgan failure;

Fig. 18 is a diagram showing C5a blockade by AON-$D21_{vD19}$ reduced lung injury wherein mice were infected by transnasal inoculation of Streptococcus pneumoniae and lung permeability was assessed by measuring the ratio of intravenously injected human serum albumin in bronchoalveolar lavage and plasma by ELISA;

(A) Lung permeability 48 h after infection in mice treated with 20 mg/kg AON-$D21_{vD19}$ or vehicle at t = 0 h and t = 24 h;

(B) 24 h after infection mice were subjected to mechanical ventilation for 6h. Lung permeability at t = 30 h in mice treated with a single dose of 20 mg/kg AON-$D21_{vD19}$ or vehicle 1 h prior to mechanical ventilation; e

Fig. 19 is a diagram showing the efficacy in the murine cecal ligation and puncture model for polymicrobial sepsis wherein sepsis was induced by cecal ligation and puncture (CLP); treatment with AON-D21, AON-$D21_{vD20}$ (1 mg/kg IP, QD) or vehicle was initiated 3 h after CLP procedure; (A) Survival of mice after CLP (n = 10 per group) was followed for 10 days; (B) Serum creatinine levels, (C) blood urea nitrogen (BUN) levels, (D) lactate dehydrogenase (LDH) levels measured 24 h after CLP;

Fig. 20 shows the 2'deoxyribonucleotides that the nucleic acid molecules according to the present invention consist of;

Fig. 21 shows the ribonucleotides that the nucleic acid molecules according to the present invention consist of;

Fig. 22 is a diagram showing PK profiles single dose AON-D21following a single 30 min IV infusion of AON-D21in healthy male participants;

Fig. 23 is a diagram showing plasma PK parameters following a single 30 min IV infusion of AON-D21 in healthy male participants;

Fig. 24 is a diagram showing exploratory pharmacodynamic assessment wherein the inhibitory activity of AON-D21 in clinical samples from a single ascending dose study are shown;

Fig. 25 A-C are diagrams showing PK/PD correlation, wherein the inhibitory activity of AON-D21 in vitro (PD) correlated to AON-D21 plasma concentrations (PK) at the respective timepoints in clinical samples from a single ascending dose study;

Fig. 26 is a diagram showing PK profiles in case of multiple dose of AON-D21 in healthy male participants; (A) 3.0 mg/kg Q2D (dosage regimen: every two days), 4 doses. (B) 0.2 mg/kg Q12h (dosage regimen: every 12 hours or twice a day), 19 doses; dotted lines indicate anticipated elimination profiles;

Fig. 27 is a diagram showing plasma PK parameters following multiple IV infusions of AON-D21 in healthy male participants;

Fig. 28 is a diagram showing exploratory pharmacodynamic assessment wherein inhibitory activity of AON-D21 in clinical samples from a multiple dose study;

Fig. 29 A-C are diagrams showing PK/PD correlation wherein inhibitory activity of AON-D21 in vitro correlated to AON-D21 plasma concentrations (PK) at the respective timepoints in clinical samples from a multiple dose study;

Fig. 30 is a diagram showing the plasma concentration of AON-D21 in the course of the various days based on a PK model for a dosing regimens of 1.6 mg/kg of AON-D21 every second days (Q2D), wherein the PK model was developed on the basis of available clinical PK data as shown in Example 13, to predict which dosing regimens can be used to maintain trough AON-D21 plasma levels ≥ 100 nmol/L throughout a treatment period while at the same time reducing the overall drug exposure of patients;

Fig. 31 is a diagram showing the plasma concentration of AON-D21 in the course of the various days based on a PK model for a dosing regimens of 0.5 mg/kg of AON-D21 every every (QD), wherein the PK model

was developed on the basis of available clinical PK data as shown in Example 13, to predict which dosing regimens can be used to maintain trough AON-D21 plasma levels ≥ 100 nmol/L throughout a treatment period while at the same time reducing the overall drug exposure of patients;

Fig. 32 is a diagram showing the plasma concentration of AON-D21 in the course of the various days based on a PK model for a dosing regimens of 0.2 mg/kg of AON-D21 twie daily (Q12h), wherein the PK model was developed on the basis of available clinical PK data as shown in Example 13, to predict which dosing regimens can be used to maintain trough AON-D21 plasma levels ≥ 100 nmol/L throughout a treatment period while at the same time reducing the overall drug exposure of patients; and

Fig. 33 is a diagram showing the plasma concentration of AON-D21 in the course of the various days based on a PK model for a dosing regimens of 0.12 mg/kg of AON-D21 every eight hour / three times daily (Q8h), wherein the PK model was developed on the basis of available clinical PK data as shown in Example 13, to predict which dosing regimens can be used to maintain trough AON-D21 plasma levels ≥ 100 nmol/L throughout a treatment period while at the same time reducing the overall drug exposure of patients.

## Example 1: Nucleic acid molecules capable of binding human and mouse C5a

Several C5a binding nucleic acid molecules and derivatives thereof were identified: the nucleotide sequences of which are depicted in Figures 1 to 5. The C5a binding nucleic acid molecules were characterized as

a) aptamers, i. e. D-nucleic acid molecules, using a direct pull-down assay (Example 3) and/or a comparative competition pull-down assay (Example 3)
b) L-aptamers, i. e. L-nucleic acid molecules, by surface plasmon resonance measurement (Example 4), and by an *in vitro* assay with cells expressing the human C5a receptor (Example 5). Moreover L-aptamers were tested for in vivo efficacy (Examples 10 to 12). The L-aptamers and aptamers were synthesized as described in Example 2.

The nucleic acid molecules thus generated exhibit slightly different sequences, whereby the sequences can be summarized or grouped as a sequence family.
For definition of ribonucleotide sequence motifs, the IUPAC abbreviations for ambiguous nucleotides are used:

| | | |
|---|---|---|
| S | strong | G or C; |
| W | weak | A or U; |
| R | purine | G or A; |
| Y | pyrimidine | C or U; |
| K | keto | G or U; |
| M | imino | A or C; |
| B | not A | C or U or G; |
| D | not C | A or G or U; |
| H | not G | A or C or U; |
| V | not U | A or C or G; |
| N | all | A or G or C or U |

If not indicated to the contrary, any nucleic acid sequence or sequence of stretches, respectively, is indicated in the 5' -> 3' direction.
For differentiation between the 2'-deoxyribonucleotides and the ribonucleotides the following abbreviations are used:
For 2'-deoxyribonucleotides: dG, dC, dT, dA and dU (see Fig. 21).
For ribonucleotides: G, C, T, U (see Fig. 22).
As depicted in Figures 1 to Fig 5 C5a binding nucleic acid molecules comprise one central stretch of nucleotides defining a potential C5a binding motif, whereby Fig. 1 shows the different sequences of the sequence family, the Figs. 2 to 5 show derivatives of the nucleic acid molecule NOX-D19001 including NOX-D20001 (also referred to as NOX-D19001-6x-DNA-020, Fig. 4A) and NOX-D21001 (also referred to as NOX-D19001-2dU-1dC-020, Fig. 5).
In general, C5a binding nucleic acid molecules comprise at the 5'-end and the 3'-end terminal stretches of nucleotides: the first terminal stretch of nucleotides and the second terminal stretch of nucleotides. The first terminal stretch of nucleotides and the second terminal stretch of nucleotides can hybridize to each other, whereby upon hybridization a double-stranded structure is formed. However, such hybridization is not necessarily given in the molecule

*in vivo* and *in vitro.*

The three stretches of nucleotides of C5a binding nucleic acid molecules - the first terminal stretch of nucleotides, the central stretch of nucleotides and the second terminal stretch of nucleotides - are arranged to each other in 5' $\rightarrow$ 3'-direction: the first terminal stretch of nucleotides - the central stretch of nucleotides - the second terminal stretch of nucleotides. However, alternatively, the first terminal stretch of nucleotides, the central stretch of nucleotides and the second terminal stretch of nucleotides are arranged to each other in 5' $\rightarrow$ 3'-direction: the second terminal stretch of nucleotides - the central stretch of nucleotides - the first terminal stretch of nucleotides.

The sequences of the defined stretches may be different between the C5a binding nucleic acid molecules which influences the binding affinity to C5a. Based on binding analysis of the different C5a binding nucleic acid molecules the central stretch of nucleotides and their nucleotide sequences as described in the following are individually and more preferably in their entirety essential for binding to human C5a.

The C5a binding nucleic acid molecules according to the present invention as shown in Fig. 1 consist of ribonucleotides and are shown in Figs. 1 to 5. The C5a binding nucleic acid molecule 274-H6-002 was tested as aptamer in a comparative competition pull-down assays (for protocol see example 3) vs. C5a binding nucleic acid 274-D5-002. C5a binding nucleic acid molecule 274-H6-002 showed weaker binding affinity in comparison to C5a binding nucleic acid molecule 274-D5-002. The C5a binding nucleic acid molecules 274-B5-002, 274-D5-002, 274-C8-002, 274-C8-002-G14 (= NOX-D19001), 274-C5-002 and 274-G6-002 were tested as L-aptamers for their ability to bind human and mouse C5a by plasmon resonance measurement (see Example 4, Fig. 1).

C5a binding nucleic acid molecule 274-C8-002-G14 (= NOX-D19001) shows the best binding affinity with a $K_D$ of 0.3 nM for mouse C5a and with a $K_D$ of 1.38 nM for human C5a (Fig. 1).

The C5a binding nucleic acid molecules 274-B5-002, 274-D5-002, 274-C8-002, 274-C8-002-G14 (= NOX-D19001), 274-C5-002, 274-G6-002 and 274-H6-002 share the sequence
5' AUGUGGUGKUGARGGGHUGUKGGGUGUCGACGCA 3' [SEQ ID NO: 69],
wherein G, A, U, C, H, K, and R are ribonucleotides.

The C5a binding nucleic acid molecules 274-C8-002, 274-C8-002-G14 (= NOX-D19001) and 274-C5-002 showed the best binding affinity to C5a and comprise the following sequences for the central stretch:

a) 274-C8-002: 5' AUGUGGUGUUGAAGGGUUGUUGGGUGUCGACGCA 3'[SEQ ID NO: 70],
b) 274-C8-002-G14: 5' AUGUGGUGGUGAAGGGUUGUUGGGUGUCGACGCA 3' [SEQ ID NO: 71],
c) 274-C5-002: 5' AUGUGGUGGUGAGGGGUUGUGGGGUGUCGACGCA 3' [SEQ ID NO: 72], wherein d G, A, U and C are ribonucleotides.

The inventors surprisingly showed that the binding affinity of C5a binding nucleic acid molecule NOX-D19001 was improved by replacing ribonucleotides by 2'-deoxyribonucleotides within the sequence of the central stretch of nucleotides and the second terminal stretch of nucleotides. In particular replacing up to six ribonucleotides by 2'-deoxyribonucleotides in the C5a binding nucleic acid molecule NOX-D19001 resulted in improved binding affinity to human C5a by a factor of 3.5. In more detail, the inventors have surprisingly found that

a) replacing one ribonucleotide by one 2'-deoxyribonucleotide at position 4, 11, 12, 25 or 27 in the central stretch of nucleotides of C5a binding nucleic acid molecule NOX-D19001 resulted in improved binding affinity to human C5a in comparison to the binding affinity of C5a binding nucleic acid molecule NOX-D19001 (see Fig. 2; L-aptamers NOX-D19001-D09, NOX-D19001-D16, NOX-D 19001-D 17, NOX-D19001-D30, NOX-D19001-D32);

b) replacing one ribonucleotide by one 2'-deoxyribonucleotide at position 1 in the second terminal stretch of nucleotides of C5a binding nucleic acid molecule NOX-D19001 resulted in improved binding affinity to human C5a in comparison to the binding affinity of C5a binding nucleic acid molecule NOX-D19001 (see Fig. 2; L-aptamers NOX-D19001-D40);

c) replacing two ribonucleotides by two 2'-deoxyribonucleotide at position 4/25, 4/27, or 25/27 in the central stretch of nucleotides of C5a binding nucleic acid molecule NOX-D19001 resulted in improved binding affinity to biotinylated C5a in comparison to the binding affinity of C5a binding nucleic acid molecule NOX-D19001 (see Fig. 3; L-aptamers NOX-D19001-D09-30, NOX-D19001-D09-32, NOX-D19001-D30-32);

d) replacing two ribonucleotides, wherein one ribonucleotide was replaced by one 2'-deoxyribonucleotide at position 1 in the second terminal stretch of nucleotides of C5a binding nucleic acid molecule NOX-D19001 and one ribonucleotide was replaced by one 2'-deoxyribonucleotide at position 4, 25 or 27 in the central stretch of nucleotides of C5a binding nucleic acid molecule NOX-D19001, resulted in improved binding affinity to human C5a in comparison to the binding affinity of C5a binding nucleic acid molecule NOX-D19001 (see Fig. 3; L-aptamers NOX-D19001-D09-40,

NOX-D19001-D30-40, NOX-D19001-D32-40);

e) replacing three ribonucleotides, wherein one ribonucleotide was replaced by one 2'-deoxyribonucleotide at position 1 in the second terminal stretch of nucleotides of C5a binding nucleic acid molecule NOX-D19001 and two ribonucleotides were replaced by two 2'-deoxyribonucleotides at position 4/25, 4/27, 25/27 in the central stretch of nucleotides of C5a binding nucleic acid molecule NOX-D19001, resulted in improved binding affinity to human C5a in comparison to the binding affinity of C5a binding nucleic acid molecule NOX-D19001 (see Fig. 3; L-aptamers , NOX-D19001-D09-30-40, NOX-D19001-D09-32-40, NOX-D19001-D30-32-40);

f) replacing three ribonucleotides by three 2'-deoxyribonucleotide at position 04/25/27 in the central stretch of nucleotides of C5a binding nucleic acid molecule NOX-D19001 resulted in improved binding affinity to biotinylated C5a in comparison to the binding affinity of C5a binding nucleic acid molecule NOX-D19001 (see Fig. 3; L-aptamer NOX-D19001-D09-30-32);

g) replacing four ribonucleotides, wherein one ribonucleotide was replaced by one 2'-deoxyribonucleotide at position 1 in the second terminal stretch of nucleotides of C5a binding nucleic acid molecule NOX-D19001 and three ribonucleotides were replaced by three 2'-deoxyribonucleotides at position 04/25/27 in the central stretch of nucleotides of C5a binding nucleic acid molecule NOX-D19001, resulted in improved binding affinity to human C5a in comparison to the binding affinity of C5a binding nucleic acid molecule NOX-D19001 (see Fig. 3; L-aptamer NOX-D19001-D09-30-32-40);

h) replacing five ribonucleotides, wherein one ribonucleotide was replaced by one 2'-deoxyribonucleotide at position 1 in the second terminal stretch of nucleotides of C5a binding nucleic acid molecule NOX-D19001 and four ribonucleotides were replaced by four 2'-deoxyribonucleotides at position 04/11/25/27 or 04/12/25/27 in the central stretch of nucleotides of C5a binding nucleic acid molecule NOX-D19001, resulted in improved binding affinity to human C5a in comparison to the binding affinity of C5a binding nucleic acid molecule NOX-D19001 (see Fig. 3; L-aptamer NOX-D19001-D09-16-30-32-40, NOX-D 19001-D09-17-30-32-40);

i) replacing six ribonucleotides, wherein one ribonucleotide was replaced by one 2'-deoxyribonucleotide at position 1 in the second terminal stretch of nucleotides of C5a binding nucleic acid molecule NOX-D19001 and five ribonucleotides were replaced by five 2'-deoxyribonucleotides at position 04/11/12/25/27 in the central stretch of nucleotides of C5a binding nucleic acid molecule NOX-D19001, resulted in improved binding affinity to human C5a in comparison to the binding affinity of C5a binding nucleic acid molecule NOX-D19001 (see Fig. 3; L-aptamer NOX-D19001-D09-16-17-30-32-40 = NOX-D19-001-6xDNA).

Based on the data shown that replacing ribonucleotides by 2'-deoxyribonucleotides at several positions of the central stretch of nucleotides of C5a binding nucleic acid molecules lead to improved binding to C5a the central stretch of all tested C5a binding nucleic acid molecules can be summarized in a the following formula
5' AUG$n_1$GGUGKU$n_2$$n_3$RGGGHUGUKGGG$n_4$G$n_5$CGACGCA 3' [SEQ ID NO: 61],

wherein $n_1$ is U or dU, $n_2$ is G or dG, $n_3$ is A or dA, $n_4$ is U or dU, $n_5$ is U or dU

and G, A, U, C, H, K,

and R are ribonucleotides, and dU, dG and dA are 2'-deoxyribonucleotides,

wherein

a) in a preferred embodiment the central stretch of nucleotides comprise the sequence 5' AUG$n_1$GGU-GUU$n_2$$n_3$AGGGUUGUGGGG$n_4$G$n_5$CGACGCA 3' [SEQ ID NO: 62] (see 274-B5-002); or
b) in a preferred embodiment the central stretch of nucleotides comprise the sequence 5' AUG$n_1$GGU-GUU$n_2$$n_3$GGGGUUGUGGGG$n_4$G$n_5$CGACGCA 3' [SEQ ID NO: 63] (see 274-D5-002); or
c) in a preferred embodiment the central stretch of nucleotides comprise the sequence 5' AUG$n_1$GGU-GUU$n_2$$n_3$AGGGUUGUUGGG$n_4$G$n_5$CGACGCA 3' [SEQ ID NO: 64] (see 274-C8-002); or
d) in a preferred embodiment the central stretch of nucleotides comprise the sequence 5' AUG$n_1$GGUG-GU$n_2$$n_3$AGGGUUGUUGGG$n_4$G$n_5$CGACGCA 3' [SEQ ID NO: 65] (see NOX-D19001); or
e) in a preferred embodiment the central stretch of nucleotides comprise the sequence 5' AUG$n_1$GGUG-GU$n_2$$n_3$GGGGUUGUGGGG$n_4$G$n_5$CGACGCA 3' [SEQ ID NO: 66] (see 274-C5-002); or

f) in a preferred embodiment the central stretch of nucleotides comprise the sequence 5' AUG$n_1$GGUGGU$n_2n_3$GGGGAUGUGGGG$n_4$G$n_5$CGACGCA 3' [SEQ ID NO: 67] (see 274-G6-002); or

g) in a preferred embodiment the central stretch of nucleotides comprise the sequence 5' AUG$n_1$GGUGUU$n_2n_3$GGGGCUGUGGGG$n_4$G$n_5$CGACGCA 3' [SEQ ID NO: 68] (see 274-H6-002).

The C5a binding nucleic acid molecules NOX-D19001-D09, NOX-D19001-D16, NOX-D19001-D17, NOX-D19001-D30, NOX-D19001-D32, NOX-D19001-D09-30, NOX-D19001-D09-32, NOX-D19001-D09-40, NOX-D19001-D30-32, NOX-D19001-D30-40, NOX-D19001-D32-40, NOX-D19001-D09-30-32, NOX-D19001-D09-30-40, NOX-D19001-D09-32-40, NOX-D19001-D30-32-40, NOX-D19001-D09-30-32-40, NOX-D19001-D09-16-30-32-40, NOX-D19001-D09-17-30-32-40, NOX-D19001-D09-16-17-30-32-40 (see Figs. 2 and 3) showed the best binding affinity to C5a and comprise the following sequence for the central stretch of nucleotides:

a) 5' AUGdUGGUGGUGAAGGGUUGUUGGGUGUCGACGCA 3' [SEQ ID NO: 73] (see NOX-D19001-D09, NOX-D19001-D09-40); or

b) 5' AUGUGGUGGUdGAAGGGUUGUUGGGUGUCGACGCA 3' [SEQ ID NO: 74] (see NOX-D19001-D16); or

c) 5' AUGUGGUGGUGdAAGGGUUGUUGGGUGUCGACGCA 3' [SEQ ID NO: 75] (see NOX-D19001-D17); or

d) 5' AUGUGGUGGUGAAGGGUUGUUGGGdUGUCGACGCA 3' [SEQ ID NO: 76] (see NOX-D19001-D30, NOX-D19001-D30-40); or

e) 5' AUGUGGUGGUGAAGGGUUGUUGGGUGdUCGACGCA 3' [SEQ ID NO: 77] (see NOX-D19001-D32, NOX-D19001-D32-40); or

f) 5' AUGdUGGUGGUGAAGGGUUGUUGGGdUGUCGACGCA 3' [SEQ ID NO: 78] (see NOX-D19001-D09-30, NOX-D19001-D09-30-40); or

g) 5' AUGdUGGUGGUGAAGGGUUGUUGGGUGdUCGACGCA 3'[SEQ ID NO: 79] (see NOX-D19001-D09-32, NOX-D19001-D09-32-40);

h) 5' AUGUGGUGGUGAAGGGUUGUUGGGdUGdUCGACGCA 3' [SEQ ID NO: 80] (see NOX-D19001-D30-32, NOX-D19001-D30-32-40); or

i) 5' AUGdUGGUGGUGAAGGGUUGUUGGGdUGdUCGACGCA 3'[SEQ ID NO: 81] (NOX-D19001-D09-30-32, NOX-D19001-D09-30-32-40); or

j) 5' AUGdUGGUGGUdGAAGGGUUGUUGGGdUGdUCGACGCA 3' [SEQ ID NO: 82] (see NOX-D19001-D09-16-30-32-40); or

k) AUGdUGGUGGUGdAAGGGUUGUUGGGdUGdUCGACGCA 3' [SEQ ID NO: 83] (see NOX-D19001-D09-17-30-32-40); or

l) 5' AUGdUGGUGGUdGdAAGGGUUGUUGGGdUGdUCGACGCA 3' [SEQ ID NO: 84] (see NOX-D19001-D09-16-17-30-32-40 = NOX-D19001-6xDNA),

wherein G, A, U and C are ribonucleotides, and dG, dA and dU are 2'-deoxyribonucleotides.

The binding affinity of C5a binding nucleic acid molecule NOX-D19001 was significantly improved by replacing one up to six ribonucleotides by 2'-deoxyribonucloetides as determined by surface plasmon resonance measurement and examplarily shown for the C5a binding nucleic acids NOX-D19001-D09 and NOX-D19001-D09-16-17-30-32-40 (also referred to as NOX-D19001-6xDNA) (Fig. 6):

NOX-D19001: $K_D$ of 1.38 nM,
NOX-D19001-D09: $K_D$ of 709 pM,
NOX-D19001-D09-16-17-30-32-40: $K_D$ of 361 pM.

NOX-D19001-6xDNA comprises a central stretch of nucleotides with five 2'-deoxyribonucleotides instead of ribonucleotides, a first terminal stretch of nucleotides with five ribonucleotides and a second terminal stretch of nucleotides with four ribonucleotides and one 2'-deoxyribonucleotide. Surprisingly, the inventors could show that the first and the second terminal stretch of nucleotides can be truncated without reduction in affinity to four or three nucleotides. As shown herein, the first and the second terminal stretch of nucleotides of NOX-D19001-6xDNA could be truncated from five to three nucleotides (see NOX-D19001-6xDNA-020 also referred to as NOX-D20001) while retaining affinity (Fig. 4A).

Fig. 4 demonstrates the successful combination of ribonucleotide-to-2'-deoxyribonucleotide substitution and truncation: The mother molecule of NOX-D19001-6xDNA and NOX-D19001-6xDNA-020 (also referred to as NOX-D20001), NOX-D19001, consisting of ribonucleotides and a first and a second terminal stretch of nucleotides with five nucleotides each has a binding affinity ($K_D$) of 1.38 nM. After six ribonucleotide-to-2'-deoxyribonucleotide substitutions (leading to NOX-D19001-6xDNA) and truncation to a first and a second terminal stretch of nucleotides with three nucleotides (leading to NOX-D19001-6xDNA-020, also referred to as NOX-D20001) the binding affinity for human C5a was improved by a more than factor four (NOX-D20001, $K_D$ of 0.3 nM). Truncation of the first or the second stretch

of nucleotides to one nucleotide led to reduced activity, but such molecules still bind to C5a with $K_D$'s lower than 10 nM (see Fig. 4A, 4B)

Another example for the successful substitution of ribonucleotides by 2'-deoxyribonucleotides is shown in Fig. 5. Molecule NOX-D19001-020 is a truncated derivative of NOX-D19001 and has a $K_D$ of 11.3 nM (see Fig. 5) instead of 1.38 nM as determined for NOX-D19001 (see Fig. 1 and 2). Both molecules comprise the identical central stretch of ribonucleotides, but NOX-D19001-020 comprises of a first terminal stretch of only three instead of five ribonucleotides and a second terminal stretch of only three instead of five ribonucleotides. By substitution of two or three ribonucleotides by 2'-deoxyribonucleotides in the central stretch of nucleotides and optionally of one ribonucleotide by 2'-deoxyribonucleotide in the second terminal stretch of nucleotides the binding affinity of NOX-D19001-020 can be improved by a factor of more than 10 (see Fig. 5, NOX-D19001-2xDNA-020, NOX-D19001-3xDNA-020, NOX-D19001-2dU-1dC-020 also referred to as NOX-D21001, NOX-D19001-3dU-1dC-020).

Taken together, the first and the second terminal stretches of C5a binding nucleic acid molecules comprise one, two, three, four or five nucleotides (Fig. 1 to Fig. 5), whereby the stretches optionally hybridize with each other, whereby upon hybridization a double-stranded structure is formed. This double-stranded structure can consist of one to five basepairs. However, such hybridization is not necessarily given in the molecule.

Analyzing the first terminal stretch of nucleotides and the second terminal stretch of nucleotides of all tested C5a binding nucleic acid molecules the generic formula for the first terminal stretch of nucleotides is 5' $Z_1Z_2Z_3Z_4$G 3' and the generic formula for the secondterminal stretch of nucleotides is 5' $Z_5Z_6Z_7Z_8$ $Z_9$ 3',
wherein

Zi is G or absent, $Z_2$ is S or absent, $Z_3$ is S or absent, $Z_4$ is B or absent, $Z_5$ is C or dC, $Z_6$ is V or absent, $Z_7$ is S or absent, $Z_8$ is S or absent, $Z_9$ is C or absent, and

G, S, B, C, V are ribonucleotides, and dC is a 2'-deoxyribonucleotide,

whereby in a first preferred embodiment

a) $Z_1$ is G, $Z_2$ is S, $Z_3$ is S, $Z_4$ is B, $Z_5$ is C or dC, $Z_6$ is V, $Z_7$ is S, $Z_8$ is S, $Z_9$ is C, or
b) $Z_1$ is absent, $Z_2$ is S, $Z_3$ is S, $Z_4$ is B, $Z_5$ is C or dC, $Z_6$ is V, $Z_7$ is S, $Z_8$ is S, $Z_9$ is absent, or
c) $Z_1$ is absent, $Z_2$ is absent, $Z_3$ is S, $Z_4$ is B, $Z_5$ is C or dC, $Z_6$ is V, $Z_7$ is S, $Z_8$ is absent, $Z_9$ is absent, or
d) $Z_1$ is absent, $Z_2$ is absent, $Z_3$ is absent, $Z_4$ is B, $Z_5$ is C or dC, $Z_6$ is V, $Z_7$ is absent, $Z_8$ is absent, $Z_9$ is absent, or
e) $Z_1$ is absent, $Z_2$ is S, $Z_3$ is S, $Z_4$ is B, $Z_5$ is C or dC, $Z_6$ is V, $Z_7$ is S, $Z_8$ is S, $Z_9$ is C, or
f) $Z_1$ is absent, $Z_2$ is absent, $Z_3$ is S, $Z_4$ is B, $Z_5$ is C or dC, $Z_6$ is V, $Z_7$ is S, $Z_8$ is S, $Z_9$ is C, or
g) $Z_1$ is absent, $Z_2$ is absent, $Z_3$ is absent, $Z_4$ is B, $Z_5$ is C or dC, $Z_6$ is V, $Z_7$ is S, $Z_8$ is S, $Z_9$ is C, or
h) $Z_1$ is absent, $Z_2$ is absent, $Z_3$ is absent, $Z_4$ is absent, $Z_5$ is C or dC, $Z_6$ is V, $Z_7$ is S, $Z_8$ is S, $Z_9$ is C, or
i) $Z_1$ is absent, $Z_2$ is absent, $Z_3$ is S, $Z_4$ is B, $Z_5$ is C or dC, $Z_6$ is V, $Z_7$ is S, $Z_8$ is S, $Z_9$ is absent, or
j) $Z_1$ is absent, $Z_2$ is absent, $Z_3$ is absent, $Z_4$ is B, $Z_5$ is C or dC, $Z_6$ is V, $Z_7$ is S, $Z_8$ is S, $Z_9$ is absent, or
k) $Z_1$ is absent, $Z_2$ is absent, $Z_3$ is absent, $Z_4$ is absent, $Z_5$ is C or dC, $Z_6$ is V, $Z_7$ is S, $Z_8$ is S, $Z_9$ is absent, or
l) $Z_1$ is absent, $Z_2$ is S, $Z_3$ is S, $Z_4$ is B, $Z_5$ is C or dC, $Z_6$ is V, $Z_7$ is S, $Z_8$ is absent, $Z_9$ is absent, or
m) $Z_1$ is absent, $Z_2$ is S, $Z_3$ is S, $Z_4$ is B, $Z_5$ is C or dC, $Z_6$ is V, $Z_7$ is absent, $Z_8$ is absent, $Z_9$ is absent, or
n) $Z_1$ is absent, $Z_2$ is absent, $Z_3$ is absent, $Z_4$ is absent, $Z_5$ is C, $Z_6$ is V, $Z_7$ is S, $Z_8$ is absent, $Z_9$ is absent, or
o) $Z_1$ is absent, $Z_2$ is absent, $Z_3$ is absent, $Z_4$ is B, $Z_5$ is C or dC, $Z_6$ is V, $Z_7$ is S, $Z_8$ is absent, $Z_9$ is absent, or
p) $Z_1$ is absent, $Z_2$ is absent, $Z_3$ is S, $Z_4$ is B, $Z_5$ is C or dC, $Z_6$ is V, $Z_7$ is absent, $Z_8$ is absent, $Z_9$ is absent, or
q) $Z_1$ is absent, $Z_2$ is absent, $Z_3$ is S, $Z_4$ is B, $Z_5$ is C or dC, $Z_6$ is absent, $Z_7$ is absent, $Z_8$ is absent, $Z_9$ is absent;

in a second preferred embodiment

a) the first terminal stretch of nucleotides comprises a nucleotide sequence of 5' GCCUG 3' and the second terminal stretch of nucleotides comprises a nucleotide sequence 5' CAGGC , or
b) the first terminal stretch of nucleotides comprises a nucleotide sequence of 5' GCCUG 3' and the second terminal stretch of nucleotides comprises a nucleotide sequence of 5' dCAGGC 3', or
c) the first terminal stretch of nucleotides comprises a nucleotide sequence of 5' CCUG 3' or 5' CUG 3' or 5' UG 3'or 5' G 3', and the second terminal stretch of nucleotides comprises a nucleotide sequence of 5' dCAGGC 3', or
d) the first terminal stretch of nucleotides comprises a nucleotide sequence of 5' GCUG 3' and the second terminal stretch of nucleotides comprises a nucleotide sequence of 5' dCAGC 3', or
e) the first terminal stretch of nucleotides comprises a nucleotide sequence of 5' GCCG 3' and the second terminal stretch of nucleotides comprises a nucleotide sequence of 5' dCGGC 3', or
f) the first terminal stretch of nucleotides comprises a nucleotide sequence of 5' GGCG 3' and the second terminal

stretch of nucleotides comprises a nucleotide sequence of 5' dCGCC 3', or
g) the first terminal stretch of nucleotides comprises a nucleotide sequence of 5' CUG 3' or 5' UG 3'or 5' CG 3' or 5' G 3', and the second terminal stretch of nucleotides comprises a nucleotide sequence of 5' dCAGC 3', or
h) the first terminal stretch of nucleotides comprises a nucleotide sequence of 5' GCUG 3', and the second terminal stretch of nucleotides comprises a nucleotide sequence of 5' dCAC 3' or 5' dCC 3' or 5' dCA 3', or
i) the first terminal stretch of nucleotides comprises a nucleotide sequence of 5' GUG 3' and the second terminal stretch of nucleotides comprises a nucleotide sequence of 5' dCAC 3', or
j) the first terminal stretch of nucleotides comprises a nucleotide sequence of 5' UG 3' and the second terminal stretch of nucleotides comprises a nucleotide sequence of 5' dCA 3', or
k) the first terminal stretch of nucleotides comprises a nucleotide sequence of 5' GCG 3' and the second terminal stretch of nucleotides comprises a nucleotide sequence of 5' dCGC 3', or
l) the first terminal stretch of nucleotides comprises a nucleotide sequence of 5' CG 3' and the second terminal stretch of nucleotides comprises a nucleotide sequence of 5' dCGC 3', or
m) the first terminal stretch of nucleotides comprises a nucleotide sequence of 5' G 3' and the second terminal stretch of nucleotides comprises a nucleotide sequence of 5' dCGC 3', or
n) the first terminal stretch of nucleotides comprises a nucleotide sequence of 5' GCG 3' and the second terminal stretch of nucleotides comprises a nucleotide sequence of 5' dCC 3', or
o) the first terminal stretch of nucleotides comprises a nucleotide sequence of 5' GCG 3' and the second terminal stretch of nucleotides comprises a nucleotide sequence of 5' dC 3', or
p) the first terminal stretch of nucleotides comprises a nucleotide sequence of 5' GG 3' and the second terminal stretch of nucleotides comprises a nucleotide sequence of 5' dCC 3', or
q) the first terminal stretch of nucleotides comprises a nucleotide sequence of 5' GCG 3' and the second terminal stretch of nucleotides comprises a nucleotide sequence of 5' CGC 3'.

In order to prove their functionality, the C5a binding nucleic acid molecules NOX-D19001, NOX-D20001 and NOX-D21001 were synthesized as a L-aptamer comprising an amino-group at its 5'-end. To said amino-modified L-aptamers a 40 kDa PEG-moiety was coupled leading to C5a binding L-aptamers NOX-D19 _(also referred to as AON-$D21_{vD19}$), NOX-D20 _(also referred to as AON-$D21_{vD20}$) and NOX-D21 (also referred to as AON-D21). Synthesis and PEGylation of the L-aptamer is described in Example 2.

The effect of improved binding affinity could be shown for the functionality of C5a binding nucleic acid molecules. As determined by a chemotaxis assay (Example 5), the C5a binding nucleic acid molecule NOX-D19 ($IC_{50}$ = 1.9 nM) exclusively consisting of ribonucleotides was less potent to inhibit the function of human C5a than the NOX-D20, a derivative C5a binding nucleic acid molecule of NOX-D19 comprising six ribonucleotide-to-2'-deoxyribonucleotide substitutions ($IC_{50}$ = 0.28 nM) (Fig. 7).

NOX-D20 showed a very high affinity binding to murine C5a with a dissociation constant $K_D$ of 19 pM, whereas for human C5a a $K_D$ of 299 pM was determined (Example 4, Fig. 8). NOX-D20 inhibits the function of human C5a with an inhibitiory constant $IC_{50}$ of 275 pM as determined by a chemotaxis assay (Example 5, Fig. 7 and 12 A). For stoichiometric reasons, the sensitivity of the chemotaxis assays for mouse C5a is limited to 150 pM due a stimulatory concentration of mouse C5a of 300 pM. Accordingly, for mouse C5a an $IC_{50}$ of 140 pM was measured for NOX-D20 (Example 5, Fig. 12 A).

NOX-D20 showed no binding to C5a from rat or rhesus monkey, indicating very high target specificity (Fig. 8). From the polypeptide sequence alignment of human, mouse, rat and rhesus monkey C5a and the determined specificity it is most likely that the residues Serine16 and Valine28 of human C5a are essential binding residues on C5a (Fig. 11). These are conserved in human and murine C5a but are different in rhesus monkey and rat C5a.

NOX-D21 (also referred to as AON-D21) contains the major affinity-improving sites of NOX-D20 and showed a high affinity to human and murine C5a as shown by Biacore measurement ($K_D$(murine C5a) = 29 pM, $K_D$(human C5a) = 815 pM, $K_D$(human C5) = 413 pM, see Fig. 11). NOX-D21 inhibits the function of human C5a with an inhibitory constant $IC_{50}$ of 476 pM, as determined by a chemotaxis assay (Example 5, Fig. 12B).

*In vivo* a truncated version of C5a is generated by enzymatic cleavage of the C-terminal arginine residue, known as des-Arg-C5a (also referred to as $C5a_{desArg}$). The biological function of des-Arg-C5a is not fully understood but there is evidence that des-Arg-C5a retains leukocyte activating functions. Therefore it was investigated, whether NOX-D20 also bound to des-Arg-C5a. NOX-D20 showed a dose-dependent binding to immobilized recombinant human des-Arg-C5a (Fig. 9).

Detailed kinetic evaluation as described showed that human des-Arg-C5a is bound by NOX-20 with comparable affinity to the full-length human C5a with a dissociation constant of 316 pM and 299 pM, respectively. NOX-D21 bound to mouse and human des-Arg-C5a with dissociation constants of 28 pM and 854 pM, respectively (Fig. 10). Thus even after cleavage of C5a to des-Arg-C5a C5a binding nucleic acid molecules such as NOX-20 and NOX-D21 still bind to their target.

Surprisingly NOX-D20 and NOX-D21 also showed binding to C5 purified from human plasma with an affinity of

164 pM and 413 pM, respectively (Figs. 9 and 10). This phenomenon could not be foreseen. However, it is plausible since C5a is a part of C5 that is cleaved off by the C5 convertase when the complement system is activated or by thrombin or other members of an activated coagulation system. Furthermore, the C5 purified from human plasma carries the native glycosylation structure on asparagine64. Glycosylation had not been present on the murine mirror image C5a polypeptide that was used for identification of NOX-D19, NOX-D20, NOX-D21 and other nucleic acid molecules according to the present invention.

Binding to C5 may influence pharmacokinetics due to the expected low clearance of the large C5 protein and a published plasma concentration of 350-390 nM. Binding to C5 may also influence pharmacodynamics. C5 is bound by C5a binding nucleic acid molecules such as NOX-20 and NOX-D21 and thus C5a is already blocked by the L-aptamer before it is liberated and may lead to receptor signaling.

In preclinical disease models AON-D21 (also referred to as NOX-D21) and two variants of AON-D21 were used: AON-D21$_{vD19}$ (also referred to as NOX-D19) and AON-D21$_{vD20}$ (also referred to as NOX-D20;) as shown in Examples 10 to 12. All three molecules have the same target-binding nucleobase sequence with varying terminal structures and differ in their affinity for the target (Fig. 13). The high molecular similarity and consistent results from *in vitro studies* (see Fig. 13, see Example 1 and 4 to 5) and *in vivo* studies (see examples 10 to 12), are indicative for the transferability of efficacy data between the different variants.

**Example 2: Synthesis and derivatization of aptamers and L-aptamers**

Small scale synthesis

Aptamers (D-RNA nucleic acids) and L-aptamers (L-RNA nucleic acids) were produced by solid-phase synthesis with an ABI 394 synthesizer (Applied Biosystems, Foster City, CA, USA) using 2'TBDMS RNA phosphoramidite chemistry (Damha and Ogilvie, 1993). rA(N-Bz)-, rC(Ac)-, rG(N-ibu)-, and rU- phosphoramidites in the D- and L-configuration were purchased from ChemGenes, Wilmington, MA. Aptamers and L-aptamers were purified by gel electrophoresis.

Large scale synthesis plus modification

L-aptamers were produced by solid-phase synthesis with an AktaPilot100 synthesizer (Amersham Biosciences; General Electric Healthcare, Freiburg) using 2'TBDMS RNA phosphoramidite chemistry (Damha and Ogilvie, 1993). L-rA(N-Bz)-, L-rC(Ac)-, L-rG(N-ibu)-, and L-rU-phosphoramidites were purchased from ChemGenes, Wilmington, MA. The 5'-amino-modifier was purchased from American International Chemicals Inc. (Framingham, MA, USA). Synthesis of the unmodified or 5'-Amino-modified L-aptamer was started on L-riboG, L-riboC, L-riboA or L-riboU modified CPG pore size 1000 Å (Link Technology, Glasgow, UK. For coupling (15 min per cycle), 0.3 M benzylthiotetrazole (CMS-Chemicals, Abingdon, UK) in acetonitrile, and 3.5 equivalents of the respective 0.1 M phosphoramidite solution in acetonitrile was used. An oxidation-capping cycle was used. Further standard solvents and reagents for oligonucleotide synthesis were purchased from Biosolve (Valkenswaard, NL). The L-aptamer was synthesized DMT-ON; after deprotection, it was purified via preparative RP-HPLC (Wincott et al., 1995) using Source15RPC medium (Amersham). The 5'DMT-group was removed with 80% acetic acid (30 min at RT). Subsequently, aqueous 2 M NaOAc solution was added and the L-aptamer was desalted by tangential-flow filtration using a 5 K regenerated cellulose membrane (Millipore, Bedford, MA).

PEGylation of L-aptamers

In order to prolong the L-aptamer's plasma residence time in vivo, L-aptamers was covalently coupled to a 40 kDa polyethylene glycol (PEG) moiety at 5'-end

For PEGylation (for technical details of the method for PEGylation see European patent application EP 1 306 382), the purified 5'-amino modified L-aptamer was dissolved in a mixture of $H_2O$ (2.5 ml), DMF (5 ml), and buffer A (5 ml; prepared by mixing citric acid • $H_2O$ [7 g], boric acid [3.54 g], phosphoric acid [2.26 ml], and 1 M NaOH [343 ml] and adding water to a final volume of 11; pH = 8.4 was adjusted with 1 M HCl).

The pH of the L-aptamer solution was brought to 8.4 with 1 M NaOH. Then, 40 kDa PEG-NHS ester (Jenkem Technology, Allen, TX, USA) was added at 37°C every 30 min in six portions of 0.25 equivalents until a maximal yield of 75 to 85% was reached. The pH of the reaction mixture was kept at 8 - 8.5 with 1 M NaOH during addition of the PEG-NHS ester.

The reaction mixture was blended with 4 ml urea solution (8 M), and 4 ml buffer B (0.1 M triethylammonium acetate in $H_2O$) and heated to 95°C for 15 min. The PEGylated L-aptamer was then purified by RP-HPLC with Source 15RPC medium (Amersham), using an acetonitrile gradient (buffer B; buffer C: 0.1 M triethylammonium acetate in acetonitrile). Excess PEG eluted at 5% buffer C, PEGylated L-aptamer at 10 - 15% buffer C. Product fractions with a purity of >95% (as assessed by HPLC) were combined and mixed with 40 ml 3 M NaOAc. The PEGylated L-aptamer was

desalted by tangential-flow filtration (5 K regenerated cellulose membrane, Millipore, Bedford MA).

**Example 3: Determination of Binding Constants to C5a for Aptamers (Pull-Down Assay)**

**Direct pull-down assay**

The affinity of C5a binding nucleic acids was measured as binding of aptamers (D-RNA nucleic acids) to biotinylated mouse D-C5a (SEQ.ID. 89) in a pull down assay format at 37°C. Aptamers were 5'-phosphate labeled by T4 polynucleotide kinase (Invitrogen) using [$\gamma$-$^{32}$P]-labeled ATP (Hartmann Analytic, Braunschweig, Germany). The specific radioactivity of labeled aptamers was 200,000 - 800,000 cpm/pmol. Assays were carried out in selection buffer (20 mM Tris-HCl pH 7.4; 150 mM NaCl; 5 mM KCl; 1 mM $MgCl_2$; 1 mM $CaCl_2$; 4 U/ml RNase inhibitor (RNaseOUT, Invitrogen); 0.1% [w/vol] Tween-20 supplemented with 50 µg/ml bovine serum albumin (Sigma), and 10 µg/ml non-specific Spiegelmer in order to prevent adsorption of binding partners with surfaces of used plasticware or the immobilization matrix). Aptamers were incubated after de- and renaturation at 0.2-1 nM concentration at 37°C in selection buffer together with varying amounts of biotinylated mouse D-C5a for 3 - 4 hours in order to reach equilibrium at low concentrations. The concentration range of biotinylated mouse D-C5a was set from 640 pM to 10 µM; total reaction volume was 80-200µl. Biotinylated mouse D-C5a and complexes of aptamer and biotinylated mouse D-C5a were immobilized on 5 µl NeutrAvidin Agarose Plus particles (Pierce Biotechnology) which had been preequilibrated with selection buffer. Particles were kept in suspension for 30 min at the 37°C in a thermomixer. Immobilized radioactivity was quantitated in a scintillation counter after detaching the supernatant and appropriate washing. The percentage of binding was plotted against the concentration of biotinylated mouse D-C5a and dissociation constants were obtained by using software algorithms (GraphPad Prism) assuming a 1:1 stoichiometry.

**Competitive pull-down assay**

In order to compare different D-C5a binding nucleic acids, a competitive ranking assay was performed. For this purpose the most affine aptamer available was radioactively labeled (see above) and served as reference. After de- and renaturation it was incubated in selection buffer at 37°C with biotinylated mouse D-C5a at conditions that resulted in around 5 - 10% binding to the biotinylated mouse D-C5a after immobilization and washing on 4µl NeutrAvidin Agarose Plus particles (Pierce Biotechnology) without competition. An excess of de- and renatured non-labeled D-RNA aptamer variants was added at concentrations ranging from 9pM - 400nM with the labeled reference aptamer to parallel binding reactions; total reaction volume was 160-400µl. After 3-4 hour incubation biotinylated mouse D-C5a and complexes of aptamer and biotinylated were immobilized and assays were analysed as described above. The aptamers to be tested competed with the reference aptamer for target binding, thus decreasing the binding signal in dependence of their binding characteristics. The aptamer that was found most active in this assay could then serve as a new reference for comparative analysis of further aptamer variants.

**Example 4: Biacore measurement of L-aptamers binding to C5a and related peptides**

The instrument was set to an enduring temperature of 37°C. The Biacore 2000 instrument was cleaned using the DESORB method before the start of each experiment/ immobilization of a new chip. After docking a maintenance chip, the instrument was consecutively primed with desorb solution 1 (0.5% sodium dodecyl sulphate, SDS), desorb solution 2 (50 mM glycine, pH 9.5) and HBS-EP buffer. Finally, the system was primed with HBS-EP buffer.

For Biacore experiments the C5a-binding L-aptamers were prepared in sterile water and had a concentration of 100 µM.

The CM5 chip was primed with HBS-EP buffer and equilibrated until a stable baseline was observed. The flow cells were immobilized beginning from flow cell 4 to flow cell 1. 100 µl of a 1:1 mixture of 0.4 M EDC and 0.1 M NHS were injected using the QUICKINJECT command at a flow of 10 µl/ min. Activation of the flow cell was monitored by an increase in RU after NHS/ EDC injection (typically 150 - 500 RU for CM5 chips). Solutions of 0.1-1 µg/ ml in 10 mM NaAc pH5.5 for C5a or 10 mM NaAc pH5.5 for human C5 were transferred to a vial and injected using the MANUALINJECT command at a flow of 10 µl/ min. 1000-3000 RU were immobilized the chip. All flow cells were then blocked with an injection of 70 µl of 1 M ethanolamine hydrochloride, pH 8 at a flow of 10 µl/ min. Injection of 30 µl of the regeneration solution (1 M NaCl) at a flow of 30 µl/ min was performed to remove unspecifically bound protein from the chip surface.

Kinetic parameters and dissociation constants were evaluated by a series of L-aptamer injections at concentrations of 2,000 - 1,000 - 500 - 200 - 125 - 62.5 - 31.3 - 15.6 (2x) - 7.8 - 3.9 - 1.95 - 0.98 - 0.48 - 0.24 - 0.12 - 0 nM diluted in running buffer, starting with the lowest concentration. In all experiments, the analysis was performed at 37°C using the Kinject command defining an association time of 240 and a dissociation time of 240 seconds at a flow of 30 µl/ min. The assay was double referenced, whereas FC1 served as (blocked) surface control (bulk contribution of each L-aptamer

concentration) and a series of buffer injections without analyte determined the bulk contribution of the buffer itself. At least one L-aptamer concentration was injected twice to monitor the regeneration efficiency and chip integrity during the experiments. Regeneration was performed by injecting 60 µl of 1M NaCl at a flow of 30 µl/ min. Stabilization time of baseline after each regeneration cycle was set to 1 min at 30 µl/ min.

Data analysis and calculation of dissociation constants (KD) was done with the BIAevaluation 3.1.1 software (BIACORE AB, Uppsala, Sweden) using a modified Langmuir 1:1 stoichiometric fitting algorithm, with a constant RI and mass transfer evaluation with a mass transport coefficient kt of $1 \times 10^7$ [RU/M*s].

## Example 5: Determination of Inhibitory Concentration in a Chemotaxis Assay

### Generation of a cell line expressing the human receptor for C5a

A stably transfected cell line expressing the human receptor for C5a was generated by transfecting BA/F3 mouse pro B cells with a plasmid coding for the human C5a receptor (NCBI accession NM_001736 in pcDNA3.1+). Cells expressing C5aR were selected by treatment with geneticin and tested for expression with RT-PCR and for functionality with chemotaxis assay.

### Chemotaxis assay

The day before the experiment, cells are seeded in a new flask at $0.3 \times 10^6$/ml. For the experiment, cells were centrifuged, washed once in HBH (HBSS, containing 1 mg/ml bovine serum albumin and 20 mM HEPES) and resuspended at $1.33 \times 10^6$ cells/ml. 75 µl of this suspension were added to the upper compartments of a 96 well Corning Transwell plate with 5 µm pores (Costar Corning, #3388; NY, USA). In the lower compartments recombinant human C5a (SEQ.ID. 50) or mouse C5a (SEQ.ID. 54)was pre-incubated together with L-aptamers in various concentrations in 235 µl HBH at 37°C for 20 to 30 min prior to addition of cells. Cells were allowed to migrate at 37°C for 3 hours. Thereafter the insert plate (upper compartments) was removed and 30 µl of 440 µM resazurin (Sigma, Deisenhofen, Germany) in phosphate buffered saline was added to the lower compartments. After incubation at 37°C for 2.5 hours, fluorescence was measured at an excitation wavelength of 544 nm and an emission wavelength of 590 nm.

Fluorescence values are corrected for background fluorescence (no C5a in well) and plotted against L-aptamer concentration. The $IC_{50}$ values are determined with non-linear regression (4 parameter fit) using GraphPad Prism. Alternatively, the value for the sample without L-aptamer (C5a only) is set 100% and the values for the samples with L-aptamer are calculated as per cent of this. The per cent-values are plotted against L-aptamer concentration and the $IC_{50}$-values are determined as described above.

### Determination of the half-maximal effective concentration ($EC_{50}$) for human and mouse C5a

After 3 hours migration of BA/F3/huC5aR cells towards various human C5a or mouse C5a concentrations, dose-response curves for human and mouse C5a were obtained, indicating half effective concentrations ($EC_{50}$) of 0.1 nM for huC5a and 0.3 nM for mC5a. For the experiments on inhibition of chemotaxis by L-aptamers 0.1 nM human C5a and 0.3 nM mouse C5a were used.

## Example 6: C5a binding nucleic acids do not interfere with complement-dependent hemolysis

The ultimate product of the complement cascade is the membrane attack complex (MAC), a pore consisting of C5b-9. MAC is believed to insert into the cytoplasmic membranes of pathogens and kill them by induction of cytoplasmic leakage.

The C5a binding nucleic acids (L-aptamers) presented here have been shown to recognize C5a in the context of C5 (see Example 1, Figs 9 and Figs 10). Therefore, it was investigated whether C5 cleavage to the anaphylatoxin C5a and C5b, which is part of the MAC is inhibited by these the Spiegelmers. This was achieved by using a complement-dependent sheep erythrocyte hemolysis test.

### Methods

Reconstituted human lyophilized serum ('Human Complement Serum' (Sigma Aldrich, Germany) was pre-incubated with PEGylated L-aptamers NOX-D19 (also referred to as AON-$D21_{vD19}$), NOX-D20 (also referred to as AON-$D21_{vD20}$) and NOX-D21 (also referred to as AON-D21) in the range of 10 nM to 10,000 nM in 96-well plates (Nunc-Immuno™ Plate, MaxiSorp Surface™). As a positive control the C5-binding aptamer C5C6 with maximal 2'OMe purine and 2'fluoro pyrimidine substitution (Biesecker *et al.* 1999) (synthesized in house) which inhibits C5 cleavage was used in the

same concentration range. As a control for potential unspecific L-aptamer effects on the assay PEGylated L-aptamers with the reverse sequence of NOX-D19 (also referred to as AON-D21$_{vD19}$) and NOX-D21 (also referred to as AON-D21), revNOX-D19 and revNOX-D21 were included. revNOX-D19 and revNOX-D21 were earlier shown not to inhibit C5a in a Biacore and cell based assays. After 1 hour incubation at 37°C sheep erythrocytes opsonized with rabbit anti-sheep erythrocyte antibodies, known as hemolytic system (Institut Virion/Serion GmbH, Germany) were added to the pre-incubated serum complement inhibitor mixture. Complement is activated via the classical pathway leading to the cleavage of C5 to C5a and C5b. C5b then associates with C6-C9 to form the lytic membrane attack complex (MAC). Sheep erythrocyte hemolysis due to MAC formation was determined 30 min later by a colorimetric measurement after spinning down intact cells. The higher the degree of hemolysis the higher the absorption at 405 nm (measured in a Fluo Star plate reader).

**Results**

The aptamer C5C6 inhibited complement-dependent lysis of the sheep erythrocytes with an $IC_{50}$ of approximately 1 μM (Figure 14 A, B). The L-aptamers tested, namely C5 and C5a binding nucleic acids NOX-D19 (also referred to as AON-D21$_{vD19}$) and NOX-D20 (also referred to as AON-D21$_{vD20}$) (Figure 14 A) and NOX-D21 (also referred to as AON-D21) (Figure 14 B) and the non-C5- or C5a-binding L-aptamers revNOX-D19 (Figure 14 A) and revNOX-D21 (Figure 14 B) did not inhibit hemolysis.

**Discussion**

The C5a binding L-aptamers NOX-D19 (also referred to as AON-D21$_{vD19}$), NOX-D20 (also referred to as AON-D21$_{vD20}$) and NOX-D21 (also referred to as AON-D21) tested were shown not to inhibit MAC formation and are therefore selective antagonists of C5a only. If used as a medicine, this may be advantageous, since inhibition of MAC-formation can compromise the body's defense mechanism to invading pathogens, mainly Gram-negative bacteria.

**Example 7 PNA-HPLC-Assay for determination of AON-D21**

For the determination of AON-D21 an anion exchange (AEX)-HPLC method with fluorescence detection was used that allowed the specific detection of the analyte AON-D21 from human plasma. The assay is based on the specific hybridization of AON-D21 to a complementary PNA-probe (name: utilized PNA-AON-D21-02; sequence: N-terminal Atto425-OO-tgc gtc gac acc caa ca C-terminal), that is N-terminally conjugated with an Atto425 fluorescent dye.

**7.1 Preparation of Stock Solution**

For method validation a reference standard of AON-D21 was dissolved in water. The optical density (OD) was determined after diluting a sample of the AON-D21 solution with TE buffer (10 mM Tris pH 8.0, 1 mM EDTA). The concentration of the stock solutions was calculated from the measured OD values using the extinction coefficient 362912 L·mole-1·cm-1 and the molar weight 13167 g/mol (corresponds to the oligonucleotide part of AON-D21).

7.1.1 Instruments and Materials

- Mettler Toledo Quantos Precision Balance System
- Mettler Toledo C30 KF Titrator with Stromboli Oven
- Glass vials suitable for Stromboli Oven (Schott M27736)
- 10 mL volumetric glass flask

7.1.2 Reagents

- Water (Honeywell, LC/MS grade, Art.-No. 39253)
- Hydranal Coulomat AG-Oven (Honeywell, Art.-No. 34739-500ML)

7.1.3 Method

- ~10 -20 mg of AON-D21 was weighed into two different 10 mL volumetric flasks and solved to 10 mL in water.
- Calculation of the final AON-D21 concentrations for up to three different solutions was performed.
- One reference standard stock solution served for the preparation of calibration standard working solutions; the second was required for preparation of QC-sample working solutions.

**7.2 Preparation of working solutions**

The PNA-HPLC-Assay for AON-D21 was validated using the following concentration levels for calibration standards and QC-samples:

- The validated assay calibration range for human urine started with an LLOQ of 2.0 ng/mL and was linear up the ULOQ at 1,000.0 ng/mL AON-D21.
- Calibration standards used during the method validation were: unspiked, 2.0, 4.0, 20.0, 100.0, 500.0 and 1,000.0 ng/mL.
- The QC-sample concentrations for analysis and used during method validation were 2.0, 6.0, 450.0 and 900.0 ng/mL.

7.2.1 Instruments and Materials

- Glass Vials (e.g. 1.5 mL HPLC autosampler vials; Altmann 22201630) for dilution of AON-D21 solutions

7.2.2 Reagents

- Water (Honeywell, LC/MS grade, Art.-No. 39253)
- Acetonitrile (ACN) (LC/MS grade)
- Tween 20 (for molecular biology; Endonuclease-exonuclease and RNase, none detected)
- 10x Phosphate Bufferend Saline pH 7.4

7.2.3 Method

- Dilution reagent for working solutions was prepared by mixing 5 mL ACN, 0.5 mL of 1% Tween 20, 5 mL 10X PBS and addition of water to a final volume of 50 mL.
- For all steps a mixture of 10% ACN / 0.01% Tween 20 / 1x PBS in water was used as dilution reagent.

**7.3 Preparation of plasma lysate**

7.3.1 Instruments and Materials

- Mastercycler (Eppendorf)
- Twin.tec PCR plate 96 (Eppendorf, Art.-No. 951020389)
- Ultra Clear 12 cap strips (Thermo Scientific, Art.-No. AB-0851)
- Multifuge X3R (Heraeus) or Centrifuge 5810 R (Eppendorf)

7.3.2 Reagents

- Proteinase K 50 mg/mL (Epicentre, Art.-No. MPRK092)
- Water (Honeywell, LC/MS grade, Art.-No. 39253)
- Acetonitrile (ACN) (LC/MS grade)
- Tween 20 (for molecular biology; Endonuclease-exonuclease and RNase, none detected)
- 0.5 M EDTA-Solution pH 8.0 (AppliChem, for molecular biology, Art.-No. A4892,0500)
- 1 M Trizma buffer pH 8.0 (BioPerformance Certified; Sigma-Aldrich, Art.-No. T2694)
- 10X Phosphate Buffered Saline pH 7.4

7.3.3 Method

- non-SDS Buffer was prepared by mixing 0.30 mL 1M Trizma buffer ph8, 0,60 0.5M EDTA-Solution pH8, 0.15 mL Tween20 and addition of water to a final volume of 10 mL.
- For each study sample lysate preparation at first 5 µL Proteinase K solution, 40 µL non-SDS buffer and 25 µL LC/MS Water and 20 µL of 10% ACN / 0.01% Tween 20 / 1x PBS were mixed. Then, 10 µL of the corresponding working solution was added.
- For preparation of calibration and QC-standards at first 10 µL of blank plasma with a mixture of 5 µL Proteinase K, 40 µL nonSDS buffer and 25 µL LC/MS Water was mixed. Then 20 µL of the corresponding working solution was added.

    o Each sample and standard were pipetted on a 96-well plate.

- The 96-well PCR-Plate was sealed with Ultra Clear 12 cap strips and treated at 37°C for 30 min in an Eppendorf Mastercycler.
- The 96-well PCR-Plate was centrifuged for at least 1 min with 3,200 rcf at 4°C and stored on ice.

7.3.3.1 Sample Dilution

- The maximal validated dilution factor was 1000.
- Samples above the ULOQ were diluted with digested human plasma.
- For 5 mL mix: 250 µL Proteinase K, 500 µL blank plasma, 2,000 µL nonSDS Verdau buffer, 1,250 µL LC-MS Water and 1,000 µL "10% ACN / 0.01% Tween 20/ 1 × PBS in water were mixed and treated at 37°C for 30 min.

**7.4 Hybridization**

7.4.1 Instruments and Materials

- Mastercycler (Eppendorf)
- Twin.tec PCR plate 96 (Eppendorf, Art.-No. 951020389)
- Multifuge X3R (Heraeus) or Centrifuge 5810 R (Eppendorf)
- Heat Sealer (Eppendorf, Art.-No.5390 000.024)
- Heat Seal Foil (Eppendorf, Art.-No. 0030-127.854)

7.4.2 Reagents

- PNA-Probe: PNA-AON-D21-02 (sequence: N-terminal Atto425-OO-tgc gtc gac acc caa ca C-terminal, complementary to AON-D21) from Panagene Inc. (Daejeon, South Korea)

    - Stock was solved in 10% ACN and 0.01% Tween 20
    - Working solution was diluted with 10% ACN and 0.01% Tween 20 to a final concentration of 2 µM

- 1 M Trizma buffer pH 8.0 (BioPerformance Certified; Sigma-Aldrich, Art.-No. T2694)

- Water (Honeywell, LC/MS grade, Art.-No. 39253)
- Acetonitrile (ACN) (LC/MS grade)
- 0.5 M EDTA-Solution pH 8.0 (AppliChem, for molecular biology, Art.-No. A4892)

7.4.3 Method

- Hybridization Master Mix :
  - For 16 mL 0.5 mL of 2 μM PNA-Probe Solution, 1.0 mL of 200 mM Trizma buffer pH=8, 2.0 mL of 99.9% ACN, 11.5 mL Water and 1.0 mL 0.5 M EDTA were mixed.
  - Multiples of the above mentioned volumes were prepared, depending on the sample numbers.
- For each sample hybridization, 160 μL of the respective hybridization master mix were mixed with 40 μL of the lysates generated from plasma (corresponds to 2 μL/mg of plasma per injection) and pipetted on a 96-well plate.
- The 96-well PCR-Plate was sealed and centrifuged for 1 min with 3,200 rcf.
- The 96-well PCR-Plate was incubated in Eppendorf Mastercycler for 15 min at 95°C and immediately placed on ice, for at least 5 min (Snap-Cool).
- The 96-well PCR-Plate was centrifuged for at least 1 min at 3,200 rcf.
- The 96-well PCR-Plate was sealed with Heat Seal Foil and put into HPLC autosampler.

**7.5 HPLC Analysis**

7.5.1 Material and Reagents

- Sodium perchlorate monohydrate (Purity >98.0%, Fe-content < 0.0003%)
- Sodium bromide (BioUltra, Purity ≥ 99.5%)
- 1 M Trizma buffer pH 8.0 (BioPerformance Certified; Sigma-Aldrich, Art.-No. T2694)
- Water (purified water TypI)
- Acetonitrile (ACN) (LC/MS grade)
- 0.5 M EDTA-Solution pH 8.0 (AppliChem, for molecular biology, Art.-No. A4892)
- Urea (Roth, p.a grade, > 99.5%)
- HPLC column: Thermo Scientific DNA Pac PA200, 4x250 mm (Art-No. 063000)

7.5.2 Instruments

- Dionex HPLC-System Ultimate 3000 consisting of: Shimadzu Fluorescence Detector RF-20AXS,Ultimate Pump LPG-3400SD, Ultimate Autosampler WPS-3000TSL, Analytical and Ultimate Column Heater TCC-3000RS

7.5.3 Method

- Eluent Buffer A (25 mM Trizma buffer pH=8, 30% Acetonitrile, 1 mM EDTA, 3 M Urea)
- Eluent Buffer B (25 mM Trizma buffer pH=8, 30% Acetonitrile, 1 mM EDTA, 3 M Urea, 600 mM Sodium bromide)
- Eluent Buffer C (10% Acetonitrile, 4 M Sodium perchlorate, 2 M Urea)
- Flow rate: 1 mL/min
- Detection: Excitation @ 436 nm; Emission @ 484 nm; Gain: Medium 16
- Injection of 100 μL of the hybridized sample (corresponds to 2 μL plasma on column)
- Column temperature: 50°C
- Autosampler temperature: 20°C

Gradient table:

| Time [min] | % Eluent A | % Eluent B | % Eluent C | Curve |
|---|---|---|---|---|
| 0.00 | 70 | 30 | 0 | linear |
| 1.00 | 70 | 30 | 0 | linear |
| 10.00 | 55 | 45 | 0 | linear |
| 10.25 | 0 | 0 | 100 | linear |
| 11.25 | 0 | 0 | 100 | linear |
| 11.50 | 70 | 30 | 0 | linear |
| 12.00 | 70 | 30 | 0 | linear |
| 12.25 | 0 | 0 | 100 | linear |
| 13.25 | 0 | 0 | 100 | linear |
| 13.50 | 70 | 30 | 0 | linear |
| 14.00 | 70 | 30 | 0 | linear |
| 14.25 | 0 | 0 | 100 | linear |
| 15.25 | 0 | 0 | 100 | linear |
| 15.50 | 70 | 30 | 0 | linear |
| 19.50 | 70 | 30 | 0 | linear |

### 7.6 Evaluation of HPLC Chromatograms

Data acquisition and integration was performed using the software package Chromeleon (Version 7.2) from Thermo Fisher Scientific.

First all HPLC chromatograms were integrated automatically by the HPLC software and the analyte concentration was calculated against the calibration curve. If more than one replicate was evaluated at each calibration standard level the average value of all replicates was used for the calculation of the calibration curve by the linear regression model.

AON-D21 is a complex molecule that was synthesized in a multi-stage process. Variants of its three dimensional structures and/or impurities from the synthesis, degradation products and metabolites which might be present could therefore lead to complex mixtures that could not be differentiated baseline-separated from each other by chromatography. For this reason, complex peak patterns could occur, of which the integration may not be detected correctly by the HPLC software. Therefore, manual re-integration was essential in such cases.

A summary of the procedure is as follows:

As a general rule, a horizontal baseline was set in the region of the peak/ peak group containing the analyte - from the lowest point before the peak/ peak group to the lowest point after the peak/ peak group. By setting the baseline, no peaks must be cut. In such a case, peaks were separated from the group, if necessary.

Overlapping peaks or peak shoulders were separated by vertical peak splits. Peak splits were set at the visual inflection point of the peak/ peak shoulder, (drop perpendicular integration).

In ambiguous cases - e.g. not distinctly recognizable inflection point, long fronting/ tailing - the Responsible Scientist must decide how to set the peak splits.

In order to ensure an equivalent, analogous integration, the chromatograms of the calibration standards, the QC samples and the study samples were overlaid with chromatograms of other calibration standards, QC samples and study samples - as applicable - with a similar profile and concentration range. If necessary, the integration was adapted, taking into account the above mentioned rules for setting baseline and peak splits.

## Example 8: Pharmacodynamic Assay

### Method Principle

AON-D21 is a PEGylated oligonucleotide (L-RNA/DNA) aptamer that binds to both C5 and C5a and prevents

C5a from binding to its receptor during complement activation. In brief, samples were thawed and spiked to 50 nM rhC5a (recombinant human C5a). Following incubation, the samples were diluted and further incubated with iLite® C5a Assay Ready Cells - genetically engineered cells that express Firefly luciferase (FL) under the control of a C5a responsive promoter and exhibit a dose-dependent increase in firefly luciferase reporter gene activity following binding of human Cb5a and its metabolite C5a des-Arg to its receptor C5aR1. C5a des-Arg is a less potent ligand of C5aR1. The firefly luciferase signal was measured in a luminometer. Inhibitory activity (% inhibition) was determined by normalizing each subject sample to the individual subject's pre-dose and pre-dose spiked with rhC5 samples.

**Equipment**

- Microtiter plate reader Tecan Infinite 200 PRO F Plex, Equipment ID: 541/542
- Microtiter plate shaker Various
- Plate reader software Magellan
- CO2 incubator
- Water bath

**Reagents**

- AON-D21: 1.3 mg/ml = 100 μM
- Control samples
- iLITE®C5a Assay Ready Cells (CS1042, SVAR Life ScienceBM4075 (Cells with a reporter gene responsive to C5a)
- Human heparin plasma pool (from healthy individuals; Lot: 2021-12-20/AZA and 2022-04-28; plasma supplied from Atreide Biosamples; pool prepared by Atreide Biosamples)
- Recombinant human C5a (rhC5a) used as positive control (MBQ27211021; Reconstituted in 1x PBS + 0.125 % BSA according to supplier's recommendation
- HBSS + 1mg/ml BSA + 20 mM HEPES
- DMEM, high glucose,GlutaMAX, pyruvate +10% HI-FBS +1% Pen/Strep
- Dual-Glo® Luciferase Assay System(1) (E2940 (100 mL) or E2980 (10 × 100 mL), Promega)
- Dual Glo Luciferase Reagent
- 1x PBS + 0.125 % BSA
- Assay plate

(1) Dual-Glo Luciferase Assay System is a kit that contains Dual-Glo Luciferase Assay Substrate (E297A), Dual-Glo Luciferase Buffer (E298), Dual-Glo Stop & Glo Substrate (E313), Dual-Glo Stop & Glo Buffer (E314). Dual-Glo Stop & Glo Buffer may precipitate upon freezing and can be resolubilized at room temperature for 3 days, or by heating to 37°C for up to 2 hours with shaking. It should be equilibrated to room temperature before use.

**Control samples**

Inhibition controls

Inhibitor control samples were prepared by spiking a human Na-heparin plasma pool with AON-D21. The plasma was aliquoted and frozen at -60°C to -90°C at least 12 hours prior to analysis. On the day of analysis, inhibition control samples were spiked with rhC5a.

Positive controls

Positive controls (PCs) were prepared on the day of analysis by spiking Na-heparin plasma pool with rhCba. rhC5a concentration serving as low positive control (LPC) and high positive control (HPC) samples were used.

Negative controls

Human Na-heparin plasma pool from healthy individuals were used as a negative control (NC). Buffer used for sample dilution (HBSS + 1 mg/ml BSA + 20 mM Hepes) was used as a blank. NCs were stored at -60°C to -90°C and blank

at < - 18°C.

List of control samples that were used to accept assay runs. Control samples were included in all runs in at least two replicates, run in duplicates (2x2 wells):

| Controls | Description |
|---|---|
| Blank | HBSS + 1 mg/ml BSA + 20 mM Hepes |
| NC | Na-heparin plasma pool |
| LPC | Na-heparin plasma pool, spiked to 50 nM rhCb5a |
| LPC 1+ μM AON-D21 | Na-heparin plasma pool + 1 μM AON-D21, spiked to 50 nM rhCb5a |
| HPC | Na-heparin plasma pool, spiked to 100 nM rhCb5a |

**Study sample preparation and assay**

Small volume, 45 ul, of pre-dose sample of each study subject was spiked to 50 nM rhCb5a, and both spiked and un-spiked pre-dose samples were analysed. All further samples from a subject were spiked to 50 nM rhCSa. Volumes for spiking study samples with rhC5a will be low, 5 μl (10 % of total volume).

Protocol

1. HBSS+1 mg/ml BSA+20 mM Hepes (buffer) and DMEM, high glucose GlutaMAX pyruvate+10% HI-FBS+1% Pen/Strep were brought to RT. rhCSa, controls and samples were brought to wet ice.
2. The controls and samples were thawed rapidly one by one in a water bath set at 37°C.
3. 500 nM and 1000 nM rhCba were prepared in buffer (HBSS+1 mg/ml BSA+20 mM Hepes)
4. Diluted rhCb5a was added to assigned wells in a 0.5 ml dilution plate:

- 5 μl of 500 nM rhC5a to wells assigned for samples or controls spiked to 50 nM rhC5a (wells assigned for LPC)
- 5 μl of 1000 nM rhC5a to wells assigned for controls spiked to 100 nM (wells assigned for HPC)

5 μl HBSS+1 mg/ml BSA+20 mM Hepes were added to i.e. wells assigned for blank controls, NC and un-spiked pre-dose samples.
5. 45 μl of controls and samples were to assigned wells in dilution plate.
6. The dilution plate was shaked at 750 rpm on a microtiter plate shaker 30 +/- 10 sec at RT.
7. The solution was carefully mixed in each well 5-10 times with a pipette.
8. The dilution plate was sealed with plate seal and incubated for 55-70 min at RT.
9. For one vial of iLite® C5a Assay Ready Cells (1 vial per assay plate), 5750 μl DMEM, high glucose, GlutaMAX pyruvate +10% HI-FBS+1% Pen/Strep was transferred to a 15-mL tube for cell suspension. The tube was warmed to 37°C in a water bath.
10. The controls and samples were diuted 1:10 in a dilution plate. 20 μl sample + 180 μl HBSS+1 mg/ml BSA+20 mM Hepes.
11. The solution was carefully mixed in each well 5-10 times with a pipette.
12. 40 μl of the diluted sample was transferred in duplicates to assigned wells in an assay plate (CulturePlate- 96, white opaque 96-well microplate). The plate was covered with the supplied lid.
13. Vial(s) with iLite® C5a Assay Ready Cells were taken out from -60°C to -90°C freezer and put on dry ice (1 vial per assay plate).
14. The iLite® C5a Assay Ready Cells were thawed for approximately 1 min in a 37°C water bath with gentle agitation.
15. The cells were resuspended by slowly pipetting up and down with a pipette set at 125 μl.
16. For each assay plate, 2x125 μl cells were added to pre-warmed 5750 μl DMEM, high glucose, GlutaMAX pyruvate +10% HI-FBS+1% Pen/Strep. The tube was tilt a few times to ensure proper mixture of cells.
17. 40 μl cell suspension was added to assigned wells.
18. The plate was shaked at 750 rpm on a microtiter plate shaker for 1 min +/- 10 sec.
19. The plate was covered with lid and incubated 5 hours + 10 min at 37°C, 5% COz(cell incubator)
20. The Dual-Glo Luciferase Reagent was thawed at RT, and let equilibrate at RT before a application to the assay.
21. Remove The plate was removed from the incubator and left it on the bench to equilibrate 10 +/- 1 min at RT.

22. 80 µl Dual-Glo Luciferase Reagent was added to all wells. The plate was protected from| light with aluminium foil and mixed on a plate shaker at 750 rpm for 10 to 12 min at RT.
23. The firefly luminescence (FL) of plate was read with the method designed for the assay as described in section 8.0.
24. During the reading of plate Dual-Glo Stop & Glo Reagent was prepared by adding a 100 µl Dual-Glo Stop & Glo Substrate to 10 ml Dual-Glo Stop & Glo Buffer.
25. 80 µl Dual-Glo Stop & Glo Reagent was added to all wells. The plate was protected from light with aluminium foil and mixed on a plate shaker at 750 rpm for 10 to 12 min at RT.
26. The renilla luminescence (RL) of plate was read.

**Criteria for an Accepted Run**

These acceptance criteria was determined run acceptance or rejection and was applied in all runs.

Controls:

- The percent coefficient of variation (%CV) from the duplicate determination of the response signal (firefly luminescence, FL) must be < 30%.
- Blank < NC < LPC < HPC
- LPC + 1uM AON-D21 < LPC

**Acceptance Criteria for Study Samples**

Samples were analysed in duplicate (1x2 wells) in each analytical run. The %CV for the duplicate determination were < 30%. If a study sample did not meet acceptance criteria, it was re-analysed.

**STATISTICS AND CALCULATIONS**

Statistics and calculations was performed using Magellan Software, Microsoft Excel, GraphPad Prism and AnalyseIT. The %inhibition of the added rhCba (%I), that was exerted by the drug in a given study sample, was calculated according to the following formula:

$$\%\ Inhibition = \left[1 - \frac{(Sample\ Results\ with\ 50\ nM\ added\ rhC5a) - (Predose\ without\ added\ rhC5a)}{(Predose\ value\ with\ 50\ nM\ added\ rhC5a) - (Predose\ without\ added\ rhC5a)}\right] x\ 100$$

**Reported results**

For sample results below the signal response value of the individual subject's predose sample without added rhC5a (%I > 100), 100 %I was reported.

For sample results above the signal response value of the individual subject's predose sample with added rhCba (%I < 0), 0 %I was reported.

For % inhibition values above or equal to 10 %I was reported without decimals, while values below 10 %I wer reported with 1 decimal, e.g. 5.0 %I.

**Example 10: In vivo study efficacy -The C5a-binding nucleic acid NOX-D19 (also referred to as AON-D21$_{vD19}$) shows efficacy in the murine cecal ligation and puncture model for polymicrobial sepsis.**

The effect of intraperitoneal injections of NOX-D19 (also referred to as AON-D21$_{vD19}$) on the course of polymicrobial sepsis was tested in a rodent cecal ligation and puncture (CLP) model.

**Methods**

***Animal model***

10-12 week old male C57BL/6 mice (Charles River Laboratories, Germany) were used for the study. Peritonitis was surgically induced under light isofluran anesthesia. Incisions were made into the left upper quadrant of the peritoneal

cavity (normal location of the cecum). The cecum was exposed and a tight ligature was placed around the cecum with sutures distal to the insertion of the small bowel (75% were ligated). One puncture wound was made with a 24-gauge needle into the cecum and small amounts of cecal contents were expressed through the wound. The cecum was replaced into the peritoneal cavity and the laparotomy site was closed. 500μl saline was given s.c. as fluid replacement. Sham animals underwent the same procedure except for ligation and puncture of the cecum. Finally, animals were returned to their cages with free access to food and water.

***Study roups***

4 groups (n=6 mice for sham surgery and n=10 mice per group for CLP surgery) were tested: (1) sham surgery with vehicle (saline) treatment, (2) CLP surgery with vehicle treatment, (3) CLP surgery with low dose NOX-D19 (1 mg/kg) treatment and (4) CLP surgery with high dose NOX D19 (10 mg/kg) treatment. The investigators were blinded to the treatment strategy and did not know which compound contains vehicle or verum. Route of administration was i.p. every day for 6 days starting at time of the CLP surgery.

***Survival***

Follow up was 7 days in each group. Mice were monitored daily and Kaplan Meier survival curves were generated using GraphPad Prism 4 software.

***Blood drawing***

Blood samples were obtained under light ether anaesthesia from the cavernous sinus with a capillary prior to surgery (baseline, day -4) and at day 1 after surgery to allow measurement of routine serum markers of acute kidney injury (serum creatinine, and blood urea nitrogen, BUN) and acute liver failure (serum alanin-aminotransferase, serum ALT). The level of aspartate aminotransferase (serum AST) was measured in serum as a marker of multiorgan failure. Measurement of clinical chemistry parameters was performed on an Olympus analyser (AU400).

***Statistics***

Statistical significance was calculated by Student's T-test. For survival Kaplan Meier curves were generated and log rank test for significance was performed. GraphPad Prism 4 software was used.

**Results**

***Survival***

As expected no mortality occurred in animals with sham surgery without CLP (Figure 15). In mice that received CLP surgery and were treated with vehicle only, median survival was 1.5 days NOX-D19 treatment after CLP surgery improved median survival (Figure 15). Mice treated with low dose NOX-D19 (1 mg/kg) showed the longest median survival (5 days, $p < 0.0001$ vs. vehicle). Mice treated with high dose NOX-D19 (10 mg/kg) had a median survival of 3 days which was significantly longer than in vehicle treated mice ($p = 0.0401$) but was not significantly different from low dose NOX-D19 treatment ($p = 0.4875$). 100% of vehicle mice died within 4 days after CLP surgery. 100% and 90% mortality occurred not before 7 days in mice treated with low and high dose NOX-D19, respectively (Figure 15).

**Clinical chemistry**

***Renal function***

The serum creatinine and blood urea nitrogen (BUN) concentration are parameters for renal function. Renal function was assessed before the start of the study (day -4) and on day 1 after CLP surgery.

By day 1 CLP induced a significant increase in serum creatinine levels in vehicle treated mice. Low dose NOX-D19 treatment (1 mg/kg) prevented this increase (Figure 16 A). In mice treated with high dose NOX-D19 (10 mg/kg) a moderate but statistically not significant increase in serum creatinine levels was observed ($p = 0.1873$ vs. vehicle) (Figure 16 A).

BUN (Figure 16 B), which is a more sensitive parameter of renal function than creatinine, was significantly increased at day 1 after CLP surgery in vehicle treated mice. Treatment of mice with low and high dose NOX-D19 significantly suppressed the increase of BUN upon CLP (Figure 16 B).

***Liver** function*

The most reliable marker of hepatocellular injury or necrosis is serum alanine aminotransferase (serum ALT). All groups showed an increase of serum ALT at day 1 after CLP surgery. However, both groups of NOX-D19 treated septic mice demonstrated improved liver function compared to vehicle treated mice (Figure 17 A).

***Multiorgan failure***

The serum level of aspartate aminotransferase (serum AST) (Figure 17 B) was measured as a marker of multiorgan failure since AST has been shown to be elevated in diseases affecting other organs besides liver, such as myocardial infarction, acute pancreatitis, acute hemolytic anemia, severe burns, acute renal disease, musculoskeletal diseases, and trauma.

Similar to ALT, all groups showed an increase of AST levels at day 1 after CLP surgery ($p < 0.001$ vs. sham). However, similar to liver function, both groups of NOX-D19 treated septic mice demonstrated less pronounced AST levels compared to vehicle treated mice (Figure 17 B).

**Example 11: In vivo efficacy study - Lung Injury Following Bacterial Pneumonia and Mechanical Ventilation**

The effect of intraperitoneal injections of AON-D21$_{vD19}$ (also referered to NOX-D19) on the course of pneumonie was tested in a mouse model of Streptococcus pneumoniae-induced pneumonia.

Material and methods

For all experiments, female C57Bl/6N mice (8 to 11 weeks old, weighing 18 to 23 g; Charles River, Germany) were used and randomly assigned to experimental groups.

*Pneumococcal Pneumonia and AON-D21$_{vD19}$ Treatment*

*S. pneumoniae* (serotype 3, strain NCTC7978) was grown to mid-log phase. Mice were anesthetized and transnasally inoculated with 5 × 106 colony-forming units of *S.pneumoniae* in 20 μl sterile phosphate-buffered saline. Sham-infected control mice received 20 μl of sterile phosphate-buffered saline.

In a first pneumonia model, at time of infection (0 h) and 24 h postinfection, mice were intraperitoneally treated with AON-D21$_{vD19}$ 20 mg/kg in 220 μl 5% glucose) or solvent (5% glucose; n = 13 each group). Twenty-four hours (all groups) or 48 h postinfection (only S. *pneumoniae-infected* groups), mice were anesthetized and exsanguinated. A subset of animals (n = 18) was subjected to assessment of specific murine pneumonia symptoms (clinical signs) at time of euthanization (24 h postinfection, sham-infected group; 48 h postinfection, S. *pneumoniae-infected* groups). C5a/C5adesArg concentrations in bronchoalveolar lavage fluid and plasma of *S. pneumoniae*-infected and control mice were quantified by plasmon resonance measurements.

In the second model of pneumonia combined with mechanical ventilation, mice received a single intraperitoneal injection of AON-D21$_{vD19}$ (20 mg/kg in 220 μl 5% glucose) or solvent (5% glucose) 23 h postinfection and were subjected to mechanical ventilation for 6 h starting 24 h postinfection, as described in detail below under "Mechanical Ventilation," when severe pneumonia had developed (n = 11 each group). In both models of pneumonia, mice were randomly assigned to the different groups by simple randomization.

*Mechanical Ventilation*

Twenty-four hours after infection, mice were subjected to mechanical ventilation as previously described. Briefly, mice were anesthetized, and body temperature was maintained at 37°C by a body temperature-controlled heating pad. Mice were tracheotomized, intubated, and ventilated with tidal volume of 12 ml/kg, respiratory rate of 120 per min, fraction of inspiratory oxygen of 0.75, and 2 cm H2O positive end-expiratory pressure. A carotid artery catheter was placed for blood pressure monitoring and infusion of a balanced electrolyte solution. Mice were ventilated for 6 h by using a special rodent ventilation system, which continuously recorded airway pressure, respiratory rate, and tidal volume (flexiVent; Scireq, Canada). All mice survived the protocol. At termination of the experiment, mice were euthanized by exsanguination *via* the carotid artery catheter. "Nonventilated mice" that served as controls were subjected to identical preparation procedures 30 h after infection and were euthanized after 5 min of mechanical ventilation (n = 5 each group).

*Lung Permeability*

Human serum albumin was injected intravenously 60 min (pneumonia alone model) or 90 min (combined model of pneumonia and mechanical ventilation) before lung preparation. The human serum albumin concentration in bronchoalveolar lavage fluid and plasma was determined by enzyme-linked immunosorbent assay. The permeability index was defined as the calculated ratio27,29 of the human serum albumin concentration in bronchoalveolar lavage fluid and plasma ($\times 10^{-3}$).

Results

In a mouse model of Streptococcus pneumoniae-induced pneumonia, AON-D21$_{vD19}$ (also referred to as NOX-D19) reduced lung injury that was assessed by measuring pulmonary vascular permeability (Fig. 18 A). AON-D21$_{vD19}$treatment was associated with less clinical symptoms of severe disease compared to vehicle treated animals. In a complementary model, AON-D21$_{vD19}$ was further able to limit the negative impact of mechanical ventilation on lung integrity (Fig. 18 B). In this model AON-D21$_{vD19}$ was applied not earlier than 23 hours after infection, highlighting its therapeutic potential.

**Example 12: In vivo efficacy study - The improved C5a-binding nucleic acid AON-D21$_{vD20}$ and AON-D21 shows efficacy in the murine cecal ligation and puncture model for polymicrobial sepsis.**

The effect of intraperitoneal injections of AON-D21$_{vD20}$ (also referred to as NOX-D20) and AON-D21 (also referred to as NOX-D21) on the course of polymicrobial sepsis was tested in a rodent cecal ligation and puncture (CLP) model.

**Methods**

***Animal model***

Polymicrobial sepsis was induced in 10-12 week old male C57BL/6 mice (Charles River Laboratories, Germany) as described in Example 10 with 60-75% of the cecum being ligated.

***Survival***

Follow up was 10 days in each group. Mice were monitored daily and Kaplan Meier survival curves were generated using GraphPad Prism 4 software.

***Study groups***

3 groups (n=10 mice per group for CLP surgery) were tested: (1) CLP surgery with vehicle treatment, (2) CLP surgery with daily low dose AON-D21$_{vD20}$ (1 mg/kg) treatment, (3) daily low dose AON-D21 (1 mg/kg) treatment. The investigators were blinded to the treatment strategy and did not know which compound contains vehicle or verum. Route of administration was i.p.

***Clinical chemistry and inflammatory parameters***

Blood samples were obtained as described in Example 10 under light ether anaesthesia from the cavernous sinus with a capillary at day 1 after surgery. Routine serum markers of acute kidney injury (serum creatinine, and blood urea nitrogen, BUN), and endothelial injury (serum lactate dehydrogenase, serum LDH) were measured.

***Statistics***

Statistical significance was calculated by one-way ANOVA and Dunnetts test. For survival long rank test for significance was performed. GraphPad Prism 4 software was used.

**Results**

In a mouse model of abdominal sepsis, AON-D21$_{vD20}$ and AON-D21 reduced multiorgan damage, assessed by measuring serum markers ofkidney injury (creatinine and urea nitrogen; Fig. 19B and Fig. 19C) and general tissue damage

(lactate dehydrogenase; Fig. 19D),. Ten-day survival was increased with AON-D21$_{vD20}$ and AON-D21 treatment initiated 3 hours after sepsis induction (Fig. 19A). In this model, head-to-head comparison of AON-D21 and AON-D21vD20 confirmed the transferability of efficacy data between different AON-D21 variants.

## Example 13: A dosing regime

As described in example 1 AON-D21 binds both C5a and C5 equally well.

The C5a level in the blood of healty indivuals is 1.8 nM (mean concentration; corresponding to 14.7 μg/L) (Zelek et al., 2020). The C5a level in the blood of patients is > 3nM, as, for example, described for COVID-19 patients with 5.2 nM (mean concentration; corresponding to 43.0 μg/L) (Zelek et al., 2020).

The C5 level in the blood of healty indivuals is, compared to the C5a level in both healthy individuals and patients, much higher, namely 455 nM (mean concentration; corresponding to 84.5 μg/L) (Zelek et al. 2020). Interestingly, the level of C5 in COVID-19 patients are unchanged compared to healthy individuals (Zelek et al. 2020).

Because of the above C5 level in healthy indiviuals and patients and the equal binding affinity of AON-D21 to C5a and C5 one had to assume AON-D21 levels ≥ 500 nmol/L were required (based on the assumption that there must be at least more than one molecule AON-D21 per molecule C5) as therapeutic dose for efficacy in humans having a disease based on elevated level by C5a.

However, the data as shown herein surprisingly indicate that trough levels ≥ 100 nmol/L are sufficient for an effective systemic blockade of C5a. This reduction of the anticipated therapeutic dose will allow dose reduction and lower exposure to AON-D21.

### 13.1 Single Ascending Dose Study (SAD Study)

SAD study was a randomized, single-centre, double-blind, placebo-controlled study, where single ascending intravenous doses of AON-D21 were administered to determine PK and pharmacodynamics (PD) in healthy male participants, aged between 22 and 55 years old.

#### 13.1.1 Study Protocol

| | | |
|---|---|---|
| Enrollment: | 40 male healthy participants (age between 18 and 55 years) for the study, for each dose group: | |
| | 6 participants received Investigational Medicinal Product (IMP) | |
| | Dose group 1: | 0.32 mg/kg (mg/kg = mg AON-D21 per kg body weight) |
| | Dose group 2: | 0.9 mg/kg (mg/kg = mg AON-D21 per kg body weight) |
| | Dose group 3: | 2.0 mg/kg (mg/kg = mg AON-D21 per kg body weight) |
| | Dose group 4: | 3.5 mg/kg (mg/kg = mg AON-D21 per kg body weight) |
| | Dose group 5: | 4.5 mg/kg (mg/kg = mg AON-D21 per kg body weight) |
| | 2 participants received placebo | |
| IMP: | AON-D21 solution for injection was a single-use, preservative-free, sterile solution of AON-D21 in 4% mannitol/0.05% EDTA. The medicinal product was filled into glass vials with a 1.5 mL fill volume. Label claim was 10 mg/mL AON-D21 (anhydrous, free acid oligonucleotide) corresponding to 759 μmol/L. | |
| Placebo: | Placebo was a single-use, preservative-free, sterile 5% glucose solution for injection filled into glass vials with a 1.5 mL fill volume. | |
| Administration: | 30 minutes intraveneous infusion, for infusion AON-D21 and placebo were diluted in 5% glucose solution for injection. | |

#### 13.1.2 SAD Study - Clinical Pharmacokinetics

Plasma samples (EDTA plasma prepared from 2 ml blood) were taken at the following timepoints:

| Day | Measurement Timepoint PK |
|---|---|
| D1 | Pre-dose<br>30 min |

(continued)

| Day | Measurement Timepoint PK |
|---|---|
| | 1h<br>2h<br>4h<br>8h<br>12h |
| D2 | 24h |
| D3 | 48h |
| D4 | 72h |
| D8 | 168 h |
| D15 | 336 h |

The plasma samples were stored for further analysis at -70 °C.

Samples were analysed using an extraction-free, fluorescence-enhanced anion exchange high performance liquid chromatography (AEX-HPLC) method validated for the quantification of total AON-D21 in human plasma (see example 7). The lower limit of quantification for AON-D21 in human plasma is 2 ng/mL (0.152 nmol/L).

All participants of the study received AON-D21 or placebo according to protocol. PK profiles are shown in Figure 22. Selected PK parameters from the study are summarized in Figure 23 and were used in Example 14.

PK data showed a dose proportional increase in maximum plasma concentration (Cmax; Cmax: Maximum observed plasma concentration over the sampling interval) from 601 nmol/L in dose group 1 to 7,280 nmol/L in dose group 2. Dose proportionality is reflected by a constant DN Cmax (DN Cmax: Dose-normalized Cmax calculated as Cmax divided by the applied dose (in mg AON D21/kg body weight)) ranging from 1,570 (nmol/L)/(mg/kg) to 1,880 (nmol/L)/(mg/kg). The elimination half-life (t½ 0-48h: Elimination half-life with the first 48 hours after dosing) within the first 48 h dose-dependently increased from 7.56 h in dose group 1 to 14.3 h in dose group 2.

As shown in the next section (section 13.1.3), an AON-D21 plasma level of 100 nM is sufficient for complete inhibition of C5a.

In the case of dose group 5 (4.5 mg AON-D21 per kg body weight), dose group 4 (3.5 mg AON-D21 per kg body weight) and dose group 3 (2.0 mg AON-D21 per kg body weight) it was shown that the AON-D21 plasma level has not fallen below 100 nmol/L (mean concentration) within 48 hours after the single dose was administered (Fig. 22).

In the case of a single dose of 0.9 mg AON-D21 per kg body weight (dose group 2) it was shown that the AON-D21 plasma level has not fallen below 100 nmol/L (mean concentration) within 24 hours after the single dose was administered (Fig. 22).

In the case of a single dose of 0.32 mg AON-D21 per kg body weight (dose group 1) it was shown that the AON-D21 plasma level has not fallen below 100 nmol/L (mean concentration) within 12 hours after the dose administered (Fig. 22).

### 13.1.3 SAD Study - Exploratory Pharmacodynamic Assessment

For the assessment of the pharmacological activity of AON-D21 in clinical blood samples, a cell-based assay was developed using a C5a receptor reporter cell line (iLite® C5a Assay Ready Cells, Wieslab, Malmö, Sweden) that generates a bioluminescence signal proportional to C5a (protocol see example 8).

The assay was designed to assess which AON-D21 plasma levels are required to block increased C5a levels observed in patients, e.g. in response to an infection. The C5a level in the blood of healty indivuals is 1.8 nM (mean concentration; corresponding to 14.7 µg/L). The C5a level in the blood of patients is > 3nM, as, for example, described for COVID-19 patients with a range of 5.2 nM (mean concentration; corresponding to 43.0 µg/L) (Zelek et al., 2020).

To mimic the increased C5a levels observed in patients, plasma samples obtained from healthy participants treated with AON-D21 or placebo were spiked with recombinant C5a to a final concentration of 50 nmol/L recombinant C5a.

Blood samples were collected at the following timepoints:

| Day | Measurement Timepoint |
|---|---|
| D1 | Pre-dose |

(continued)

| Day | Measurement Timepoint |
|---|---|
| | 1h<br>12h |
| D2 | 24h |
| D3 | 48h |
| D4 | 72h |
| D8 | 168 h |

Samples were spiked with C5a (50 nmol/L final concentration) and C5a activity was analysed using the cell-based assay. The pre-dose sample (no AON-D21 present) was analysed both spiked with C5a and not spiked with C5a to define maximum C5a activity and baseline C5a activity, respectively, in each healthy individual.

Results (as shown in Fig. 24 and 25) were normalized to the C5a activity in the pre-dose sample of each individual participant with and without spiked C5a. Pre-dose samples (no AON-D21 present) spiked with C5a (50 nmol/L final concentration) show a high C5a activity, i.e. a high bioluminescence signal, which was defined as no (0%) inhibition.

Pre-dose samples (no AON-D21 present) that were not spiked with C5a show a low C5a activity, i.e. a low bioluminescence signal, that derives from baseline C5a levels (< 3 nmol/L) present in every healthy individual. Because it is the aim of AON-D21 therapy to block C5a activity in patients to levels that would normally be observed in healthy individuals, the baseline C5a activity was defined as complete (100%) inhibition.

In the SAD study, dose- and time-dependent blockade of C5a was observed (Figure 24). All AON-D21 dose groups showed complete inhibition of added C5a at 30 min and 12 h after the start of the infusion. In the two highest dose groups (dose group 4 and 5: 3.5 mg/kg and 4.5 mg/kg) complete inhibition was maintained up to 48h with approximately 80% inhibition at 72 h. For a singe dose of 2.0 mg/kg AON-D21 (dose group 3) complete inhibition was maintained up to 24 h, with > 90% inhibition at 48 h and approximately 50% inhibition at 72 h. For a single dose of 0.9 mg/kg AON-D21 (dose group 2) and 0.32 mg/kg AON-D21 (dose group 1) complete inhibition was maintained less than 24 h with less than 80% inhibition at 24 h and less than 50% inhibition at 48 h. At the follow-up visit one week after dosing, inhibition of the added C5a in all dose groups was at the background levels comparable to placebo participants.

Correlating AON-D21 plasma levels with the *in vitro* inhibitory activity surpringly showed that AON-D21 plasma levels of ≥ 100 nmol/L were sufficient for 100% inhibition of C5a (50 nmol/L added in vitro) (Figure 25 A).

In summary, these data surprisingly showed that an effective systemic blockade of C5a is achieved with trough AON-D21 plasma levels of ≥ 100 nmol/L suggesting that these plasma levels are sufficient for pharmacological efficacy in patients.

Lower AON-D21 plasma levels led to a reduced *in vitro* inhibitory activity. AON-D21 plasma levels of ≥ 90 nmol/L were sufficient for 90% inhibition of C5a (50 nmol/L added in vitro) (Figure 25 B). AON-D21 plasma levels of ≥ 80 nmol/L were sufficient for 80% inhibition of C5a (50 nmol/L added in vitro) (Figure 25 C).

### 13.2 Multiple Dose Study

The Multiple Dose Study was a randomized, single-centre, double-blind, placebo-controlled study, with multiple intravenous dosing of AON-D21 to determine pharmacokinetics (abbr. PK) and pharmacodynamics (abbr.) PD in healthy male participants, between 23 and 51 years of age. Two dose regimens were tested: 3.0 mg/kg every other day (abbr. for every other day: Q2D) and 0.2 mg/kg every 12 hours (abbr. for every 12 hours: Q12h).

#### 13.2.1 Study Protocol

| | |
|---|---|
| Enrollment: | 20 male participants (age between 23 and 51 years) for the study, for each dose group:<br>8 participants received Investigational Medicinal Product (IMP:<br>Dose group 1: 3.0 mg/kg (mg/kg = mg AON-D21 per kg body weight) administered every other day (Q2D), seven doses administered<br>Dose group 2: 0.2 mg/kg (mg/kg = mg AON-D21 per kg body weight) every 12 hours (Q12h), 19 doses administered<br>2 participants received placebo. |

(continued)

| | |
|---|---|
| IMP: | AON-D21 solution for injection was a single-use, preservative-free, sterile solution of AON-D21 in 4% mannitol/0.05% EDTA. The medicinal product was filled into glass vials with a 1.5 mL fill volume. Label claim was 10 mg/mL AON-D21 (anhydrous, free acid oligonucleotide) corresponding to 759 μmol/L. |
| Placebo: | Placebo was a single-use, preservative-free, sterile 5% glucose solution for injection filled into glass vials with a 1.5 mL fill volume. |
| Administration: | 30 min (dose group 1) or 15 min (dose group 2) intraveneous infusion;for infusion AON-D21 and placebo were diluted in 5% glucose solution |

### 13.2.2 Multiple Dose Study - Clinical Pharmacokinetics

Plasma samples (EDTA plasma prepared from 2 ml blood) were taken at the following points of time:

**3.0 mg/kg Q2D (every two days)**

| Day | Dosis | Measurement timepoint |
|---|---|---|
| D1 | Dosis 1 | Pre-dose<br>30 min<br>1h<br>2h<br>4h<br>8h<br>12h |
| D2 | | 24h |
| D3 | Dosis 2 | Pre-dose = trough 30 min |
| D5 | Dosis 3 | Pre-dose = trough 30 min |
| D7 | Dosis 4 | Pre-dose = trough 30 min<br>1h |
| D8 | | 24h |
| D9 | | 48h |
| D10 | | 72h |
| D11 | | 96h |
| D20 | | 312h |
| D27 | | 480h |

**0.2 mg/kg Q12h**

| Day | Dosis | Measurement timepoint |
|---|---|---|
| D1 | Dosis 1 | Pre-dose<br>15 min<br>1h<br>2h<br>4h<br>8h |

(continued)

| Day | Dosis | Measurement timepoint |
|---|---|---|
| | Dosis 2 | 12h = pre-dose = trough 13h (1h post-dose) |
| D2 | Dosis 3<br>Dosis 4 | pre-dose = trough 1h<br>pre-dose = trough 1h |
| D3 | Dosis 5 | pre-dose = trough 1h |
| D5 | Dosis 9 | Pre-dose = trough 1h |
| D7 | Dosis 13 | Pre-dose = trough 1h |
| D9 | Dosis 17<br>Dosis 18 | Pre-dose = trough<br>Pre-dose = trough |
| D10 | Dosis 19 | Pre-dose = trough<br>15 min<br>1h<br>2h<br>4h<br>8h<br>12h |
| D11 | | 24h |
| D12 | | 48h |
| D18 | | 192h |

The plasma samples were stored for further analysis at -70 °C.

Samples were analysed using an extraction-free, fluorescence-enhanced anion exchange high performance liquid chromatography (AEX-HPLC) method validated for the quantification of total AON-D21 in human plasma (see example 7). The lower limit of quantification for AON-D21 in human plasma is 2 ng/mL (0.152 nmol/L).

All participants in dose group 1of the study Multiple Dose Study received 4 doses of 3.0 mg/kg Q2D AON-D21 or placebo and were included in PK analyses. PK profiles are shown in Fig. 26. Selected PK parameters are summarized in Fig. 27.

In dose group 1, the mean Cmax ($C_{max}$: Maximum observed plasma concentration over the sampling interval) increased from 6,420 nmol/L after the first dose to 7,300 nmol/L after the fourth dose. In dose group 2, the mean Cmax increased from 364 nmol/L after the first dose to 504 nmol/L after the last dose. For both dose groups, DN Cmax (DN $C_{max}$: Dose-normalized $C_{max}$ calculated as Cmax divided by the applied dose (in mg/kg)) values were comparable to those observed in the SAD study (Figure 23). Cmax was reached 1h after the start of infusion in dose group 1 and 0.625 h after the start of infusion in dose group 2 (median tmax values). In dose group 1, the mean elimination half-life within the first 48 h after dosing (t½ 0-tau) was 11.5h. In dose group 2, the mean elimination half-life within the first 12 h after dosing (t½ 0-tau) was 6.95h and 6.82 after the first and the last dose, respectively. These values are consistent with the dose-dependent elimination half-lives observed in the SAD study (Figure 23). With doses of 3.0 mg/kg Q2D (dose group 1), AON-D21 Ctrough levels were 356 nmol/L and 525 nmol/L after the first and the last dose, respectively. Lower Ctrough levels of 122 nmol/L and 155 nmol/L after the first and at steady state, respectively, were observed with doses of 0.2 mg/kg Q12h (dose group 2).

In the case of dose group 1 (3.0 mg AON-D21 per kg body weight every other day), it was shown that the AON-D21 plasma level has not fallen below 100 nmol/L (mean concentration) within the treatment period. After the last dosing the AON-D21 plasma level has fallen below 100 nmol/L (mean concentration) within 96 h (Day 11) (Fig. 26).

In the case of dose group 2 (0.2 mg AON-D21 per kg body weight every 12 hours), it was shown that the AON-D21 plasma level has not fallen below 100 nmol/L (mean concentration) within the treatment period. After the last dosing the AON-D21 plasma level has fallen to below 100 nmol/L (mean concentration) between 12 and 24 hours (Fig. 26).

### 13.2.3 Multiple Dose Study - Exploratory Pharmacodynamic Assessment -

For the assessment of the pharmacological activity of AON-D21 in clinical blood samples, a cell-based assay was used as described in section ' 13.1.3 SAD Study - Exploratory Pharmacodynamic Assessment'.

The following measurement timepoints were used:

**3.0 mg/kg Q2D (every two days)**

| Day | Dosis | Measurement timepoint |
|---|---|---|
| D1 | Dosis 1 | Pre-dose<br>1h |
| D2 | | - |
| D3 | Dosis 2 | Pre-dose = trough |
| D5 | | Pre-dose = trough |
| D7 | Dosis 3 | Pre-dose = trough<br>1h |
| D8 | | 24h |
| D9 | | 48h |
| D10 | | 72h |
| D11 | | 96h |
| D20 | | 312h |
| D27 | | - |

**0.2 mg/kg Q12h (every 12 hours)**

| **Day** | **Dosis** | **Measurement timepoint** |
|---|---|---|
| D1 | Dosis 1 | Pre-dose<br>1h |
| | Dosis 2 | 12h = pre-dose = trough |
| D2 | Dosis 3<br>Dosis 4 | 12h = pre-dose = trough<br>12h = pre-dose = trough |
| D3 | Dosis 5 | Pre-dose = trough |
| D5 | Dosis 9 | Pre-dose = trough |
| D7 | Dosis 13 | Pre-dose = trough<br>1h |
| D9 | Dosis 17<br>Dosis 18 | -<br>- |
| D10 | Dosis 19 | 12h = pre-dose = trough<br>1h<br>12h |
| D11 | | 24h |
| D12 | | 48h |
| D18 | | |

In the Multiple Dose Study, analysis of trough samples (see measurement timepoint above) showed that permanent, complete (100%) inhibition of spiked C5a is achieved in both multiple dose regimens, i.e. with 3.0 mg/kg Q2D (dose group 1) as well as with 0.2 mg/kg Q12h (dose group 2) (Figure 28).

After the last dose on Day 7, in dose group 1 (3.0 mg/kg Q2D), 100% inhibition is maintained for another 48h (Day

9) with approximately 90% inhibition after 72 h (Day 10). At Day 20, inhibition of the added C5a was at the background levels comparable to placebo participants (Figure 28).

In dose group 2 (0.2 mg/kg Q12h), 100% inhibition is still present 12 hours after the last dose (Day 10) and decreases to about 50% inhibition within the next 12 hours (24h post-dose). 48 hours after the last dose, inhibition of the added C5a was at the background levels (Figure 28).

Correlating AON-D21 plasma levels with the *in vitro* inhibitory activity surpringly showed that AON-D21 plasma levels of > 100 nmol/L were generally sufficient for 100% inhibition of C5a (50 nmol/L added vitro) (Figure 29 A).

In summary, these data surpringly showed that an effective systemic blockade of C5a is achieved with trough AON-D21 plasma levels of ≥ 100 nmol/L suggesting that these plasma levels are sufficient for pharmacological efficacy in patients.

Lower AON-D21 plasma levels led to a reduced *in vitro* inhibitory activity. AON-D21 plasma levels of ≥ 90 nmol/L were sufficient for 90% inhibition of C5a (50 nmol/L added in vitro) (Figure 29 B). AON-D21 plasma levels of ≥ 80 nmol/L were sufficient for 90% inhibition of C5a (50 nmol/L added in vitro) (Figure 29 C).

## Example 14: Different dosage regimens

As described in Example 13, a minimal AON-D21 plasma or trough AON-D21 plasma level ≥ 100 nmol/L was sufficient for an effective systemic blockade of C5a.

A PK model was developed on the basis of available clinical PK data as shown for the SAD Study (section 13.1) and the dosing 3.0 mg/kg Q2D (every two days) of the Multiple Dose Study (section 13.2) in Example 13, to predict which dosing regimens can be used to maintain minimal or trough AON-D21 plasma levels ≥ 100 nmol/L. For example, the dose group 2 of the Multiple Dose Study (administration of 0.2 mg AON-D21 per kg body weight every 12 hours (Q12h)) was identified by this model. As shown in example 13, the validity of the model was confirmed.

The model is based on (1) the calculation of Cmax values for a selected dose and (2) the calculation minimal or trough AON-D21 concentrations ($C_{trough}$) at the end of dosing interval of 8h, 12h, 24h or 48h by correlating $C_{max}$ to clinically observed AON-D21 plasma concentrations at the given timepoints, as explained below.

(1) Figures 23 and 27 show that $C_{max}$ was proportional to the administered dose of AON-D21. The mean DN $C_{max}$ of the SAD Study (section 13.1) and the dosing 3.0 mg/kg Q2D (every two days) of the Multiple Dose Study (section 13.2) was 1780 (nmol/L)/(mg/kg) and the following equation can be used to determine $C_{max}$ for any selected dose:

$$C_{max} = \text{Dose} \cdot \text{DN } C_{max}$$

*Example: Selected dose 0.5 mg/kg*

$$C_{max} = Dose \cdot DN\ C_{max} = 0.5\ mg/kg \cdot 1{,}780\ (nmol/L)/(mg/kg) = 890\ nmol/L$$

(2) PK data showed a linear correlation between individual AON-D21 $C_{max}$ values and AON-D21 plasma concentrations at 8h, 12h, 24h and 48h sampling timepoints. This correlation is described by the following equations:

8 h timepoint:

$$\log (C_{8h}) = 1.100 \cdot \log (C_{max}) - 0.558.$$

12 h timepoint:

$$\log (C_{12h}) = 1.224 \cdot \log (C_{max}) - 1.105.$$

24 h timepoint:

$$\log (C_{24h}) = 1.452 \cdot \log (C_{max}) - 2.211.$$

48 h timepoint:

$$\log (C_{48h}) = 1.707 \cdot \log (C_{max}) - 3.864.$$

($C_{8h}$, $C_{12h}$, $C_{24h}$, $C_{48h}$: AON-D21 plasma concentration 8h, 12h, 24h, 48h after dosing)

These equations were used to calculate $C_{trough}$ levels at the end of a dosing interval (8h, 12h, 24h, or 48h) from any $C_{max}$.

*Example: Selected dose 0.5 mg/kg*

$$C_{trough}\ at\ 8h = 10^{(1.100 \cdot \log (Cmax) - 0.558)} = 10^{(1.100 \cdot \log (890\ nmol/L) - 0.558)} = 10^{2.69} = 486\ nmol/L.$$

$$C_{trough}\ at\ 12h = 10^{(1.224 \cdot \log (Cmax) - 1.105)} = 10^{(1.224 \cdot \log (890\ nmol/L) - 1.105)} = 10^{2.51} = 320\ nmol/L.$$

$$C_{trough}\ at\ 24h = 10^{(1.452 \cdot \log (Cmax) - 2.211)} = 10^{(1.452 \cdot \log (890\ nmol/L) - 2.211)} = 10^{2.07} = 118\ nmol/L.$$

$$C_{trough}\ at\ 48h = 10^{(1.707 \cdot \log (Cmax) - 3.864)} = 10^{(1.707 \cdot \log (890\ nmol/L) - 3.864)} = 10^{1.17} = 14.8\ nmol/L.$$

The model was further used for calculating $C_{trough}$ values for multiple dosings. For this purpose, repeated dose $C_{max}$ was calculated as follows: $C_{max}$(2nd dose) = $C_{trough}$ (1st dose) + Dose · DN $C_{max}$. $C_{trough}$ calculations were done as described above.

An exponential elimination model was then used to calculate plasma elimination half-lives and to model PK elimination profiles shown in Figures 31-34:

$$C_{\text{trough}} = C_{max} \cdot 0.5^{\Delta t / t_{1/2}}$$

$$t_{1/2} = \Delta t \cdot \left( \frac{\ln 0.5}{\ln\left(\frac{C_{trough}}{C_{max}}\right)} \right)$$

(with $\Delta t$ = (dosing interval) - $t_{max}$).

As described in Example 13, correlating AON-D21 plasma levels with the *in vitro* inhibitory activity surprisingly showed that AON-D21 plasma levels of ≥ 100 nmol/L were sufficient for 100% inhibition of C5a (50 nmol/L added in vitro) (Figure 25 A). AON-D21 plasma levels of ≥ 90 nmol/L were sufficient for 90% inhibition of C5a (50 nmol/L added in vitro) (Figure 25 B). AON-D21 plasma levels of ≥ 80 nmol/L were sufficient for 80% inhibition of C5a (50 nmol/L added in vitro vitro) (Figure 25 C).

The PK model identified the following dosage regimens that provide trough AON-D21 plasma levels of 80 to 300 nmol/L (nmol/L = nM) that are reached after administering the first dose before administering the second dose, defined as 'first dose trough level' herein.

Moreover, the PK model identified the following dosage regimens that provide trough AON-D21 plasma levels of 80 to 300 nmol/L (nmol/L = nM) that are reached after one or more doses corresponding to the dosage regimen, defined as 'steady state trough level' herein.

The following tables show the dose to be used for different dosage regimens (administering a dose of AON-D21 every two days, every day, twice a day or every 8 hours) to reach the minimal trough level (e.g. 80 nM. 90 nM or 100 nM) or the maximum trough level (e.g. 200 nM or 300 nM), wherein the doses were individually calculated for 'first dose trough level' (table row 'first dose trough level') and 'steady state trough level' (table row 'steady state trough level') and summarized in the table as 'trough level' (table row 'trough level'). To achieve 100% inhibition of C5a activity within the treatment period a dosage regimen leading to a minimum trough level of ≥ 100 nmol/L should be used. To minimize potential adverse effect due to the blockage of C5a the a dosage regimen should be selected that leads to low trough level. This is the reason why we have chosen 300 nM as the maximum value for trough AON-D21 plasma level, wherein 300 nM as the maximum value for trough AON-D21 plasma level is preferred.

The following two tables show the minimal and maximal doses of AON-D21 for the dosage regimen of administering AON-D21 every two days to a subject (dosage regimen 'every two days') to reach the minimal trough

level or the maximum trough level. Table 1A shows the values of the doses in mg/kg (mg AON-D21 per kg body weight). Table 1B shows the values of the doses in mmol/kg (nmol AON-D21 per kg body weight).

**dosage regimen: every two days**

Table 1A: Minimal and maximal doses of AON-D21 to reach the minimal trough level or the maximum trough level for the dosage regimen 'every two days' (doses in mg/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [mg/kg]** | **min. dosis [mg/kg]** | **min. dosis [mg/kg]** | **max. dosis [mg/kg]** | **max. dosis [mg/kg]** |
| trough level | 1.30 | 1.40 | 1.50 | 2.30 | 2.90 |
| trough level 'first dose' | 1.35 | 1.45 | 1.60 | 2.30 | 2.90 |
| trough level 'steady state' | 1.30 | 1.40 | 1.50 | 2.10 | 2.70 |

**dosage regimen: every two days**

Table 1B: Minimal and maximal doses of AON-D21 to reach the minimal trough level or the maximum trough level for the dosage regimen 'every two days' (doses in nmol/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [nmol/kg]** | **min. dosis [nmol/kg]** | **min. dosis [nmol/kg]** | **max. dosis [nmol/kg]** | **max. dosis [nmol/kg]** |
| trough level | 98.73 | 106.33 | 113.92 | 174.68 | 220.25 |
| trough level 'first dose' | 102.53 | 110.12 | 121.52 | 174.68 | 220.25 |
| trough level 'steady state' | 98.73 | 106.33 | 113.92 | 159.49 | 205.06 |

The following two tables show the minimal and maximal doses of AON-D21 for the dosage regimen of administering AON-D21 every day (dosage regimen 'every day') to a subject (dosage regimen 'every day') to reach the minimal trough level or the maximum trough level. Table 2A shows the values of the doses in mg/kg (mg AON-D21 per kg body weight). Table 2B shows the values of the doses in nmol/kg (nmol AON-D21 per kg body weight).

**dosage regimen: every day**

Table 2A: Minimal and maximal doses of AON-D21 to reach the minimal trough level or the maximum trough level for the dosage regimen 'every day' (doses in mg/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [mg/kg]** | **min. dosis [mg/kg]** | **min. dosis [mg/kg]** | **max. dosis [mg/kg]** | **max. dosis [mg/kg]** |
| trough level | 0.34 | 0.37 | 0.39 | 0.72 | 0.95 |
| trough level 'first dose' | 0.39 | 0.42 | 0.50 | 0.72 | 0.95 |
| trough level 'steady state' | 0.34 | 0.37 | 0.39 | 0.60 | 0.78 |

**dosage regimen: every day**

Table 2B: Minimal and maximal doses of AON-D21 to reach the minimal trough level or the maximum trough level for the dosage regimen ‘every day’ (doses in nmol/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [nmol/kg]** | **min. dosis [nmol/kg]** | **min. dosis [nmol/kg]** | **max. dosis [nmol/kg]** | **max. dosis [nmol/kg]** |
| trough level | 25.82 | 28.10 | 29.62 | 54.68 | 72.15 |
| trough level ’first dose’ | 29.62 | 31.90 | 37.97 | 54.68 | 72.15 |
| trough level ’steady state’ | 25.82 | 28.10 | 29.62 | 45.57 | 59.24 |

The following two tables show the minimal and maximal doses of AON-D21 for the dosage regimen of administering AON-D21 twice day to a subject (dosage regimen ‘twice a day’) to reach the minimal trough level or the maximum trough level. Table 3A shows the values of the doses in mg/kg (mg AON-D21 per kg body weight). Table 3B shows the values of the doses in nmol/kg (nmol AON-D21 per kg body weight).

**dosage regimen: twice a day**

Table 3A: Minimal and maximal doses of AON-D21 to reach the minimal trough level or the maximum trough level for the dosage regimen ’twice a day’ (doses in mg/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [mg/kg]** | **min. dosis [mg/kg]** | **min. dosis [mg/kg]** | **max. dosis [mg/kg]** | **max. dosis [mg/kg]** |
| trough level | 0.12 | 0.12 | 0.12 | 0.34 | 0.47 |
| trough level ’first dose’ | 0.16 | 0.18 | 0.20 | 0.34 | 0.47 |
| trough level ’steady state’ | 0.12 | 0.13 | 0.14 | 0.22 | 0.30 |

**dosage regimen: twice a day**

Table 3B: Minimal and maximal doses of AON-D21 to reach the minimal trough level or the maximum trough level for the dosage regimen ’twice a day’ (doses in nmol/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [nmol/kg]** | **min. dosis [nmol/kg]** | **min. dosis [nmol/kg]** | **max. dosis [nmol/kg]** | **max. dosis [nmol/kg]** |
| trough level | 9.11 | 9.11 | 9.11 | 25.82 | 35.70 |
| trough level ’first dose’ | 12.15 | 13.67 | 15.19 | 25.82 | 35.70 |
| trough level ’steady state’ | 9.11 | 9.87 | 10.63 | 16.71 | 22.78 |

According to this model, administration of 0.2 mg AON-D21 per kg body weight every 12 hours (Q12h; dose group 2 of the Multiple Dose Study) leads to trough level of 100 nM (‘first dose trough level’) and of 170 nM (’steady state trough level’).

As shown in example 13, the validity of the model was confirmed.

The following two tables show the minimal and maximal doses of AON-D21 for the dosage regimen of administering AON-D21 every 8 hours to a subject (dosage regimen ‘every 8 hours’) to reach the minimal trough level or the maximum trough level. Table 4A shows the values of the doses in mg/kg (mg AON-D21 per kg body weight). Table 4B shows the values of the doses in nmol/kg (nmol AON-D21 per kg body weight).

**dosage regimen: every 8 hours**

Table 4A: Minimal and maximal doses of AON-D21 to reach the minimal trough level or the maximum trough level for the dosage regimen ‘every 8 hours’ (doses in mg/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [mg/kg]** | **min. dosis [mg/kg]** | **min. dosis [mg/kg]** | **max. dosis [mg/kg]** | **max. dosis [mg/kg]** |
| trough level | 0.055 | 0.06 | 0.07 | 0.22 | 0.32 |
| trough level ’first dose’ | 0.10 | 0.11 | 0.12 | 0.22 | 0.32 |
| trough level ’steady state’ | 0.055 | 0.06 | 0.07 | 0.11 | 0.15 |

**dosage regimen: every 8 hours**

Table 4B: Minimal and maximal doses of AON-D21 to reach the minimal trough level or the maximum trough level for the dosage regimen ‘every 8 hours’ (doses in nmol/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [nmol/kg]** | **min. dosis [nmol/kg]** | **min. dosis [nmol/kg]** | **max. dosis [nmol/kg]** | **max. dosis [nmol/kg]** |
| trough level | 4.18 | 4.56 | 5.32 | 16.71 | 24.30 |
| trough level ’first dose’ | 7.59 | 8.35 | 9.11 | 16.71 | 24.30 |
| trough level ’steady state’ | 4.18 | 4.56 | 5.32 | 8.35 | 11.39 |

Converting the different dosages of the dosage regimens ‘every 8 hours’ and ‘twice a day’ to a daily dose and considering these dosages together with dosages of the dosage regimen ‘every day’ results in minimal and maximal daily doses of AON-D21 to a subject to reach the minimal trough level or the maximum trough level. Table 5A shows the values of the daily doses in mg/kg (mg AON-D21 per kg body weight). Table 5B shows the values of the daily doses in nmol/kg (nmol AON-D21 per kg body weight).

**Daily dosis**

Table 5A: Minimal and maximal daily doses of AON-D21 to reach the minimal trough level or the maximum trough level (doses in mg/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [mg/kg]** | **min. dosis [mg/kg]** | **min. dosis [mg/kg]** | **max. dosis [mg/kg]** | **max. dosis [mg/kg]** |
| trough level | 0.165 | 0.18 | 0.21 | 0.72 | 0.95 |
| trough level ’first dose’ | 0.30 | 0.33 | 0.36 | 0.72 | 0.95 |
| trough level ’steady state’ | 0.165 | 0.18 | 0.21 | 0.60 | 0.78 |

**Daily dosis**

Table 5B: Minimal and maximal daily doses of AON-D21 to reach the minimal trough level or the maximum trough level (doses in nmol/kg).

| **min. or max. trough level (TL)** | **min. TL: 80 nM** | **min. TL: 80 nM** | **min. TL: 80 nM** | **max. TL: 200 nM** | **max. TL: 300 nM** |
|---|---|---|---|---|---|
| **min. or max. dosis for:** | **min. dosis [nmol/kg]** | **min. dosis [nmol/kg]** | **min. dosis [nmol/kg]** | **max. dosis [nmol/kg]** | **max. dosis [nmol/kg]** |
| trough level | 12.53 | 13.67 | 15.95 | 54.68 | 72.15 |

(continued)

| min. or max. trough level (TL) | min. TL: 80 nM | min. TL: 80 nM | min. TL: 80 nM | max. TL: 200 nM | max. TL: 300 nM |
|---|---|---|---|---|---|
| min. or max. dosis for: | min. dosis [nmol/kg] | min. dosis [nmol/kg] | min. dosis [nmol/kg] | max. dosis [nmol/kg] | max. dosis [nmol/kg] |
| trough level 'first dose' | 22.78 | 25.06 | 27.34 | 54.68 | 72.15 |
| trough level 'steady state' | 12.53 | 13.67 | 15.95 | 45.57 | 59.24 |

If you consider different dosages of the dosage regimens 'every 8 hours', 'twice a day', 'every day' and 'every 2 days' together, this results in minimal and maximal doses of AON-D21 for administering to a subject within 8 to 48 hours. Table 6A shows the values of these minimal and maximal doses of AON-D21 in mg/kg (mg AON-D21 per kg body weight). Table 6B shows the values of these minimal and maximal doses of AON-D21 in nmol/kg (nmol AON-D21 per kg body weight).

**Minimal and maximal doses within 8 - 48 hours**

Table 6A: Minimal and maximal doses of AON-D21 to reach the minimal trough level or the maximum trough level within 8 - 48 hours (doses in mg/kg).

| min. or max. trough level (TL) | min. TL: 80 nM | min. TL: 80 nM | min. TL: 80 nM | max. TL: 200 nM | max. TL: 300 nM |
|---|---|---|---|---|---|
| min. or max. dosis for: | min. dosis [mg/kg] | min. dosis [mg/kg] | min. dosis [mg/kg] | max. dosis [mg/kg] | max. dosis [mg/kg] |
| trough level | 0.055 | 0.06 | 0.07 | 2.30 | 2.90 |
| trough level 'first dose' | 0.10 | 0.11 | 0.12 | 2.30 | 2.90 |
| trough level 'steady state' | 0.055 | 0.06 | 0.07 | 2.10 | 2.70 |

**Minimal and maximal doses within 8 - 48 hours**

Table 6B: Minimal and maximal doses of AON-D21 to reach the minimal trough level or the maximum trough level within 8 - 48 hours (doses in nmol/kg).

| min. or max. trough level (TL) | min. TL: 80 nM | min. TL: 80 nM | min. TL: 80 nM | max. TL: 200 nM | max. TL: 300 nM |
|---|---|---|---|---|---|
| min. or max. dosis for: | min. dosis [nmol/kg] | min. dosis [nmol/kg] | min. dosis [nmol/kg] | max. dosis [nmol/kg] | max. dosis [nmol/kg] |
| trough level | 4.18 | 4.56 | 5.32 | 174.68 | 220.25 |
| trough level 'first dose' | 7.59 | 8.35 | 9.11 | 174.68 | 220.25 |
| trough level 'steady state' | 4.18 | 4.56 | 5.32 | 159.49 | 205.06 |

As an example, if you set a minimum trough level for AON-D21 or an C5a binding agent of ≥ 80 nmol/L and a maximum trough level for or an C5a binding agent of 300 nM, the different dosage regimens would comprise administering to the subject

a) 98.73to 220.25 nmol of the C5a binding agent per kg body weight of the subject every two days,
b) 25.82 to 72.12 nmol of the C5a binding agent per kg body weight of the subject every day,
c) 4.18 to 24.30 nmol of the C5a binding agent per kg body weight of the subject twice a day,
d) 4.175 to 24.288 nmol of the C5a binding agent per kg body weight of the subject three times a day,
e) 1.3 to 2.9 mg of the C5a binding agent per kg body weight of the subject every two days,
f) 0.34 to 0.95 mg of the C5a binding agent per kg body weight of the subject every day,
g) 0.12 to 0.47 mg of the C5a binding agent per kg body weight of the subject twice a day, or
h) 0.055 to 0.32 mg of the C5a binding agent per kg body weight of the subject three times a day.

Over a period of 8 - 48 hours such dosage regimens would lead to

a) a total amount of 4.18 to 22025 nmol of the C5a binding agent per kg bodyweight of the subj ect, or
b) a total amount of 0.055 to 2.9 mg of the C5a binding agent per kg bodyweight of the subject.

When calculating the daily dose, such dosage regimens would lead to

a) a total amount of 12.53 to 72.12 nmol of the C5a binding agent per kg bodyweight of the subject every day, or
b) a total amount of 0.165 to 0.95 mg of the C5a binding agent per kg bodyweight of the subject every day.

Alternatively, a continuous infusion can be used to maintain minimal AON-D21 levels ≥ 100 nmol/L. To establish AON-D21 plasma levels ≥ 100 nmol/L, an initial loading dose of 0.06 mg/kg body weight ist required:

$$C_{max} = \text{Dose} \cdot \text{DN } C_{max} = 0.06 \text{ mg/kg} \cdot 1{,}780 \text{ (nmol/L)/(mg/kg)} = 107 \text{ nmol/L.}$$

The PK model estimates a $C_{trough}$ at 8h of 47 nmol/L:

$$C_{trough} \text{ at 8h} = 10^{(1.100 \cdot \log(\text{Cmax}) - 0.558)} = 10^{(1.100 \cdot \log(107 \text{ nmol/L}) - 0.558)} = 10^{1.67} = 47 \text{ nmol/L.}$$

An exponential elimination can be used to calculate the half-life (t½) from $C_{max}$ and $C_{trough}$:

$$t_{1/2} = \Delta t \cdot \left( \frac{\ln 0.5}{\ln\left(\frac{C_{trough}}{C_{max}}\right)} \right) = 7h \cdot \left( \frac{\ln 0.5}{\ln\left(\frac{47\frac{nmol}{L}}{107\frac{nmol}{L}}\right)} \right) = 7h \cdot \left( \frac{\ln 0.5}{\ln\left(\frac{47\frac{nmol}{L}}{107\frac{nmol}{L}}\right)} \right) = 5.9h$$

with Δt = 8h - $t_{max}$ 7h (for $t_{max}$ refer to Figure 27).

With a half-life of 5.9 h AON-D21 plasma will drop to 95 nmol/L within the first hour after dosing:

$$C_{1h} = C_{max} \cdot 0.5^{\Delta t/t_{1/2}} = 107\frac{nmol}{L} \cdot 0.5^{1h/5.9h} = 95\, nmol/L$$

This is equivalent to an hourly loss of 107 nmol/L - 95 nmol/L = 12 nmol/L. To compensate for this elimination, continuous infusion of 0.007 mg/kg (= [12 nmol/L] / [1780 (nmol/L)/(mg/kg)]) AON-D21 per hour is required (daily dose: 0.16 mg/kg). Expressed in moles, a loading dose of 0.06 mg/kg corresponds to a loading dose of 4.56 nmol/kg, a continuous infusion of 0.007 mg/kg corresponds to a continuous infusion of 0.53 nmol/kg and a daily dose of 0.16 mg/kg corresponds to a daily dose of 12.15 nmol/kg.

To establish AON-D21 plasma levels ≥ 90 nmol/L, an initial loading dose of 0.065mg/kg body weight AON-D21 (=4.94nmol/kg) and a continuous infusion 0.006 mg/kg AON-D21 (0.49 nmol/kg) is required. Such a dosage regimen leads a daily dose AON-D21of 0.14 mg/kg (= 10.63 nmol/kg).

To establish AON-D21 plasma levels ≥ 80 nmol/L, an initial loading dose of 0.045mg/kg body weight AON-D21 (= 3.42 nmol/kg) and a continuous infusion 0.005 mg/kg AON-D21 (0.38 nmol/kg) is required. Such a dosage regimen leads a daily dose AON-D21 of 0.12 mg/kg (= 9.11 nmol/kg).

**Example 15: Comparison of C5a selective inhibitors**

As shown in Example 13 AON-D21 plasma levels ≥ 100 nmol/L are sufficient for an effective systemic blockade of C5a.

For a single dose of 2 mg/kg AON-D21 (Figure 22) and for a dosage regimen of 1.6 mg/kg AON-D21 every two days (Figure 30) it was shown that the AON-D21 plasma level has not fallen below 100 nmol/L within 48 hours after the end of treatment, whereas within approximately 72 hours after the end of treatment the plasma level fell to such an extent that only 50% of C5a's activity was still inhibited (single dose of 2 mg/kg AON-D21; Figure 24).

Other dosage regimen with lower amounts of AON-D21 allows these times to be shortened even further. For example:

For a single dose of 0.9 mg/kg AON-D21 (Figure 22) and for a dosage regimen of 0.5 mg/kg AON-D21 every day (Figure

31) it was shown that the AON-D21 plasma level has not fallen below 100 nmol/L within 24 hours after the end of treatment, whereas within approximately 48 hours after the end of treatment the plasma level fell to such an extent that only less 50% of C5a's activity was still inihibited (single dose of 0.9 mg/kg AON-D21; Figure 24).

For a single dose of 0.32 mg/kg AON-D21 (Figure 22) and for a dosage regimen of 0.2 mg/kg AON-D21 every 12 hours (Figures 26 and 31) it was shown that the AON-D21 plasma level has not fallen below 100 nmol/L within 12 hours after the end of treatment, whereas within between 24 and 48 hours after the end of treatment the plasma level fell to such an extent that only less 50% of C5a's activity was still inihibited (single dose of 0.32 mg/kg AON-D21: Fig. 24; dosage regimen of 0.2 mg/kg AON-D21 every 12 hours: Fig. 28).

If a dosage regimen of with a dose of 0.12 mg/kg AON-D21 was used, it was shown that the AON-D21 plasma level has not fallen below 100 nmol/Lwithin 8 hours after the end of treatment, whereas one can assume that within 24 hours after the end of treatment the plasma level fell to such an extent that only less 50% of C5a's activity will be still inihibited.

This means that if a dosage regimen according to the present invention is used, the AON-D21 plasma levels will fall below 100 nmol/L (≥ 100 nmol/L are sufficient for an effective systemic blockade of C5a) within 8 to 48 hours after end of treatment depending on the dosage regimen used. Moreover, if a dosage regimen according to the present invention is used, 50% of C5a's activity is restored after just 24 to 72 hours after end of treatment.

This short wash-out period is linked to AON-D21 elimnation half-lives of less than 15 hours within the first 12-48 hours after dosing (Figure 23 and 27).

In contrast thereto, the elimination half-life for IgG1, IgG2 and IgG4 antibodies is approx. 18-21 days, which is substantially longer that the half-life of other proteins with similar weight (Ryman and Meibohm, 2017).

Such longer half-life may result in treament-emergent adverse events (TEAEs) as shown in a study using C5a binding antibody vilobelimab for patients with SARS-Co V-2 infection (Vlaar et al., 2022). In this study, the patients with SARS-Co V-2 infection received standard of care and vilobelimab at a dose of 800 mg intraveneously for a maximum of six dosis and observed treament-emergent adverse events (TEAEs) were as follows: pneumonia (38 [21,7%] vs 26 [14,8%]), herpes simplex (11 [6,3%] vs 5 [2,6%], Staphylococcus pneumonia (10 [5,7%] vs 5 [2,6%] and Herpes simplex reactivation (4 [2,3%] vs 2 [1,1%]. It is strongly assumed that the TEAEs result from are a prolonged blockade of C5a after end of treatment due to high terminal half life as known for antibodies in the human body and/or the high dosis of Vilobelimab used (800 mg intraveneously), thereby suppressing or diminishing C5a's positive role in the defense against infections based microbial invasion for a lengthy period of time after end of treatment.

As shown above, AON-D21 having a short half life within the pharmacologically relevant and allowing a dosage and thus a treatment regimen according to the present invention results in significantly fewer side effects.

## References

The complete bibliographic data of the documents recited herein the disclosure of which is incorporated by reference is, if not indicated to the contrary, as follows.


Altschul SF, Gish W, Miller W, Myers EW, Lipman DJ (1990), Basic local alignment search tool. J Mol Biol. 215(3):403-10.

Altschul SF, Madden TL, Schaffer AA, Zhang J, Zhang Z, Miller W, Lipman DJ (1997). Gapped BLAST and PSI-BLAST: a new generation of protein database search programs. Nucleic Acids Res. 25(17):3389-402.

Arumugam TV, Shiels IA, Strachan AJ, Abbenante G, Fairlie DP, Taylor SM (2003) A small molecule C5a receptor antagonist protects kidneys from ischemia/reperfusion injury in rats. Kidney Int 63(1): 134-142

Arumugam TV, Woodruff TM, Stocks SZ, Proctor LM, Pollitt S, Shiels IA, Reid RC, Fairlie DP, Taylor SM (2004) Protective effect of a human C5a receptor antagonist against hepatic ischaemia-reperfusion injury in rats. J Hepatol 40(6) : 934-941

Bekker et al, PLoS One. 2016 Oct 21; 11(10): e 0164646

Bergh K, Iversen OJ, Lysvand H. (1993). Surprisingly high levels of anaphylatoxin C5a des Arg are extractable from psoriatic scales. Arch Dermatol Res 285(3):131-134.

Biesecker G, Dihel L, Enney K, Bendele RA (1999) Derivation of RNA aptamer inhibitors of human complement C5. Immunopharmacology 42(1-3): 219-230.

Bonifati DM, Kishore U. (2007) Role of complement in neurodegeneration and neuroinflammation. Mol Immunol 44(5): 999-1010.

Bosmann M, Ward PA (2012) Role of C3, C5 and anaphylatoxin receptors in acute lung injury and in sepsis. Adv Exp Med Biol 946: 147-159

Breivik T, Gundersen Y, Gjermo P, Taylor SM, Woodruff TM, Opstad PK (2011) Oral treatment with complement factor C5a receptor (CD88) antagonists inhibits experimental periodontitis in rats. J Periodontal Res 46(6): 643-647

Chen M, Daha MR, Kallenberg CG (2010) The complement system in systemic autoimmune disease. J Autoimmun

34(3): J276-286
Copland DA, Hussain K, Baalasubramanian S, Hughes TR, Morgan BP, Xu H, Dick AD, Nicholson LB (2010) Systemic and local anti-C5 therapy reduces the disease severity in experimental autoimmune uveoretinitis. Clin Exp Immunol 159(3) : 303-314
Czermak BJ, Sarma V, Pierson CL, Warner RL, Huber-Lang M, Bless NM, Schmal H, Friedl HP, Ward PA (1999) Protective effects of C5a blockade in sepsis. Nat Med 5(7): 788-792
Damha MJ and Ogilvie KK, Methods in Molecular Biology, Vol. 20 Protocols for oligonucleotides and analogs, ed. S. Agrawal, p. 81-114, Humana Press Inc. (1993)
Ehrnthaller C, Ignatius A, Gebhard F, Huber-Lang M (2011) New Insights of an Old Defense System: Structure, Function, and Clinical Relevance of the Complement System. Mol Med 17(3-4): 317-329
Farkas I, Baranyi L, Liposits ZS, Yamamoto T, Okada H (1998) Complement C5a anaphylatoxin fragment causes apoptosis in TGW neuroblastoma cells. Neuroscience 86(3): 903-911
Fernandez HN, Hugli TE (1978) Primary structural analysis of the polypeptide portion of human C5a anaphylatoxin. Polypeptide sequence determination and assignment of the oligosaccharide attachment site in C5a. J Biol Chem 253(19): 6955-6964
Flierl MA, Rittirsch D, Nadeau BA, Day DE, Zetoune FS, Sarma JV, Huber-Lang MS, Ward PA (2008) Functions of the complement components C3 and C5 during sepsis. FASEB J 22(10): 3483-3490
Fonseca MI, Ager RR, Chu SH, Yazan O, Sanderson SD, LaFerla FM, Taylor SM, Woodruff TM, Tenner AJ (2009) Treatment with a C5aR antagonist decreases pathology and enhances behavioral performance in murine models of Alzheimer's disease. J Immunol 183(2): 1375-1383
Gueler F, Rong S, Gwinner W, Mengel M, Brocker V, Schon S, Greten TF, Hawlisch H, Polakowski T, Schnatbaum K, Menne J, Haller H, Shushakova N (2008) Complement 5a receptor inhibition improves renal allograft survival. J Am Soc Nephrol 19(12): 2302-2312
Huber-Lang M, Sarma VJ, Lu KT, McGuire SR, Padgaonkar VA, Guo RF, Younkin EM, Kunkel RG, Ding J, Erickson R, Curnutte JT, Ward PA (2001) Role of C5a in multiorgan failure during sepsis. J Immunol 166(2): 1193-1199
Huber-Lang MS, Younkin EM, Sarma JV, McGuire SR, Lu KT, Guo RF, Padgaonkar VA, Curnutte JT, Erickson R, Ward PA (2002) Complement-induced impairment of innate immunity during sepsis. J Immunol 169(6): 3223-3231
Jacob A, Hack B, Bai T, Brorson JR, Quigg RJ, Alexander JJ (2010a) Inhibition of C5a receptor alleviates experimental CNS lupus. J Neuroimmunol 221(1-2):46-52
Jacob A, Hack B, Chiang E, Garcia JG, Quigg RJ, Alexander JJ (2010b) C5a alters blood-brain barrier integrity in experimental lupus. FASEB J 24(6):1682-8
Kambas K, Markiewski MM, Pneumatikos IA, Rafail SS, Theodorou V, Konstantonis D, Kourtzelis I, Doumas MN, Magotti P, Deangelis RA, Lambris JD, Ritis KD (2008) C5a and TNF-alpha up-regulate the expression of tissue factor in intra-alveolar neutrophils of patients with the acute respiratory distress syndrome. J Immunol 180(11): 7368-7375
Khan MA, Jiang X, Dhillon G, Beilke J, Holers VM, Atkinson C, Tomlinson S, Nicolls MR (2011) CD4+ T cells and complement independently mediate graft ischemia in the rejection of mouse orthotopic tracheal transplants. Circ Res 109(11): 1290-1301
Klos A, Tenner AJ, Johswich KO, Ager RR, Reis ES, Kohl J (2009) The role of the anaphylatoxins in health and disease. Mol Immunol 46(14): 2753-2766
Klussmann S. (2006). "The Aptamer Handbook - Functional Oligonucleotides and their Applications." Edited by S. Klussmann. WILEY-VCH, Weinheim, Germany, ISBN 3-527-31059-2
Kohl J. (2001) Anaphylatoxins and infectious and non-infectious inflammatory diseases. Mol Immunol 38(2-3):175-187.
Laudes IJ, Chu JC, Sikranth S, Huber-Lang M, Guo RF, Riedemann N, Sarma JV, Schmaier AH, Ward PA (2002) Anti-c5a ameliorates coagulation/fibrinolytic protein changes in a rat model of sepsis. Am J Pathol 160(5) : 1867-1875
Lewis AG, Kohl G, Ma Q, Devaraj an P, Kohl J. (2008) Pharmacological targeting of C5a receptors during organ preservation improves kidney graft survival. Clin Exp Immunol 153(1): 117-126.
Li K, Wang N, Peng Q, Lu B, Ma L, Lambris JD, Sacks S, Zhou W (2012) C5a/C5aR signaling is an important negative regulator of murine nature killer cell homeostasis and effector function. Immunobiology 217(11): 1146
Manderson AP, Botto M, Walport MJ. (2004) The role of complement in the development of systemic lupus erythematosus. Annu Rev Immunol 22 : 431-456
Markiewski MM, DeAngelis RA, Benencia F, Ricklin-Lichtsteiner SK, Koutoulaki A, Gerard C, Coukos G, Lambris JD (2008) Modulation of the antitumor immune response by complement. Nat Immunol.(11): 1225-35
McGinnis S, Madden TL (2004) BLAST: at the core of a powerful and diverse set of sequence analysis tools. Nucleic Acids Res. 32(Web Server issue):W20-5.
McNamara, L.A., et al., High Risk for Invasive Meningococcal Disease Among Patients Receiving Eculizumab (Soliris) Despite Receipt of Meningococcal Vaccine. MMWR Morb Mortal Wkly Rep, 2017. 66(27): p. 734-737;
Min X, Wei L, Li Z, Sacks S, Zhou W, Li K (2012) Negative regulation of human nature killer cells by complement.

Immunobiology 217(11): 1189

Muller-Ladner U, Jones JL, Wetsel RA, Gay S, Raine CS, Barnum SR. (1996) Enhanced expression of chemotactic receptors in multiple sclerosis lesions. J Neurol Sci 144(1-2):135-141.

Needleman & Wunsch (1970) A general method applicable to the search for similarities in the amino acid sequence of two proteins. J Mol Biol. 48(3):443-53.

Nozaki M, Raisler BJ, Sakurai E, Sarma JV, Barnum SR, Lambris JD, Chen Y, Zhang K, Ambati BK, Baffi JZ, Ambati J. (2006) Drusen complement components C3a and C5a promote choroidal neovascularization. Proc Natl Acad Sci U S A 103(7): 2328-2333

Ostrand-Rosenberg S (2008) Cancer and complement. Nat Biotechnol. 26(12):1348-9.

Patel SN, Berghout J, Lovegrove FE, Ayi K, Conroy A, Serghides L, Min-oo G, Gowda DC, Sarma JV, Rittirsch D, Ward PA, Liles WC, Gros P, Kain KC (2008) C5 deficiency and C5a or C5aR blockade protects against cerebral malaria. J Exp Med 205(5): 1133-1143

Pearson & Lipman (1988) Improved tools for biological sequence comparison. Proc. Nat'l. Acad. Sci. USA 85: 2444

Piccolo MT, Wang Y, Sannomiya P, Piccolo NS, Piccolo MS, Hugli TE, Ward PA, Till GO. (1999) Chemotactic mediator requirements in lung injury following skin burns in rats. Exp Mol Pathol 66(3):220-226.

Ryman and Meibohm, Pharmacokinetics of Monoclonal Antibodies, CPT Pharmacometrics Syst Pharmacol, 2017 Sep;6(9):576-588

Ricklin D, Lambris JD. (2007) Complement-targeted therapeutics. Nat Biotechnol 25(11): 1265-1275

Ricklin, D., et al., Complement: a key system for immune surveillance and homeostasis. Nat Immunol, 2010. 11(9): p. 785-97

Riley RD, Sato H, Zhao ZQ, Thourani VH, Jordan JE, Fernandez AX, Ma XL, Hite DR, Rigel DF, Pellas TC, Peppard J, Bill KA, Lappe RW, Vinten-Johansen J. (2000) Recombinant human complement C5a receptor antagonist reduces infarct size after surgical revascularization. J Thorac Cardiovasc Surg 120(2):350-358.

Rittirsch D, Flierl MA, Nadeau BA, Day DE, Huber-Lang M, Mackay CR, Zetoune FS, Gerard NP, Cianflone K, Kohl J, Gerard C, Sarma JV, Ward PA (2008) Functional roles for C5a receptors in sepsis. Nat Med 14(5): 551-557

Schiela, B., et al., Active Human Complement Reduces the Zika Virus Load via Formation of the Membrane-Attack Complex. Front Immunol, 2018. 9: p. 2177

Smith & Waterman (1981) Adv. Appl. Math. 2: 482

Socie, G., et al., Eculizumab in paroxysmal nocturnal haemoglobinuria and atypical haemolytic uraemic syndrome: 10-year pharmacovigilance analysis. Br J Haematol, 2019. 185(2): p. 297-310

Sprong T, Brandtzaeg P, Fung M, Pharo AM, Hoiby EA, Michaelsen TE, Aase A, van der Meer JW, van Deuren M, Mollnes TE (2003) Inhibition of C5a-induced inflammation with preserved C5b-9-mediated bactericidal activity in a human whole blood model of meningococcal sepsis. Blood 102(10): 3702-3710

Tokodai K, Goto M, Inagaki A, Nakanishi W, Ogawa N, Satoh K, Kawagishi N, Sekiguchi S, Nilsson B, Okada N, Okada H, Satomi S (2010) Attenuation of cross-talk between the complement and coagulation cascades by C5a blockade improves early outcomes after intraportal islet transplantation. Transplantation 90(12): 1358-1365

van der Pals J, Koul S, Andersson P, Gotberg M, Ubachs JF, Kanski M, Arheden H, Olivecrona GK, Larsson B, Erlinge D (2010) Treatment with the C5a receptor antagonist ADC-1004 reduces myocardial infarction in a porcine ischemia-reperfusion model. BMC Cardiovasc Disord 10:45

Vlaar, A.P.J., et al., Anti-C5a antibody (vilobelimab) therapy for critically ill, invasively mechanically ventilated patients with COVID-19 (PANAMO): a multicentre, double-blind, randomised, placebo-controlled, phase 3 trial. Lancet Respir Med. 2022 Dec;10(12):1137-1146

Wang Y. (2006) Complementary therapies for inflammation. Nat Biotechnol 24(10): 1224-1226

Ward PA (2010a) The harmful role of c5a on innate immunity in sepsis. J Innate Immun 2(5): 439-445

Ward PA (2010b) Role of C5 activation products in sepsis. ScientificWorldJournal 10: 2395-2402 Wincott F, DiRenzo A, Shaffer C, Grimm S, Tracz D, Workman C, Sweedler D, Gonzalez C, Scaringe S, and Usman N (1995). Synthesis, deprotection, analysis and purification of RNA and ribosomes. Nucleic Acids Res. 23:2677-2684.

Woodruff TM, Arumugam TV, Shiels IA, Reid RC, Fairlie DP, Taylor SM. (2003) A potent human C5a receptor antagonist protects against disease pathology in a rat model of inflammatory bowel disease. J Immunol 171(10):5514-5520.

Woodruff TM, Strachan AJ, Dryburgh N, Shiels IA, Reid RC, Fairlie DP, Taylor SM. (2002) Antiarthritic activity of an orally active C5a receptor antagonist against antigen-induced monarticular arthritis in the rat. Arthritis Rheum 46(9):2476-2485.

Yao YM, Redl H, Bahrami S, Schlag G. (1998) The inflammatory basis of trauma/shock-associated multiple organ failure. Inflamm Res 47(5): 201-210.

Zelek et al, 2020, Complement Inhibition with the C5 Blocker LFG316 in Severe COVID-19, Am J Respir Crit Care Med, Nov 1;202(9):1304-1308

Zheng X, Zhang X, Feng B, Sun H, Suzuki M, Ichim T, Kubo N, Wong A, Min LR, Budohn ME, Garcia B, Jevnikar AM,

Min WP (2008) Gene silencing of complement C5a receptor using siRNA for preventing ischemia/reperfusion injury. Am J Pathol 173(4): 973-980

The features of the present invention disclosed in the specification, the claims, the sequence listing and/or the drawings may both separately and in any combination thereof be material for realizing the invention in various forms thereof.

## Claims

1. A C5a binding agent for use in a method of treating and/or preventing a disease in a subject, wherein the disease is associated with or caused by C5a, wherein the method comprises administering to the subject multiple doses of the C5a binding agent, wherein a dose of the multiple doses is administered to the subject when a level of the C5a binding agent in the blood of the subject is a level of 80 to 300 nM.

2. The C5a binding agent for use of claim 1, wherein the level of the C5a binding agent is a trough level of the C5a binding agent.

3. The C5a binding agent for use of any one of claims 1 to 2, wherein the multiple doses comprise a first dose, at least a second dose and optionally one or more subsequent dose(s), wherein

   the first dose of the multiple doses of the C5a binding agent is administered to the subject at a first point in time in the method of treating and/or preventing a disease when the level of the C5a binding agent in the blood of the subject is a level of 80 to 300 nM,
   the second dose of the C5a binding agent is administered to the subject at a second point in time in the method of treating and/or preventing a disease when the level of the C5a binding agent in the blood of the subject is a level of 80 to 300 nM, and the one or more subsequent doses is/are administered to the subject at one or at more subsequent points in time in the method of treating and/or preventing a disease when the level of the C5a binding agent in the blood of the subject is a level of 80 to 300 nM.

4. The C5a binding agent for use of any of claims 1 to 3, wherein at a level of the C5a binding agent in the blood of the subject of ≥ about 100 nM 100% of an activity of C5a is inhibited, wherein preferably at a level of the C5a binding agent in the blood of the subject of ≥ 100 nM 100% of an activity of C5a in the blood of the subject is inhibited.

5. A C5a binding agent for use in a method for treating and/or preventing a disease in a subject, wherein the disease is associated with or caused by C5a, wherein the method comprises administering the C5a binding agent to the subject, wherein an activity of C5a in the blood of the subject is reduced by 50% or less within 24 to 72 hours after administering the C5a binding agent to the subject.

6. The C5a binding agent for use of any one of claims 1 to 5, wherein the C5a binding agent used has a blood plasma half-life of 6 to 15 hours within the first 12 to 48 after administering the C5a binding agent to the subject as determined in the blood of the subject.

7. The C5a binding agent for use of any one of claims 1 to 6, wherein the method comprises administering to the subject

   a) a total amount of 4.18 to 220.25 nmol of the C5a binding agent per kg body weight of the subject over a period of 8 to 48 hours, or
   b) a total amount of 0.055 to 2.9 mg of the C5a binding agent per kg bodyweight of the subject over a period of 8 to 48 hours.

8. The C5a binding agent for use of any one of claims 1 to 7, wherein the method comprises administering to the subject

   a) 121.52 nmol of the C5a binding agent per kg body weight of the subject every two days,
   b) 37.97 nmol of the C5a binding agent per kg body weight of the subject every day,
   c) 15.19 nmol of the C5a binding agent per kg body weight of the subject twice a day,
   d) 9.11 nmol of the C5a binding agent per kg body weight of the subject three times a day,
   e) 1.6 mg of the C5a binding agent per kg body weight of the subject every two days,
   f) 0.5 mg of the C5a binding agent per kg body weight of the subject every day,

g) 0.2 mg of the C5a binding agent per kg body weight of the subject twice a day, or
h) 0.12 mg of the C5a binding agent per kg body weight of the subject three times a day.

**9.** The C5a binding agent for use of any one of claims 1 to 8, wherein the method comprises administering to the subject a dose of 15.19 nmol of AON-D21 per kg body weight of the subject or a dose of 0.2 mg of AON-D21 per kg body weight of the subject every 11 to 13 hours, preferably every 12 hours,

wherein the route of administration is an intravenous infusion, wherein the intravenous infusion is a central intravenous infusion or intravenous peripheral infusion,
wherein the duration of infusion is 15 to 30 minutes,
preferably the 0.2 mg is based on the molecular weight of the oligonucleotide part of AON-D21 as an anhydrous free acid, more preferably of the oligonucleotide part of AON-D21 as an anhydrous free acid has a molecular weight of 13167 Da.

**10.** The C5a binding agent for use of any one of claims 1 to 9, wherein the methods avoids or reduces an adverse effect arising from or associated with the administering of an anti-C5a therapy C5a.

**11.** The C5a binding agent for use of any one of claims 1 to 10, wherein the adverse effect is selected from the group comprising of shock, lung edema, postoperative kidney damage, pneumonia, herpes simplex, Staphylococcus pneumonia and Herpes simplex reactivation.

**12.** The C5a binding agent for use of any of claims 1 to 11, wherein the C5a binding agent is capable of binding to C5a and C5, preferably the C5a binding agent is an agent having a binding affinity to C5a with a $K_D$ of about 0.1 to 5 nM, more preferably of 0.1 to 2 nM, most preferably of 0.1 to 1.0 nM, and/or preferably the C5a binding agent has a binding affinity to C5 with a $K_D$ of about 0.1 to 5 nM, preferably 0.1 to 2 nM, more preferably 0.1 to 1.0 nM.

**13.** The C5a binding agent for use of any one of claims 1 to 12, wherein the C5a binding agent is a C5a binding L-nucleic acid molecule, optionally comprising a modification, wherein the L-nucleic acid molecule comprises a central stretch of nucleotides, wherein the central stretch of nucleotides comprises a nucleotide sequence of
wherein the nucleic acid molecule comprises a central stretch of nucleotides, wherein the central stretch of nucleotides comprises a nucleotide sequence of 5' AUG$n_1$GGUGKU$n_2n_3$RGGGHUGUKGGG$n_4$G$n_5$CGACGCA 3' [SEQ ID NO: 61], wherein

$n_1$ is U or dU, $n_2$ is G or dG, $n_3$ is A or dA, $n_4$ is U or dU, $n_5$ is U or dU and
G, A, U, C, H, K, and R are ribonucleotides, and
dU, dG and dA are 2'-deoxyribonucleotides.

**14.** The C5a binding agent for use of claim 13, wherein the C5a binding agent is compound AON-D21 comprises a nucleotide sequence according to SEQ ID NO: 92 and is represented by the following structural formula:

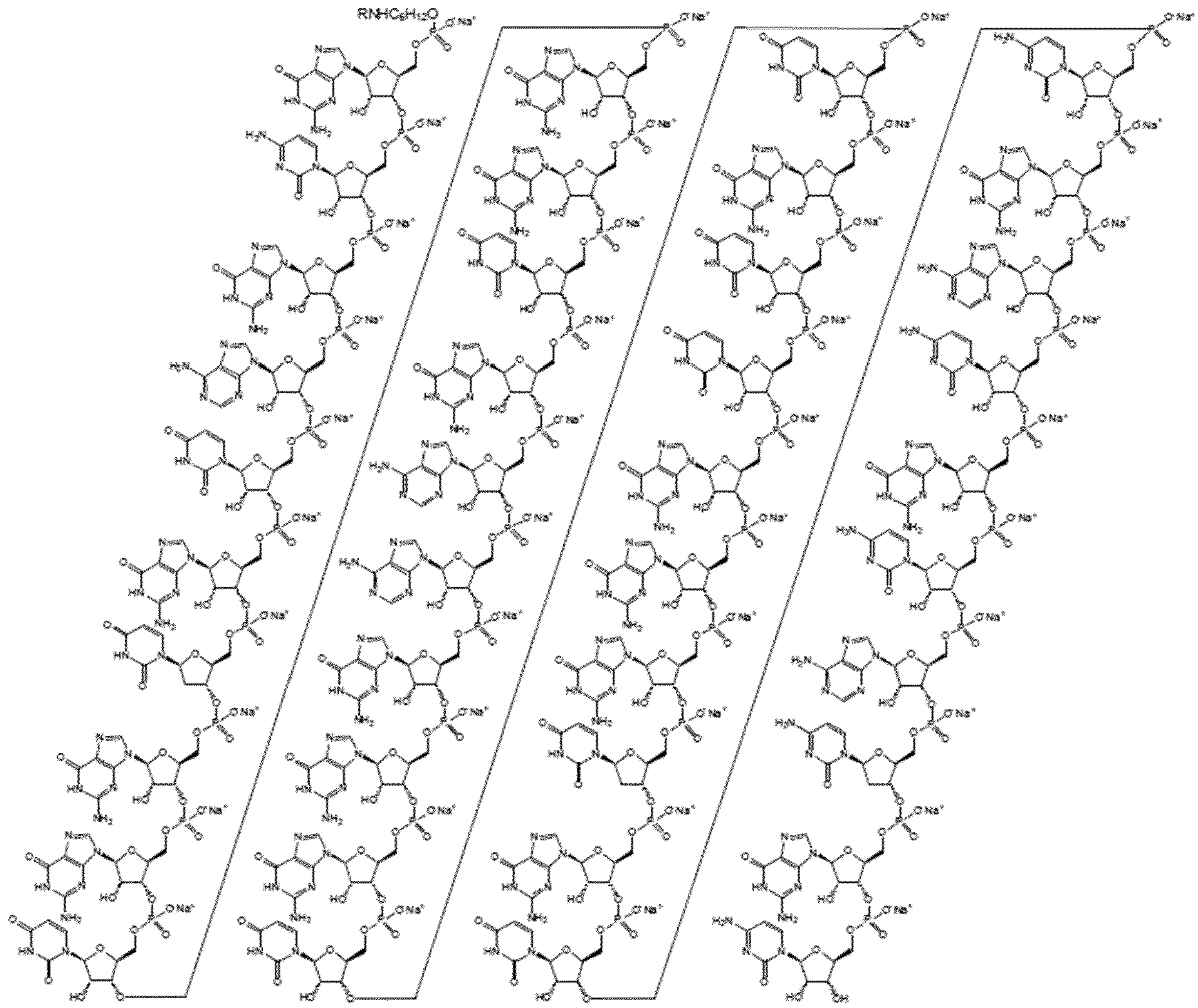

(structural formula I)

wherein $NHC_6H_{12}O$ is a hexylamino linker and R is

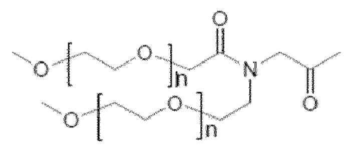

and n is approximately 450,
wherein the molecular formula is $C_{398}H_{458}N_{160}Na_{40}O_{287}P_{40}[C_2H_4O]_{2n}$, wherein n is approximately 450.

**15.** The C5a binding agent for use of any one of claims 1 to 14, wherein administering the C5a binding agent to the subject comprises administering the C5a binding agent to the subject by intravenous administration, subcutaneous administration or administration by inhalation, wherein preferably administering the C5a binding agent is administering the C5a binding agent by intravenous administration.

**16.** The C5a binding agent for use of any one of claim 1 to 15, wherein the disease associated with or caused by C5a is a disease selected from the group comprising a disease associated with complement activation and C5a-mediated pathogenic mechanisms, and/or is selected from the group comprising an autoimmune disease, an inflammatory disease, a systemic inflammatory response syndrome, a disease of the eye, an ischemia/reperfusion injuries, a delayed graft function, a transplant rejection, a cardiovascular disease, a respiratory disease, an acute reactions, an infectious disease, a neurological disease, a neurodegenerative disease, a fibrotic disease, a hematological disease,

a metabolic disease, a tumor and a clinical complication associated with complement activation by biomaterials, wherein preferably the systemic inflammatory response syndrome is selected from the group comprising sepsis and a secondary damage of trauma or a severe burn; the tumor is caner, more preferably lung cancer; and the respiratory disease is pneumonia.

| Name | SEQ ID NO. | Sequence: 5′-3′ | KD [nM] | |
|---|---|---|---|---|
| | | | mC5a | huC5a |
| 274-B5-002 | 001 | **GCCUG**AUGUGGUGUUGAAGGGUUGUGGGGUGUCGACGCA**CAGGC** | 3.5 | 12.1 |
| 274-D5-002 | 002 | **GCCUG**AUGUGGUGUUGAGGGGUUGUGGGGUGUCGACGCA**CAGGC** | 2.2 | 7.8 |
| 274-C8-002 | 003 | **GCCUG**AUGUGGUGUUGAAGGGUUGUUGGGUGUCGACGCA**CAGGC** | 0.9 | 5.1 |
| 274-C8-002-G14 (=NOX-D19001) | 004 | **GCCUG**AUGUGGUGGUGAAGGGUUGUUGGGUGUCGACGCA**CAGGC** | 0.3 | 1.4 |
| 274-C5-002 | 005 | **GCCUG**AUGUGGUGGUGAGGGGUUGUGGGGUGUCGACGCA**CAGGC** | 0.3 | 1.6 |
| 274-G6-002 | 006 | **GCCUG**AUGUGGUGGUGAGGGGAUGUGGGGUGUCGACGCA**CAGGC** | 1.0 | 4.8 |
| 274-H6-002 | 007 | **GCCUG**AUGUGGUGUUGAGGGGCUGUGGGGUGUCGACGCA**CAGGC** | n.d. | n.d. |

any of G, C, U and A is a ribonucleotide; nucleotides edged by ☐ represent a C5a-binding motif;

**KD**: Dissociation constant $K_D$ of Spiegelmers (L-nucleic acid), molecules of the indicated sequence were tested as Spiegelmers (L-nucleic acid) measured as surface plasmon resonance on Biacore using direct binding to covalently immobilized human C5a (huC5a) or mouse C5a (mC5a)

**Fig.1**

| Name | SEQ ID NO. | Sequence: 5'-3' | KD [nM] huC5a |
|---|---|---|---|
| NOX-D19001 | **004** | **GCCUG**AUGUGGUGGUGAAGGGUUGUUGGGUGUCGACGCA**CAGGC** | 1.38 |
| NOX-D19001-D09 | 008 | **GCCUG**AUGdUGGUGGUGAAGGGUUGUUGGGUGUCGACGCA**CAGGC** | 0.71 |
| NOX-D19001-D16 | 009 | **GCCUG**AUGUGGUGGUdGAAGGGUUGUUGGGUGUCGACGCA**CAGGC** | 1.03 |
| NOX-D19001-D17 | 010 | **GCCUG**AUGUGGUGGUGdAAGGGUUGUUGGGUGUCGACGCA**CAGGC** | 0.91 |
| NOX-D19001-D30 | 011 | **GCCUG**AUGUGGUGGUGAAGGGUUGUUGGGdUGUCGACGCA**CAGGC** | 0.77 |
| NOX-D19001-D32 | 012 | **GCCUG**AUGUGGUGGUGAAGGGUUGUUGGGUGdUCGACGCA**CAGGC** | 0.92 |
| NOX-D19001-D40 | 013 | **GCCUG**AUGUGGUGGUGAAGGGUUGUUGGGUGUCGACGCA**dCAGGC** | 0.88 |

any of G, C, U and A is a ribonucleotide; any of dG, dC, dU and dA is a 2'-desoxyribonucleotide;

nucleotides edged by ☐ represent a C5a-binding motif;

**KD**: Dissociation constant $K_D$ of Spiegelmers (L-nucleic acid), molecules of the indicated sequence were tested as Spiegelmers (L-nucleic acid) measured as surface plasmon resonance on Biacore using direct binding to covalently immobilized human C5a (huC5a)

**Fig. 2**

| Name | SEQ ID NO. | Sequence: 5′-3′ | KD [nM] |
|---|---|---|---|
| 2 RNA-to-DNA substitutions: | | | |
| NOX-D19001-D09-30 | 014 | **GCCUG**AUGdUGGUGGUGAAGGGUUGUUGGGdUGUCGACGCA**CAGGC** | 0.461 |
| NOX-D19001-D09-32 | 015 | **GCCUG**AUGdUGGUGGUGAAGGGUUGUUGGGUGdUCGACGCA**CAGGC** | 0.566 |
| NOX-D19001-D09-40 | 016 | **GCCUG**AUGdUGGUGGUGAAGGGUUGUUGGGUGUCGACGCA**dCAGGC** | 0.570 |
| NOX-D19001-D30-32 | 017 | **GCCUG**AUGUGGUGGUGAAGGGUUGUUGGGdUGdUCGACGCA**CAGGC** | 0.855 |
| NOX-D19001-D30-40 | 018 | **GCCUG**AUGUGGUGGUGAAGGGUUGUUGGGdUGUCGACGCA**dCAGGC** | 0.624 |
| NOX-D19001-D32-40 | 019 | **GCCUG**AUGUGGUGGUGAAGGGUUGUUGGGUGdUCGACGCA**dCAGGC** | 0.652 |
| 3 RNA-to-DNA substitutions: | | | |
| NOX-D19001-D09-30-32 | 020 | **GCCUG**AUGdUGGUGGUGAAGGGUUGUUGGGdUGdUCGACGCA**CAGGC** | 0.526 |
| NOX-D19001-D09-30-40 | 021 | **GCCUG**AUGdUGGUGGUGAAGGGUUGUUGGGdUGUCGACGCA**dCAGGC** | 0.400 |
| NOX-D19001-D09-32-40 | 022 | **GCCUG**AUGdUGGUGGUGAAGGGUUGUUGGGUGdUCGACGCA**dCAGGC** | 0.448 |
| NOX-D19001-D30-32-40 | 023 | **GCCUG**AUGUGGUGGUGAAGGGUUGUUGGGdUGdUCGACGCA**dCAGGC** | 0.880 |
| 4 RNA-to-DNA substitutions: | | | |
| NOX-D19001-D09-30-32-40 | 024 | **GCCUG**AUGdUGGUGGUGAAGGGUUGUUGGGdUGdUCGACGCA**dCAGGC** | 0.385 |
| 5 RNA-to-DNA substitutions: | | | |
| NOX-D19001-D09-16-30-32-40 | 025 | **GCCUG**AUGdUGGUGGUdGAAGGGUUGUUGGGdUGdUCGACGCA**dCAGGC** | 0.351 |
| NOX-D19001-D09-17-30-32-40 | 026 | **GCCUG**AUGdUGGUGGUGdAAGGGUUGUUGGGdUGdUCGACGCA**dCAGGC** | 0.308 |
| 6 RNA-to-DNA substitutions: | | | |
| NOX-D19001-D09-16-17-30-32-40 (= NOX-D19001-6xDNA) | 027 | **GCCUG**AUGdUGGUGGUdGdAAGGGUUGUUGGGdUGdUCGACGCA**dCAGGC** | 0.36 |

Fig. 3

any of G, C, U and A is a ribonucleotide; any of dG, dC, dU and dA is a 2'-desoxyribonucleotide;

nucleotides edged by □ represent a C5a-binding motif;

**KD**: Dissociation constant $K_D$ of Spiegelmers (L-nucleic acid), molecules of the indicated sequence were tested as Spiegelmers (L-nucleic acid) measured as surface plasmon resonance on Biacore using direct binding to covalently immobilized human C5a (huC5a)

**Fig. 3 continuation**

| Name | SEQ ID NO. | Sequence: 5'-3' | nt | KD [nM] huC5a |
|---|---|---|---|---|
| **NOX-D19001-6xDNA** | **027** | **GCCUG**AUGdUGGUGGUdGdAAGGGUUGUUGGGdUGdUCGACGCA**dCAGGC** | 44 | 0.36 |
| NOX-D19001-6xDNA-007 | 028 | **CCUG**AUGdUGGUGGUdGdAAGGGUUGUUGGGdUGdUCGACGCA**dCAGGC** | 43 | 0.46 |
| NOX-D19001-6xDNA-008 | 029 | **CUG**AUGdUGGUGGUdGdAAGGGUUGUUGGGdUGdUCGACGCA**dCAGGC** | 42 | 0.57 |
| NOX-D19001-6xDNA-009 | 030 | **UG**AUGdUGGUGGUdGdAAGGGUUGUUGGGdUGdUCGACGCA**dCAGGC** | 41 | 3.51 |
| NOX-D19001-6xDNA-010 | 031 | **G**AUGdUGGUGGUdGdAAGGGUUGUUGGGdUGdUCGACGCA**dCAGGC** | 40 | 6.89 |
| NOX-D19001-6xDNA-011 | 032 | **GCUG**AUGdUGGUGGUdGdAAGGGUUGUUGGGdUGdUCGACGCA**dCAGC** | 42 | 0.34 |
| NOX-D19001-6xDNA-012 | 033 | **GUG**AUGdUGGUGGUdGdAAGGGUUGUUGGGdUGdUCGACGCA**dCAC** | 40 | 0.81 |
| NOX-D19001-6xDNA-013 | 034 | **UG**AUGdUGGUGGUdGdAAGGGUUGUUGGGdUGdUCGACGCA**dCA** | 38 | 8.22 |
| NOX-D19001-6xDNA-018 | 035 | **GCCG**AUGdUGGUGGUdGdAAGGGUUGUUGGGdUGdUCGACGCA**dCGGC** | 42 | 0.30 |
| NOX-D19001-6xDNA-019 | 036 | **GGCG**AUGdUGGUGGUdGdAAGGGUUGUUGGGdUGdUCGACGCA**dCGCC** | 42 | 0.24 |
| NOX-D19001-6xDNA-020 (=NOX-D20001) | 037 | **GCG**AUGdUGGUGGUdGdAAGGGUUGUUGGGdUGdUCGACGCA**dCGC** | 40 | 0.42 |
| NOX-D19001-6xDNA-021 | 038 | **CUG**AUGdUGGUGGUdGdAAGGGUUGUUGGGdUGdUCGACGCA**dCAGC** | 41 | 0.70 |
| NOX-D19001-6xDNA-022 | 039 | **UG**AUGdUGGUGGUdGdAAGGGUUGUUGGGdUGdUCGACGCA**dCAGC** | 40 | 3.27 |

any of G, C, U and A is a ribonucleotide; any of dG, dC, dU and dA is a 2'-desoxyribonucleotide;

nucleotides edged by ☐ represent a C5a-binding motif; **nt.**: number of nucleotides;

**KD**: Dissociation constant $K_D$ of Spiegelmers (L-nucleic acid), molecules of the indicated sequence were tested as Spiegelmers (L-nucleic acid) measured as surface plasmon resonance on Biacore using direct binding to covalently immobilized human C5a (huC5a)

**Fig. 4A**

| Name | SEQ ID NO. | Sequence: 5'-3' | nt | KD [nM] huC5a |
|---|---|---|---|---|
| **NOX-D19001-6xDNA** | **027** | **GCCUG**AUGdUGGUGGUdGdAAGGGUUGUUGGGdUGdUCGACGCA**dCAGGC** | 44 | 0.42 |
| NOX-D19001-6xDNA-023 | 040 | **CG**AUGdUGGUGGUdGdAAGGGUUGUUGGGdUGdUCGACGCA**dCAGC** | 40 | 9.38 |
| NOX-D19001-6xDNA-024 | 041 | **G**AUGdUGGUGGUdGdAAGGGUUGUUGGGdUGdUCGACGCA**dCAGC** | 39 | 8.29 |
| NOX-D19001-6xDNA-025 | 042 | **GCUG**AUGdUGGUGGUdGdAAGGGUUGUUGGGdUGdUCGACGCA**dCAC** | 41 | 5.01 |
| NOX-D19001-6xDNA-026 | 043 | **GCUG**AUGdUGGUGGUdGdAAGGGUUGUUGGGdUGdUCGACGCA**dCC** | 40 | 16.8 |
| NOX-D19001-6xDNA-027 | 044 | **GCUG**AUGdUGGUGGUdGdAAGGGUUGUUGGGdUGdUCGACGCA**dCA** | 40 | 4.71 |
| NOX-D19001-6xDNA-028 | 045 | **CG**AUGdUGGUGGUdGdAAGGGUUGUUGGGdUGdUCGACGCA**dCGC** | 39 | 3.36 |
| NOX-D19001-6xDNA-029 | 046 | **G**AUGdUGGUGGUdGdAAGGGUUGUUGGGdUGdUCGACGCA**dCGC** | 38 | 6.89 |
| NOX-D19001-6xDNA-030 | 047 | **GCG**AUGdUGGUGGUdGdAAGGGUUGUUGGGdUGdUCGACGCA**dCC** | 39 | 16.1 |
| NOX-D19001-6xDNA-032 | 048 | **GCG**AUGdUGGUGGUdGdAAGGGUUGUUGGGdUGdUCGACGCA**dC** | 38 | 5.58 |
| NOX-D19001-6xDNA-033 | 049 | **GG**AUGdUGGUGGUdGdAAGGGUUGUUGGGdUGdUCGACGCA**dCC** | 38 | 5.47 |

any of G, C, U and A is a ribonucleotide; any of dG, dC, dU and dA is a 2'-desoxyribonucleotide;

nucleotides edged by ☐ represent a C5a-binding motif; **nt.**: number of nucleotides;

**KD**: Dissociation constant $K_D$ of Spiegelmers (L-nucleic acid), molecules of the indicated sequence were tested as Spiegelmers (L-nucleic acid) measured as surface plasmon resonance on Biacore using direct binding to covalently immobilized human C5a (huC5a)

**Fig. 4B**

| Name | SEQ ID NO. | Sequence: 5'-3' | KD [nM] | $IC_{50}$ [nM] |
|---|---|---|---|---|
| NOX-D19001-020 | 55 | **GCG**AUGUGGUGGUGAAGGGUUGUUGGGUGUCGACGCA**CGC** | 11.3 | 9.4 |
| **1 RNA-to-DNA substitutions:** | | | | |
| NOX-D19001-1xDNA-020 | 56 | **GCG**AUGdUGGUGGUGAAGGGUUGUUGGGUGUCGACGCA**CGC** | 1.34 | 3 |
| **2 RNA-to-DNA substitutions:** | | | | |
| NOX-D19001-2xDNA-020 | 57 | **GCG**AUGdUGGUGGUGAAGGGUUGUUGGGdUGUCGACGCA**CGC** | 0.73 | 2.2 |
| **3 RNA-to-DNA substitutions:** | | | | |
| NOX-D19001-3xDNA-020 | 58 | **GCG**AUGdUGGUGGUGAAGGGUUGUUGGGdUGdUCGACGCA**CGC** | 0.91 | 3.7 |
| NOX-D19001-2dU-1dC-020 (=NOX-D21001) | 59 | **GCG**AUGdUGGUGGUGAAGGGUUGUUGGGdUGUCGACGCA**dCGC** | 0.82 | 0.6 |
| **4 RNA-to-DNA substitutions:** | | | | |
| NOX-D19001-3dU-1dC-020 | 60 | **GCG**AUGdUGGUGGUGAAGGGUUGUUGGGdUGdUCGACGCA**dCGC** | 0.87 | 0.6 |

any of G, C, U and A is a ribonucleotide; any of dG, dC, dU and dA is a 2'-desoxyribonucleotide;

nucleotides edged by ☐ represent a C5a-binding motif;

**KD**: Dissociation constant $K_D$ of Spiegelmers (L-nucleic acid), molecules of the indicated sequence were tested as Spiegelmers (L-nucleic acid) measured as surface plasmon resonance on Biacore using direct binding to covalently immobilized human C5a (huC5a)

**$IC_{50}$**: Half maximal inhibitory concentration (IC50) of Spiegelmers (L-nucleic acid), molecules of the indicated sequence were tested as Spiegelmers in a cell culture *in vitro* taxis assy to inhibit C5a

**Fig. 5**

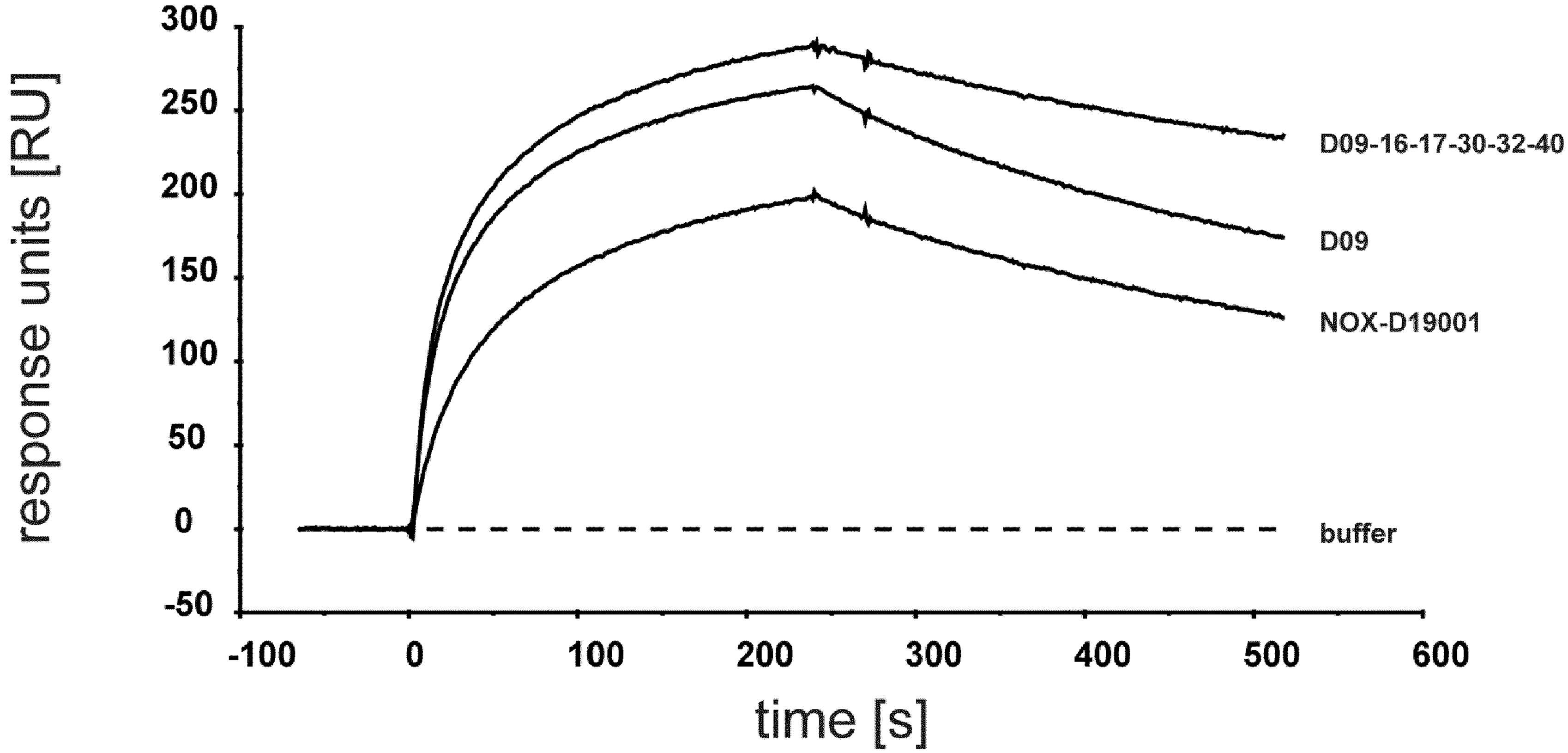


| | $K_D$ [nM] |
|---|---|
| NOX-D19001 | 1.38 |
| NOX-D19001-D09 | 0.71 |
| NOX-D19001-D09-16-17-30-32-40 | 0.36 |

Fig. 6

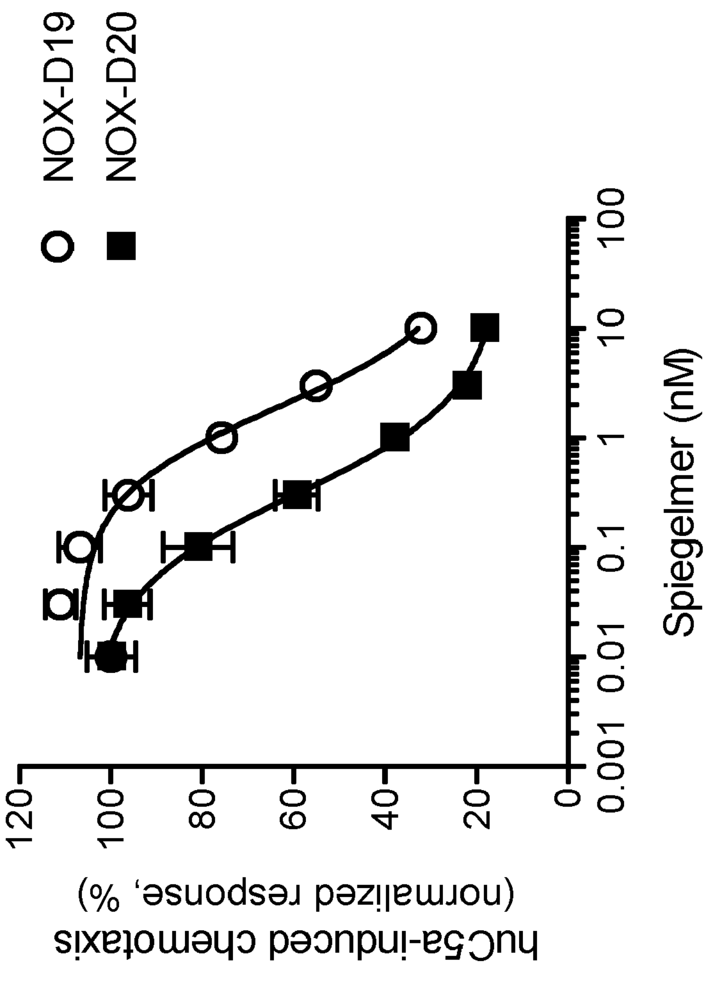
NOX-D19
NOX-D20
huC5a-induced chemotaxis
(normalized response, %)
120
100
80
60
40
20
0
0.001
0.01
0.1
1
10
100
Spiegelmer (nM)

Fig. 7

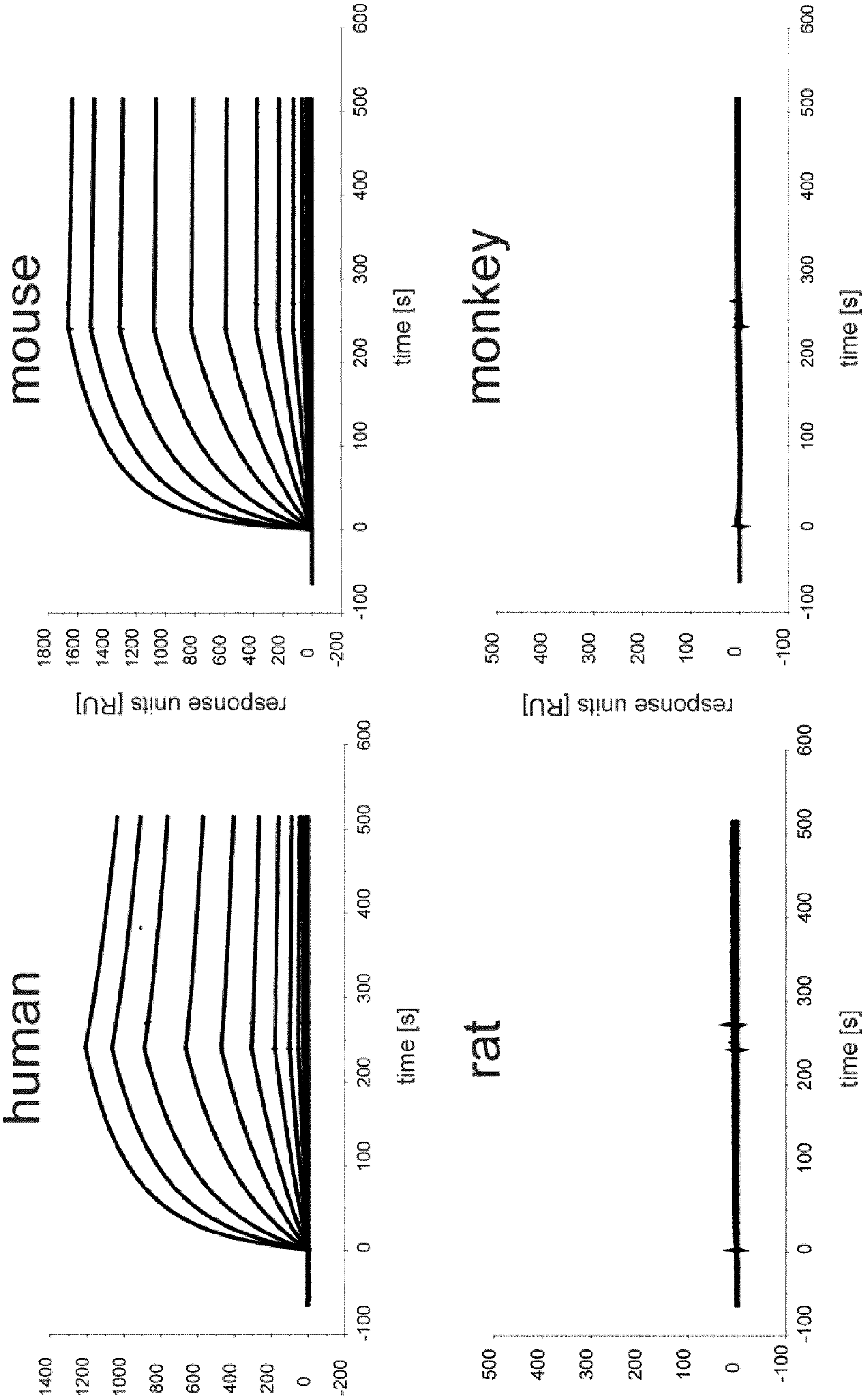


Fig. 8

| NOX-D20 binding to: | $K_D$ [pM] |
|---|---|
| mouse C5a | 19.1 |
| human C5a | 299 |
| rat C5a | -- |
| rhesus monkey C5a | -- |

**Fig. 8 continuation**

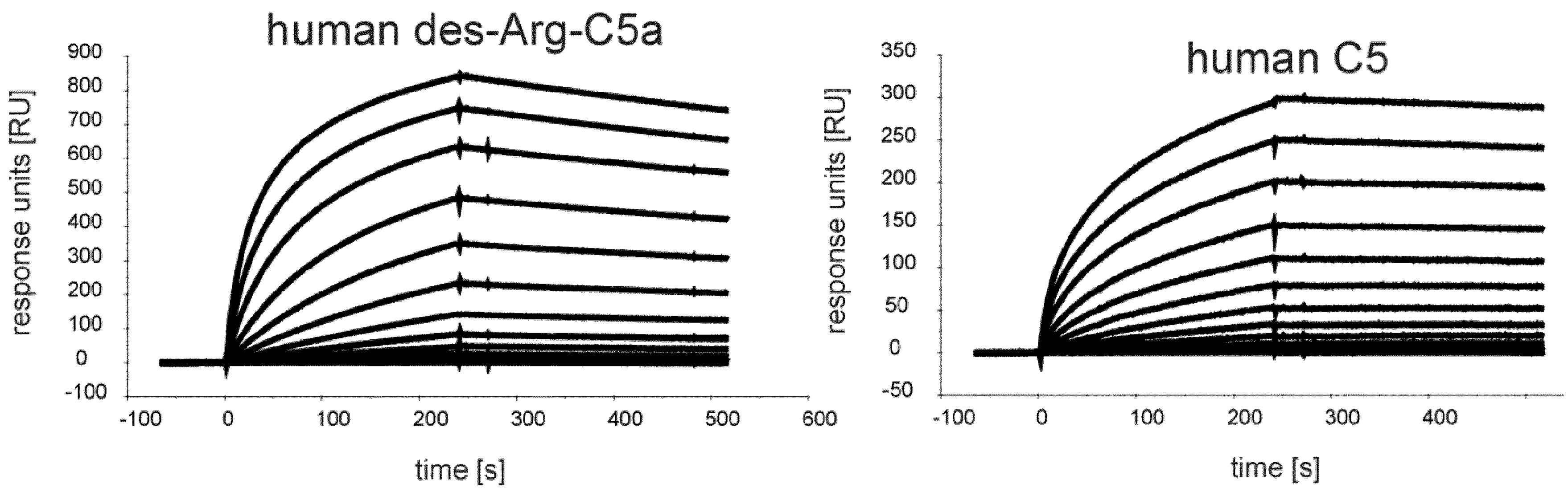


| NOX-D20 binding to: | $K_D$ [pM] |
|---|---|
| human desArg-C5a | 316 |
| human C5 | 164 |

Fig. 9

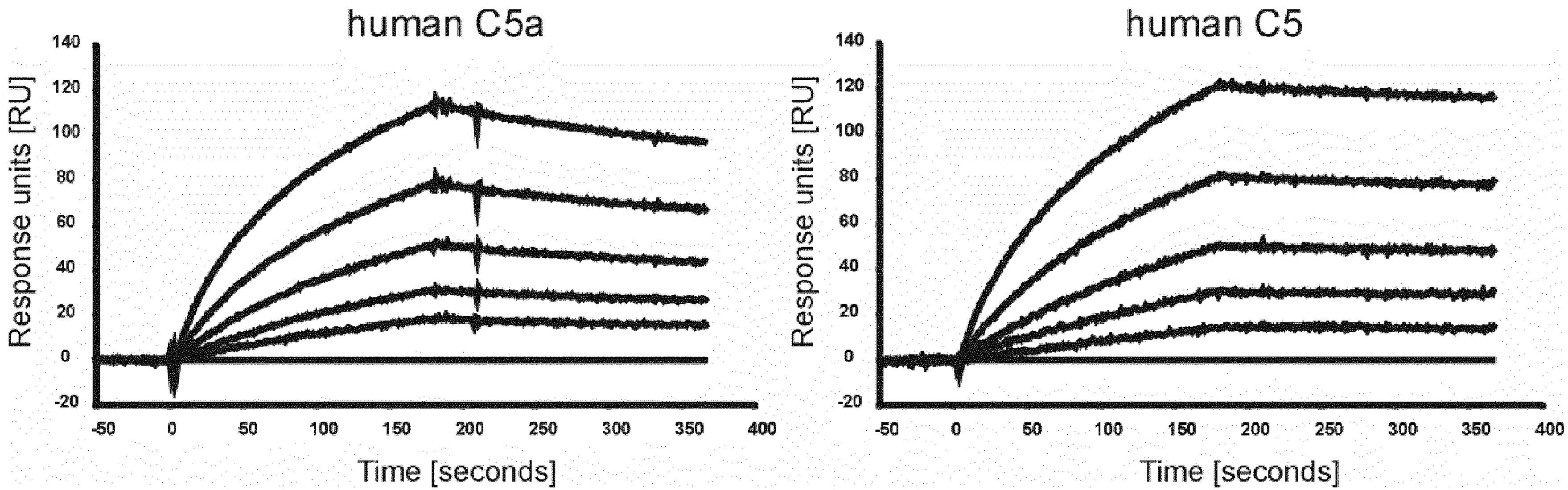


| NOX-D21 binding to: | $K_D$ [pM] |
|---|---|
| **human C5a** | 815 |
| **human C5** | 413 |
| **human des-ArgC5a** | 854 |
| **mouseC5a** | 29.4 |
| **mouse des-ArgC5a** | 27.6 |

**Fig. 10**

**Alignment of C5a from selected species**

```
human:       TLQKKIEEIAAKYKHSVVKKCCYDGACVNNDETCEQRAARISLGPRCIKAFTECCVVASQLRANISHKDMQLGR
[SEQ ID NO: 050]

rhesus:      MLQEKIEEIAAKYKHLVVKKCCYDGVRINHDETCEQRAARISVGPRCVKAFTECCVVASQLRANNSHKDLQLGR
[SEQ ID NO: 054]

mouse:    NLHLLRQKIEEQAAKYKHSVPKKCCYDGARVNFYETCEERVARVTIGPLCIRAFNECCTIANKIRKESPHKPVQLGR
[SEQ ID NO: 052]

rat:      DLQLLHQKVEEQAAKYKHRVPKKCCYDGARENKYETCEQRVARVTIGPHCIRAFNECCTIADKIRKESHHKGMLLGR
[SEQ ID NO: 051]
```

**Fig. 11**

A

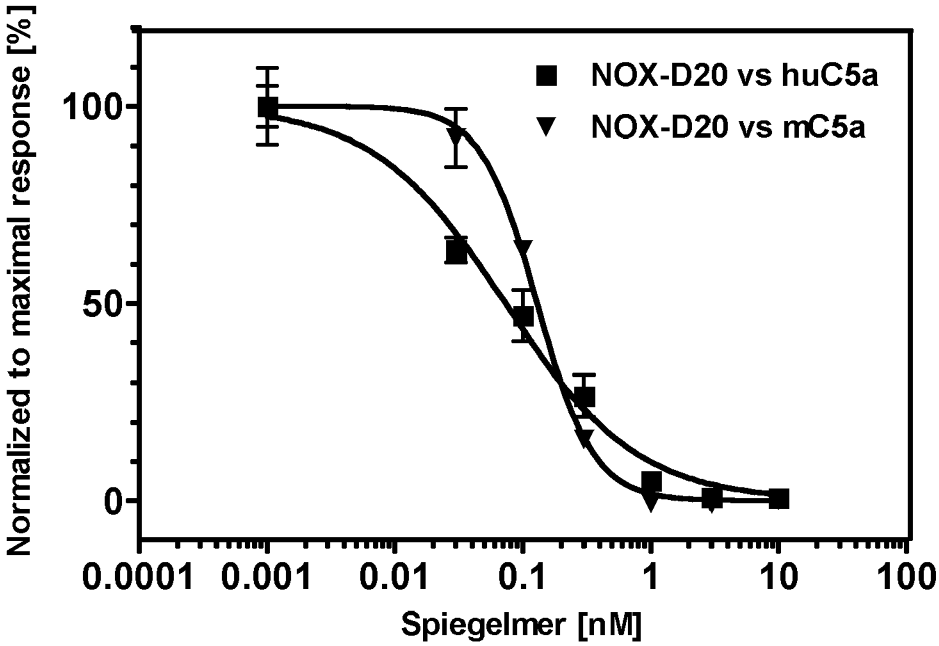
NOX-D20 vs huC5a
NOX-D20 vs mC5a
Normalized to maximal response [%]
100
50
0
0.0001 0.001 0.01 0.1 1 10 100
Spiegelmer [nM]

B

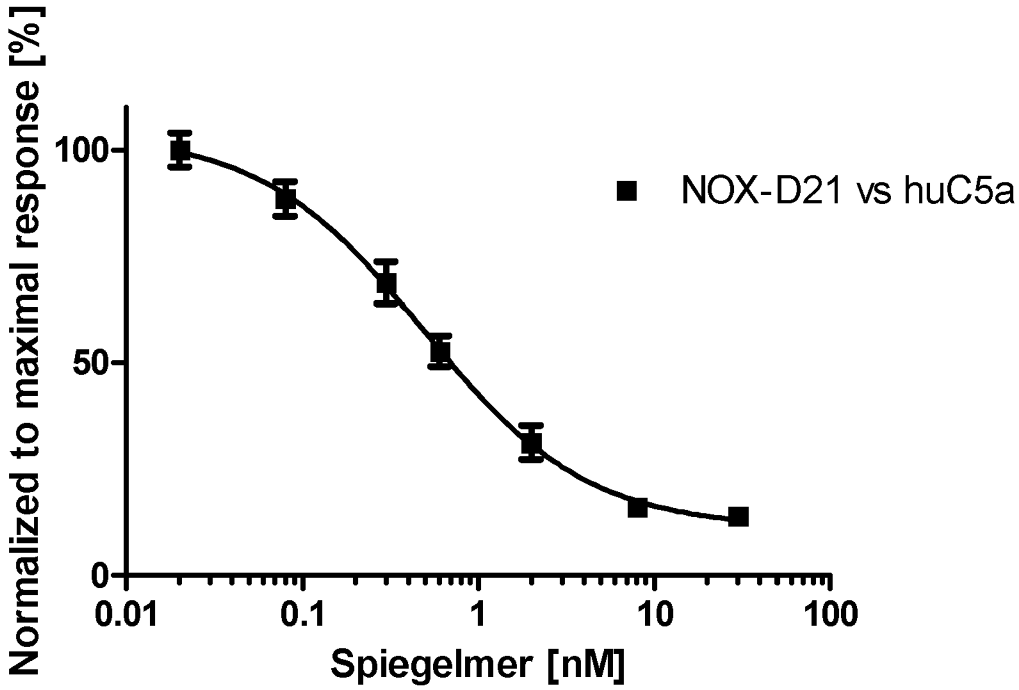
NOX-D21 vs huC5a
Normalized to maximal response [%]
100
50
0
0.01 0.1 1 10 100
Spiegelmer [nM]

Fig. 12

| Name | Sequence: 5′-3′ | Binding (KD) Human C5a | Inhibition (IC50) Human C5a |
|---|---|---|---|
| **AON-D21$_{vD19}$** | **GCCUG**AUGUGGUGGUGAAGGGUUGUUGGGUGUCGACGCA**CAGGC** | 1.38 nM | 1.9 nM |
| **AON-D21$_{vD20}$** | **GCG**AUGdUGGUGGUdGdAAGGGUUGUUGGGdUGdUCGACGCA**dCGC** | 0.299 nM | 0.28 nM |
| **AON-D21** | **GCG**AUGdUGGUGGUGAAGGGUUGUUGGGdUGUCGACGCA**dCGC** | 0.815 nM | 0.476 nM |

**Fig. 13**

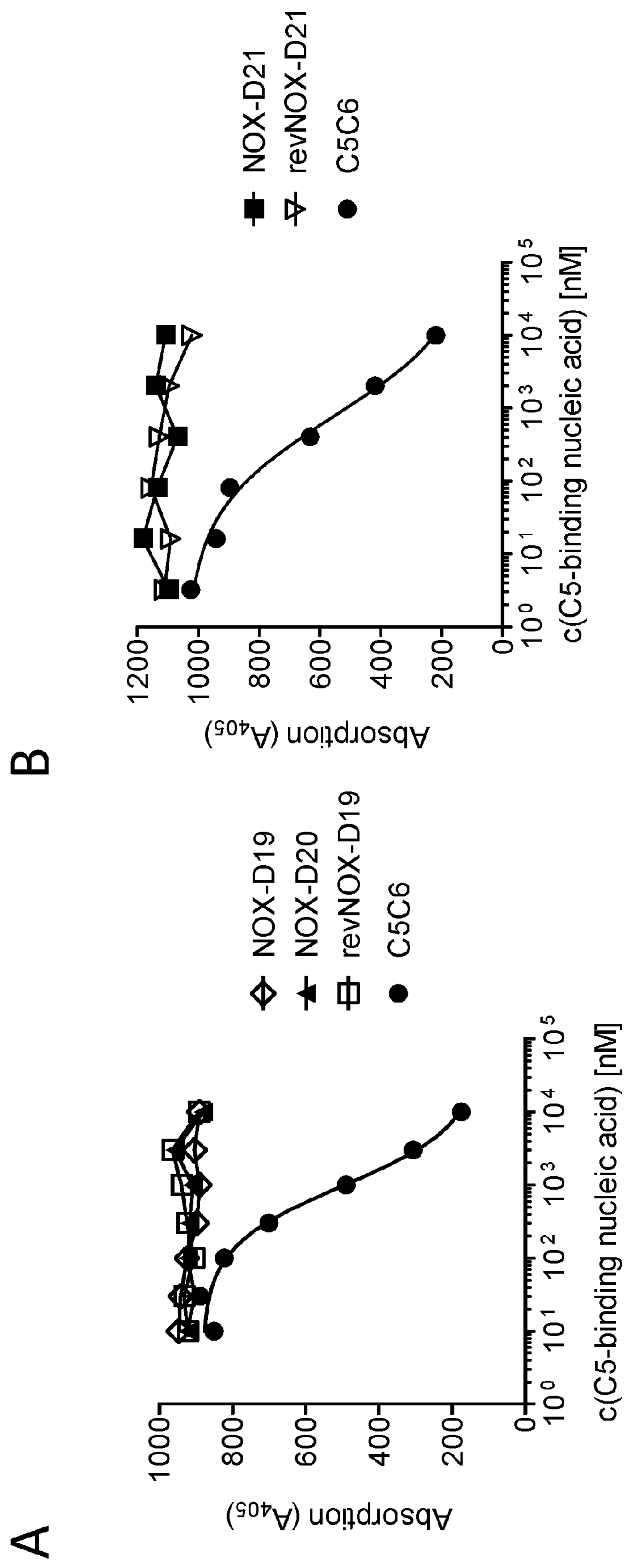
A
Absorption ($A_{405}$)
c(C5-binding nucleic acid) [nM]
NOX-D19
NOX-D20
revNOX-D19
C5C6
B
Absorption ($A_{405}$)
c(C5-binding nucleic acid) [nM]
NOX-D21
revNOX-D21
C5C6

Fig. 14

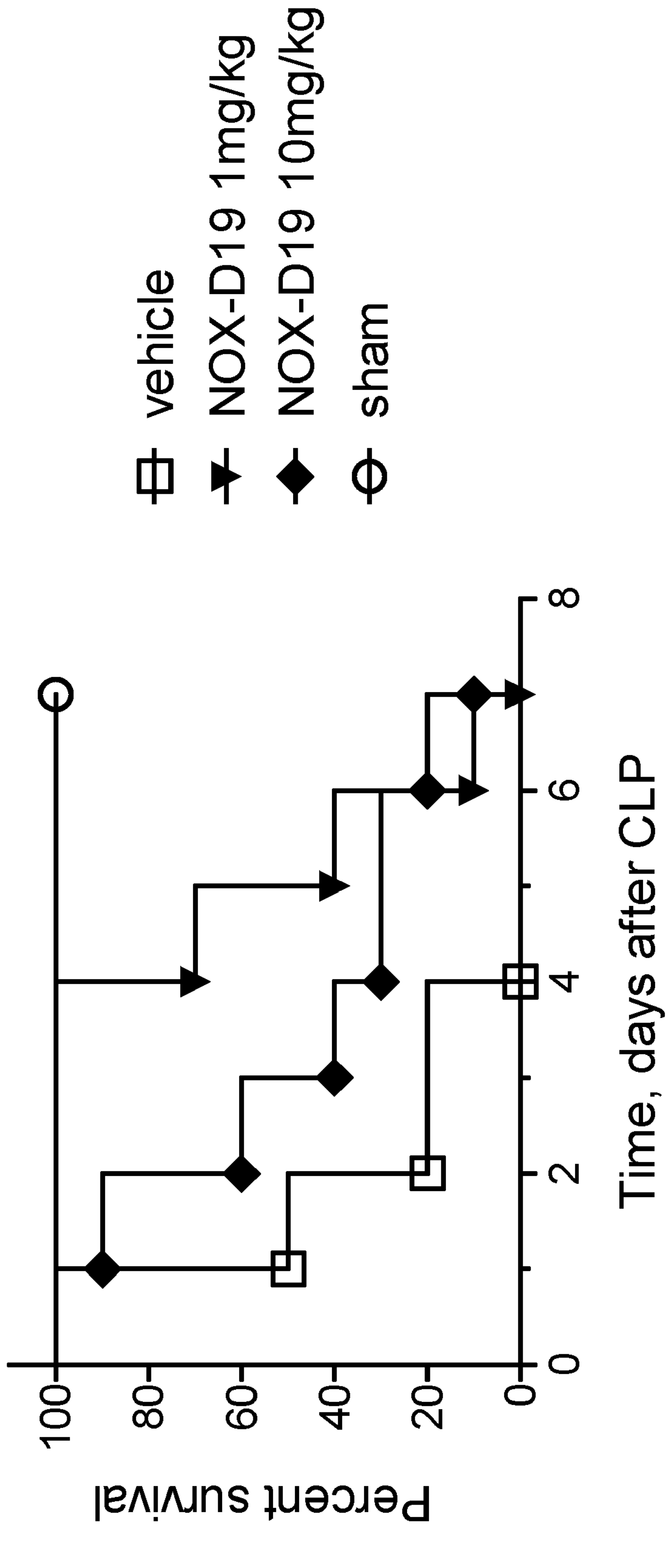
vehicle
NOX-D19 1mg/kg
NOX-D19 10mg/kg
sham
Percent survival
100
80
60
40
20
0
0
2
4
6
8
Time, days after CLP

Fig. 15

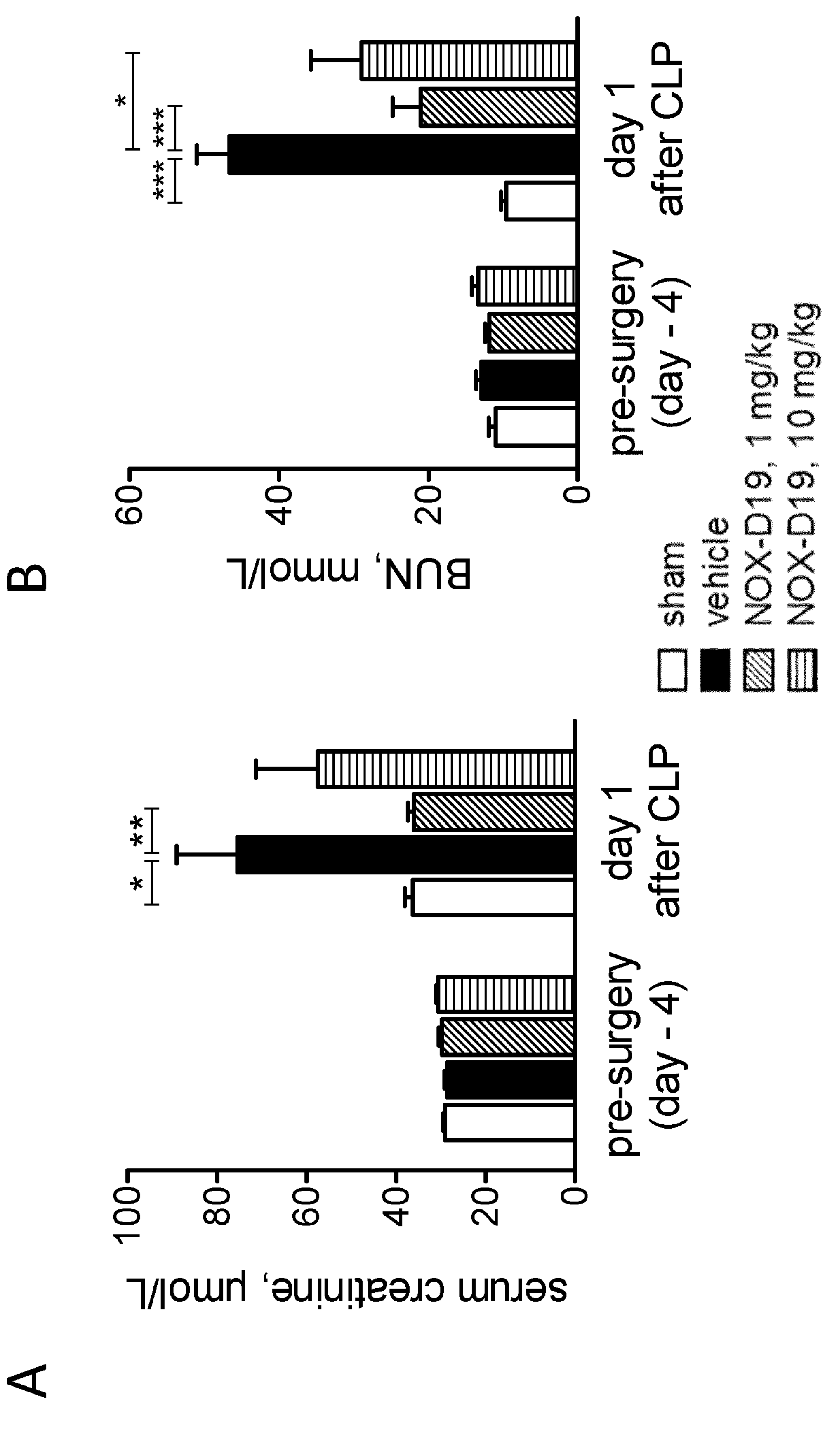
A
serum creatinine, µmol/L
100
80
60
40
20
0
pre-surgery
(day - 4)
day 1
after CLP
*
**
B
BUN, mmol/L
60
40
20
0
pre-surgery
(day - 4)
day 1
after CLP
***
***
*
sham
vehicle
NOX-D19, 1 mg/kg
NOX-D19, 10 mg/kg

Fig. 16

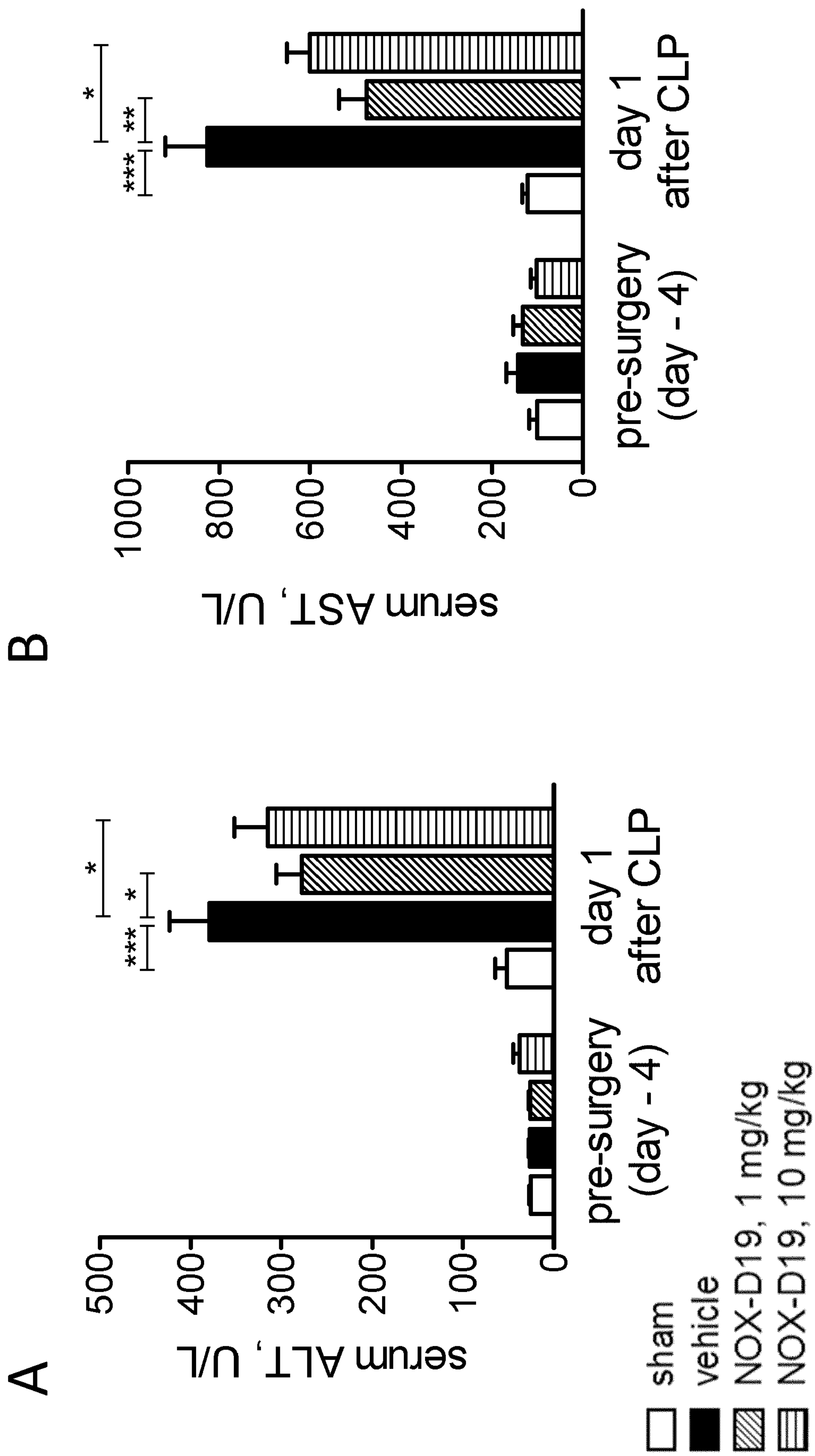
A
serum ALT, U/L
500
400
300
200
100
0
pre-surgery (day - 4)
day 1 after CLP
***
*
*
sham
vehicle
NOX-D19, 1 mg/kg
NOX-D19, 10 mg/kg
B
serum AST, U/L
1000
800
600
400
200
0
pre-surgery (day - 4)
day 1 after CLP
***
**
*

Fig. 17

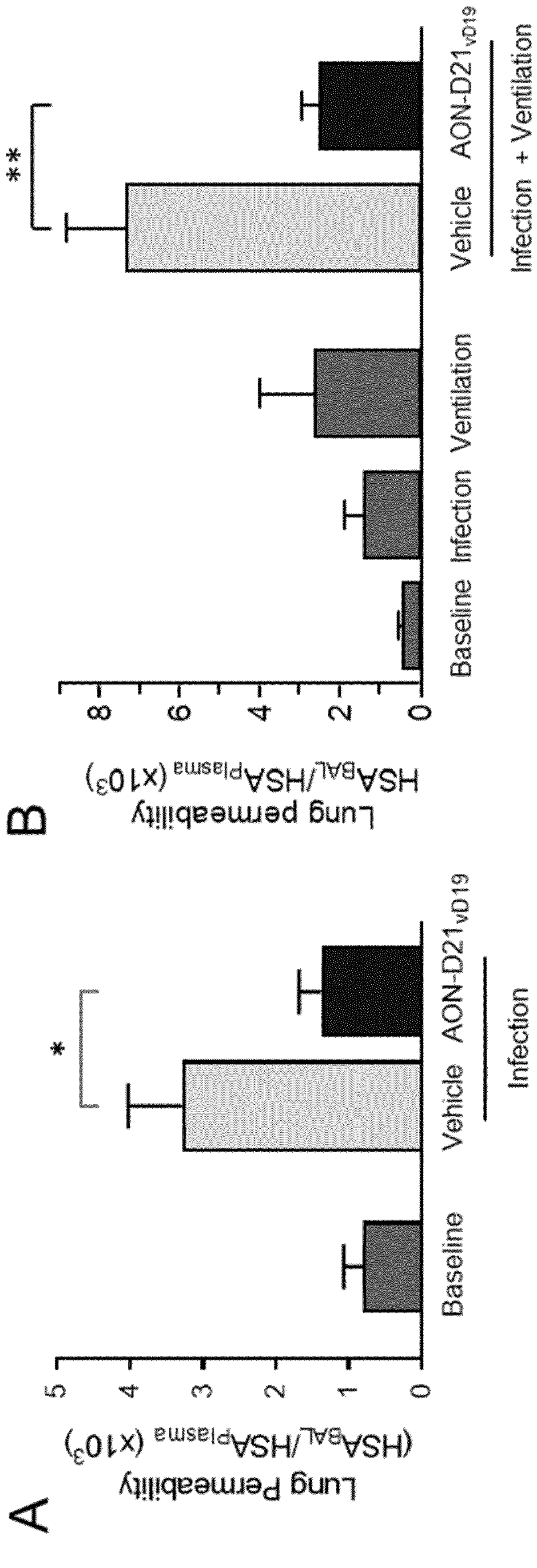
A
Lung Permeability
(HSA_BAL/HSA_Plasma (x10^3)
0
1
2
3
4
5
Baseline
Vehicle
AON-D21vD19
Infection
*
B
Lung permeability
HSA_BAL/HSA_Plasma (x10^3)
0
2
4
6
8
Baseline
Infection
Ventilation
Vehicle
AON-D21vD19
Infection + Ventilation
**

Fig. 18

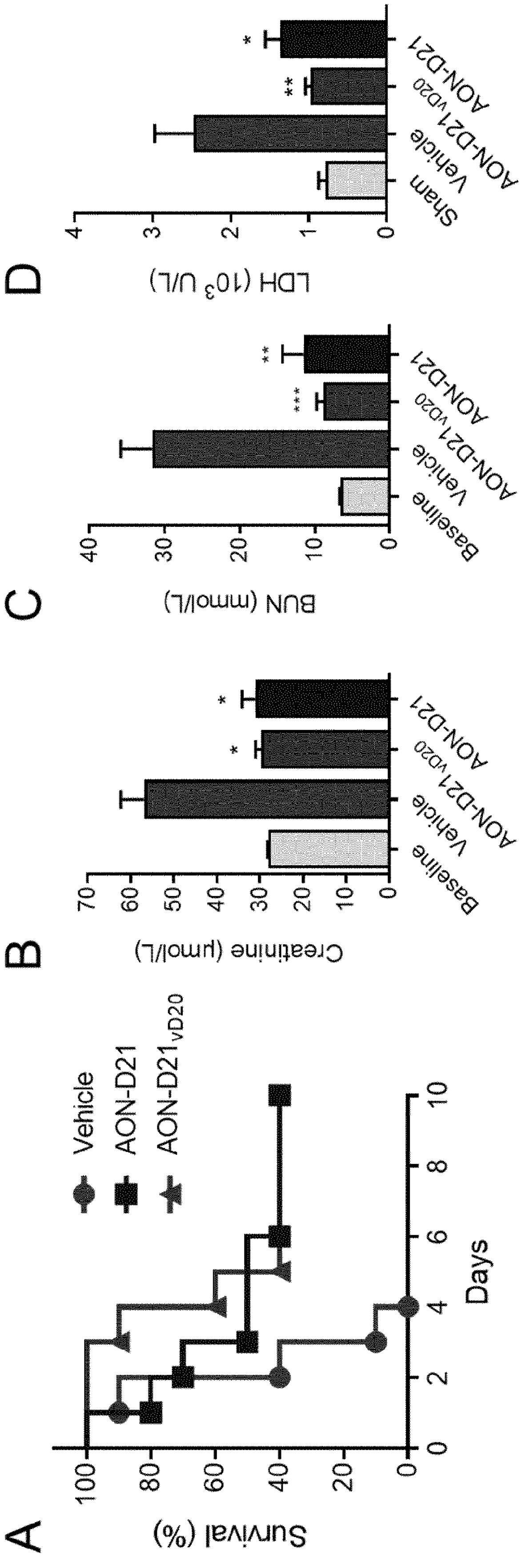
A
Survival (%)
Days
Vehicle
AON-D21
AON-D21vD20
B
Creatinine (μmol/L)
C
BUN (mmol/L)
D
LDH (10^3 U/L)
Baseline
Sham
Vehicle
AON-D21vD20
AON-D21

Fig. 19

2'-Deoxyribonucleotides

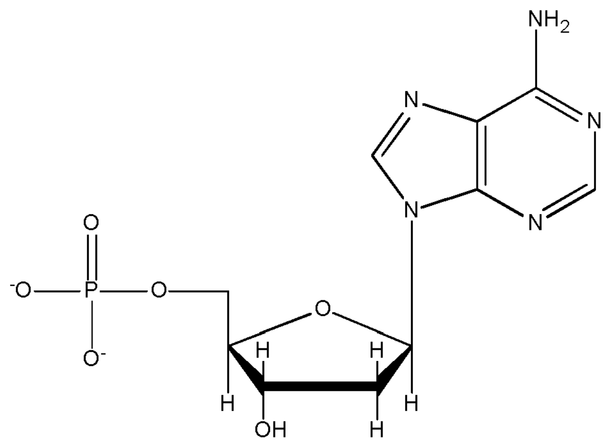

2'Deoxy-adenosine 5'monophosphate (abbr. dA)

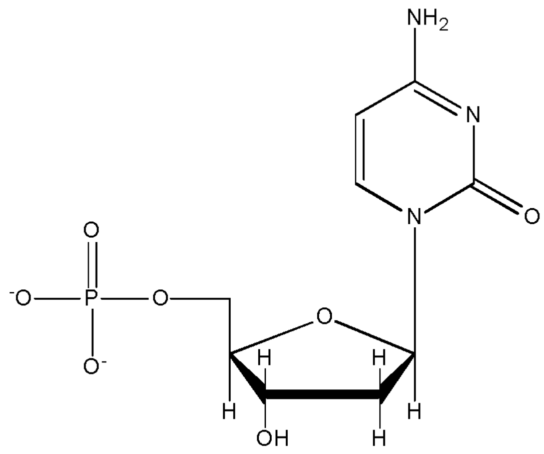

2'Deoxy-cytidine 5'monophosphate (abbr. dC)

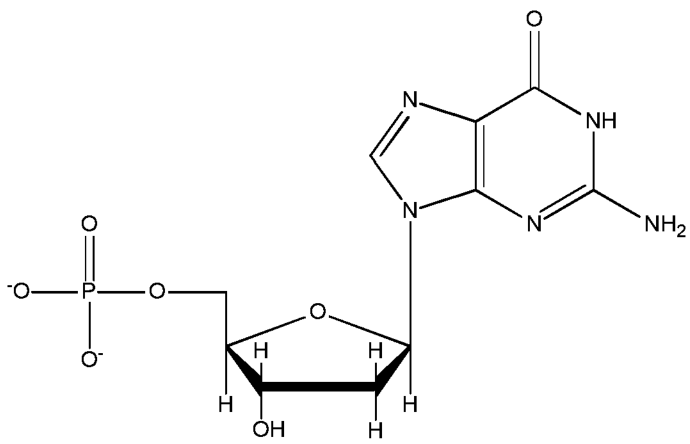

2'Deoxy-guanosine 5'monophosphate (abbr. dG)

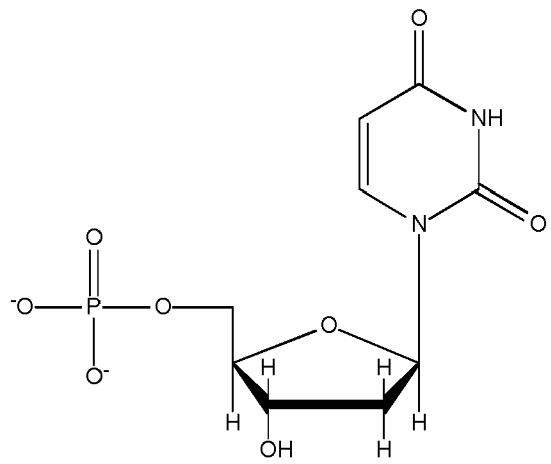

2'Deoxy-uridine 5'monophosphate (abbr. dU)

Fig. 20

**Ribonucleotides**

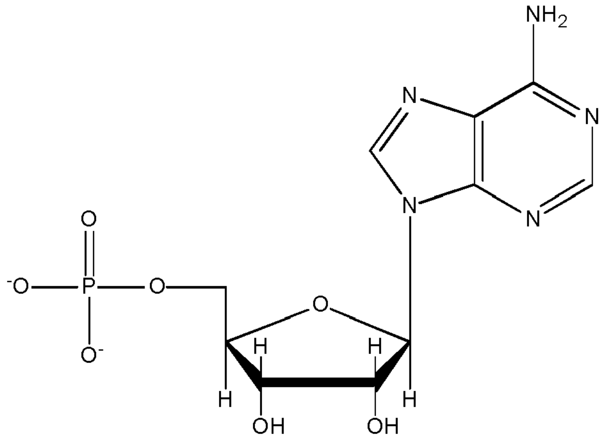

**Adenosine 5'monophosphate (abbr. A)**

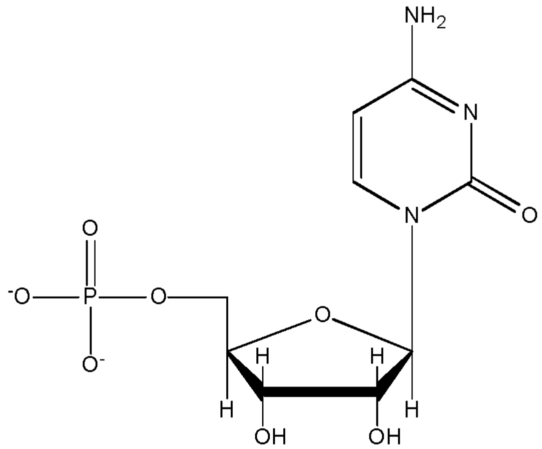

**Cytidine 5'monophosphate (abbr. C)**

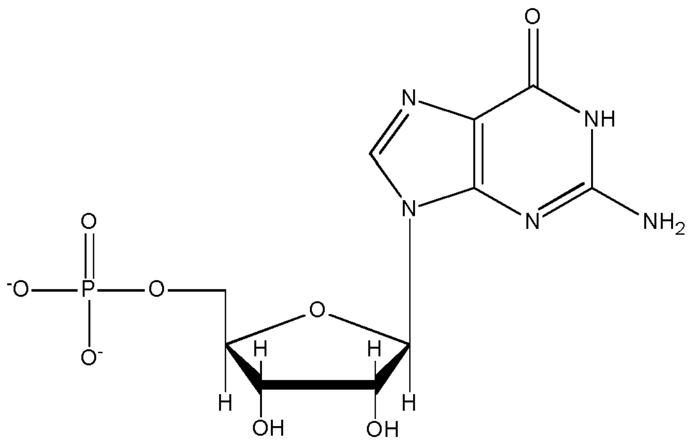

**Guanosine 5'monophosphate (abbr, G)**

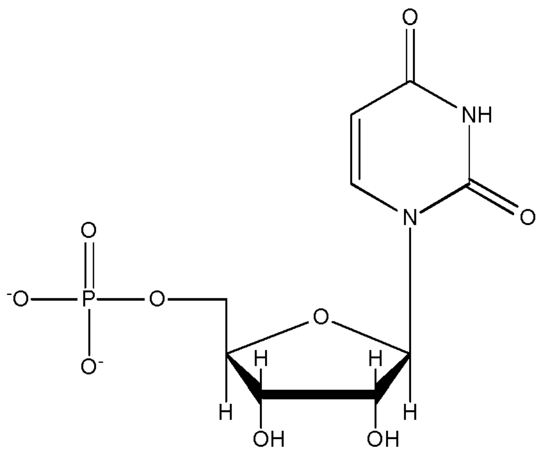

**Uridine 5'monophosphate (abbr. U)**

**Fig. 21**

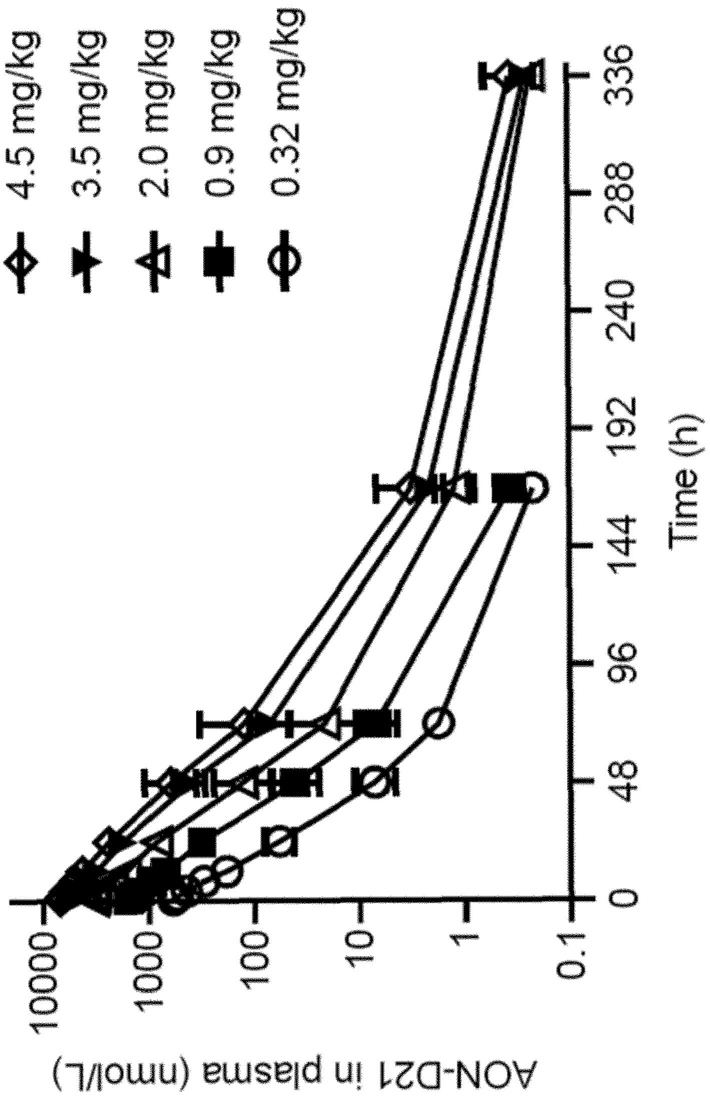
4.5 mg/kg
3.5 mg/kg
2.0 mg/kg
0.9 mg/kg
0.32 mg/kg
AON-D21 in plasma (nmol/L)
10000
1000
100
10
1
0.1
0
48
96
144
192
240
288
336
Time (h)

Fig. 22

| Parameter | Statistics | Treatment<br>0.32 mg/kg SD (n=6) | 0.9 mg/kg SD (n=5) | 2.0 mg/kg SD (n=6) | 3.5 mg/kg SD (n=6) | 4.5 mg/kg SD (n=6) |
|---|---|---|---|---|---|---|
| $C_{max}$ (nmol/L) | Mean | 601 | 1,580 | 3,440 | 5,480 | 7,280 |
| | SD | 96.3 | 228 | 389 | 941 | 1,040 |
| | CV% | 16.0 | 14.4 | 11.3 | 17.2 | 14.3 |
| | Minimum | 412 | 1,270 | 2,800 | 4,300 | 6,060 |
| | Median | 631 | 1,670 | 450 | 5,580 | 7,260 |
| | Maximum | 671 | 1,850 | 3,950 | 6,470 | 8,540 |
| DN $C_{max}$ (nmol/L)/(mg/kg) | Mean | 1,880 | 1,760 | 1,720 | 1,570 | 1,620 |
| | SD | 301 | 253 | 194 | 269 | 231 |
| $t_{½}$ 0-48h (h) | Mean | 7.56 | 9.13 | 10.2 | 12.7 | 14.3 |
| | SD | 0.893 | 1.28 | 1.55 | 1.84 | 3.20 |
| | CV% | 11.8 | 14.0 | 15.2 | 14.5 | 22.4 |
| | Minimum | 6.29 | 7.20 | 8.47 | 10.3 | 10.5 |
| | Median | 7.39 | 9.43 | 10.2 | 13.4 | 13.1 |
| | Maximum | 8.76 | 10.7 | 11.9 | 14.3 | 19.2 |

$C_{max}$: Maximum observed plasma concentration over the sampling interval

DN $C_{max}$: Dose-normalized $C_{max}$ calculated as $C_{max}$ divided by the applied dose (in mg AON-D21/kg body weight)

$t_{½}$ 0-48h: Elimination half-life with the first 48 hours after dosing.

Fig. 23

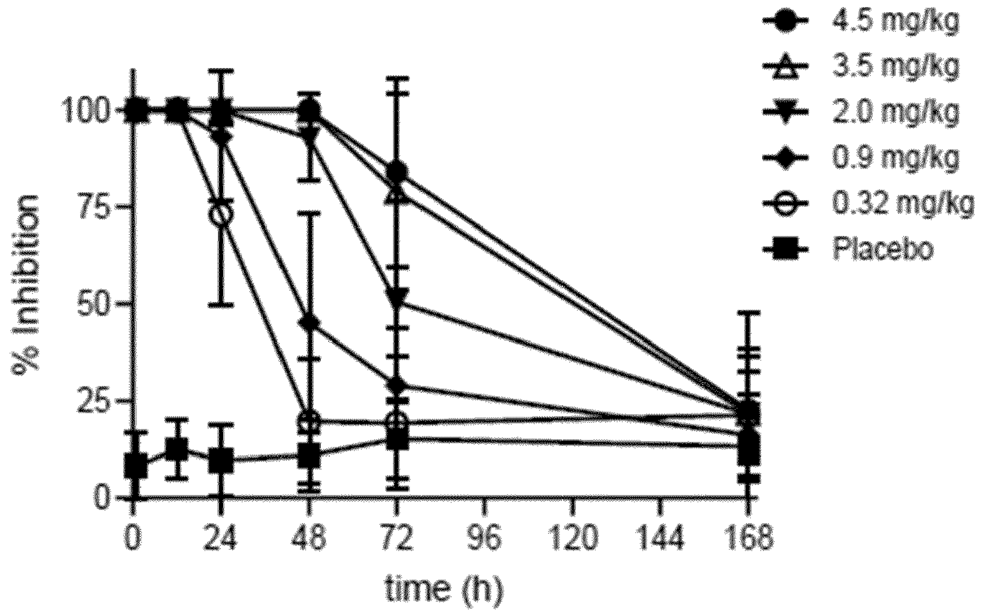
4.5 mg/kg
3.5 mg/kg
2.0 mg/kg
0.9 mg/kg
0.32 mg/kg
Placebo
% Inhibition
100
75
50
25
0
0
24
48
72
96
120
144
168
time (h)

Fig. 24

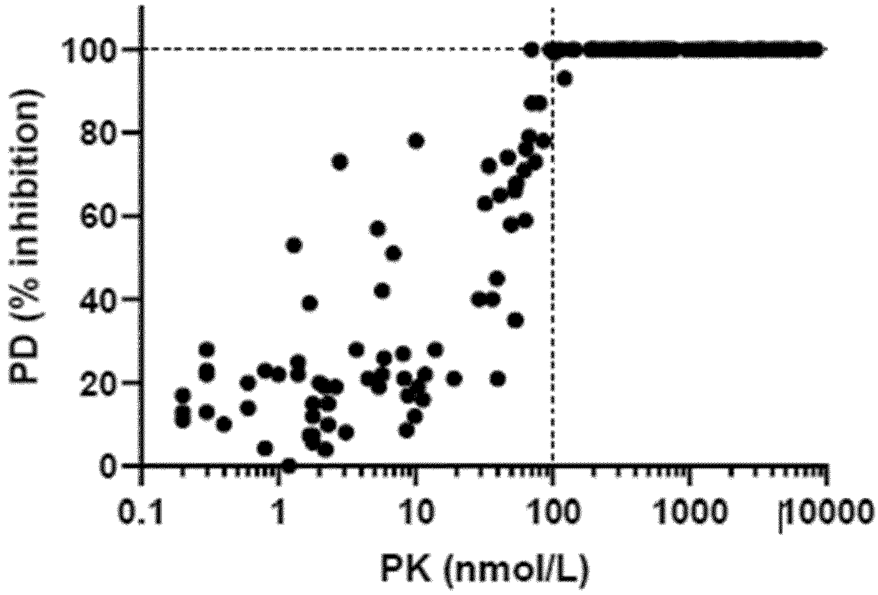


Fig. 25 A

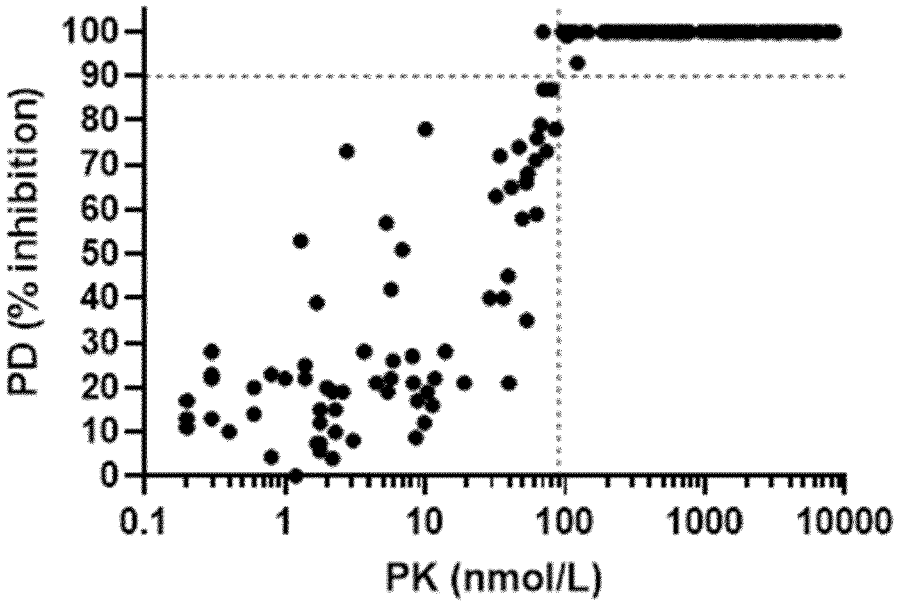


Fig. 25 B

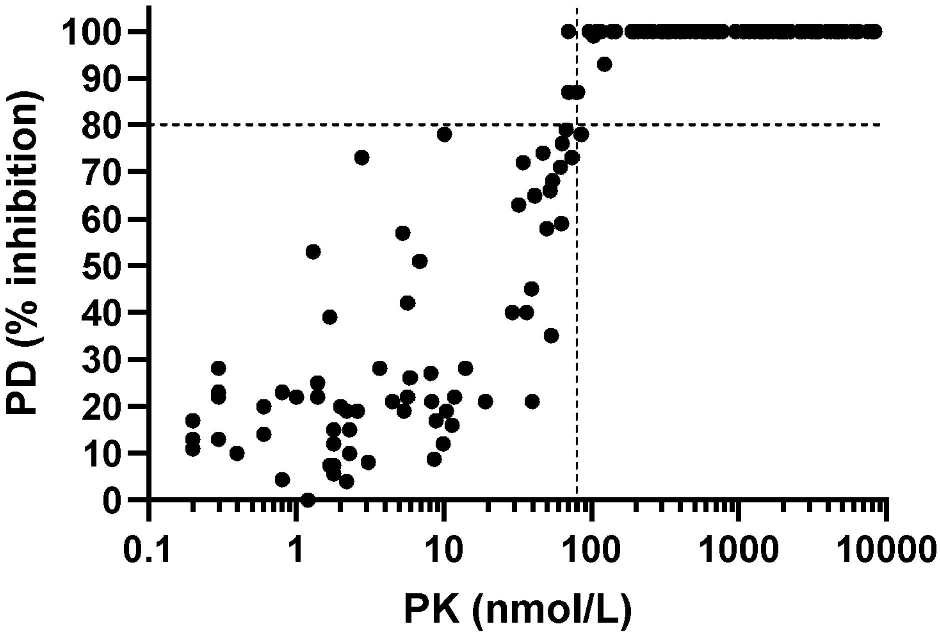


Fig. 25 C

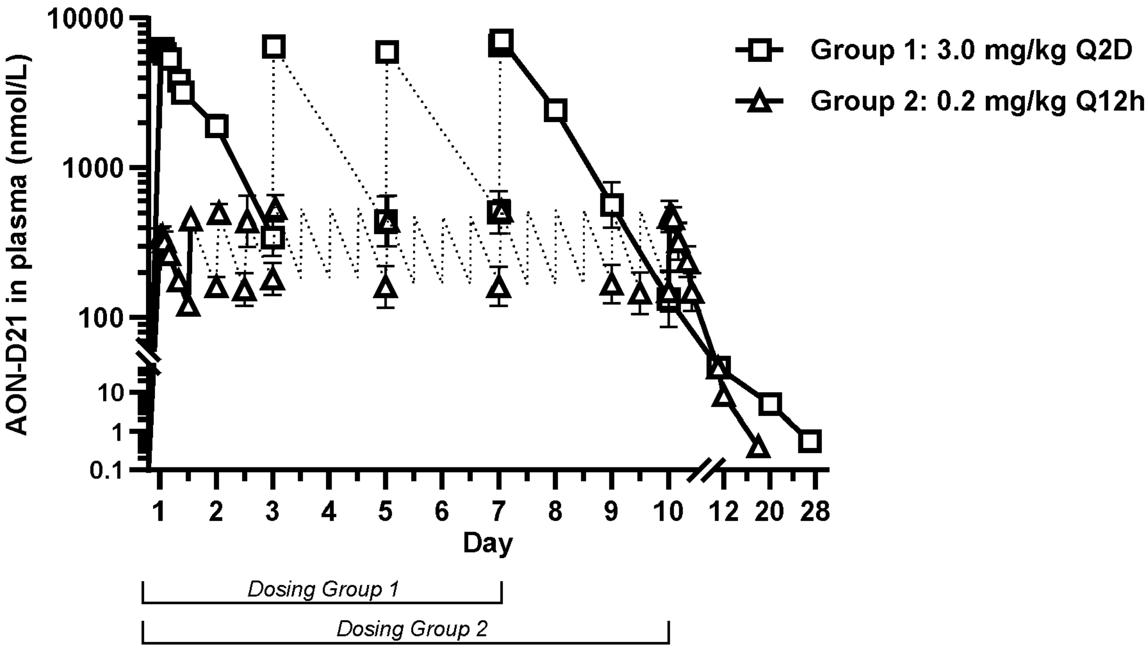


Fig. 26

| Parameter | Statistics | Treatment: 3.0 mg/kg Q2D (n=6) | | Treatment: 0.2 mg/kg Q12h (n=6) | |
|---|---|---|---|---|---|
| | | 1st Dose (Day 1) | 4th Dose (Day 7) | 1st Dose (Day 1) | 19st Dose (Day 10) |
| $C_{max}$ (nmol/L) | Mean | 6,420 | 7,300 | 364 | 504 |
| | SD | 673 | 835 | 58 | 79 |
| | CV% | 10.5 | 11.4 | 15.9 | 15.6 |
| | Minimum | 5,510 | 5,970 | 280 | 402 |
| | Median | 6,580 | 7,540 | 392 | 527 |
| | Maximum | 7,270 | 7,990 | 412 | 602 |
| DN $C_{max}$ (nmol/L)/(mg/kg) | Mean | 2,139 | N.A. | 1,820 | N.A. |
| | SD | 224 | | 290 | |
| $t_{max}$ (h) | Minimum | 0,92 | 0.52 | 0.25 | 0.25 |
| | Median | 1 | 1 | 0.625 | 0.625 |
| | Maximum | 2 | 1 | 1 | 1 |
| $t_{½}$ 0-tau (h) | Mean | 11.5 | No data | 6.95 | 6.82 |
| | SD | 1.4 | | 1.28 | 0.87 |
| | CV% | 12.2 | | 18.3 | 12.7 |
| | Minimum | 9.53 | | 5.73 | 6.05 |
| | Median | 11.6 | | 6.55 | 6.58 |
| | Maximum | 13.6 | | 9.34 | 8.28 |
| | | Day 3 (pre 2nd dose) | Day 7 (pre 4th dose) | Day 1 (pre 2nd dose) | Day 10 (pre 19th dose) |
| $C_{trough}$ (nmol/L) | Mean | 356 | 525 | 122 | 155 |
| | SD | 87 | 142 | 13.7 | 49.2 |
| | CV% | 24.5 | 27.0 | 11.2 | 31.7 |
| | Minimum | 205 | 276 | 105 | 95 |
| | Median | 383 | 554 | 121 | 154 |
| | Maximum | 443 | 677 | 138 | 242 |

$C_{max}$: Maximum observed plasma concentration over the sampling interval

DN $C_{max}$: Dose-normalized $C_{max}$ calculated as Cmax divided by the applied dose (in mg/kg)

$t_{max}$: Time corresponding to occurrence of $C_{max}$

t½ 0-tau: Elimination half-life with the dosing interval (48h for dose group 1, 12h for dose group 2).

$C_{trough}$: Measured concentration at the end of a dosing interval (taken directly before next administration)

**Fig. 27**

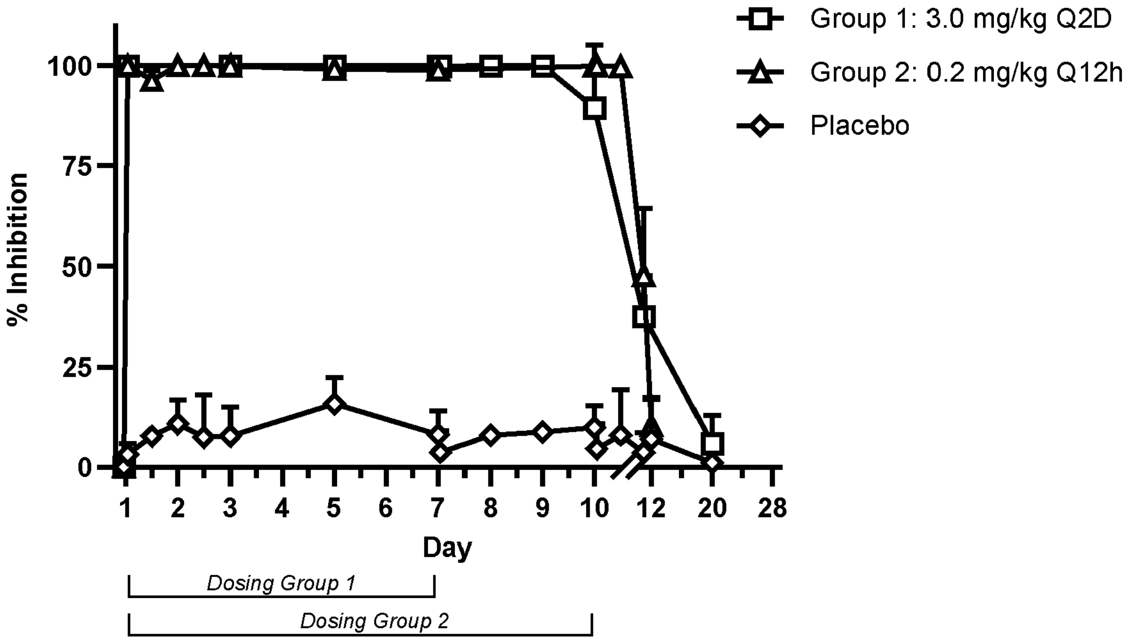


Fig. 28

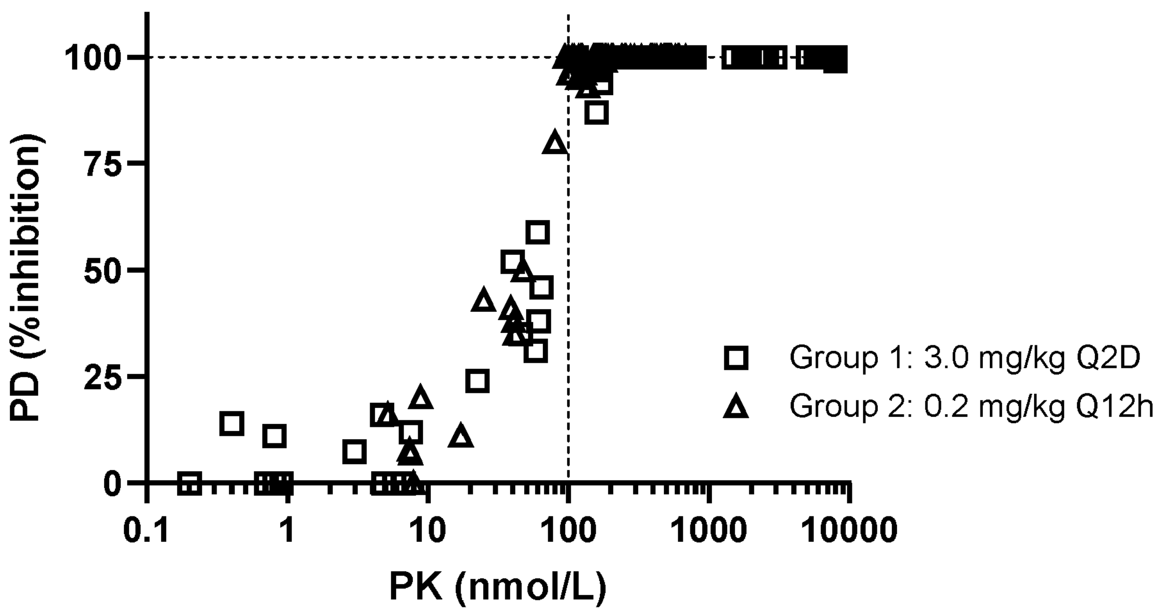


Fig. 29 A

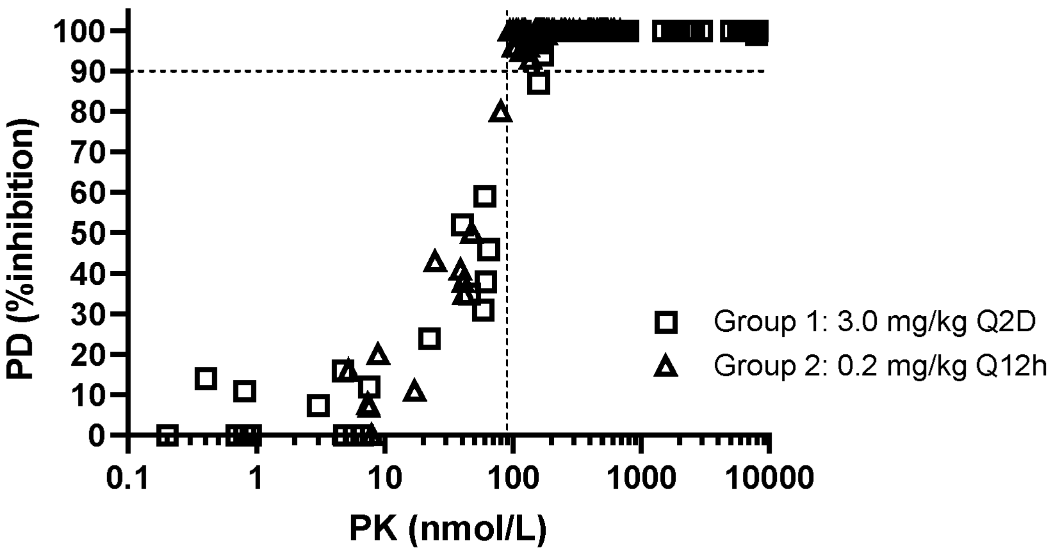


Fig. 29 B

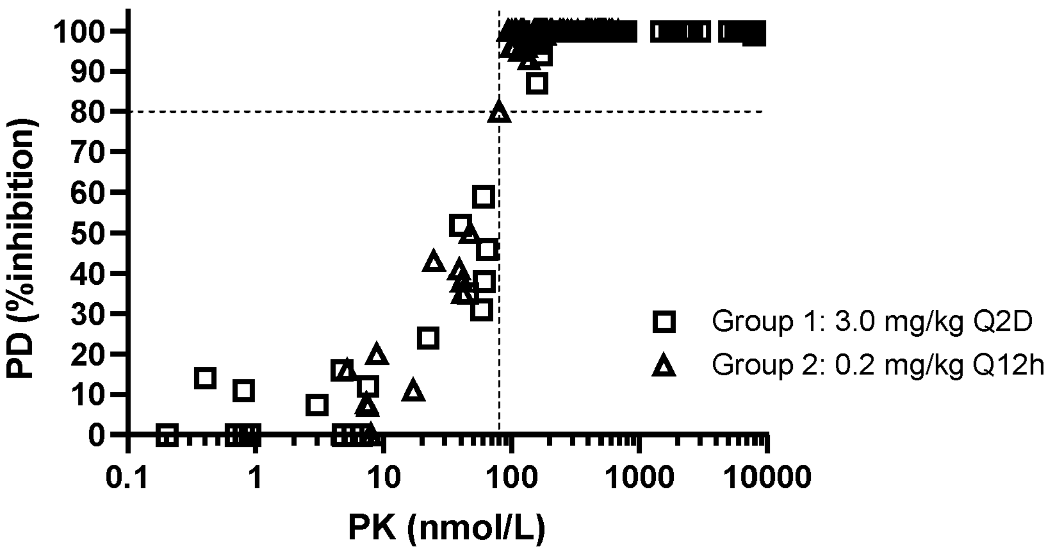


Fig. 29 C

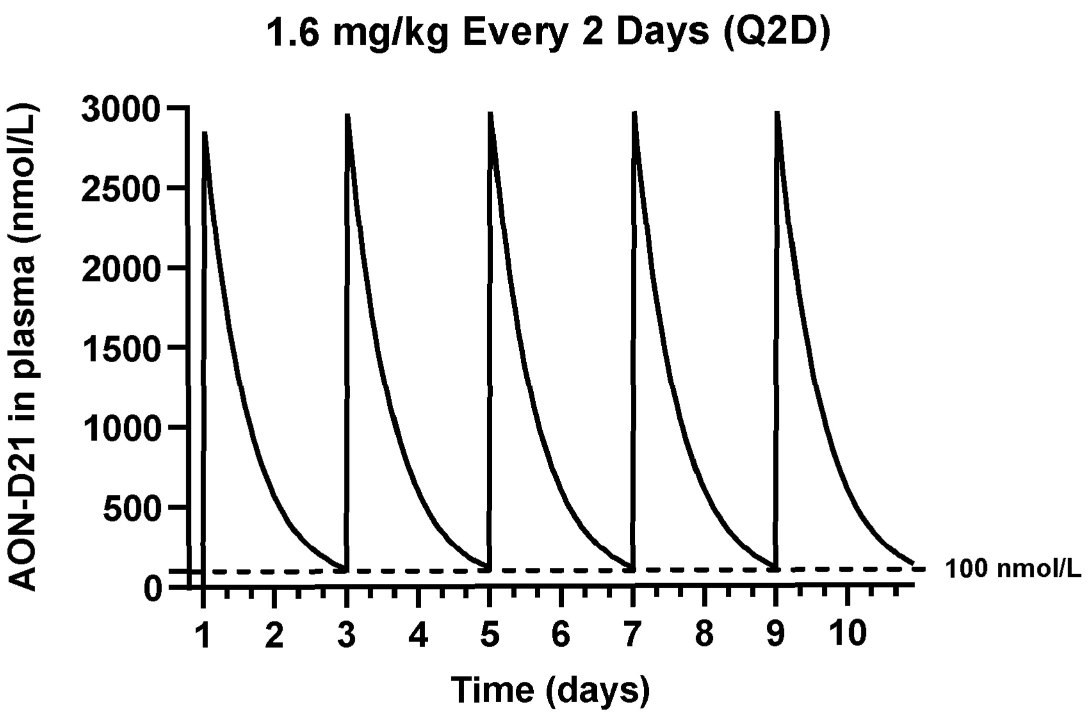
1.6 mg/kg Every 2 Days (Q2D)
AON-D21 in plasma (nmol/L)
3000
2500
2000
1500
1000
500
0
100 nmol/L
1
2
3
4
5
6
7
8
9
10
Time (days)

Fig. 30

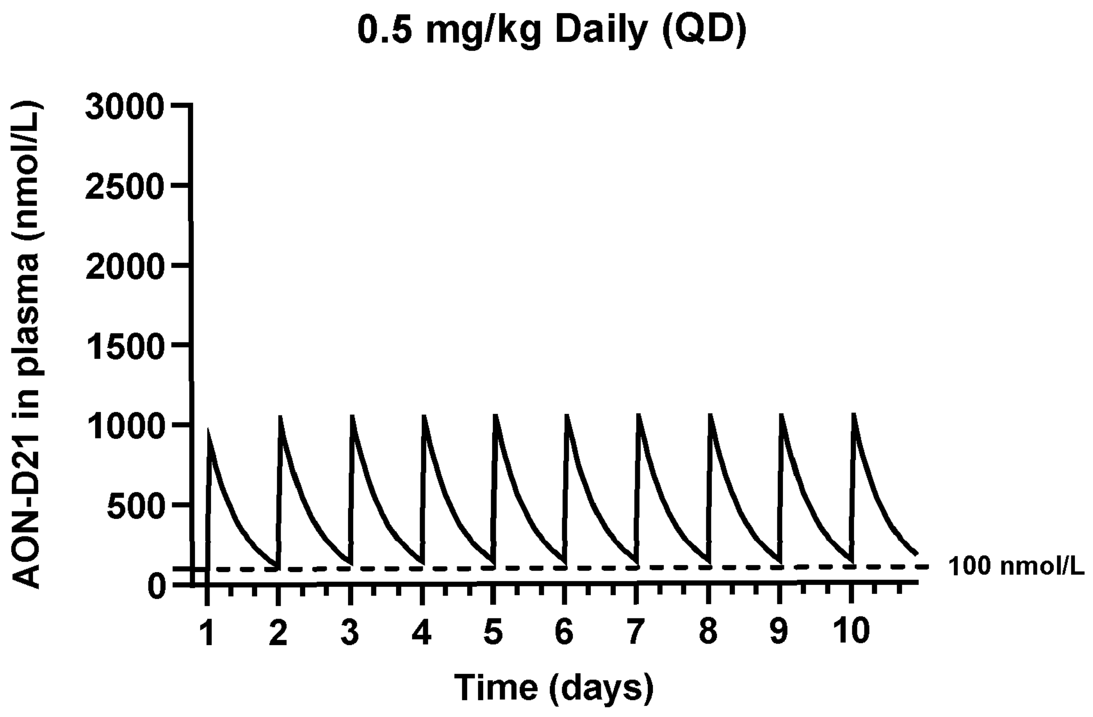
0.5 mg/kg Daily (QD)
AON-D21 in plasma (nmol/L)
3000
2500
2000
1500
1000
500
0
100 nmol/L
1
2
3
4
5
6
7
8
9
10
Time (days)

Fig. 31

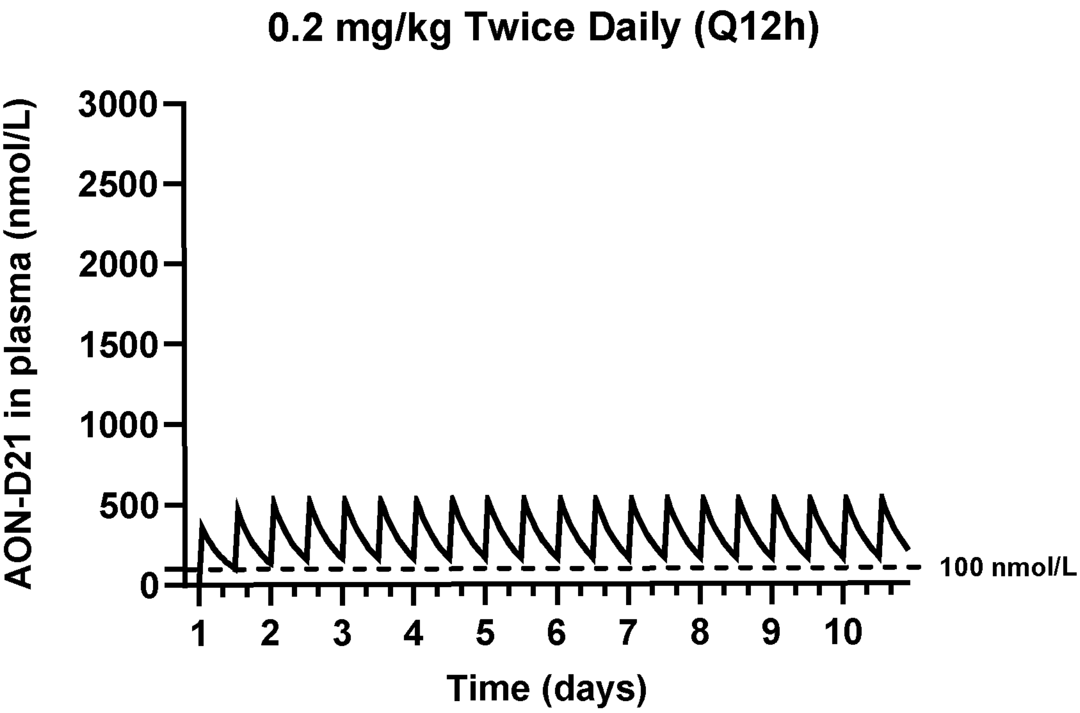
0.2 mg/kg Twice Daily (Q12h)
AON-D21 in plasma (nmol/L)
3000
2500
2000
1500
1000
500
0
100 nmol/L
1
2
3
4
5
6
7
8
9
10
Time (days)

Fig. 32

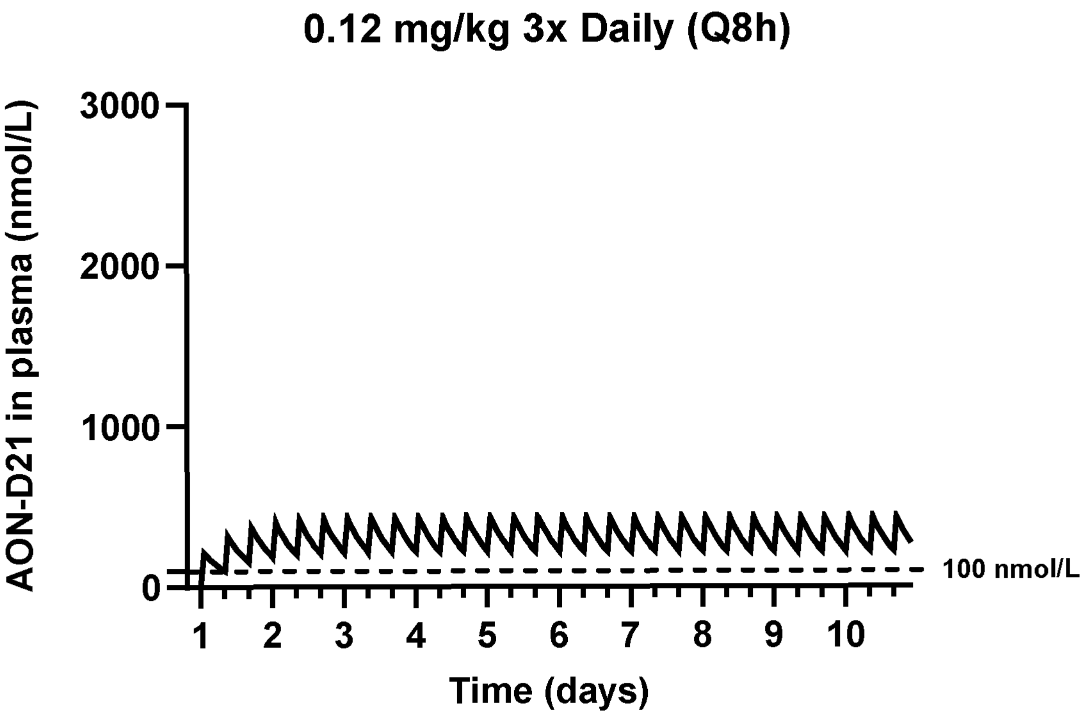
0.12 mg/kg 3x Daily (Q8h)
AON-D21 in plasma (nmol/L)
3000
2000
1000
0
100 nmol/L
1
2
3
4
5
6
7
8
9
10
Time (days)

Fig. 33

Europäisches Patentamt
European Patent Office
Office européen des brevets

# PARTIAL EUROPEAN SEARCH REPORT

under Rule 62a and/or 63 of the European Patent Convention. This report shall be considered, for the purposes of subsequent proceedings, as the European search report

Application Number
EP 24 16 4977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HOEHLIG KAI ET AL: "A Novel C5a-neutralizing Mirror-image (l-)Aptamer Prevents Organ Failure and Improves Survival in Experimental Sepsis", MOLECULAR THERAPY, vol. 21, no. 12, 1 December 2013 (2013-12-01), pages 2236-2246, XP093190076, US ISSN: 1525-0016, DOI: 10.1038/mt.2013.178 * the entire document and in particular, the abstract * ----- | 1-16 | INV. A61K31/7088 C07K14/47 |
| X | US 2021/163946 A1 (HOHLIG KAI [DE] ET AL) 3 June 2021 (2021-06-03) * paragraph [0378]; figure 21 * ----- -/-- | 1-16 | |

TECHNICAL FIELDS SEARCHED (IPC)

A61K
C07K

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

3

| Place of search | Date of completion of the search | Examiner |
|---|---|---|
| Munich | 10 October 2024 | Potthast, Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 24 16 4977

| Category | DOCUMENTS CONSIDERED TO BE RELEVANT<br>Citation of document with indication, where appropriate, of relevant passages | Relevant to claim | CLASSIFICATION OF THE APPLICATION (IPC) |
|---|---|---|---|
| A | DANIEL AJONA ET AL: "A Combined PD-1/C5a Blockade Synergistically Protects against Lung Cancer Growth and Metastasis", CANCER DISCOVERY, vol. 7, no. 7, 1 July 2017 (2017-07-01), pages 694-703, XP055768191, US ISSN: 2159-8274, DOI: 10.1158/2159-8290.CD-16-1184<br>* abstract, (supplementary data, chapter "Pharmacokinetics of AON-D21 in mice", results subchapter "C5a Inhibition synergizes with PD-1 Blockade to Prevent Cancer Growth and Metastasis" *<br>----- | 1-16 | |
| | | | TECHNICAL FIELDS SEARCHED (IPC) |

3

EPO FORM 1503 03.82 (P04C10)

Europäisches Patentamt
European Patent Office
Office européen des brevets

# INCOMPLETE SEARCH SHEET C

Application Number
EP 24 16 4977

Claim(s) searched incompletely:
1-16

Reason for the limitation of the search:

see separate sheet

## ANNEX TO THE EUROPEAN SEARCH REPORT ON EUROPEAN PATENT APPLICATION NO. EP 24 16 4977

This annex lists the patent family members relating to the patent documents cited in the above-mentioned European search report. The members are as contained in the European Patent Office EDP file on
The European Patent Office is in no way liable for these particulars which are merely given for the purpose of information.

10-10-2024

| Patent document cited in search report | Publication date | Patent family member(s) | Publication date |
|---|---|---|---|
| US 2021163946 A1 | 03-06-2021 | AU 2013209131 A1 | 10-07-2014 |
| | | AU 2018271388 A1 | 20-12-2018 |
| | | BR 112014016877 A2 | 29-10-2019 |
| | | CA 2860806 A1 | 18-07-2013 |
| | | CN 104145018 A | 12-11-2014 |
| | | DK 2802660 T3 | 18-05-2020 |
| | | EP 2802660 A1 | 19-11-2014 |
| | | ES 2786007 T3 | 08-10-2020 |
| | | HK 1198371 A1 | 10-04-2015 |
| | | JP 6415327 B2 | 31-10-2018 |
| | | JP 2015509705 A | 02-04-2015 |
| | | KR 20140111703 A | 19-09-2014 |
| | | MX 362061 B | 07-01-2019 |
| | | PL 2802660 T3 | 27-07-2020 |
| | | PT 2802660 T | 21-04-2020 |
| | | RU 2014132708 A | 27-02-2016 |
| | | SG 10201605594U A | 29-09-2016 |
| | | SG 11201403771S A | 30-07-2014 |
| | | US 2014364487 A1 | 11-12-2014 |
| | | US 2018223285 A1 | 09-08-2018 |
| | | US 2021163946 A1 | 03-06-2021 |
| | | WO 2013104540 A1 | 18-07-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009040113 A
- WO 2010108657 A
- WO 2013104540 A
- US 20030118592 A
- US 20030133939 A
- EP 1306382 A
- DE 12004006249
- WO 2005074993 A
- WO 2003035665 A
- WO 2006052790 A
- WO 2008034122 A
- WO 2004092191 A
- WO 2005099768 A
- WO 2002065963 A
- WO 2003070823 A
- WO 2004113394 A
- WO 200041647 A
- US 6011020 A


### Non-patent literature cited in the description


- The Aptamer Handbook. 2006
- **ALTSCHUL SF** ; **GISH W** ; **MILLER W** ; **MYERS EW** ; **LIPMAN DJ**. Basic local alignment search tool.. *J Mol Biol.*, 1990, vol. 215 (3), 403-10
- **ALTSCHUL SF** ; **MADDEN TL** ; **SCHAFFER AA** ; **ZHANG J** ; **ZHANG Z** ; **MILLER W** ; **LIPMAN DJ**. Gapped BLAST and PSI-BLAST: a new generation of protein database search programs.. *Nucleic Acids Res.*, 1997, vol. 25 (17), 3389-402
- **ARUMUGAM TV** ; **SHIELS IA** ; **STRACHAN AJ** ; **ABBENANTE G** ; **FAIRLIE DP** ; **TAYLOR SM**. A small molecule C5a receptor antagonist protects kidneys from ischemia/reperfusion injury in rats. *Kidney Int*, 2003, vol. 63 (1), 134-142
- **ARUMUGAM TV** ; **WOODRUFF TM** ; **STOCKS SZ** ; **PROCTOR LM** ; **POLLITT S** ; **SHIELS IA** ; **REID RC** ; **FAIRLIE DP** ; **TAYLOR SM**. Protective effect of a human C5a receptor antagonist against hepatic ischaemia-reperfusion injury in rats.. *J Hepatol*, 2004, vol. 40 (6), 934-941
- **BEKKER et al.** *PLoS One.*, 21 October 2016, vol. 11 (10), e 0164646
- **BERGH K** ; **IVERSEN OJ** ; **LYSVAND H.** Surprisingly high levels of anaphylatoxin C5a des Arg are extractable from psoriatic scales. *Arch Dermatol Res*, 1993, vol. 285 (3), 131-134
- **BIESECKER G** ; **DIHEL L** ; **ENNEY K** ; **BENDELE RA**. Derivation of RNA aptamer inhibitors of human complement C5. *Immunopharmacology*, 1999, vol. 42 (1-3), 219-230
- **BONIFATI DM** ; **KISHORE U.** Role of complement in neurodegeneration and neuroinflammation. *Mol Immunol*, 2007, vol. 44 (5), 999-1010
- **BOSMANN M** ; **WARD PA**. Role of C3, C5 and anaphylatoxin receptors in acute lung injury and in sepsis. *Adv Exp Med Biol*, 2012, vol. 946, 147-159
- **BREIVIK T** ; **GUNDERSEN Y** ; **GJERMO P** ; **TAYLOR SM** ; **WOODRUFF TM** ; **OPSTAD PK**. Oral treatment with complement factor C5a receptor (CD88) antagonists inhibits experimental periodontitis in rats.. *J Periodontal Res*, 2011, vol. 46 (6), 643-647
- **CHEN M** ; **DAHA MR** ; **KALLENBERG CG**. The complement system in systemic autoimmune disease.. *J Autoimmun*, 2010, vol. 34 (3), J276-286
- **COPLAND DA** ; **HUSSAIN K** ; **BAALASUBRAMANIAN S** ; **HUGHES TR** ; **MORGAN BP** ; **XU H** ; **DICK AD** ; **NICHOLSON LB**. Systemic and local anti-C5 therapy reduces the disease severity in experimental autoimmune uveoretinitis. *Clin Exp Immunol*, 2010, vol. 159 (3), 303-314
- **CZERMAK BJ** ; **SARMA V** ; **PIERSON CL** ; **WARNER RL** ; **HUBER-LANG M** ; **BLESS NM** ; **SCHMAL H** ; **FRIEDL HP** ; **WARD PA**. Protective effects of C5a blockade in sepsis.. *Nat Med*, 1999, vol. 5 (7), 788-792
- Protocols for oligonucleotides and analogs. **DAMHA MJ** ; **OGILVIE KK**. Methods in Molecular Biology. Humana Press Inc., 1993, vol. 20, 81-114
- **EHRNTHALLER C** ; **IGNATIUS A** ; **GEBHARD F** ; **HUBER-LANG M**. New Insights of an Old Defense System: Structure, Function, and Clinical Relevance of the Complement System. *Mol Med*, 2011, vol. 17 (3-4), 317-329

- **FARKAS I** ; **BARANYI L** ; **LIPOSITS ZS** ; **YAMAMOTO T** ; **OKADA H**. Complement C5a anaphylatoxin fragment causes apoptosis in TGW neuroblastoma cells. *Neuroscience*, 1998, vol. 86 (3), 903-911
- **FERNANDEZ HN** ; **HUGLI TE**. Primary structural analysis of the polypeptide portion of human C5a anaphylatoxin. Polypeptide sequence determination and assignment of the oligosaccharide attachment site in C5a.. *J Biol Chem*, 1978, vol. 253 (19), 6955-6964
- **FLIERL MA** ; **RITTIRSCH D** ; **NADEAU BA** ; **DAY DE** ; **ZETOUNE FS** ; **SARMA JV** ; **HUBER-LANG MS** ; **WARD PA**. Functions of the complement components C3 and C5 during sepsis. *FASEB J*, 2008, vol. 22 (10), 3483-3490
- **FONSECA MI** ; **AGER RR** ; **CHU SH** ; **YAZAN O** ; **SANDERSON SD** ; **LAFERLA FM** ; **TAYLOR SM** ; **WOODRUFF TM** ; **TENNER AJ**. Treatment with a C5aR antagonist decreases pathology and enhances behavioral performance in murine models of Alzheimer's disease.. *J Immunol*, 2009, vol. 183 (2), 1375-1383
- **GUELER F** ; **RONG S** ; **GWINNER W** ; **MENGEL M** ; **BROCKER V** ; **SCHON S** ; **GRETEN TF** ; **HAWLISCH H** ; **POLAKOWSKI T** ; **SCHNATBAUM K**. Complement 5a receptor inhibition improves renal allograft survival.. *J Am Soc Nephrol*, 2008, vol. 19 (12), 2302-2312
- **HUBER-LANG M** ; **SARMA VJ** ; **LU KT** ; **MCGUIRE SR** ; **PADGAONKAR VA** ; **GUO RF** ; **YOUNKIN EM** ; **KUNKEL RG** ; **DING J** ; **ERICKSON R**. Role of C5a in multiorgan failure during sepsis.. *J Immunol*, 2001, vol. 166 (2), 1193-1199
- **HUBER-LANG MS** ; **YOUNKIN EM** ; **SARMA JV** ; **MCGUIRE SR** ; **LU KT** ; **GUO RF** ; **PADGAONKAR VA** ; **CURNUTTE JT** ; **ERICKSON R** ; **WARD PA**. Complement-induced impairment of innate immunity during sepsis.. *J Immunol*, 2002, vol. 169 (6), 3223-3231
- **JACOB A** ; **HACK B** ; **BAI T** ; **BRORSON JR** ; **QUIGG RJ** ; **ALEXANDER JJ**. Inhibition of C5a receptor alleviates experimental CNS lupus.. *J Neuroimmunol*, 2010, vol. 221 (1-2), 46-52
- **JACOB A** ; **HACK B** ; **CHIANG E** ; **GARCIA JG** ; **QUIGG RJ** ; **ALEXANDER JJ**. C5a alters blood-brain barrier integrity in experimental lupus. *FASEB J*, 2010, vol. 24 (6), 1682-8
- **KAMBAS K** ; **MARKIEWSKI MM** ; **PNEUMATIKOS IA** ; **RAFAIL SS** ; **THEODOROU V** ; **KONSTANTONIS D** ; **KOURTZELIS I** ; **DOUMAS MN** ; **MAGOTTI P** ; **DEANGELIS RA**. C5a and TNF-alpha up-regulate the expression of tissue factor in intra-alveolar neutrophils of patients with the acute respiratory distress syndrome.. *J Immunol*, 2008, vol. 180 (11), 7368-7375
- **KHAN MA** ; **JIANG X** ; **DHILLON G** ; **BEILKE J** ; **HOLERS VM** ; **ATKINSON C** ; **TOMLINSON S** ; **NICOLLS MR**. CD4+ T cells and complement independently mediate graft ischemia in the rejection of mouse orthotopic tracheal transplants. *Circ Res*, 2011, vol. 109 (11), 1290-1301
- **KLOS A** ; **TENNER AJ** ; **JOHSWICH KO** ; **AGER RR** ; **REIS ES** ; **KOHL J**. The role of the anaphylatoxins in health and disease. *Mol Immunol*, 2009, vol. 46 (14), 2753-2766
- **KLUSSMANN S.** The Aptamer Handbook - Functional Oligonucleotides and their Applications.. WILEY-VCH, 2006
- **KOHL J.** Anaphylatoxins and infectious and non-infectious inflammatory diseases. *Mol Immunol*, 2001, vol. 38 (2-3), 175-187
- **LAUDES IJ** ; **CHU JC** ; **SIKRANTH S** ; **HUBER-LANG M** ; **GUO RF** ; **RIEDEMANN N** ; **SARMA JV** ; **SCHMAIER AH** ; **WARD PA**. Anti-c5a ameliorates coagulation/fibrinolytic protein changes in a rat model of sepsis. *Am J Pathol*, 2002, vol. 160 (5), 1867-1875
- **LEWIS AG** ; **KOHL G** ; **MA Q** ; **DEVARAJ AN P** ; **KOHL J**. Pharmacological targeting of C5a receptors during organ preservation improves kidney graft survival.. *Clin Exp Immunol*, 2008, vol. 153 (1), 117-126
- **LI K** ; **WANG N** ; **PENG Q** ; **LU B** ; **MA L** ; **LAMBRIS JD** ; **SACKS S** ; **ZHOU W**. C5a/C5aR signaling is an important negative regulator of murine nature killer cell homeostasis and effector function.. *Immunobiology*, 2012, vol. 217 (11), 1146
- **MANDERSON AP** ; **BOTTO M** ; **WALPORT MJ**. The role of complement in the development of systemic lupus erythematosus. *Annu Rev Immunol*, 2004, vol. 22, 431-456
- **MARKIEWSKI MM** ; **DEANGELIS RA** ; **BENENCIA F** ; **RICKLIN-LICHTSTEINER SK** ; **KOUTOULAKI A** ; **GERARD C** ; **COUKOS G** ; **LAMBRIS JD**. Modulation of the antitumor immune response by complement.. *Nat Immunol*, 2008, vol. 11, 1225-35
- **MCGINNIS S** ; **MADDEN TL**. BLAST: at the core of a powerful and diverse set of sequence analysis tools.. *Nucleic Acids Res.*, 2004, vol. 32, W20-5
- **MCNAMARA, L.A. et al.** High Risk for Invasive Meningococcal Disease Among Patients Receiving Eculizumab (Soliris) Despite Receipt of Meningococcal Vaccine. *MMWR Morb Mortal Wkly Rep*, 2017, vol. 66 (27), 734-737
- **MIN X** ; **WEI L** ; **LI Z** ; **SACKS S** ; **ZHOU W** ; **LI K**. Negative regulation of human nature killer cells by complement. *Immunobiology*, 2012, vol. 217 (11), 1189

- **MULLER-LADNER U** ; **JONES JL** ; **WETSEL RA** ; **GAY S** ; **RAINE CS** ; **BARNUM SR**. Enhanced expression of chemotactic receptors in multiple sclerosis lesions.. *J Neurol Sci*, 1996, vol. 144 (1-2), 135-141
- **NEEDLEMAN** ; **WUNSCH**. A general method applicable to the search for similarities in the amino acid sequence of two proteins.. *J Mol Biol.*, 1970, vol. 48 (3), 443-53
- **NOZAKI M** ; **RAISLER BJ** ; **SAKURAI E** ; **SARMA JV** ; **BARNUM SR** ; **LAMBRIS JD** ; **CHEN Y** ; **ZHANG K** ; **AMBATI BK** ; **BAFFI JZ**. Drusen complement components C3a and C5a promote choroidal neovascularization.. *Proc Natl Acad Sci U S A*, 2006, vol. 103 (7), 2328-2333
- **OSTRAND-ROSENBERG S**. Cancer and complement.. *Nat Biotechnol.*, 2008, vol. 26 (12), 1348-9
- **PATEL SN** ; **BERGHOUT J** ; **LOVEGROVE FE** ; **AYI K** ; **CONROY A** ; **SERGHIDES L** ; **MIN-OO G** ; **GOWDA DC** ; **SARMA JV** ; **RITTIRSCH D**. C5 deficiency and C5a or C5aR blockade protects against cerebral malaria.. *J Exp Med*, 2008, vol. 205 (5), 1133-1143
- **PEARSON** ; **LIPMAN**. Improved tools for biological sequence comparison.. *Proc. Nat'l. Acad. Sci. USA*, 1988, vol. 85, 2444
- **PICCOLO MT** ; **WANG Y** ; **SANNOMIYA P** ; **PICCOLO NS** ; **PICCOLO MS** ; **HUGLI TE** ; **WARD PA** ; **TILL GO**. Chemotactic mediator requirements in lung injury following skin burns in rats. *Exp Mol Pathol*, 1999, vol. 66 (3), 220-226
- **RYMAN** ; **MEIBOHM**. Pharmacokinetics of Monoclonal Antibodies. *CPT Pharmacometrics Syst Pharmacol*, September 2017, vol. 6 (9), 576-588
- **RICKLIN D** ; **LAMBRIS JD**. Complement-targeted therapeutics.. *Nat Biotechnol*, 2007, vol. 25 (11), 1265-1275
- **RICKLIN, D. et al.** Complement: a key system for immune surveillance and homeostasis.. *Nat Immunol*, 2010, vol. 11 (9), 785-97
- **RILEY RD** ; **SATO H** ; **ZHAO ZQ** ; **THOURANI VH** ; **JORDAN JE** ; **FERNANDEZ AX** ; **MA XL** ; **HITE DR** ; **RIGEL DF** ; **PELLAS TC**. Recombinant human complement C5a receptor antagonist reduces infarct size after surgical revascularization.. *J Thorac Cardiovasc Surg*, 2000, vol. 120 (2), 350-358
- **RITTIRSCH D** ; **FLIERL MA** ; **NADEAU BA** ; **DAY DE** ; **HUBER-LANG M** ; **MACKAY CR** ; **ZETOUNE FS** ; **GERARD NP** ; **CIANFLONE K** ; **KOHL J**. Functional roles for C5a receptors in sepsis.. *Nat Med*, 2008, vol. 14 (5), 551-557
- **SCHIELA, B. et al.** Active Human Complement Reduces the Zika Virus Load via Formation of the Membrane-Attack Complex. *Front Immunol*, 2018, vol. 9, 2177
- **SMITH** ; **WATERMAN**. *Adv. Appl. Math.*, 1981, vol. 2, 482
- **SOCIE, G. et al.** Eculizumab in paroxysmal nocturnal haemoglobinuria and atypical haemolytic uraemic syndrome: 10-year pharmacovigilance analysis. *Br J Haematol*, 2019, vol. 185 (2), 297-310
- **SPRONG T** ; **BRANDTZAEG P** ; **FUNG M** ; **PHARO AM** ; **HOIBY EA** ; **MICHAELSEN TE** ; **AASE A** ; **VAN DER MEER JW** ; **VAN DEUREN M** ; **MOLLNES TE**. Inhibition of C5a-induced inflammation with preserved C5b-9-mediated bactericidal activity in a human whole blood model of meningococcal sepsis. *Blood*, 2003, vol. 102 (10), 3702-3710
- **TOKODAI K** ; **GOTO M** ; **INAGAKI A** ; **NAKANISHI W** ; **OGAWA N** ; **SATOH K** ; **KAWAGISHI N** ; **SEKIGUCHI S** ; **NILSSON B** ; **OKADA N**. Attenuation of cross-talk between the complement and coagulation cascades by C5a blockade improves early outcomes after intraportal islet transplantation. *Transplantation*, 2010, vol. 90 (12), 1358-1365
- **VAN DER PALS J** ; **KOUL S** ; **ANDERSSON P** ; **GOTBERG M** ; **UBACHS JF** ; **KANSKI M** ; **ARHEDEN H** ; **OLIVECRONA GK** ; **LARSSON B** ; **ERLINGE D**. Treatment with the C5a receptor antagonist ADC-1004 reduces myocardial infarction in a porcine ischemia-reperfusion model.. *BMC Cardiovasc Disord*, 2010, vol. 10, 45
- **VLAAR, A.P.J. et al.** Anti-C5a antibody (vilobelimab) therapy for critically ill, invasively mechanically ventilated patients with COVID-19 (PANAMO): a multicentre, double-blind, randomised, placebo-controlled, phase 3 trial. *Lancet Respir Med.*, December 2022, vol. 10 (12), 1137-1146
- **WANG Y**. Complementary therapies for inflammation.. *Nat Biotechnol*, 2006, vol. 24 (10), 1224-1226
- **WARD PA**. The harmful role of c5a on innate immunity in sepsis.. *J Innate Immun*, 2010, vol. 2 (5), 439-445
- **WARD PA**. Role of C5 activation products in sepsis. *ScientificWorldJournal*, 2010, vol. 10, 2395-2402
- **WINCOTT F** ; **DIRENZO A** ; **SHAFFER C** ; **GRIMM S** ; **TRACZ D** ; **WORKMAN C** ; **SWEEDLER D** ; **GONZALEZ C** ; **SCARINGE S** ; **USMAN N**. Synthesis, deprotection, analysis and purification of RNA and ribosomes.. *Nucleic Acids Res.*, 1995, vol. 23, 2677-2684
- **WOODRUFF TM** ; **ARUMUGAM TV** ; **SHIELS IA** ; **REID RC** ; **FAIRLIE DP** ; **TAYLOR SM.** A potent human C5a receptor antagonist protects against disease pathology in a rat model of inflammatory bowel disease.. *J Immunol*, 2003, vol. 171 (10), 5514-5520

- **WOODRUFF TM** ; **STRACHAN AJ** ; **DRYBURGH N** ; **SHIELS IA** ; **REID RC** ; **FAIRLIE DP** ; **TAYLOR SM**. Antiarthritic activity of an orally active C5a receptor antagonist against antigen-induced monarticular arthritis in the rat. *Arthritis Rheum*, 2002, vol. 46 (9), 2476-2485
- **YAO YM** ; **REDL H** ; **BAHRAMI S** ; **SCHLAG G.** The inflammatory basis of trauma/shock-associated multiple organ failure. *Inflamm Res*, 1998, vol. 47 (5), 201-210
- **ZELEK et al.** Complement Inhibition with the C5 Blocker LFG316 in Severe COVID-19. *Am J Respir Crit Care Med*, 01 November 2020, vol. 202 (9), 1304-1308
- **ZHENG X** ; **ZHANG X** ; **FENG B** ; **SUN H** ; **SUZUKI M** ; **ICHIM T** ; **KUBO N** ; **WONG A** ; **MIN LR** ; **BUDOHN ME**. Gene silencing of complement C5a receptor using siRNA for preventing ischemia/reperfusion injury.. *Am J Pathol*, 2008, vol. 173 (4), 973-980